(19) Europäisches Patentamt
European Patent Office
Office européen des brevets

(11) EP 4 194 553 A1

(12) EUROPEAN PATENT APPLICATION
published in accordance with Art. 153(4) EPC

(43) Date of publication:
**14.06.2023 Bulletin 2023/24**

(21) Application number: **21852482.5**

(22) Date of filing: **04.08.2021**

(51) International Patent Classification (IPC):
***C12N 15/113** (2010.01)* ***A61K 31/713** (2006.01)*
***A61K 48/00** (2006.01)*

(52) Cooperative Patent Classification (CPC):
**A61K 31/713; A61K 48/00; C12N 15/113**

(86) International application number:
**PCT/CN2021/110509**

(87) International publication number:
**WO 2022/028462 (10.02.2022 Gazette 2022/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.08.2020 CN 202010772542**
**05.03.2021 CN 202110244977**
**02.04.2021 CN 202110361502**

(71) Applicant: **Tuojie Biotech (Shanghai) Co., Ltd.**
**Shanghai 201203 (CN)**

(72) Inventors:
- **HUANG, Jinyu**
**Shanghai 201203 (CN)**
- **LUO, Min**
**Shanghai 201203 (CN)**
- **YIN, Ke**
**Shanghai 201203 (CN)**

(74) Representative: **Regimbeau**
**20, rue de Chazelles**
**75847 Paris Cedex 17 (FR)**

(54) **MODIFIED SIRNA WITH REDUCED OFF-TARGET ACTIVITY**

(57) Disclosed is a modified siRNA with a reduced off-target activity. The siRNA comprises a sense strand and an antisense strand, wherein the antisense strand contains a chemical modification as represented by formula (I) or a tautomeric modification thereof in at least one nucleotide position from position 2 to position 8 of 5' region thereof. A conjugate, a pharmaceutical composition, a cell or a kit containing the siRNA, and the medical use of the siRNA, the conjugate and/or the pharmaceutical composition thereof are also disclosed. Further disclosed are compounds as represented by formula (II) and formula (III) or tautomers thereof, and preparation methods therefor.

EP 4 194 553 A1

## Description

The present application claims priority to the Chinese Patent Application CN202010772542.6 filed on Aug. 4, 2020, the Chinese Patent Application CN202110244977.8 filed on Mar. 5, 2021 and the Chinese Patent Application CN202110361502.7 filed on Apr. 2, 2021, which are incorporated herein by reference in their entirety.

## TECHNICAL FIELD

The present disclosure relates to an siRNA that inhibits expression of a target gene, and particularly to a modified siRNA with reduced off-target activity.

## BACKGROUND

RNA interference (RNAi) is an effective way to silence gene expression. Statistically, about more than 80% of the proteins related to diseases in humans are non-druggable proteins as they cannot be targeted by the conventional small-molecule drugs and biomacromolecule formulations. By using the RNA interference technology, proper siRNAs can be designed according to the mRNAs coding for these proteins to specifically target and degrade the target mRNAs so the generation of the related proteins is inhibited. Therefore, siRNAs have very important prospects for drug development.

However, siRNAs often have varying degrees of off-target effects. One off-target effect is the miRNA-like off-target effect, i.e., the inhibitory activity against mRNA caused by complete or incomplete pairing of the seed region (positions 2-8 at the 5' end) of the siRNA's antisense strand (also known as the AS strand) with the target mRNA. The off-target effect of one siRNA molecule may affect multiple mRNAs. Thus, unpredictable toxic side effects may be produced. This is the main cause of the toxic side effects produced by siRNA drugs (Janas, M.M., Schlegel, M.K., Harbison, C.E. et al. Selection of GalNAc-conjugated siRNAs with limited off-target-driven rat hepatotoxicity. Nat Commun 9, 723 (2018)).

## SUMMARY

siRNA

The present disclosure provides an siRNA that incorporates a chemical modification in the seed region thereof to inhibit or reduce siRNA off-target activity while maintaining (or even increasing) siRNA on-target activity.

The present disclosure provides an siRNA, which comprises a sense strand and an antisense strand, wherein each of the strands has 15 to 35 nucleotides; the antisense strand comprises a chemical modification of formula (I) or a tautomer modification thereof in at least one of nucleotide positions 2 to 8 (e.g., positions 2, 3, 4, 5, 6, 7 and 8) of the 5' region thereof:

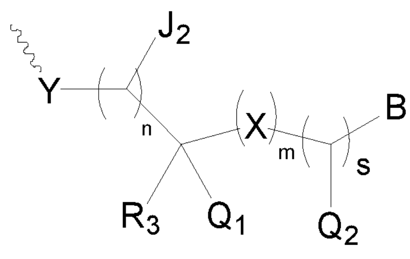

(I)

wherein Y is selected from the group consisting of O, NH and S;

each X is independently selected from the group consisting of $CR_4(R_4')$, S, $NR_5$ and NH-CO, wherein $R_4$, $R_4'$ and $R_5$ are each independently H or $C_1$-$C_6$ alkyl;

$J_2$ is H or $C_1$-$C_6$ alkyl;

n = 0, 1 or 2; m = 0, 1 or 2; s = 0 or 1;

$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, $S$-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;

$Q_1$ is

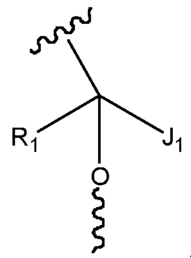

,

and $Q_2$ is $R_2$; or $Q_1$ is $R_2$, and $Q_2$ is

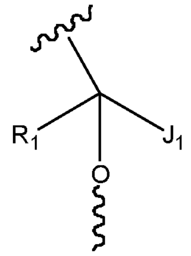

;

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$J_1$ is H or $C_1$-$C_6$ alkyl;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is a base or a base analog;
wherein the chemical modification of formula (I) is not

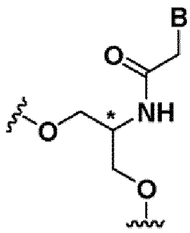

.

In some embodiments, the antisense strand comprises a chemical modification of formula (I-1) or a tautomer modification thereof in at least one of nucleotide positions 2 to 8 (e.g., positions 2, 3, 4, 5, 6, 7 and 8) of the 5' region thereof:

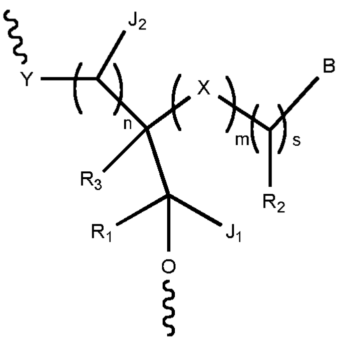

(I-1)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
each $J_1$ and each $J_2$ is independently H or $C_1$-$C_6$ alkyl;
n = 0, 1 or 2; m = 0, 1 or 2; s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring.

In some embodiments, the antisense strand comprises a chemical modification of formula (I-2) or a tautomeric modification thereof in at least one of nucleotide positions 2 to 8 (e.g., positions 2, 3, 4, 5, 6, 7 and 8) of the 5' region thereof:

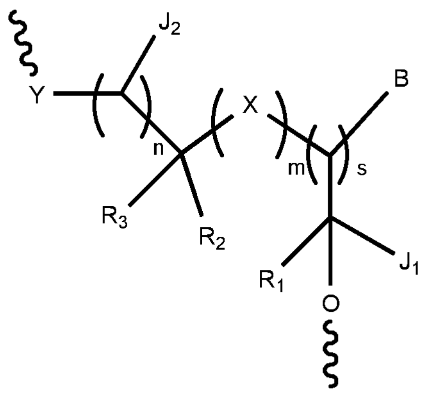

(I-2)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
n = 0, 1 or 2; m = 0, 1 or 2; s = 0 or 1;
each $J_1$ and each $J_2$ is independently H or $C_1$-$C_6$ alkyl;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$,

wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;

$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;

optionally, $R_1$ and $R_2$ are directly linked to form a ring.

In some embodiments, a nucleotide comprising a chemical modification of formula (I) or a tautomer modification thereof is a nucleotide comprising a chemical modification of formula (I') or a tautomer modification thereof,

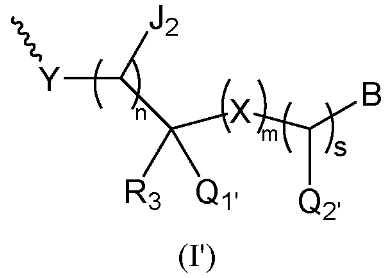

(I')

wherein Y is selected from the group consisting of O, NH and S;

each X is independently selected from the group consisting of $CR_4(R_4')$, S, $NR_5$ and NH-CO, wherein $R_4$, $R_4'$ and $R_5$ are each independently H or $C_1$-$C_6$ alkyl;

$J_2$ is H or $C_1$-$C_6$ alkyl;

n = 0, 1 or 2; m = 0, 1 or 2; s = 0 or 1;

$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;

$Q_{1'}$ is

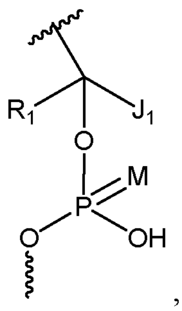

,

and $Q_{2'}$ is $R_2$; or $Q_{1'}$ is $R_2$, and $Q_{2'}$ is

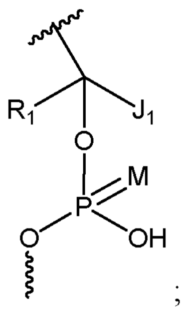

;

wherein $R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;

$J_1$ is H or $C_1$-$C_6$ alkyl;

$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
M is O or S;
optionally, $R_1$ and $R_2$ are directly linked to form a ring;
B is a base or a base analog;
wherein the chemical modification of formula (I') is not

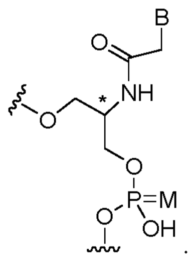

In some embodiments, the antisense strand comprises a chemical modification of formula (I-3) or a tautomer modification thereof in at least one of nucleotide positions 2 to 8 (e.g., positions 2, 3, 4, 5, 6, 7 and 8) of the 5' region thereof:

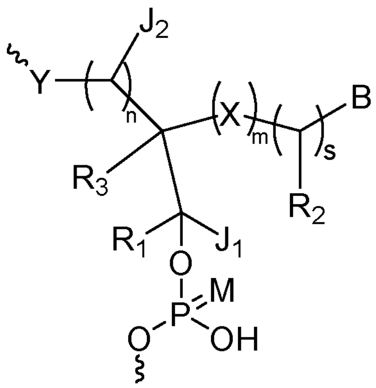

(I-3)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
each $J_1$ and each $J_2$ is independently H or $C_1$-$C_6$ alkyl;
n = 0, 1 or 2; m = 0, 1 or 2; s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
M is O or S;
optionally, $R_1$ and $R_2$ are directly linked to form a ring.

In some embodiments, the antisense strand comprises a chemical modification of formula (I-4) or a tautomeric modification thereof in at least one of nucleotide positions 2 to 8 (e.g., positions 2, 3, 4, 5, 6, 7 and 8) of the 5' region thereof:

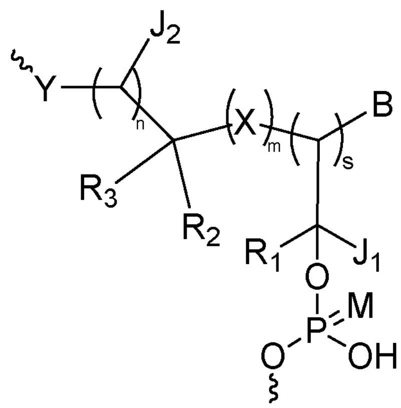

(I-4)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
n = 0, 1 or 2; m = 0, 1 or 2; s = 0 or 1;
each $J_1$ and each $J_2$ is independently H or $C_1$-$C_6$ alkyl;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
M is O or S;
optionally, $R_1$ and $R_2$ are directly linked to form a ring.

In some embodiments, the chemical modification described above is not

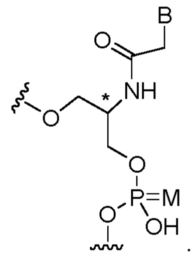

In some embodiments, when X is NH-CO, $R_1$ is not H.

In some embodiments, each X is independently selected from the group consisting of $CR_4(R_4')$, S, $NR_5$ and NH-CO, wherein $R_4$, $R_4'$ and $R_5$ are each independently H or $C_1$-$C_3$ alkyl;

n = 0, 1 or 2; m = 0, 1 or 2; s = 0 or 1;
each $J_1$ and each $J_2$ is independently H or $C_1$-$C_3$ alkyl;
$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_3$ alkyl, $C_1$-$C_3$ alkoxy, $C_2$-$C_4$ alkenyl, $C_2$-$C_4$ alkynyl and $(CH_2)_qR_7$, wherein $R_7$ is selected from the group consisting of OH, halogen, methoxy, ethoxy, $N_3$, $C_2$-$C_4$ alkenyl and $C_2$-$C_4$ alkynyl, and q = 1, 2 or 3;
$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$

alkynyl, $S\text{-}CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_4$ alkenyl and $C_2$-$C_4$ alkynyl, and r = 1, 2 or 3;
optionally, $R_1$ and $R_2$ are directly linked to form a ring;
B is selected from the group consisting of purine bases, pyrimidine bases, indole, 5-nitroindole and 3-nitropyrrole.

In some embodiments, each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;

n = 0, 1 or 2; m = 0, 1 or 2; s = 0 or 1;
each $J_1$ and each $J_2$ is independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring;
B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;

n = 0, 1 or 2; m = 0, 1 or 2; s = 0 or 1;
each $J_1$ and each $J_2$ is independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring;
B is selected from the group consisting of purine, adenine, guanine, isoguanine, hypoxanthine, xanthine, 2,6-diaminopurine, 6-dimethylaminopurine, 2-aminopurine, 7-deazapurine, cytosine, 5-methylcytosine, isocytosine, pseudocytosine, uracil, pseudouracil, 2-thiouridine, 4-thiouridine, thymine, indole, 5-nitroindole and 3-nitropyrrole.

In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and $CH_2OH$;
$R_2$ is selected from the group consisting of H, OH, $NH_2$, methyl and $CH_2OH$;
$R_3$ is selected from the group consisting of H, OH, $NH_2$, methyl and $CH_2OH$;
optionally, $R_1$ and $R_2$ are directly linked to form a ring;
B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and $CH_2OH$;
$R_2$ is selected from the group consisting of H, methyl and $CH_2OH$;
$R_3$ is selected from the group consisting of H, OH, $NH_2$, methyl and $CH_2OH$;
optionally, $R_1$ and $R_2$ are directly linked to form a ring;
B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some embodiments, the chemical modification of formula (I) is selected from the group consisting of:

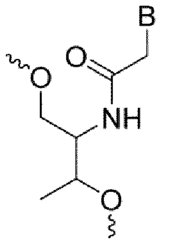, 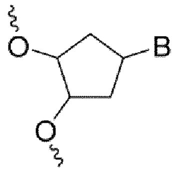, 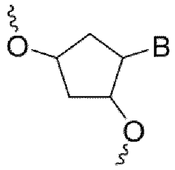, 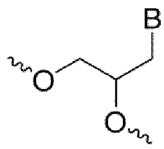, 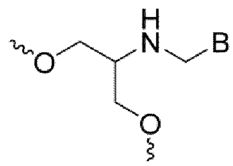,

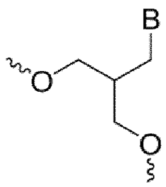, 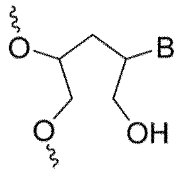, 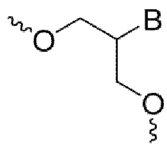, 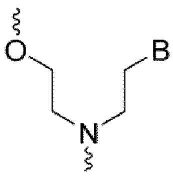, 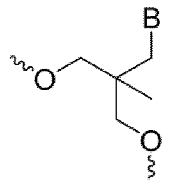,

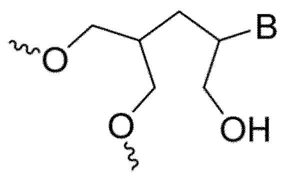, 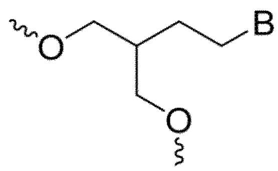,

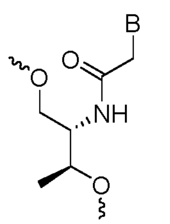, 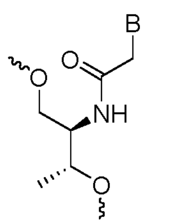, 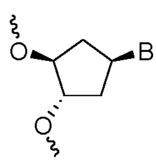, 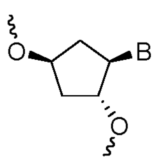, 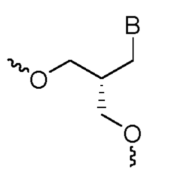,

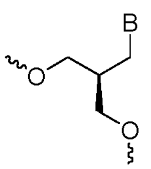, 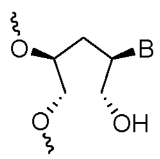, 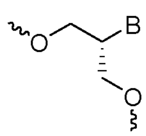, 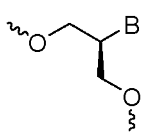, 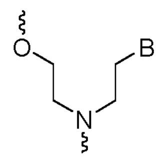,

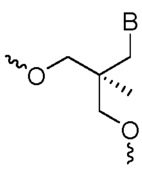 , 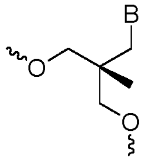 , 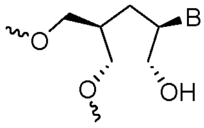 , 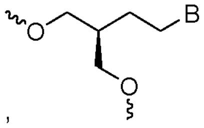 , 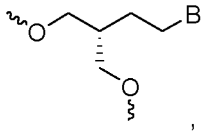 ,

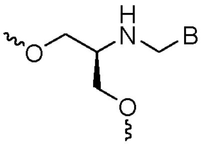 , 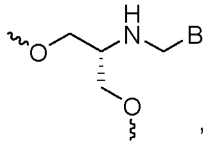 , 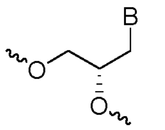 , 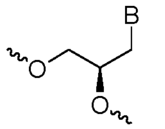 ,

wherein B is a base or a base analog; for example, B is selected from the group consisting of purine bases, pyrimidine bases, indole, 5-nitroindole and 3-nitropyrrole.

In some embodiments, B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some other embodiments, B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some embodiments, B is a base in a corresponding position among positions 2 to 8 (e.g., positions 2, 3, 4, 5, 6, 7 and 8) of the 5' region of the antisense strand.

In some specific embodiments, B is a natural base in a corresponding position among positions 2 to 8 (e.g., positions 2, 3, 4, 5, 6, 7 and 8) of the 5' region of the antisense strand.

In some embodiments, the chemical modification of formula (I) is selected from the group consisting of:

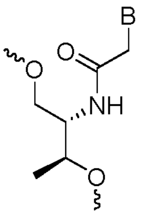 , 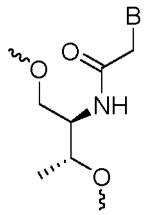 , 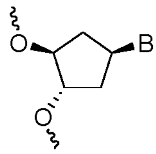 , 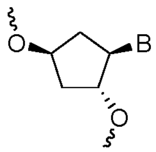 , 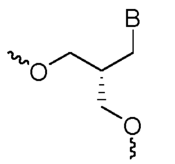 ,

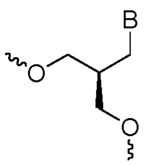 , 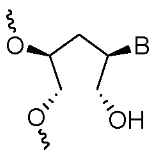 , 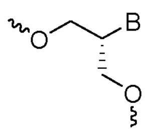 , 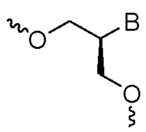 , 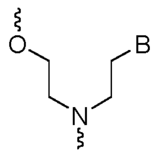 ,

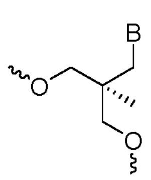 , 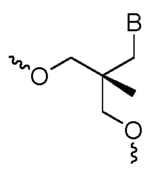 ,

wherein B is a base or a base analog; for example, B is selected from the group consisting of purine bases, pyrimidine bases, indole, 5-nitroindole and 3-nitropyrrole.

In some embodiments, B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some other embodiments, B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some embodiments, the chemical modification of formula (I) is selected from the group consisting of:

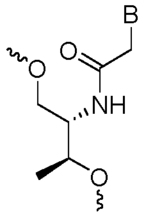 , 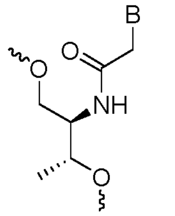 , 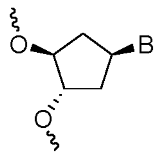 , 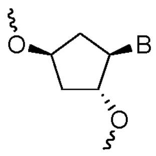 , 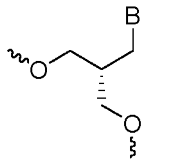 ,

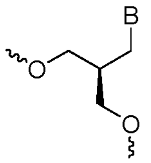 ,

wherein B is a base or a base analog; for example, B is selected from the group consisting of purine bases, pyrimidine bases, indole, 5-nitroindole and 3-nitropyrrole. In some embodiments, B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some other embodiments, B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some other embodiments, B is selected from the group consisting of adenine, guanine, cytosine, uracil and thymine.

In some embodiments, the chemical modification of formula (I) is selected from the group consisting of:

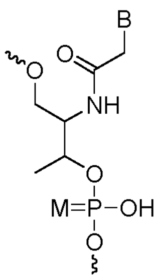 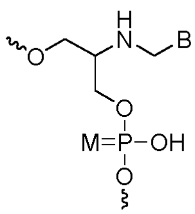 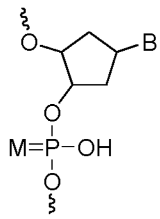 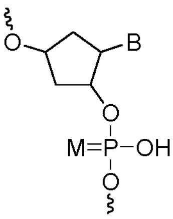 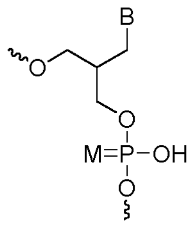

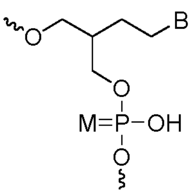 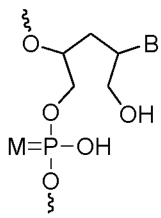 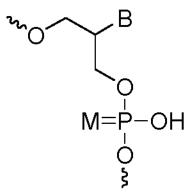 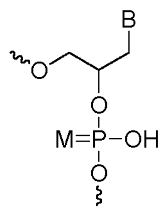 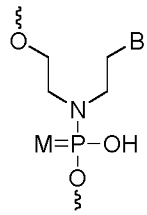

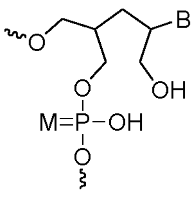 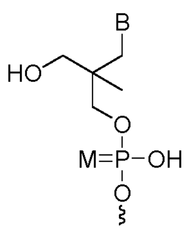

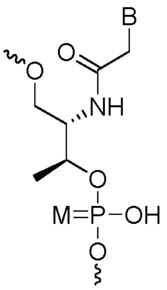 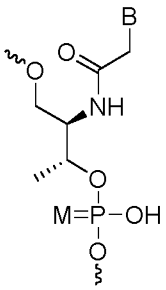 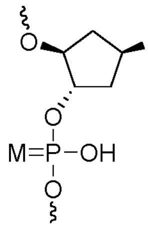 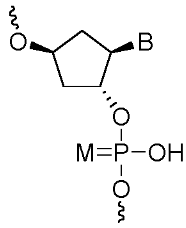 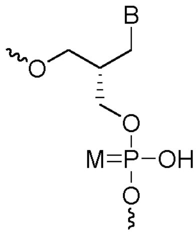

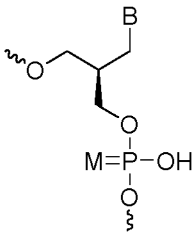 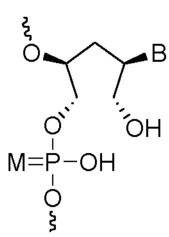 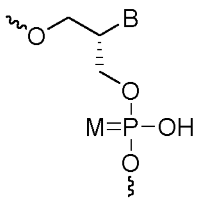 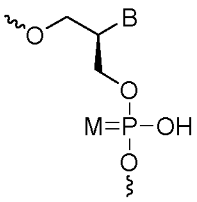 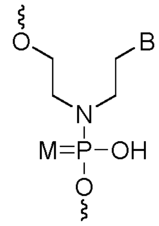

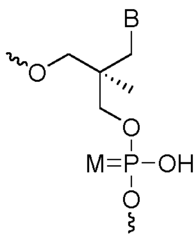 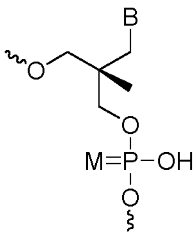 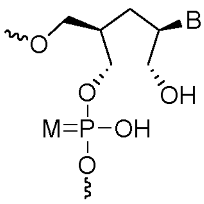 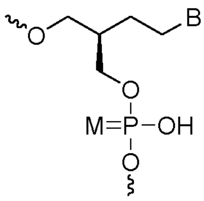 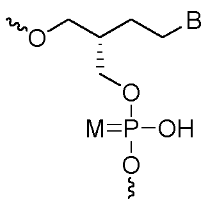

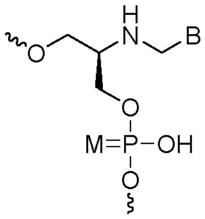 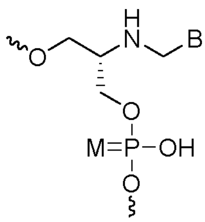 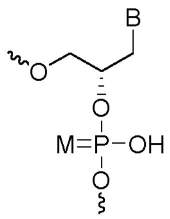 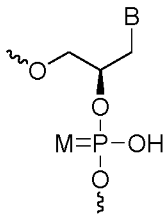 ,

wherein M is O or S;
wherein B is a base or a base analog; for example, B is selected from the group consisting of purine bases, pyrimidine bases, indole, 5-nitroindole and 3-nitropyrrole. In some embodiments, B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some other embodiments, B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some embodiments, the chemical modification of formula (I) is selected from the group consisting of:

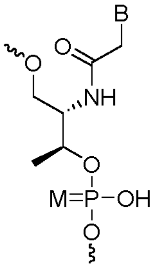 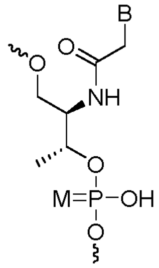 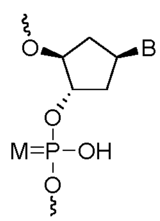 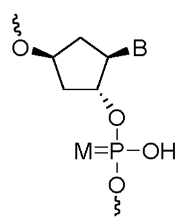 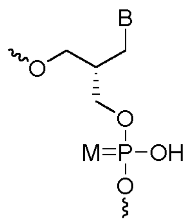

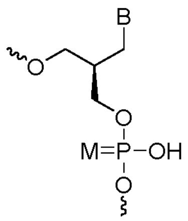 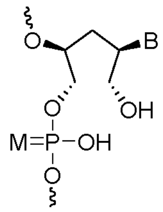 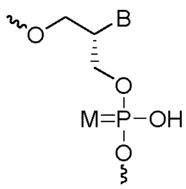 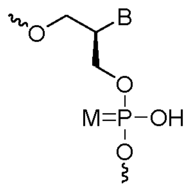 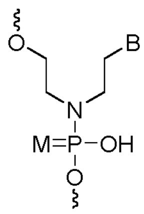

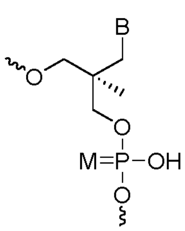 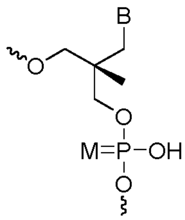

wherein M is O or S;

B is a base or a base analog; for example, B is selected from the group consisting of purine bases, pyrimidine bases, indole, 5-nitroindole and 3-nitropyrrole.

In some embodiments, B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some other embodiments, B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some embodiments, the chemical modification of formula (I) is selected from the group consisting of:

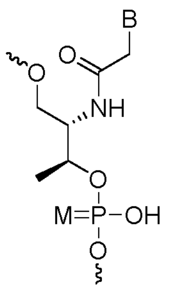 , 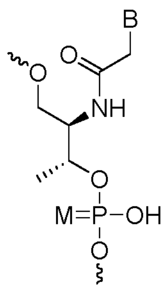 , 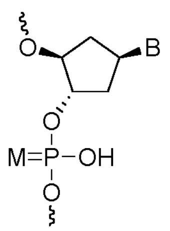 , 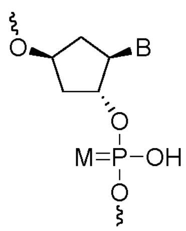 , 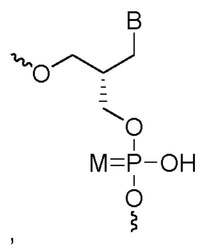 ,

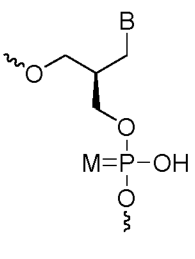 ,

wherein M is O or S;

B is a base or a base analog; for example, B is selected from the group consisting of purine bases, pyrimidine bases, indole, 5-nitroindole and 3-nitropyrrole.

In some embodiments, B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some other embodiments, B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some other embodiments, B is selected from the group consisting of adenine, guanine, cytosine, uracil and thymine.

In some embodiments, the chemical modification of formula (I) includes, but is not limited to:

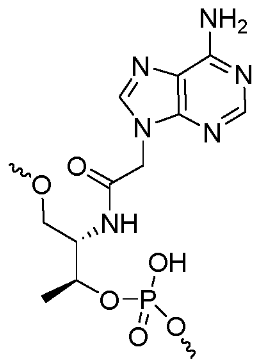 , 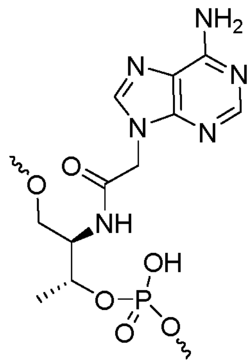 , 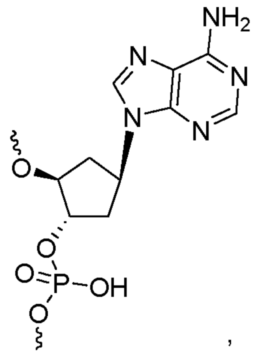 , 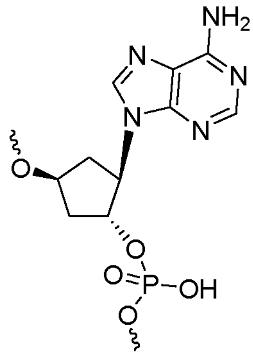 ,

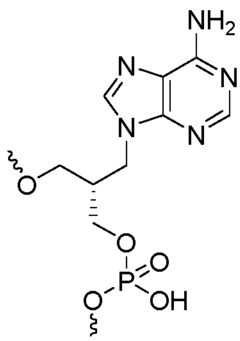 , 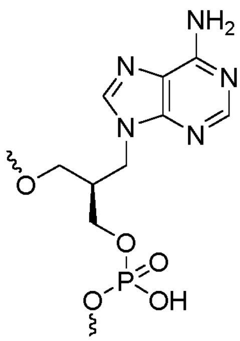 , 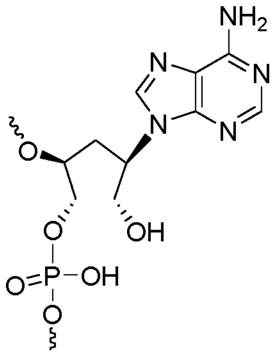

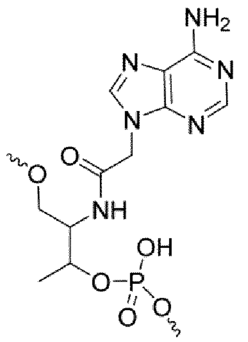 , 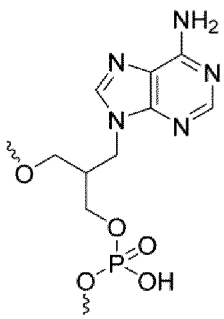 , 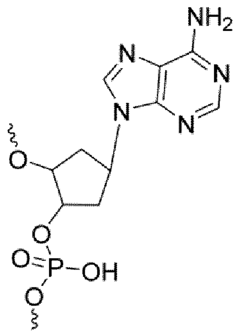 , 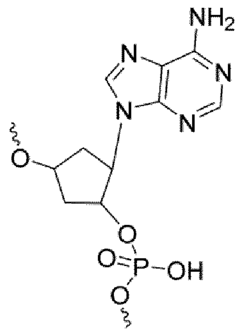 ,

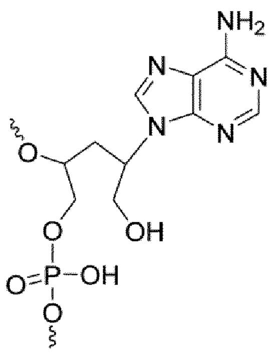 ,

and those where adenine in the structure is replaced with guanine, cytosine, uracil or thymine.

In some embodiments, the antisense strand comprises the chemical modification of formula (I) or the tautomeric modification thereof described above in at least one of nucleotide positions 2 to 8, 3 to 8, 4 to 8, 5 to 8, or 5 to 7 of the 5' region thereof.

In some embodiments, the antisense strand comprises the chemical modification of formula (I) or the tautomeric modification thereof described above in nucleotide positions 5, 6 and 7 of the 5' region thereof.

In some embodiments, the antisense strand comprises the chemical modification of formula (I) or the tautomeric modification thereof described above in nucleotide position 7 of the 5' region thereof.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides.

In some embodiments, the sense strand and the antisense strand are identical or different in length; the sense strand is 19-23 nucleotides in length, and the antisense strand is 19-26 nucleotides in length. A ratio of the length of the sense strand to the length of the antisense strand of the siRNA provided by the present disclosure can be 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, a ratio of the length of the sense strand to the length of the antisense strand of the siRNA is 19/21, 21/23 or 23/25. In some embodiments, a ratio of the length of the sense strand to the length of the antisense strand of the siRNA is 19/21.

In some embodiments, the antisense strand is at least partially reverse complementary to the target sequence to mediate RNA interference; in some embodiments, there are no more than 5, no more than 4, no more than 3, no more than 2, or no more than 1 mismatch between the antisense strand and the target sequence; in some embodiments, the antisense strand is fully reverse complementary to the target sequence.

In some embodiments, the sense strand is at least partially reverse complementary to the antisense strand so they form a double-stranded region; in some embodiments, there are no more than 5, no more than 4, no more than 3, no more than 2, or no more than 1 mismatch between the sense strand and the antisense strand; in some embodiments, the sense strand is fully reverse complementary to the antisense strand.

The present disclosure also provides an siRNA that is the siRNA described above with modifications, wherein in addition to the nucleotide modified by the chemical modification of formula (I) or the tautomer modification thereof described above, at least one otherwise modified nucleotide is also comprised in at least one of the sense strand and/or antisense strand.

In some embodiments, in addition to the nucleotide modified by the chemical modification of formula (I) or the tautomer modification thereof described above, the other nucleotides in the sense strand and/or antisense strand are otherwise modified nucleotides.

In some embodiments, the otherwise modified nucleotides are each independently selected from the group consisting of a deoxy-nucleotide, a 3'-end deoxy-thymine nucleotide, a 2'-O-methyl-modified nucleotide, a 2'-fluoro-modified nucleotide, a 2'-deoxy-modified nucleotide, a locked nucleotide, an unlocked nucleotide, a conformationally constrained nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nonnatural base-comprising nucleotide, a tetrahydropyran-modified nucleotide, a 1,5-anhydrohexitol-modified nucleotide, a cyclohexenyl-modified nucleotide, a phosphorothioate group-comprising nucleotide, a methylphosphonate group-comprising nucleotide, a 5'-phosphate-comprising nucleotide, and a 5'-phosphate mimic-comprising nucleotide.

In some embodiments, the otherwise modified nucleotides are each independently selected from the group consisting of a 2'-alkoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, a 2'-fluoro-modified nucleotide, a 2'-deoxynucleotide, a 2'-deoxy-2'-fluoro-modified nucleotide, a 3'-deoxy-thymine nucleotide, an isonucleotide, LNA, ENA, cET, UNA and GNA.

In some embodiments, the otherwise modified nucleotides are each independently selected from the group consisting of a 2'-methoxy-modified nucleotide, a 2'-fluoro-modified nucleotide, and a 2'-deoxy-modified nucleotide.

In the context of the present disclosure, a fluoro-modified nucleotide refers to a nucleotide in which the hydroxy group in the 2' position of the ribosyl group of the nucleotide is substituted with fluorine. In some embodiments, the 2'-alkoxy-modified nucleotide is a methoxy-modified nucleotide (2'-OMe). In some embodiments, the 2'-substituted alkoxy-modified nucleotide can be, for example, a 2'-O-methoxyethyl-modified nucleotide (2'-MOE) or a 2'-amino modified nucleotide (2'-$NH_2$).

In some embodiments, in a 5'-end to 3'-end direction, nucleotides in positions 2, 6, 14 and 16 of the antisense strand are each independently a 2'-deoxynucleotide or a 2'-fluoro-modified nucleotide.

In some embodiments, in a 5'-end to 3'-end direction, nucleotides in positions 2, 6, 9, 12 and 14 of the antisense strand are each independently a 2'-deoxynucleotide or a 2'-fluoro-modified nucleotide.

In some embodiments, in a 5'-end to 3'-end direction, nucleotides in positions 2, 4, 6, 9, 12, 14 and 18 of the antisense strand are each independently a 2'-deoxynucleotide or a 2'-fluoro-modified nucleotide.

In some embodiments, in a 5'-end to 3'-end direction, nucleotides in positions 2, 4, 6, 9, 12, 14, 16 and 18 of the antisense strand are each independently a 2'-deoxynucleotide or a 2'-fluoro-modified nucleotide.

In some embodiments, at least one phosphoester group in the sense strand and/or the antisense strand is a phosphoester group with a modification group. The modification group makes the siRNA have increased stability in a biological sample or environment. In some embodiments, the phosphoester group with a modification group is a phosphorothioate group. Specifically, a phosphorothioate group refers to a phosphodiester group modified by replacing one non-bridging oxygen atom with a sulfur atom.

In some embodiments, the phosphorothioate group is present in at least one of the positions selected from the group consisting of:

a position between the 1st and 2nd nucleotides of the 5' end of the sense strand;
a position between the 2nd and 3rd nucleotides of the 5' end of the sense strand;
a position between the 1st and 2nd nucleotides of the 3' end of the sense strand;
a position between the 2nd and 3rd nucleotides of the 3' end of the sense strand;
a position between the 1st and 2nd nucleotides of the 5' end of the antisense strand;
a position between the 2nd and 3rd nucleotides of the 5' end of the antisense strand;
a position between the 1st and 2nd nucleotides of the 3' end of the antisense strand; and
a position between the 2nd and 3rd nucleotides of the 3' end of the antisense strand.

In some embodiments, the sense strand has a nucleotide sequence of the formula shown below:
$5'-N_aN_aN_aN_aN_bN_aN_bN_bN_bN_aN_aN_aN_aN_aN_aN_aN_aN_aN_a-3'$

wherein each $N_a$ and each $N_b$ independently represents a modified nucleotide or an unmodified nucleotide, and modifications on $N_a$ and $N_b$ are different; and/or
the antisense strand has a nucleotide sequence of the formula shown below:
5'-Na'Nb'Na'X'Na'Nb'W'Na'Nb'Na'Na'Nb'Na'Nb'Na'Y'Na'X'Na'Na'Na'-3'
wherein each $N_a$' and each $N_b$' independently represents a modified nucleotide or an unmodified nucleotide, wherein modifications on $N_a$' and $N_b$' are different; each X' is independently $N_a$' or $N_b$'; Y' is $N_a$' or $N_b$'; W' represents a nucleotide comprising the chemical modification of formula (I) or the tautomer modification thereof described above.

In some embodiments, the sense strand has a nucleotide sequence of the formula shown below:

$5'-N_aN_aN_aN_aN_bN_aN_bN_bN_bN_aN_aN_aN_aN_aN_aN_aN_aN_aN_a-3'$

wherein each $N_a$ and each $N_b$ independently represents a modified nucleotide or an unmodified nucleotide, and modifications on $N_a$ and $N_b$ are different; and/or
the antisense strand has a nucleotide sequence of the formula shown below:
5'-Na'Nb'Na'X'Na'W'Na'Na'Nb'Na'Na'Nb'Na'Nb'Na'Y'Na'X'Na'Na'Na'-3'
wherein each $N_a$' and each $N_b$' independently represents a modified nucleotide or an unmodified nucleotide, wherein modifications on $N_a$' and $N_b$' are different; each X' is independently $N_a$' or $N_b$'; Y' is $N_a$' or $N_b$'; W' represents a nucleotide comprising the chemical modification of formula (I) or the tautomer modification thereof described above.

In some embodiments, the sense strand has a nucleotide sequence of the formula shown below:
$5'-N_aN_aN_aN_aN_bN_aN_bN_bN_bN_aN_aN_aN_aN_aN_aN_aN_aN_aN_a-3'$

wherein each $N_a$ and each $N_b$ independently represents a modified nucleotide or an unmodified nucleotide, and modifications on $N_a$ and $N_b$ are different; and/or
the antisense strand has a nucleotide sequence of the formula shown below:
$5'-N_a'N_b'N_a'X'W'N_b'N_a'N_a'N_b'N_a'N_a'N_b'N_a'N_b'N_a'Y'N_a'X'N_a'N_a'N_a'-3'$
wherein each $N_a$' and each $N_b$' independently represents a modified nucleotide or an unmodified nucleotide, wherein modifications on $N_a$' and $N_b$' are different; each X' is independently $N_a$' or $N_b$'; Y' is $N_a$' or $N_b$'; W' represents a nucleotide comprising the chemical modification of formula (I) or the tautomer modification thereof described above.

In some embodiments, $N_a$ is a 2'-methoxy-modified nucleotide and $N_b$ is a 2'-fluoro-modified nucleotide or a 2'-deoxy-modified nucleotide.

In some embodiments, $N_a$' is a 2'-methoxy-modified nucleotide and $N_b$' is a 2'-fluoro-modified nucleotide or a

2'-deoxy-modified nucleotide.

In some embodiments, at least one phosphoester group in the sense strand and/or the antisense strand is a phosphoester group with a modification group that provides the siRNA with increased stability in a biological sample or environment.

In some embodiments, the phosphoester group with a modification group is a phosphorothioate group. Specifically, a phosphorothioate group refers to a phosphodiester group modified by replacing one non-bridging oxygen atom with a sulfur atom.

In some embodiments, the phosphorothioate group is present in at least one of the positions selected from the group consisting of:

a position between the 1st and 2nd nucleotides of the 5' end of the sense strand;
a position between the 2nd and 3rd nucleotides of the 5' end of the sense strand;
a position between the 1st and 2nd nucleotides of the 3' end of the sense strand;
a position between the 2nd and 3rd nucleotides of the 3' end of the sense strand;
a position between the 1st and 2nd nucleotides of the 5' end of the antisense strand;
a position between the 2nd and 3rd nucleotides of the 5' end of the antisense strand;
a position between the 1st and 2nd nucleotides of the 3' end of the antisense strand; and
a position between the 2nd and 3rd nucleotides of the 3' end of the antisense strand.

In some embodiments, the sense strand has a nucleotide sequence of the formula shown below:
5'-NmsNmsNmNmNfNmNfNfNfNmNmNmNmNmNmNmNmNmNm-3'

wherein Nm represents any methoxy-modified nucleotide, such as methoxy-modified C, G, U, A or T; Nf represents any fluoro-modified nucleotide, such as fluoro-modified C, G, U, A or T; the lowercase letter s indicates that the two nucleotides adjacent to the letter s are linked by a phosphorothioate group; and/or
the antisense strand has a nucleotide sequence of the formula shown below:
5'-Nms'Nfs'Nm'Nm'Nm'NfW'Nm'Nm'Nm'Nm'Nm'Nm'NfNm'NfNm'Nm'Nms'Nms'Nm'-3'
wherein Nm' represents any methoxy-modified nucleotide, such as methoxy-modified C, G, U, A or T; Nf represents any fluoro-modified nucleotide, such as fluoro-modified C, G, U, A or T; the lowercase letter s indicates that the two nucleotides adjacent to the letter s are linked by a phosphorothioate group; W' represents a nucleotide comprising the chemical modification of formula (I) or the tautomer modification thereof described above.

In some embodiments, in a 5'-end to 3'-end direction, nucleotides in positions 2, 6 and 14 of the antisense strand are each independently a 2'-deoxynucleotide or a 2'-fluoro-modified nucleotide.

In some embodiments, in a 5'-end to 3'-end direction, nucleotides in positions 2, 6, 14 and 16 of the antisense strand are each independently a 2'-deoxynucleotide or a 2'-fluoro-modified nucleotide.

In some embodiments, in a 5'-end to 3'-end direction, nucleotides in positions 2, 6, 9, 12 and 14 of the antisense strand are each independently a 2'-deoxynucleotide or a 2'-fluoro-modified nucleotide.

In some embodiments, in a 5'-end to 3'-end direction, nucleotides in positions 2, 6, 10, 12 and 14 of the antisense strand are each independently a 2'-deoxynucleotide or a 2'-fluoro-modified nucleotide.

In some embodiments, in a 5'-end to 3'-end direction, nucleotides in positions 2, 4, 6, 9, 12, 14 and 18 of the antisense strand are each independently a 2'-deoxynucleotide or a 2'-fluoro-modified nucleotide.

In some embodiments, in a 5'-end to 3'-end direction, nucleotides in positions 2, 4, 6, 10, 12, 14 and 18 of the antisense strand are each independently a 2'-deoxynucleotide or a 2'-fluoro-modified nucleotide.

In some embodiments, in a 5'-end to 3'-end direction, nucleotides in positions 2, 4, 6, 9, 12, 14, 16 and 18 of the antisense strand are each independently a 2'-deoxynucleotide or a 2'-fluoro-modified nucleotide.

In some embodiments, in a 5'-end to 3'-end direction, nucleotides in positions 2, 4, 6, 10, 12, 14, 16 and 18 of the antisense strand are each independently a 2'-deoxynucleotide or a 2'-fluoro-modified nucleotide.

In some embodiments, in a 5'-end to 3'-end direction, nucleotides in positions 2, 4, 6, 9, 10 12, 14, 16 and 18 of the antisense strand are each independently a 2'-deoxynucleotide or a 2'-fluoro-modified nucleotide.

In some embodiments, in a 5'-end to 3'-end direction, nucleotides in positions 2, 6 and 14 of the antisense strand are each independently a 2'-fluoro-modified nucleotide.

In some embodiments, in a 5'-end to 3'-end direction, nucleotides in positions 2, 6, 14 and 16 of the antisense strand are each independently a 2'-fluoro-modified nucleotide. In some embodiments, in a 5'-end to 3'-end direction, nucleotides in positions 2, 6, 12 and 14 of the antisense strand are each independently a 2'-fluoro-modified nucleotide. In some embodiments, in a 5'-end to 3'-end direction, nucleotides in positions 2, 4, 6, 12, 14, 16 and 18 of the antisense strand are each independently a 2'-fluoro-modified nucleotide.

In some embodiments, in a 5'-end to 3'-end direction, nucleotides in positions 2, 4, 6, 9, 12, 14, 16 and 18 of

the antisense strand are each independently a 2’-fluoro-modified nucleotide.

In some embodiments, in a 5’-end to 3’-end direction, nucleotides in positions 2, 4, 6, 10, 12, 14, 16 and 18 of the antisense strand are each independently a 2’-fluoro-modified nucleotide.

In some embodiments, the sense strand of the siRNA of the present disclosure has a nucleotide sequence of the formula shown below:

5’-$N_aN_aN_aN_aXN_aN_bN_bN_bN_aN_aN_aN_aN_aN_aN_aN_aN_aN_a$-3’

wherein each $N_a$ and each $N_b$ independently represents a modified nucleotide or an unmodified nucleotide, and modifications on $N_a$ and $N_b$ are different; each X is independently $N_a$ or $N_b$.

In some embodiments, the antisense strand of the siRNA of the present disclosure has a nucleotide sequence of the formula shown below:

5’-$N_a'N_b'N_a'X'N_a'N_b'W'N_a'X'Y'N_a'X'N_a'N_b'N_a'X'N_a'X'N_a'N_a'N_a'$-3’;

wherein each $N_a'$ and each $N_b'$ independently represents a modified nucleotide or an unmodified nucleotide, wherein modifications on $N_a'$ and $N_b'$ are different; each X’ is independently $N_a'$ or $N_b'$; Y’ is $N_a'$ or $N_b'$; W’ represents a nucleotide comprising any one of the chemical modifications of formula (I) or the tautomer modifications thereof of the present disclosure.

In some embodiments, modifications on X’ and Y’ are different.

In some embodiments, $N_a$ is a 2’-methoxy-modified nucleotide and $N_b$ is a 2’-fluoro-modified nucleotide or a 2’-deoxy-modified nucleotide.

In some embodiments, $N_a'$ is a 2’-methoxy-modified nucleotide and $N_b'$ is a 2’-fluoro-modified nucleotide or a 2’-deoxy-modified nucleotide.

In some specific embodiments, $N_a$ is a 2’-methoxy-modified nucleotide and $N_b$ is a 2’-fluoro-modified nucleotide.

In some specific embodiments, $N_a'$ is a 2’-methoxy-modified nucleotide and $N_b'$ is a 2’-fluoro-modified nucleotide.

In some embodiments, the antisense strand of the siRNA of the present disclosure has a nucleotide sequence of the formula shown below:

5’-$N_a'N_b'N_a'N_b'N_a'N_b'W'N_a'X'Y'N_a'N_b'N_a'N_b'N_a'N_b'N_a'N_b'N_a'N_a'N_a'$-3’;

wherein each X’ is independently $N_a'$ or $N_b'$, Y’ is $N_a'$ or $N_b'$, and modifications on X’ and Y’ are different; $N_a'$ is a 2’-methoxy-modified nucleotide, and $N_b'$ is a 2’-fluoro-modified nucleotide; W’ represents a nucleotide comprising any one of the chemical modifications of formula (I) or the tautomer modifications thereof of the present disclosure.

In some embodiments, the sense strand of the siRNA of the present disclosure has a nucleotide sequence of the formula shown below:

5’-$N_aN_aN_aN_aN_aN_aN_bN_bN_bN_aN_aN_aN_aN_aN_aN_aN_aN_aN_a$-3’; or,
5’-$N_aN_aN_aN_aN_bN_aN_bN_bN_bN_aN_aN_aN_aN_aN_aN_aN_aN_aN_a$-3’;
wherein $N_a$ is a 2’-methoxy-modified nucleotide, and $N_b$ is a 2’-fluoro-modified nucleotide.

In some embodiments, the antisense strand of the siRNA of the present disclosure has a nucleotide sequence of the formula shown below:

5’-$N_a'N_b'N_a'N_b'N_a'N_b'W'N_a'N_a'N_b'N_a'N_b'N_a'N_b'N_a'N_b'N_a'N_b'N_a'N_a'N_a'$-3’; or,
5’-$N_a'N_b'N_a'N_b'N_a'N_b'W'N_a'N_b'N_a'N_a'N_b'N_a'N_b'N_a'N_b'N_a'N_b'N_a'N_a'N_a'$-3’;
wherein $N_a$ is a 2’-methoxy-modified nucleotide, and $N_b$ is a 2’-fluoro-modified nucleotide; and/or $N_a'$ is a 2’-methoxy-modified nucleotide, and $N_b'$ is a 2’-fluoro-modified nucleotide.

W’ represents a nucleotide comprising any one of the chemical modifications of formula (I) or the tautomer modifications thereof of the present disclosure.

In some specific embodiments, W’ represents a nucleotide comprising a chemical modification or a tautomer modification thereof; the chemical modification is selected from the group consisting of:

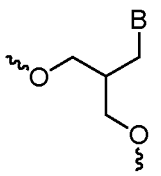 , 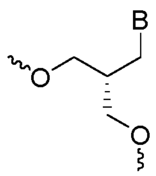 and 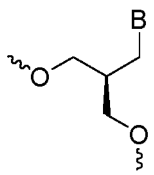 ;

wherein B is selected from the group consisting of guanine, adenine, cytosine and uracil; in some specific embodiments, B is selected from the base corresponding to position 7 of the 5’ region of the antisense strand.

In some specific embodiments, W’ represents a nucleotide comprising a chemical modification or a tautomer

modification thereof; the chemical modification is selected from the group consisting of:

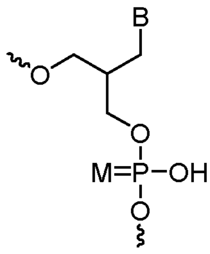 , 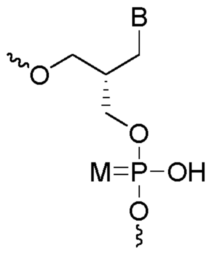 and 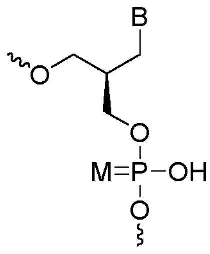 ;

wherein M is O or S; wherein B is selected from the group consisting of guanine, adenine, cytosine and uracil; in some specific embodiments, B is selected from the base corresponding to position 7 of the 5' region of the antisense strand.

In some specific embodiments, M is S. In some specific embodiments, M is O.

In some embodiments, at least one phosphoester group in the sense strand and/or the antisense strand is a phosphoester group with a modification group that provides the siRNA with increased stability in a biological sample or environment; in some embodiments, the phosphoester group with a modification group is a phosphorothioate group. Specifically, a phosphorothioate group refers to a phosphodiester group modified by replacing one non-bridging oxygen atom with a sulfur atom.

In some embodiments, the phosphorothioate group is present in at least one of the positions selected from the group consisting of:

a position between the 1st and 2nd nucleotides of the 5' end of the sense strand;
a position between the 2nd and 3rd nucleotides of the 5' end of the sense strand;
an end of the 1st nucleotide of the 3' end of the sense strand;
a position between the 1st and 2nd nucleotides of the 3' end of the sense strand;
a position between the 2nd and 3rd nucleotides of the 3' end of the sense strand;
a position between the 1st and 2nd nucleotides of the 5' end of the antisense strand;
a position between the 2nd and 3rd nucleotides of the 5' end of the antisense strand;
an end of the 1st nucleotide of the 3' end of the antisense strand;
a position between the 1st and 2nd nucleotides of the 3' end of the antisense strand; and
a position between the 2nd and 3rd nucleotides of the 3' end of the antisense strand.

In some embodiments, the sense strand and/or the antisense strand comprise a plurality of phosphorothioate groups that are present in:

a position between the 1st and 2nd nucleotides of the 5' end of the sense strand;
a position between the 2nd and 3rd nucleotides of the 5' end of the sense strand;
a position between the 1st and 2nd nucleotides of the 5' end of the antisense strand;
a position between the 2nd and 3rd nucleotides of the 5' end of the antisense strand;
a position between the 1st and 2nd nucleotides of the 3' end of the antisense strand;
a position between the 2nd and 3rd nucleotides of the 3' end of the antisense strand;
optionally an end of the 1st nucleotide of the 3' end of the sense strand, and/or
optionally a position between the 1st and 2nd nucleotides of the 3' end of the sense strand.

In some embodiments, the sense strand is selected from the nucleotide sequence of the formula shown below:

5'-NmsNmsNmNmNfNmNfNfNfNmNmNmNmNmNmNmNmNmNm-3', or
5'-NmsNmsNmNmNmNmNfNfNfNmNmNmNmNmNmNmNmNmNm-3', or
5'-NmsNmsNmNmNfNmNfNfNfNmNmNmNmNmNmNmNmNmNms-3', or
5'-NmsNmsNmNmNmNmNfNfNfNmNmNmNmNmNmNmNmNmNms-3', or
5'-NmsNmsNmNmNfNmNfNfNfNmNmNmNmNmNmNmNmNmsNm-3', or
5'-NmsNmsNmNmNmNmNfNfNfNmNmNmNmNmNmNmNmNmsNm-3', or
5'-NmsNmsNmNmNfNmNfNfNfNmNmNmNmNmNmNmNmNmsNms-3', or
5'-NmsNmsNmNmNmNmNfNfNfNmNmNmNmNmNmNmNmNmsNms-3',
wherein Nm represents any 2'-methoxy-modified nucleotide, such as 2'-methoxy-modified C, G, U, A or T; Nf represents any 2'-fluoro-modified nucleotide, such as 2'-fluoro-modified C, G, U, A or T;

the lowercase letter s, when present between uppercase letters, indicates that the two nucleotides adjacent to the letter s are linked by a phosphorothioate group; the lowercase letter s, when being the first at the 3’ end, indicates that the left nucleotide adjacent to the letter s ends in a phosphorothioate group.

In some embodiments, the antisense strand has a nucleotide sequence of the formula shown below:

5’-Nms’Nfs’Nm’Nf’Nm’Nf’W’Nm’Nm’Nf’Nm’Nf’Nm’Nf’Nm’Nf’Nm’Nf’Nms’Nms’Nm’-3’, or
5’-Nms’Nfs’Nm’Nf’Nm’Nf’W’Nm’Nf’Nm’Nm’Nf’Nm’Nf’Nm’Nf’Nm’Nf’Nms’Nms’Nm’-3’ wherein Nm’ represents any 2’-methoxy-modified nucleotide, such as 2’-methoxy-modified C, G, U, A or T; Nf represents any 2’-fluoro-modified nucleotide, such as 2’-fluoro-modified C, G, U, A or T;
the lowercase letter s, when present between uppercase letters, indicates that the two nucleotides adjacent to the letter s are linked by a phosphorothioate group, and the lowercase letter s, when being the first at the 3’ end, indicates that the left nucleotide adjacent to the letter s ends in a phosphorothioate group;
W’ represents a nucleotide comprising a chemical modification or a tautomer modification thereof; the chemical modification is selected from the group consisting of:

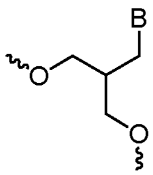 , 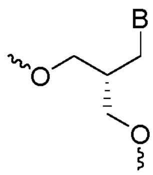 and 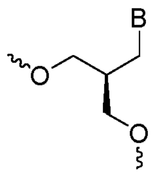 ;

wherein B is selected from the group consisting of guanine, adenine, cytosine and uracil; in some embodiments, B is selected from the base corresponding to position 7 of the 5’ region of the antisense strand.

In some specific embodiments, W’ represents a nucleotide comprising a chemical modification or a tautomer modification thereof; the chemical modification is selected from the group consisting of:

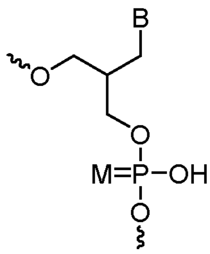 , 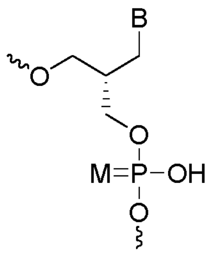 and 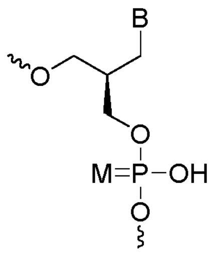 ;

wherein M is O or S; wherein B is selected from the group consisting of guanine, adenine, cytosine and uracil; in some specific embodiments, B is selected from the base corresponding to position 7 of the 5’ region of the antisense strand.
In some specific embodiments, M is S. In some specific embodiments, M is O.
In some embodiments, the siRNA comprises a sense strand selected from Table 5.
In some embodiments, the siRNA comprises any antisense strand selected from Table 5.
In some embodiments, the siRNA comprises any sense strand selected from Table 8.
In some embodiments, the siRNA comprises any antisense strand selected from Table 8.
In some embodiments, the siRNA comprises any antisense strand selected from Table 9.
In some embodiments, the siRNA comprises any sense strand selected from Table 13.
In some embodiments, the siRNA comprises any antisense strand selected from Table 13.
In some embodiments, the siRNA comprises any sense strand selected from Table 15.
In some embodiments, the siRNA comprises any antisense strand selected from Table 15.
In some embodiments, the siRNA comprises any sense strand selected from Table 24.
In some embodiments, the siRNA comprises any antisense strand selected from Table 24.
In some embodiments, the siRNA comprises any sense strand selected from Table 25.
In some embodiments, the siRNA comprises any antisense strand selected from Table 25.
In some embodiments, the siRNA comprises any sense strand selected from Table 26.
In some embodiments, the siRNA comprises any antisense strand selected from Table 26.
In some embodiments, the siRNA comprises any sense strand selected from Table 66.

In some embodiments, the siRNA comprises any antisense strand selected from Table 66.

In some embodiments, the siRNA described above, when in contact with a target gene-expressing cell, inhibits the target gene expression by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, the siRNA described above, when in contact with a target gene-expressing cell, results in a percent remaining expression of the target gene's mRNA of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, the siRNA comprising the chemical modification of the present disclosure, e.g., the chemical modification of formula (I) or formula (II), when in contact with a target gene-expressing cell, reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, the siRNA comprising the chemical modification of the present disclosure, e.g., the chemical modification of formula (I) or formula (II), when in contact with a target gene-expressing cell, reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psi CHECK activity screening and luciferase reporter gene assay, other methods such as PCR or branched DNA (bDNA)-based methods, or protein-based methods such as immunofluorescence assay, e.g., western blot or flow cytometry.

In some embodiments, the siRNA comprising the chemical modification of the present disclosure, e.g., the chemical modification of formula (I) or formula (II), when in contact with a target gene-expressing cell, reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

Conjugate

The present disclosure also provides an siRNA conjugate, which comprises any siRNA described above and a conjugated group linked to the siRNA.

In some embodiments, the conjugated group comprises a pharmaceutically acceptable targeting ligand and optionally a linker, and the siRNA, the linker and the targeting ligand are covalently or non-covalently linked in sequence.

In some embodiments, the linker is linked to the 3' end of the sense strand of the siRNA. The present disclosure also provides an siRNA conjugate, which comprises any siRNA described above and a targeting ligand linked to the siRNA.

In some embodiments, the siRNA and the targeting ligand are linked covalently or non-covalently.

In some embodiments, the targeting ligand is linked to the 3' end of the sense strand of the siRNA.

In some embodiments, the targeting ligand targets the liver.

In some embodiments, the targeting ligand binds to an asialoglycoprotein receptor (ASGPR).

In some embodiments, the targeting ligand is selected from the group consisting of a galactose cluster and a galactose derivative cluster, wherein the galactose derivative is selected from the group consisting of N-acetyl-galactosamine, N-trifluoroacetylgalactosamine, N-propionylgalactosamine, N-n-butyrylgalactosamine and N-isobutyrylgalactosamine.

In some embodiments, to promote entry of the siRNA into a cell, a lipophilic group such as cholesterol can be introduced into an end of the sense strand of the siRNA, and the lipophilic group is covalently bonded to a small interfering

nucleic acid; for example, cholesterol, lipoprotein, vitamin E, etc., are introduced to the end to facilitate going through the cell membrane consisting of a lipid bilayer and interacting with the mRNA in the cell. Meanwhile, the siRNA can also be modified by non-covalent bonding, for example, bonding to a phospholipid molecule, a polypeptide, a cationic polymer, etc, by a hydrophobic bond or an ionic bond to increase stability and biological activity.

In some embodiments, the targeting ligand is linked to an end of the siRNA by a phosphoester group, a phosphorothioate group, or a phosphonic acid group.

In some embodiments, the targeting ligand is indirectly linked to an end of the siRNA by a phosphoester group, a phosphorothioate group, or a phosphonic acid group.

In some embodiments, the targeting ligand is directly linked to an end of the siRNA by a phosphoester group, a phosphorothioate group, or a phosphonic acid group.

In some embodiments, the targeting ligand is directly linked to an end of the siRNA by a phosphoester group or a phosphorothioate group.

In some embodiments, the targeting ligand is directly linked to the 3' end of the sense strand of the siRNA by a phosphoester group or a phosphorothioate group.

In some embodiments, the targeting ligand has a structure of formula (IV) shown below,

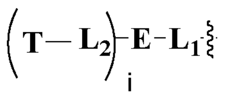

(IV)

wherein T is a targeting moiety, E is a branching group, $L_1$ is a linker moiety, and $L_2$ is a tether moiety between the targeting moiety and the branching group, wherein i is selected from an integer from 1 to 10, e.g., 1, 2, 3, 4, 5, 6, 7, 8, 9 or 10.

In some embodiments, i is selected from an integer from 2 to 8.

In some embodiments, i is selected from an integer from 3 to 5.

In some embodiments, $L_1$ is

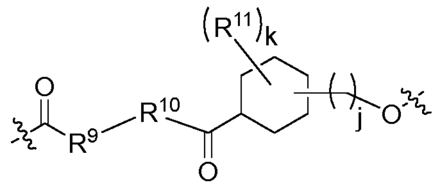

(C-1)

,

wherein $R^9$ and $R^{10}$ are each independently selected from the group consisting of -S-, - NH-, -O-, -C(O)-, -OC(O)-, -C(O)O-, -NHC(O)-, -C(O)NH-, $-CH_2-$, $-CH_2NH-$, $-CH_2O-$, $-NH-C(O)-CH_2-$, $-C(O)-CH_2-NH-$, -NH(CO)NH-, and 3- to 12-membered heterocyclyl, wherein the $-CH_2-$ is optionally substituted with a substituent selected from the group consisting of halogen, alkyl, alkoxy, and alkylamino, and the alkyl is optionally further substituted with a substituent selected from the group consisting of hydroxy, amino, and halogen;

$R^{11}$ is selected from the group consisting of deuterium, halogen, alkyl, amino, cyano, nitro, alkenyl, alkynyl, carboxyl, hydroxy, sulfhydryl, alkylsulfhydryl, alkoxy, alkylamino, -C(O)-alkyl, -C(O)-O-alkyl, $-CONH_2$, -CONH-alkyl, -OC(O)-alkyl, -NH-C(O)-alkyl, -S(O)O-alkyl, $-S(O)ONH_2$, and -S(O)ONH-alkyl, wherein the alkyl, alkenyl, alkynyl, alkylsulfhydryl, alkoxy, -C(O)-alkyl, -C(O)-O-alkyl, -CONH-alkyl, - OC(O)-alkyl, -NH-C(O)-alkyl, -S(O)O-alkyl and -S(O)ONH-alkyl are optionally further substituted with a substituent selected from the group consisting of halogen, hydroxy, amino, and sulfhydryl;

the k is selected from the group consisting of 0, 1, 2, 3 and 4;

the j is selected from an integer from 1 to 20 (e.g., 0, 1, 2, 3, 4, 5, 6, 7, 8, 9, 10, 11, 12, 13, 14, 15, 16, 17, 18, 19 or 20).

In some embodiments, $L_1$ is

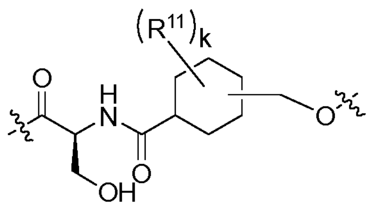

(C-2) or

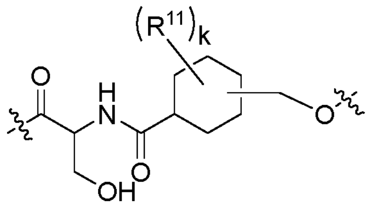

(C-2') ,

wherein $R^{11}$ is selected from the group consisting of deuterium, halogen, alkyl, amino, cyano, nitro, alkenyl, alkynyl, carboxyl, hydroxy, sulfhydryl, alkylsulfhydryl, alkoxy, alkylamino, -C(O)-alkyl, -C(O)-O-alkyl, $-CONH_2$, -CONH-alkyl, -OC(O)-alkyl, -NH-C(O)-alkyl, -S(O)O-alkyl, $-S(O)ONH_2$, and -S(O)ONH-alkyl, wherein the alkyl, alkenyl, alkynyl, carboxy, alkylsulfhydryl, alkoxy, -C(O)-alkyl, -C(O)-O-alkyl, -CONH-alkyl, -OC(O)-alkyl, -NH-C(O)-alkyl, -S(O)O-alkyl and -S(O)ONH-alkyl are optionally further substituted with a substituent selected from the group consisting of halogen, hydroxy, amino, and sulfhydryl;

the k is selected from the group consisting of 0, 1, 2, 3 and 4;

In some embodiments, $L_1$ is

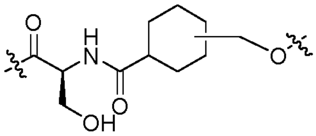

(C-3) or

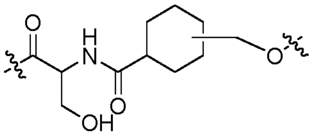

(C-3') .

In some embodiments, $L_1$ is

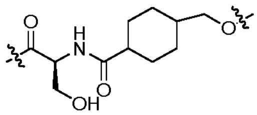

(C-4) or

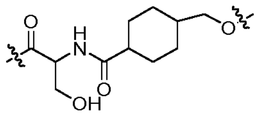

(C-4') .

In some embodiments, $L_1$ is

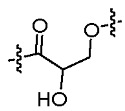

(C-5) .

In some embodiments, E in the targeting ligand is

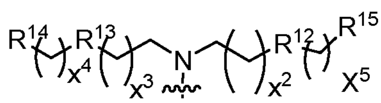

(E-1) .

wherein the $R^{12}$, $R^{13}$, $R^{14}$ and $R^{15}$ are each independently selected from the group consisting of -C(O)NH- and -C(O)-, wherein the carbonyl is optionally further substituted with alkyl, and the alkyl is optionally further substituted with a group selected from the group consisting of alkyl, hydroxy, -C(O)O-, -C(O)O-alkyl-, and - C(O)NH-;

the $X^2$, $X^3$, $X^4$ and $X^5$ are each independently selected from an integer from 0 to 10 (e.g., 0, 1, 2, 3, 4, 5, 6, 7, 8, 9 or 10).

In some embodiments, E in the targeting ligand is

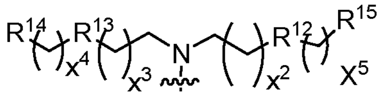

(E-1) ,

wherein the $R^{12}$, $R^{13}$, $R^{14}$ and $R^{15}$ are each independently selected from the group consisting of -C(O)NH- and -C(O)-, wherein the -C(O)NH- and -C(O)- are optionally further substituted with alkyl, and the alkyl is optionally further substituted with a group selected from the group consisting of alkyl, hydroxy, -C(O)O-, -C(O)O-alkyl-, and - C(O)NH-;

the $X^2$, $X^3$, $X^4$ and $X^5$ are each independently selected from an integer from 0 to 10 (e.g., 0, 1, 2, 3, 4, 5, 6, 7, 8, 9 or 10).

In some embodiments, E in the targeting ligand is

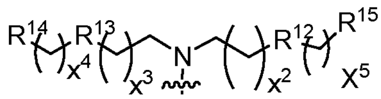

(E-1)

wherein the $R^{12}$, $R^{13}$, $R^{14}$ and $R^{15}$ are each independently selected from the group consisting of -C(O)NH-, -C(O)-,

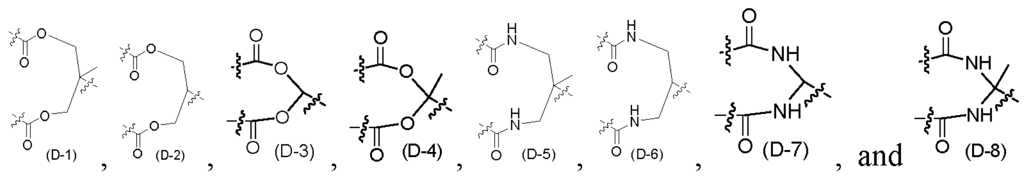

(D-1) , (D-2) , (D-3) , (D-4) , (D-5) , (D-6) , (D-7) , and (D-8) ;

the $X^2$, $X^3$, $X^4$ and $X^5$ are each independently selected from an integer from 0 to 10 (e.g., 0, 1, 2, 3, 4, 5, 6, 7, 8, 9 or 10).

In some embodiments, E in the targeting ligand is

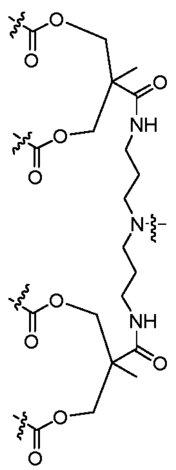

(E-2) .

In some embodiments, E in the targeting ligand is selected from the group consisting of

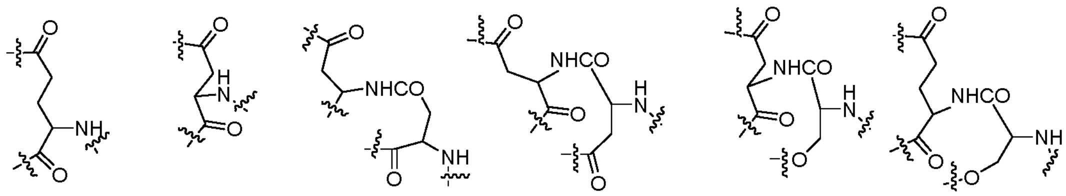

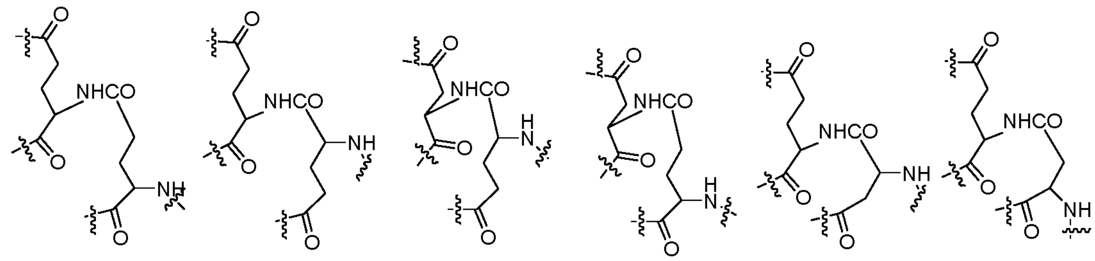

,

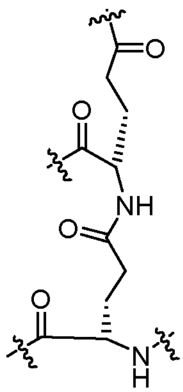

,

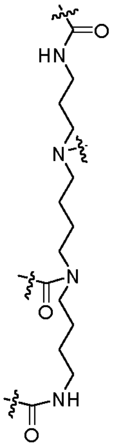

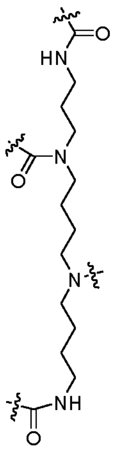

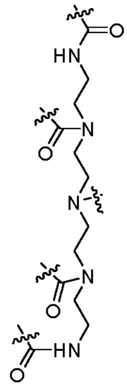

, and

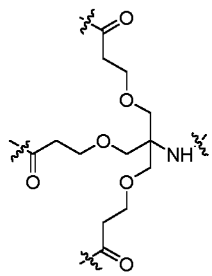

.

In some embodiments, E in the targeting ligand is selected from the group consisting of

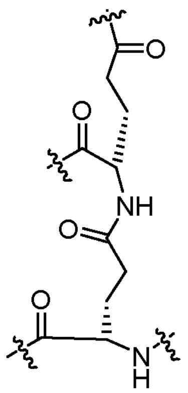

and

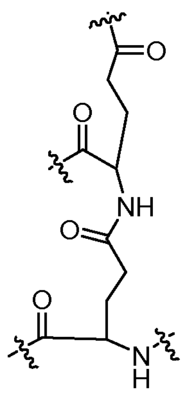

.

In some embodiments, E in the targeting ligand is selected from

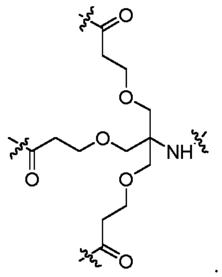

.

In some embodiments, E in the targeting ligand is

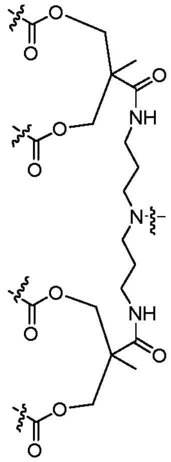

(E-2) ,

and $L_1$ is selected from the group consisting of the following structures:

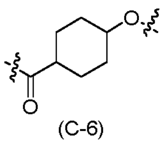

(C-6) ,

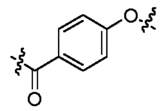

(C-7) ,

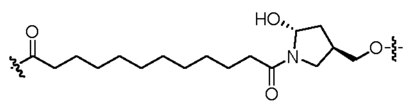

(C-8) ,

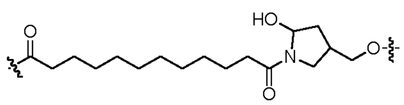

(C'-8) ,

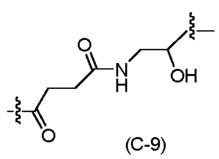

(C-9) ,

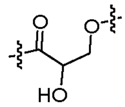

(C-5)

and

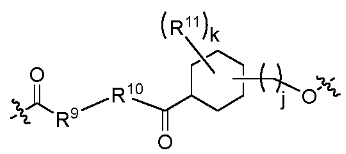

(C-1) ,

wherein $R^9$ and $R^{10}$ are each independently selected from the group consisting of -S-, - NH-, -O-, -S-, -C(O)-, -OC(O)-, -C(O)O-, -NHC(O)-, -C(O)NH-, $-CH_2-$, $-CH_2NH-$, $-CH_2O-$, $-NH-C(O)-CH_2-$, $-C(O)-CH_2-NH-$, -NH(CO)NH-, and 3- to 12-membered heterocyclyl, wherein the $-CH_2-$ is optionally substituted with a substituent selected from the group consisting of halogen, alkyl, alkoxy, and alkylamino, and the alkyl is optionally further substituted with a

substituent selected from the group consisting of hydroxy, amino, and halogen;

$R^{11}$ is selected from the group consisting of deuterium, halogen, alkyl, amino, cyano, nitro, alkenyl, alkynyl, carboxyl, hydroxy, sulfhydryl, alkylsulfhydryl, alkoxy, alkylamino, -C(O)-alkyl, -C(O)-O-alkyl, $-CONH_2$, -CONH-alkyl, -OC(O)-alkyl, -NH-C(O)-alkyl, -S(O)O-alkyl, $-S(O)ONH_2$, and -S(O)ONH-alkyl, wherein the alkyl, alkenyl, alkynyl, alkylsulfhydryl, alkoxy, -C(O)-alkyl, -C(O)-O-alkyl, -CONH-alkyl, - OC(O)-alkyl, -NH-C(O)-alkyl, -S(O)O-alkyl and -S(O)ONH-alkyl are optionally further substituted with a substituent selected from the group consisting of halogen, hydroxy, amino, and sulfhydryl;

the k is selected from the group consisting of 0, 1, 2, 3 and 4;

the j is selected from the group consisting of 0, 1, 2, 3, 4, 5, 6, 7, 8, 9, 10, 11, 12, 13, 14, 15, 16, 17, 18, 19 and 20.

In some embodiments, E in the targeting ligand is selected from the group consisting of:

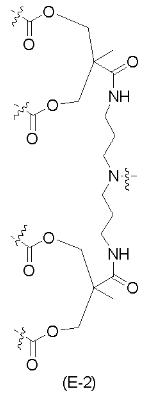
(E-2) ,
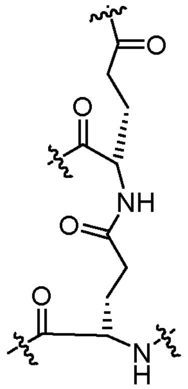
and
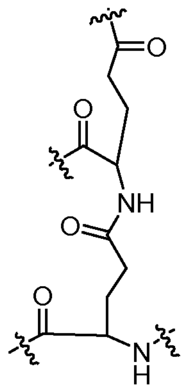
,

and $L_1$ is selected from the group consisting of:

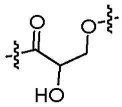
(C-5) ,
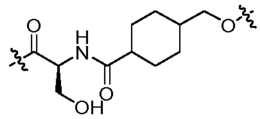
(C-4) and
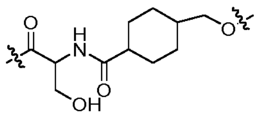
(C-4') .

In some embodiments, E in the targeting ligand is selected from the group consisting of:

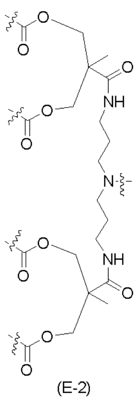
(E-2) ,

and $L_1$ is selected from the group consisting of:

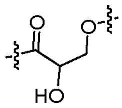
(C-5) .

-that is, E-$L_1$ is

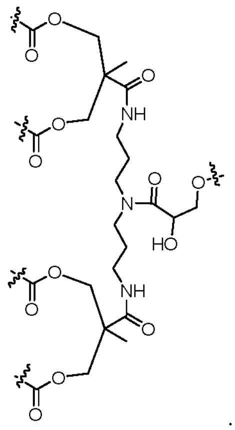

.

In some embodiments, E in the targeting ligand is selected from the group consisting of:

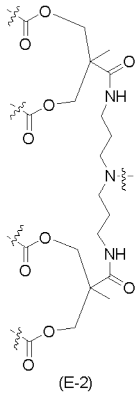

(E-2)

,

and $L_1$ is selected from the group consisting of

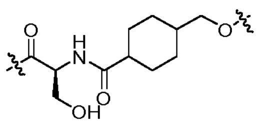

(C-4)

and

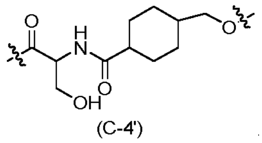

(C-4')

-that is, E-$L_1$ is

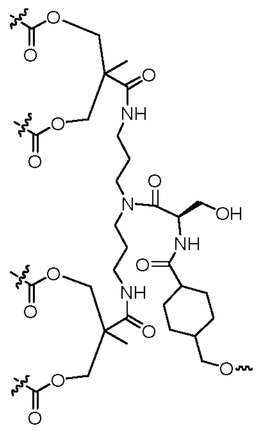
or
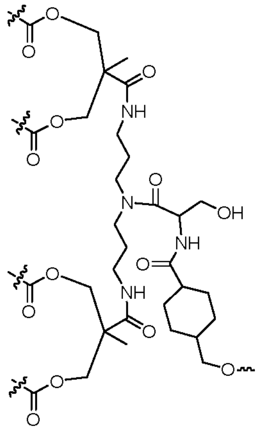
.

In some embodiments, E in the targeting ligand is selected from the group consisting of

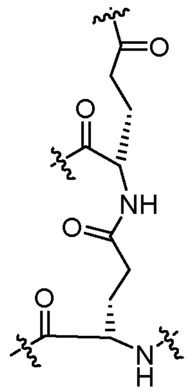
and
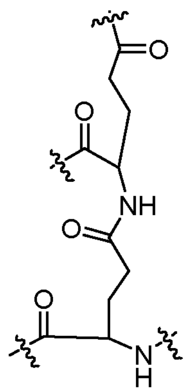
,

and $L_1$ is selected from

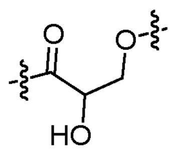

(C-5)

-that is, E-$L_1$ is

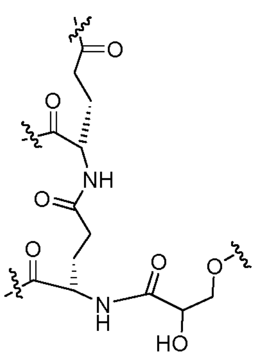
or
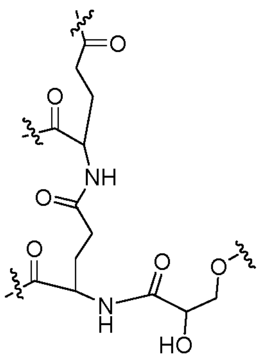
.

In some embodiments, E in the targeting ligand is selected from the group consisting of

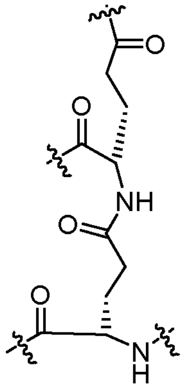 and 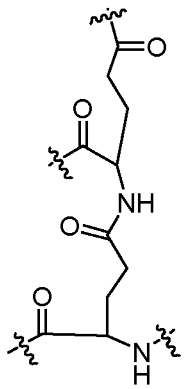 ,

and $L_1$ is selected from the group consisting of

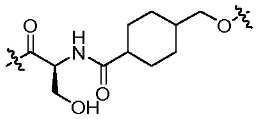

(C-4)

and

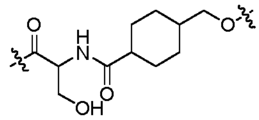

(C-4')

-that is, $E\text{-}L_1$ is

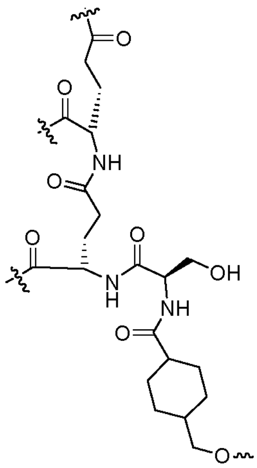

or

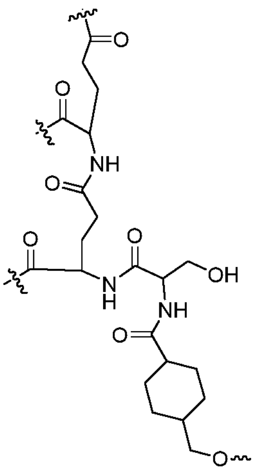

In the present disclosure, $L_2$ is a tether moiety between the targeting moiety and the branching group, and $L_2$ links and spaces the targeting moiety and the branching group. In some embodiments, one end of $L_2$ is directly linked to the targeting ligand and the other end is directly linked to the branching group E.

In some embodiments, one end of $L_2$ is directly linked to the targeting ligand and the other end is indirectly linked to the branching group E.

In some embodiments, one end of $L_2$ is indirectly linked to the targeting ligand and the other end is indirectly linked to the branching group E.

In some embodiments, the targeting ligand disclosed herein comprises two $L_2$ and two targeting moieties.

In some embodiments, the targeting ligand disclosed herein comprises three $L_2$ and three targeting moieties.

In some embodiments, the targeting ligand disclosed herein comprises four $L_2$ and four targeting moieties.

In some embodiments, the targeting ligand disclosed herein comprises a plurality of $L_2$ and a plurality of targeting moieties.

In some embodiments, $L_2$ in the present disclosure is selected from one of or a combination of 2-20 of the following groups covalently linked (e.g., 1, 2, 3, 4, 5, 6, 7, 8, 9, 10, 11, 12, 13, 14, 15, 16, 17, 18, 19 or 20):

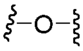 (F-1) 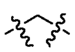 (F-2) 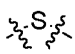 (F-3) 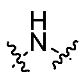 (F-4) 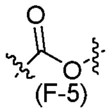 (F-5) 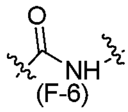 (F-6) 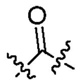 (F-7) 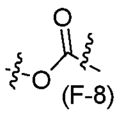 (F-8) 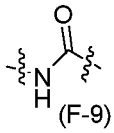 (F-9)

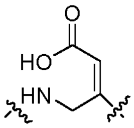 (F-10) 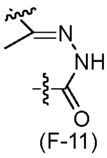 (F-11) 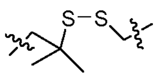 (F-12) 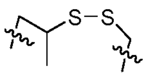 (F-13) 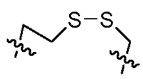 (F-14) ,

substituted or unsubstituted cycloalkyl (e.g., cyclohexyl, cyclopropyl, cyclobutyl, cyclopentyl, cycloheptyl, or cyclooctyl), substituted or unsubstituted cycloalkenyl (e.g., cyclohexenyl, cyclobutenyl, cyclopentenyl, cycloheptenyl, cyclooctenyl, cyclohexadienyl, cyclopentadienyl, cycloheptadienyl, or cyclooctadienyl), substituted or unsubstituted aryl (e.g., phenyl, naphthyl, binaphthyl, or anthracenyl), substituted or unsubstituted heteroaryl (e.g., pyridyl, pyrimidinyl, pyrrole, imidazole, furan, benzofuran, or indole), and substituted or unsubstituted heterocyclyl (e.g., tetrahydrofuran, tetrahydropyran, piperidine, or pyrrolidine) covalently linked in combinations.

In some embodiments, $L_2$ in the present disclosure is selected from one of or a combination of 2-20 of the following groups covalently linked (e.g., 1, 2, 3, 4, 5, 6, 7, 8, 9, 10, 11, 12, 13, 14, 15, 16, 17, 18, 19 or 20):

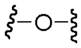 (F-1) 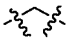 (F-2) 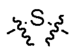 (F-3) 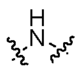 (F-4) 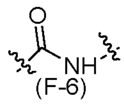 (F-6) 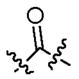 (F-7) 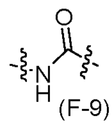 (F-9) .

In some embodiments, the targeting ligand comprises $L_2$ of the structure below,

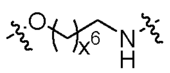

(G-1) ,

wherein $x^6$ is an integer from 1 to 20 (e.g., 1, 2, 3, 4, 5, 6, 7, 8, 9, 10, 11, 12, 13, 14, 15, 16, 17, 18, 19 or 20).

In some embodiments, the targeting ligand comprises $L_2$ of the structure below,

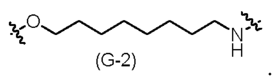

(G-2) .

In some embodiments, the targeting ligand comprises $L_2$ of the structure below,

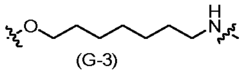

(G-3).

In some embodiments, the targeting ligand comprises $L_2$ of the structure below,

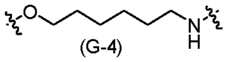

(G-4).

In some embodiments, the targeting ligand comprises $L_2$ of the structure below,

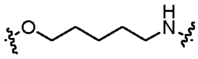

(G-5).

In some embodiments, the targeting ligand comprises $L_2$ of the structure below,

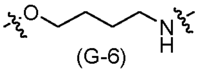

(G-6).

In some embodiments, the targeting ligand comprises $L_2$ of the structure below,

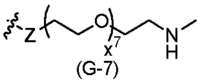

(G-7),

wherein $x^7$ is an integer from 1 to 20 (e.g., 1, 2, 3, 4, 5, 6, 7, 8, 9, 10, 11, 12, 13, 14, 15, 16, 17, 18, 19 or 20), and Z is

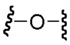

(F-1)

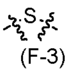

(F-3)

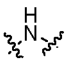

(F-4).

In some embodiments, the targeting ligand comprises $L_2$ of the structure below,

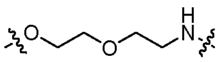

(G-8).

In some embodiments, the targeting ligand comprises $L_2$ of the structure below,

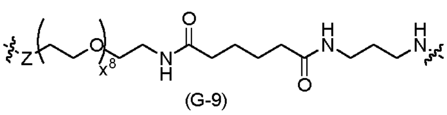

(G-9).

wherein $x^8$ is an integer from 1 to 20 (e.g., 1, 2, 3, 4, 5, 6, 7, 8, 9, 10, 11, 12, 13, 14, 15, 16, 17, 18, 19 or 20), and Z is

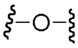

(F-1)

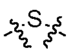

(F-3)

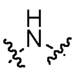

(F-4).

In some embodiments, the targeting ligand comprises $L_2$ of the structure below,

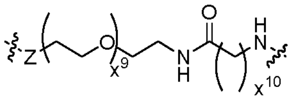

(G-10) ,

wherein $x^9$ and $X^{10}$ are each independently selected from an integer from 1 to 20 (e.g., 1, 2, 3, 4, 5, 6, 7, 8, 9, 10, 11, 12, 13, 14, 15, 16, 17, 18, 19 or 20), and Z is

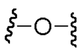 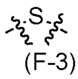 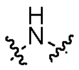

(F-1) (F-3) (F-4).

In some embodiments, the targeting ligand comprises $L_2$ of the structure below,

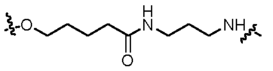

(G-11) .

In some embodiments, the targeting ligand comprises $L_2$ of the structure below,

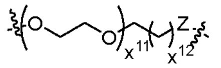

(G-12) .

wherein $x^7$ and $X^8$ are each independently selected from an integer from 1 to 20 (e.g., 1, 2, 3, 4, 5, 6, 7, 8, 9, 10, 11, 12, 13, 14, 15, 16, 17, 18, 19 or 20), and Z is

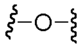 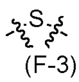 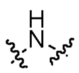

(F-1) (F-3) (F-4).

In some specific embodiments, the targeting ligand has the structure below:

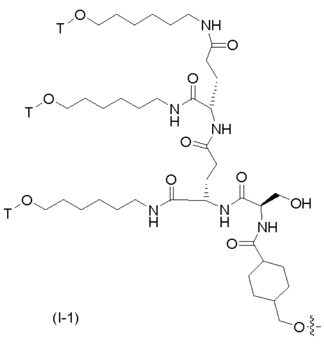

(I-1) or

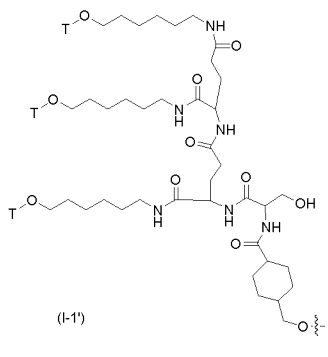

(I-1').

In some specific embodiments, the targeting ligand has the structure below:

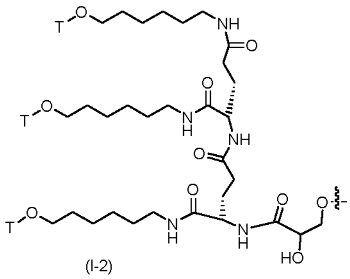

(I-2) or

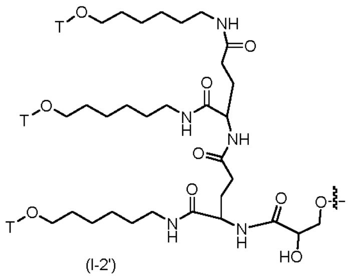

(I-2') .

In some specific embodiments, the targeting ligand has the structure below:

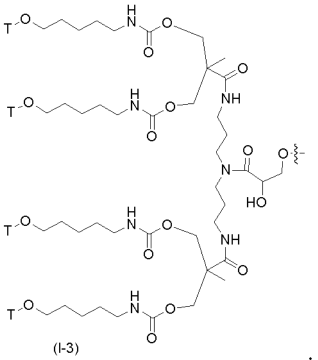

(I-3) .

In some specific embodiments, the targeting ligand has the structure below:

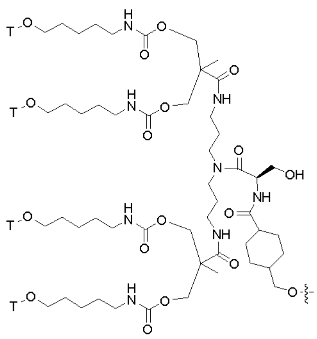

(I-4) or

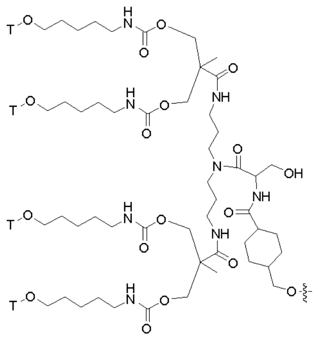

(I-4') .

In some embodiments, the targeting moiety of the targeting ligand consists of one or more targeting groups, and the targeting ligand assists in directing the delivery of the therapeutic agent linked thereto to the desired target location. In some cases, the targeting moiety can bind to a cell or cellular receptor and initiate endocytosis to facilitate entry of the therapeutic agent into the cell. The targeting moiety can comprise a compound with affinity for a cellular receptor or a cell surface molecule or an antibody. Various targeting ligands comprising targeting moieties can be linked to therapeutic agents and other compounds to target the agents at cells and specific cellular receptors.

In some embodiments, the types of the targeting moieties include carbohydrates, cholesterol, and cholesterol groups or steroids. Targeting moieties that can bind to cellular receptors include saccharides such as galactose, galactose derivatives (e.g., N-acetyl-galactosamine, N-trifluoroacetylgalactosamine, N-propionylgalactosamine, N-n-butyrylgalactosamine, and N-isobutyrylgalactosamine), mannose, and mannose derivatives.

Targeting moieties that bind to asialoglycoprotein receptors (ASGPR) are known to be particularly used for directing the delivery of oligomeric compounds to the liver. Asialoglycoprotein receptors are extensively expressed on liver cells (hepatocytes). The targeting moieties of cellular receptors targeting ASCPR include galactose and galactose derivatives. Specifically, clusters of galactose derivatives, including clusters consisting of 2, 3, 4 or more than 4 N-acetyl-galactosamines (GalNAc or NAG), can promote the uptake of certain compounds in hepatocytes. The GalNAc cluster coupled to the oligomeric compound is used for directing the composition to the liver where the N-acetyl-galactosamine saccharide can bind to the asialoglycoprotein receptors on the liver cell surface. It is believed that the binding to the asialoglycoprotein receptors will initiate receptor-mediated endocytosis, thereby promoting entry of the compound into

the interior of the cell.

In some embodiments, the targeting ligand can include 2, 3, 4 or more than 4 targeting moieties.

In some embodiments, the targeting ligand disclosed herein can include 1, 2, 3, 4 or more than 4 targeting moieties linked to the branching group by $L_2$.

In some embodiments, the targeting ligand is in the form of a galactose cluster.

In some embodiments, each targeting moiety comprises a galactosamine derivative, which is N-acetyl-galactosamine. Other sugars that can be used as targeting moieties and that have affinity for asialoglycoprotein receptors can be selected from the group consisting of galactose, galactosamine, N-formyl-galactosamine, N-acetyl-galactosamine, N-propionyl-galactosamine, N-n-butyryl-galactosamine, N-isobutyrylgalactosamine, etc.

In some embodiments, the targeting ligand in the present disclosure comprises N-acetylgalactosamine as a targeting moiety,

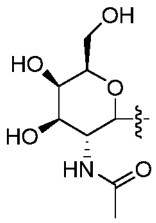

(H-2)

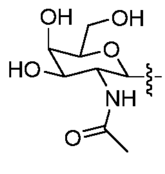

(H-2).

In some embodiments, the targeting ligand comprises three terminal galactosamines or galactosamine derivatives (such as N-acetyl-galactosamine), each of which has affinity for asialoglycoprotein receptors. In some embodiments, the targeting ligand comprises three terminal N-acetyl-galactosamine (GalNAc or NAG) as targeting moieties.

In some embodiments, the targeting ligand comprises four terminal galactosamines or galactosamine derivatives (such as N-acetyl-galactosamine), each of which has affinity for asialoglycoprotein receptors. In some embodiments, the targeting ligand comprises four terminal N-acetyl-galactosamine (GalNAc or NAG) as targeting moieties.

The terms commonly used in the art when referring to three terminal N-acetyl-galactosamines include tri-antennary, tri-valent and trimer.

The terms commonly used in the art when referring to four terminal N-acetyl-galactosamines include tetra-antennary, tetra-valent and tetramer.

In some specific embodiments, the targeting ligand of the present disclosure has the structure below,

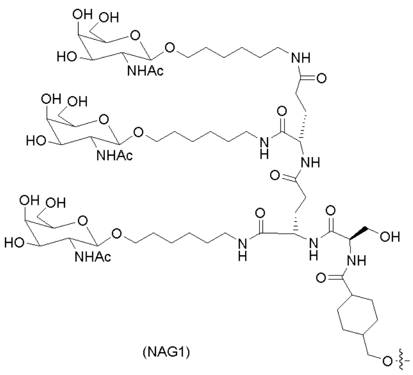

(NAG1) or

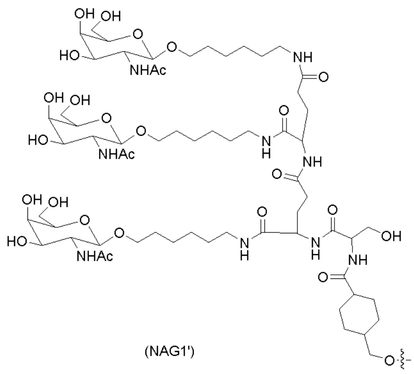

(NAG1').

In some specific embodiments, the targeting ligand provided by the present disclosure has the structure below,

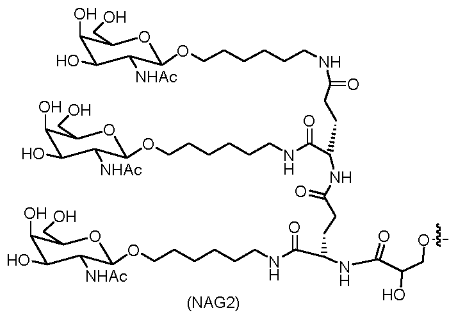

(NAG2) or

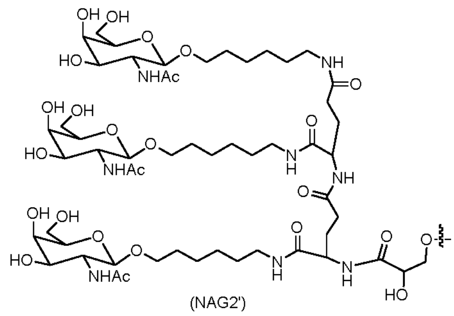

(NAG2').

In some specific embodiments, the targeting ligand provided by the present disclosure has the structure below,

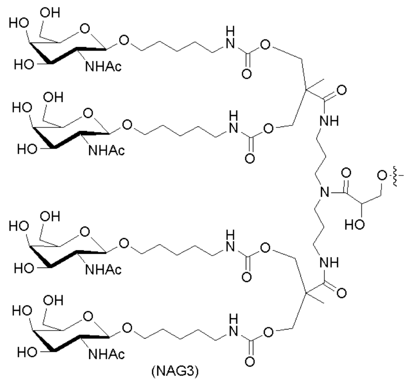

(NAG3).

In some specific embodiments, the targeting ligand provided by the present disclosure has the structure below,

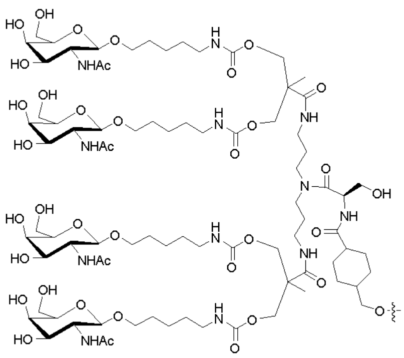

(NAG4) or

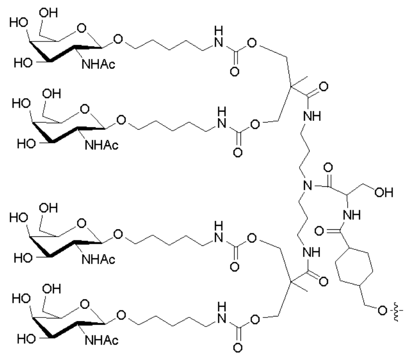

(NAG4').

In some embodiments, the siRNA of the present disclosure is linked to the targeting ligand of the present disclosure, forming an siRNA conjugate as shown below,

, forming an siRNA conjug

$$\left(T - L_2\right)_i - E - L_1 - D,$$

, wherein T is a targeting moiety, E is a branching group, $L_1$ is a linker moiety, and $L_2$ is a tether moiety between the targeting moiety and the branching group, wherein x is selected from an integer from 1 to 10, and D is the siRNA according to any one of the embodiments described above.

In some embodiments, D is an siRNA targeting ApoC3.

In some embodiments, D is an siRNA targeting HBV-X.

In some embodiments, D is an siRNA targeting F11.

In some embodiments, D is an siRNA targeting HBV-S.

In some embodiments, D is an siRNA targeting angiopoietin-like protein-3 (ANGPTL3).

In some embodiments, D is an siRNA targeting the transthyretin (TTR) gene.

In some embodiments, D is any siRNA of the present disclosure.

In some embodiments, the $L_1$ is linked to the 3' end of the sense strand of the siRNA.

In some embodiments, the targeting ligand is linked to an end of the siRNA by a phosphoester group, a phosphorothioate group, or a phosphonic acid group.

In some embodiments, the targeting ligand is indirectly linked to an end of the siRNA by a phosphoester group, a phosphorothioate group, or a phosphonic acid group.

In some embodiments, the targeting ligand is directly linked to an end of the siRNA by a phosphoester group, a phosphorothioate group, or a phosphonic acid group.

In some embodiments, the targeting ligand is directly linked to an end of the siRNA by a phosphoester group or a phosphorothioate group.

In some embodiments, the targeting ligand is directly linked to the 3' end of the sense strand of the siRNA by a phosphoester group or a phosphorothioate group.

In some specific embodiments, the siRNA conjugate described in the present disclosure is shown below,

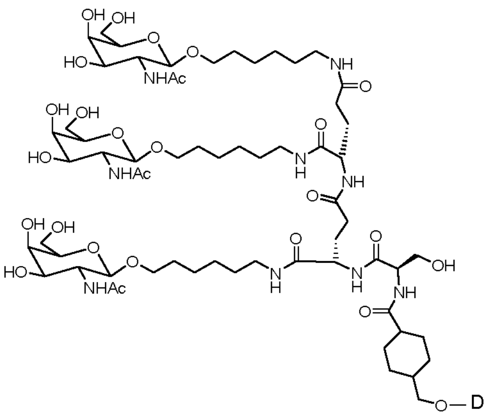

or

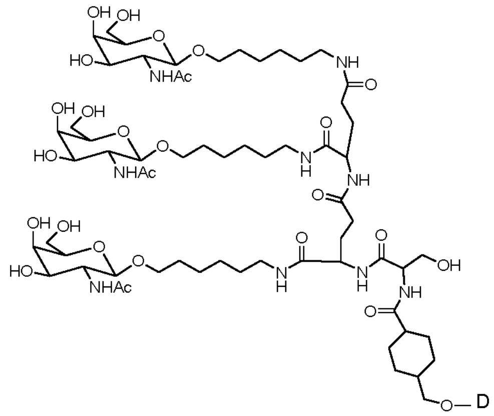

,

wherein D is an siRNA according to any one of the embodiments described above.

In some embodiments, D is an siRNA targeting ApoC3.

In some embodiments, D is an siRNA targeting HBV-X.

In some embodiments, D is an siRNA targeting F11.

In some embodiments, D is an siRNA targeting HBV-S.

In some embodiments, D is an siRNA targeting angiopoietin-like protein-3 (ANGPTL3).

In some embodiments, D is an siRNA targeting the transthyretin (TTR) gene.

In some specific embodiments, the targeting ligand is directly linked to the 3' end of the sense strand of the siRNA by a phosphoester group or a phosphorothioate group.

In some specific embodiments, the siRNA conjugate described in the present disclosure is shown below,

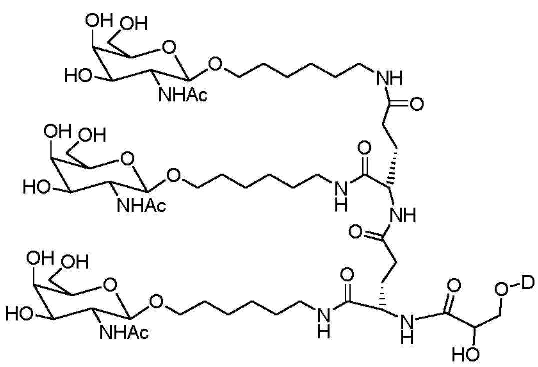

or

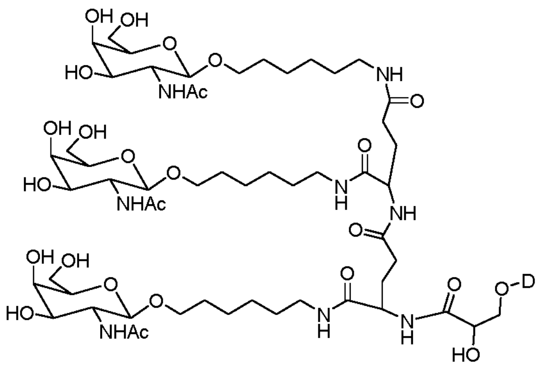

,

wherein D is an siRNA according to any one of the embodiments described above.

In some embodiments, D is an siRNA targeting ApoC3.

In some embodiments, D is an siRNA targeting HBV-X.

In some embodiments, D is an siRNA targeting F11.

In some embodiments, D is an siRNA targeting HBV-S.

In some embodiments, D is an siRNA targeting angiopoietin-like protein-3 (ANGPTL3).

In some embodiments, D is an siRNA targeting the transthyretin (TTR) gene.

In some specific embodiments, the targeting ligand is directly linked to the 3' end of the sense strand of the siRNA by a phosphoester group or a phosphorothioate group.

In some specific embodiments, the siRNA conjugate described in the present disclosure is shown below,

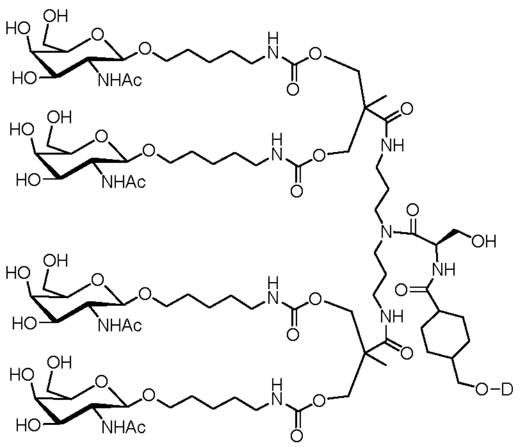

or

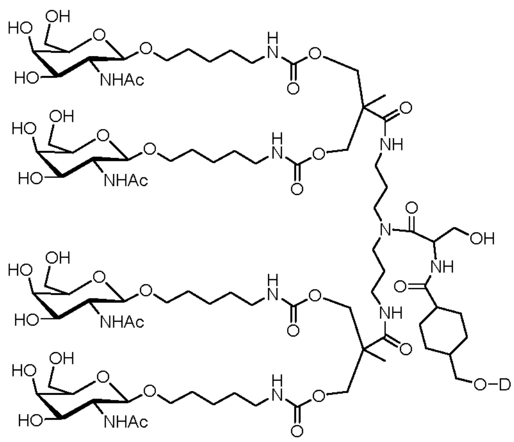

,

wherein D is an siRNA according to any one of the embodiments described above.

In some embodiments, D is an siRNA targeting ApoC3.

In some embodiments, D is an siRNA targeting HBV-X.

In some embodiments, D is an siRNA targeting F11.

In some embodiments, D is an siRNA targeting HBV-S.

In some embodiments, D is an siRNA targeting angiopoietin-like protein-3 (ANGPTL3).

In some embodiments, D is an siRNA targeting the transthyretin (TTR) gene.

In some specific embodiments, the targeting ligand is directly linked to the 3' end of the sense strand of the siRNA by a phosphoester group or a phosphorothioate group.

In some specific embodiments, the siRNA conjugate described in the present disclosure is shown below,

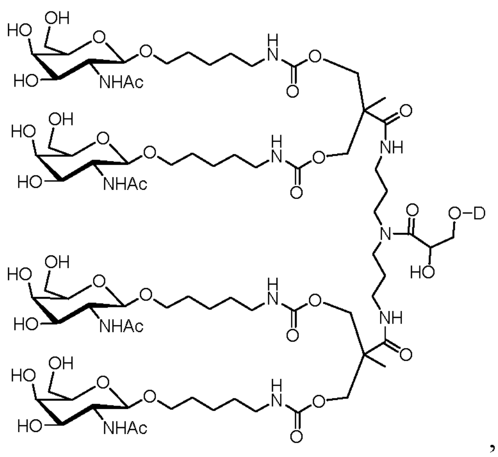

,

wherein D is an siRNA according to any one of the embodiments described above.

In some embodiments, D is an siRNA targeting ApoC3.

In some embodiments, D is an siRNA targeting HBV-X.

In some embodiments, D is an siRNA targeting F11.

In some embodiments, D is an siRNA targeting HBV-S.

In some embodiments, D is an siRNA targeting angiopoietin-like protein-3 (ANGPTL3).
In some embodiments, D is an siRNA targeting the transthyretin (TTR) gene.
In some specific embodiments, the targeting ligand is directly linked to the 3' end of the sense strand of the siRNA by a phosphoester group or a phosphorothioate group.
In some specific embodiments, $L_1$ is linked to D by a phosphoester group, a phosphorothioate group, or a phosphonic acid group.
In some specific embodiments, $L_1$ is linked to the 3' end of the D's sense strand by a phosphoester group, a phosphorothioate group, or a phosphonic acid group.
In some specific embodiments, $L_1$ is directly linked to the 3' end of the D's sense strand by a phosphoester group, or a phosphorothioate group.
In some specific embodiments, $L_1$ is indirectly linked to the 3' end of the D's sense strand by a phosphoester group, or a phosphorothioate group. In some embodiments, to promote entry of the siRNA into a cell, a lipophilic group such as cholesterol can be introduced into an end of the sense strand of the siRNA, and the lipophilic group is covalently bonded to a small interfering nucleic acid; for example, cholesterol, lipoprotein, vitamin E, etc., are introduced to the end to facilitate going through the cell membrane consisting of a lipid bilayer and interacting with the mRNA in the cell. Meanwhile, the siRNA can also be modified by non-covalent bonding, for example, bonding to a phospholipid molecule, a polypeptide, a cationic polymer, etc, by a hydrophobic bond or an ionic bond to increase stability and biological activity.

Composition

Another aspect of the present disclosure provides a composition, which comprises the conjugate described above, and one or more pharmaceutically acceptable excipients, such as a carrier, a vehicle, a diluent, and/or a delivery polymer.
The present disclosure also provides a pharmaceutical composition, which comprises the siRNA or siRNA conjugate of the present disclosure.
In some embodiments, the pharmaceutical composition can further comprise a pharmaceutically acceptable auxiliary material and/or adjuvant; the auxiliary material can be one or more of various formulations or compounds conventionally used in the art. For example, the pharmaceutically acceptable auxiliary material can include at least one of a pH buffer, a protective agent, and an osmotic pressure regulator.

Use and Method

Another aspect of the present disclosure provides use of the conjugate or the composition comprising the conjugate described above in manufacturing a medicament for treating a disease in a subject; in some embodiments, the disease is selected from a hepatic disease.
Another aspect of the present disclosure provides a method for treating a disease in a subject, which comprises administering to the subject the conjugate or the composition described above.
Another aspect of the present disclosure provides a method for inhibiting mRNA expression in a subject, which comprises administering to the subject the conjugate or the composition described above.
Another aspect of the present disclosure provides a method for delivering an expression-inhibiting oligomeric compound to the liver *in vivo*, which comprises administering to a subject the conjugate or the composition described above.
The conjugate, the composition and the methods disclosed herein can reduce the target mRNA level in a cell, a cell population, a tissue or a subject, which comprises administering to the subject a therapeutically effective amount of the expression-inhibiting oligomer described herein. The expression-inhibiting oligomer is linked to a targeting ligand, thereby inhibiting target mRNA expression in the subject.
In some embodiments, the subject has been previously identified as having pathogenic upregulation of the target gene in the targeted cell or tissue.
The subject described in the present disclosure refers to a subject having a disease or condition that would benefit from reduction or inhibition of target mRNA expression. Delivery can be accomplished by topical administration (e.g., direct injection, implantation or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In optional embodiments, the pharmaceutical composition provided by the present disclosure can be administered by injection, for example, intravenous, intramuscular, intradermal, subcutaneous, intraduodenal, or intraperitoneal injection.
In optional embodiments, the conjugate can be packaged in a kit.
In some embodiments, the siRNA conjugate or pharmaceutical composition described above, when in contact with a target gene-expressing cell, inhibits the target gene expression by at least 5%, at least 10%, at least 15%, at least

20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, the siRNA conjugate or pharmaceutical composition described above, when in contact with a target gene-expressing cell, results in a percent remaining expression of the target gene's mRNA of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA conjugate or pharmaceutical composition is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA conjugate or pharmaceutical composition is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA conjugate or pharmaceutical composition is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

The present disclosure also provides a method for silencing a target gene or the mRNA of a target gene in a cell, which comprises the step of introducing into the cell the siRNA, the siRNA conjugate, and/or the pharmaceutical composition of the present disclosure.

The present disclosure also provides a method for silencing a target gene or the mRNA of a target gene in a cell *in vivo* or *in vitro*, which comprises the step of introducing into the cell the siRNA, the siRNA conjugate, and/or the pharmaceutical composition according to the present disclosure.

The present disclosure also provides a method for inhibiting a target gene or the expression of the mRNA of a target gene, which comprises administering to a subject in need an effective amount or effective dose of the siRNA, the siRNA conjugate, and/or the pharmaceutical composition according to the present disclosure.

In some embodiments, administration is carried out through routes of administration including intramuscular, intrabronchial, intrapleural, intraperitoneal, intraarterial, lymphatic, intravenous, subcutaneous, cerebrospinal, or combinations thereof.

In some embodiments, the effective amount or effective dose of the siRNA, the siRNA conjugate and/or the pharmaceutical composition is from about 0.001 mg/kg body weight to about 200 mg/kg body weight, from about 0.01 mg/kg body weight to about 100 mg/kg body weight, or from about 0.5 mg/kg body weight to about 50 mg/kg body weight.

In some embodiments, the target gene is the hepatitis B virus (HBV) gene, the angiopoietin-like protein-3 (ANGPTL3) gene, or the transthyretin (TTR) gene.

The present disclosure also provides use of the aforementioned siRNA and/or pharmaceutical composition and/or siRNA conjugate in manufacturing a medicament for preventing and/or treating pathological conditions and diseases caused by the hepatitis B virus.

The present disclosure also provides use of the aforementioned siRNA and/or pharmaceutical composition and/or siRNA conjugate in manufacturing a medicament for preventing and/or treating hepatitis B.

The present disclosure also provides a method for treating hepatitis B, which comprises administering to a

patient in need the aforementioned siRNA and/or pharmaceutical composition and/or siRNA conjugate.

The present disclosure also provides a method for inhibiting HBV gene expression in a hepatitis cell infected with chronic HBV, which comprises introducing an effective amount or effective dose of the aforementioned siRNA and/or pharmaceutical composition and/or siRNA conjugate into the hepatitis cell infected with chronic HBV The present disclosure also provides use of the aforementioned siRNA and/or pharmaceutical composition and/or siRNA conjugate in manufacturing a medicament for preventing and/or treating pathological conditions and diseases caused by aberrant expression of the ANGPTL3 gene or TTR gene in mammals (e.g., humans).

The present disclosure also provides a method for treating pathological conditions and diseases caused by aberrant expression of the ANGPTL3 gene or TTR gene, which comprises administering an effective amount or dose of the aforementioned siRNA and/or pharmaceutical composition and/or siRNA conjugate.

Pathological conditions and diseases caused by aberrant expression of the ANGPTL3 gene include cardiovascular and/or metabolic diseases, such as hyperlipidemia, hypertriglyceridemia, hypercholesterolemia, obesity, diabetes and/or ischemic heart disease.

Pathological conditions and diseases caused by aberrant expression of the TTR gene include sensory neuropathy (e.g., sensory abnormalities in the distal limbs, or sensory decline), autonomic neuropathy (e.g., gastrointestinal dysfunction such as gastric ulcer, or orthostatic hypotension), motor neuropathy, seizures, dementia, myelopathy, polyneuropathy, carpal tunnel syndrome, autonomic defects, cardiomyopathy, vitreous opacity, renal insufficiency, renal disease, a substantial decrease in mBMI (change in body mass index), cranial nerve dysfunction, and lattice corneal dystrophy.

In some embodiments, the aforementioned siRNA and/or pharmaceutical composition and/or siRNA conjugate exhibits excellent on-target activity and reduced off-target activity in regulating genes expressed in the liver, or in treating pathological conditions or diseases caused by aberrant expression of genes in liver cells. Genes expressed in the liver include, but are not limited to, the ApoB, ApoC, ANGPTL3, PCSK9, SCD1, TIMP-1, Col1A1, FVII, STAT3, p53, HBV and HCV genes, etc. In some embodiments, the specific gene is selected from the group consisting of the hepatitis B virus gene, the angiopoietin-like protein 3 gene, or the apolipoprotein C3 gene. Accordingly, the disease is selected from the group consisting of chronic liver disease, hepatitis, liver fibrosis disease, liver proliferative disease and dyslipidemia. In some embodiments, the dyslipidemia is hypercholesterolemia, hypertriglyceridemia or atherosclerosis.

In some embodiments, the aforementioned siRNA, siRNA conjugate and/or pharmaceutical composition may also be used to treat other liver diseases, including diseases characterized by unwanted cellular proliferation, hematological diseases, metabolic diseases, and diseases characterized by inflammation. A proliferative disease of the liver may be a benign or malignant disease, such as cancer, hepatocellular carcinoma (HCC), liver metastasis or hepatoblastoma. Hematologic or inflammatory diseases of the liver may be diseases relating to coagulation factors and complement-mediated inflammation or fibrosis. Metabolic diseases of the liver include dyslipidemia and irregularities in glucose regulation.

Host Cell

The present disclosure also provides a cell, which comprises the siRNA or siRNA conjugate of the present disclosure.

Kit

The present disclosure also provides a kit, which comprises the siRNA or siRNA conjugate of the present disclosure.

Intermediate

The present disclosure also provides a compound of formula (II) or a tautomer thereof,

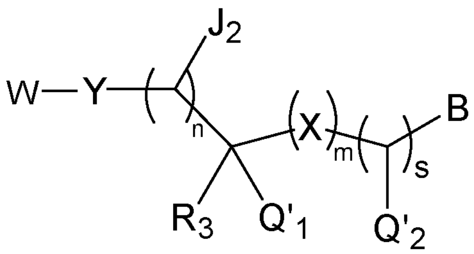

(II)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
$J_2$ is H or $C_1$-$C_6$ alkyl;
n = 0, 1 or 2; m = 0, 1 or 2; s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$Q'_1$ is

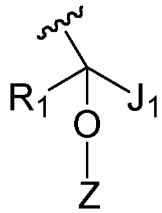

,

and $Q_2$ is $R_2$; or $Q_1$ is $R_2$, and $Q'_2$ is

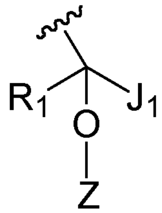

;

wherein $R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$J_1$ is H or $C_1$-$C_6$ alkyl;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is a base or a base analog; and
W is a leaving group, and Z is a phosphorus-containing active reaction group.

In some embodiments, W is MMTr or DMTr.

In some embodiments, Z is

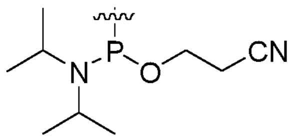

.

In some embodiments, the compound described above is not

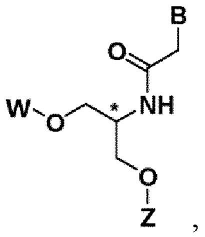

,

wherein W, B and Z are as defined above.

In some embodiments, when X is NH-CO, $R_1$ is not H.

In some embodiments, the compound described above is not:

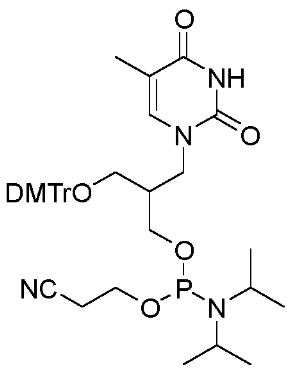

or

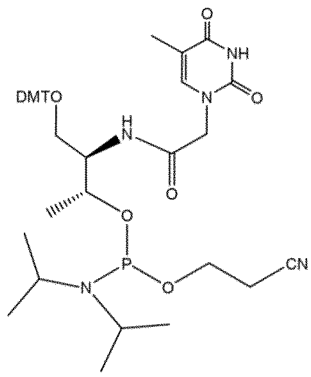

.

In some embodiments, the compound described above is not:

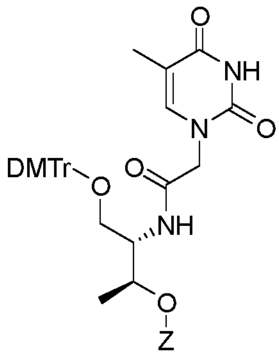

or

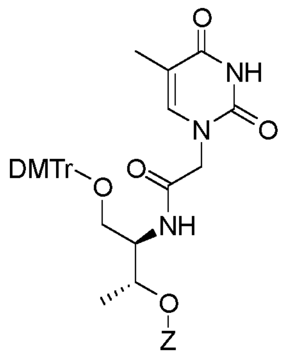

,

wherein Z is as defined above.

In some embodiments, the compound of formula (II) or the tautomer thereof is specifically a compound of formula (II-1) or a tautomer thereof,

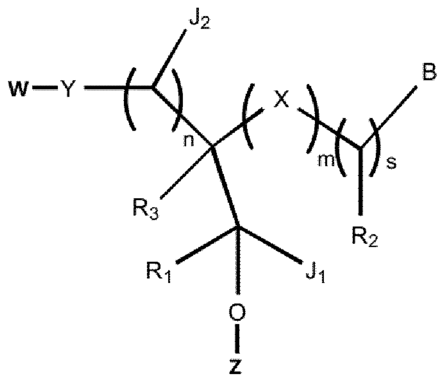

(II-1)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
each $J_1$ and each $J_2$ is independently H or $C_1$-$C_6$ alkyl;
n = 0, 1 or 2; m = 0, 1 or 2; s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is a base or a base analog; and
W is a leaving group, and Z is a phosphorus-containing active reaction group.

In some embodiments, W is MMTr or DMTr.

In some embodiments, Z is

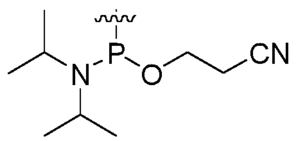

.

In some embodiments, the compound described above is not

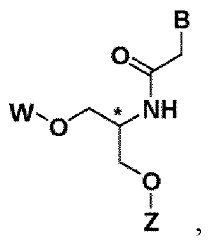

,

wherein W, B and Z are as defined above.

In some embodiments, when X is NH-CO, $R_1$ is not H.

In some embodiments, the compound described above is not:

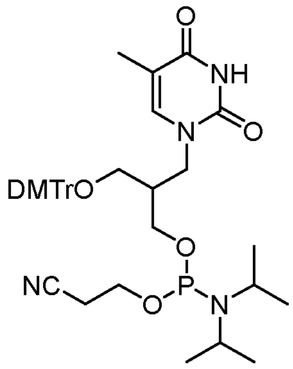

or

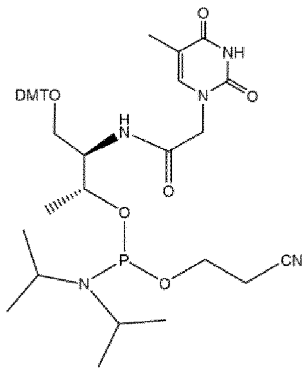

.

In some embodiments, the compound described above is not:

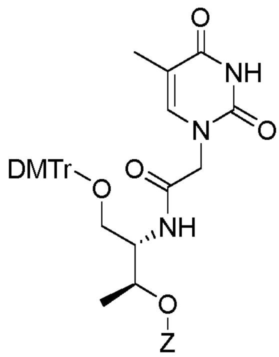

or

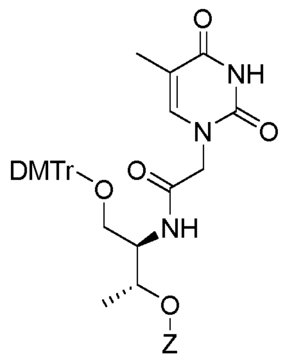

,

wherein Z is as defined above.

In some embodiments, the compound of formula (II) or the tautomer thereof is specifically a compound of formula (II-2) or a tautomer thereof,

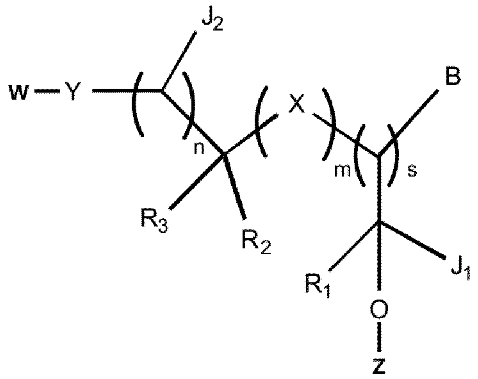

(II-2)

wherein Y is selected from the group consisting of O, NH and S;

each X is independently selected from the group consisting of $CR_4(R_4')$, S, $NR_5$ and NH-CO, wherein $R_4$, $R_4'$ and $R_5$ are each independently H or $C_1$-$C_6$ alkyl;

n = 0, 1 or 2; m = 0, 1 or 2; s = 0 or 1;

each $J_1$ and each $J_2$ is independently H or $C_1$-$C_6$ alkyl;

$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;

$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is a base or a base analog; and
W is a leaving group, and Z is a phosphorus-containing active reaction group.

In some embodiments, W is MMTr or DMTr.

In some embodiments, Z is

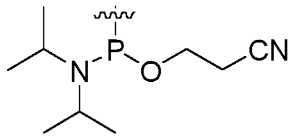

.

In some embodiments, the compound described above is not

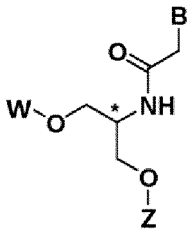

,

wherein W, B and Z are as defined above.

In some embodiments, when X is NH-CO, $R_1$ is not H.

In some embodiments, the compound described above is not:

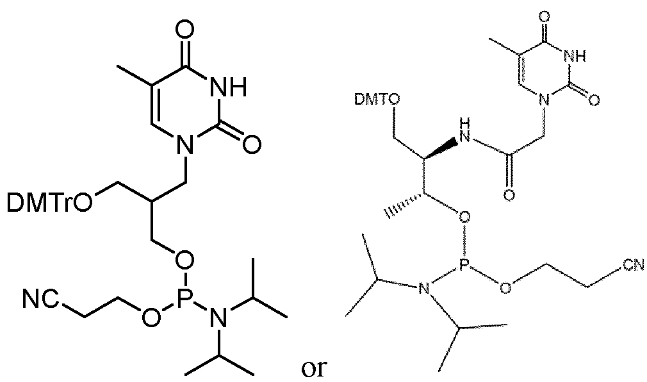

or

.

In some embodiments, the compound described above is not:

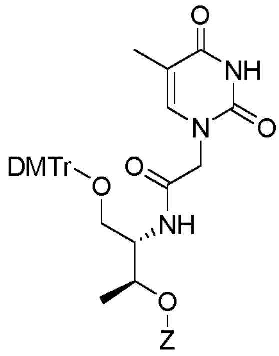 or 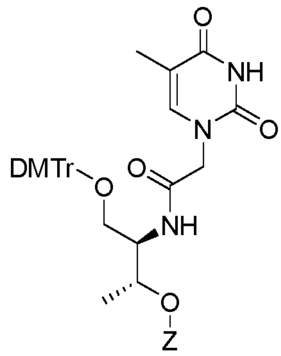 ,

wherein Z is as defined above.

In some embodiments, each X is independently selected from the group consisting of $CR_4(R_4')$, S, $NR_5$ and NH-CO, wherein $R_4$, $R_4'$ and $R_5$ are each independently H or $C_1$-$C_3$ alkyl;

n = 0, 1 or 2; m = 0, 1 or 2; s = 0 or 1;
each $J_1$ and each $J_2$ is independently H or $C_1$-$C_3$ alkyl;
$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_3$ alkyl, $C_1$-$C_3$ alkoxy, $C_2$-$C_4$ alkenyl, $C_2$-$C_4$ alkynyl and $(CH_2)_qR_7$, wherein $R_7$ is selected from the group consisting of OH, halogen, methoxy, ethoxy, $N_3$, $C_2$-$C_4$ alkenyl and $C_2$-$C_4$ alkynyl, and q = 1, 2 or 3;
$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_4$ alkenyl and $C_2$-$C_4$ alkynyl, and r = 1, 2 or 3;
optionally, $R_1$ and $R_2$ are directly linked to form a ring;
B is selected from the group consisting of purine bases, pyrimidine bases, indole, 5-nitroindole and 3-nitropyrrole.

In some embodiments, each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;

n = 0, 1 or 2; m = 0, 1 or 2; s = 0 or 1;
each $J_1$ and each $J_2$ is independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring;
B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;

n = 0, 1 or 2; m = 0, 1 or 2; s = 0 or 1;
each $J_1$ and each $J_2$ is independently H or methyl;

$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring;
B is selected from the group consisting of purine, adenine, guanine, isoguanine, hypoxanthine, xanthine, 2,6-diaminopurine, 6-dimethylaminopurine, 2-aminopurine, 7-deazapurine, cytosine, 5-methylcytosine, isocytosine, pseudocytosine, uracil, pseudouracil, 2-thiouridine, 4-thiouridine, thymine, indole, 5-nitroindole and 3-nitropyrrole.

In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and CHzOH;
$R_2$ is selected from the group consisting of H, OH, $NH_2$, methyl and CHzOH;
$R_3$ is selected from the group consisting of H, OH, $NH_2$, methyl and CHzOH; optionally, $R_1$ and $R_2$ are directly linked to form a ring;
B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and CHzOH;
$R_2$ is selected from the group consisting of H, methyl and CHzOH;
$R_3$ is selected from the group consisting of H, OH, $NH_2$, methyl and CHzOH;
optionally, $R_1$ and $R_2$ are directly linked to form a ring;
B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some other embodiments, B is selected from the group consisting of adenine, guanine, cytosine, uracil and thymine.

In some embodiments, the compound described above includes, but is not limited to:

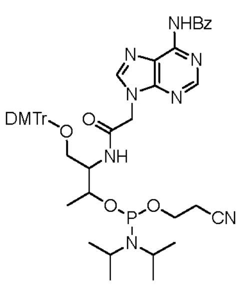,
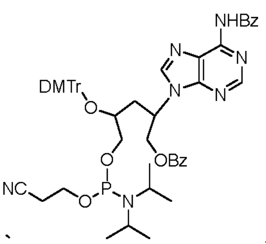,
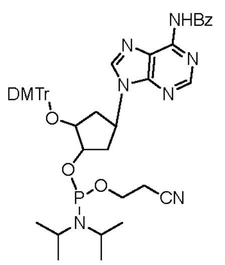,
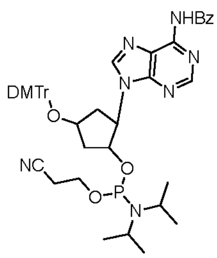,

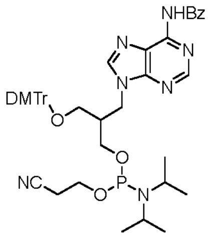

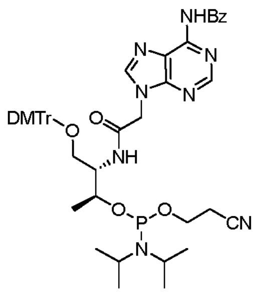,
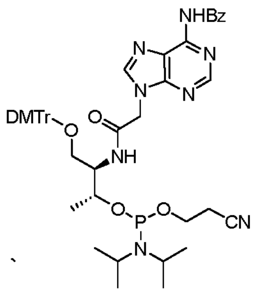,
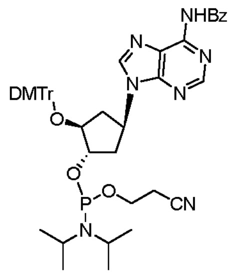,
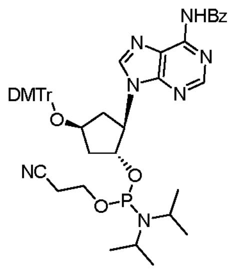,

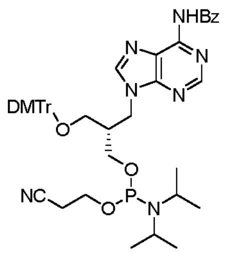,
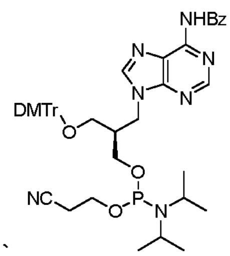,
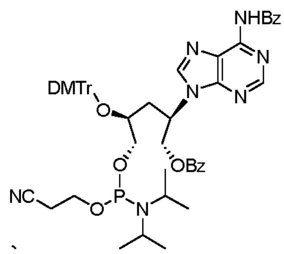

, and those compounds where adenine is replaced with guanine, cytosine, uracil or thymine.

The present disclosure also provides a compound of formula (III) or a tautomer thereof,

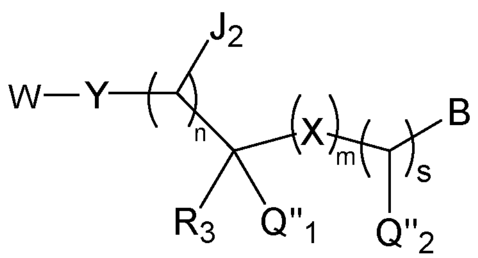

(III)

wherein Y is selected from the group consisting of O, NH and S;

each X is independently selected from the group consisting of $CR_4(R_4')$, S, $NR_5$ and NH-CO, wherein $R_4$, $R_4'$ and $R_5$ are each independently H or $C_1$-$C_6$ alkyl;

$J_2$ is H or $C_1$-$C_6$ alkyl;

n = 0, 1 or 2; m = 0, 1 or 2; s = 0 or 1;

$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;

$Q''_1$ is

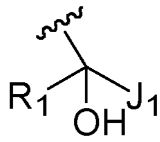

and $Q_2$ is $R_2$; or $Q_1$ is $R_2$, and $Q''_2$ is

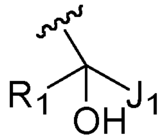

wherein $R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;

$J_1$ is H or $C_1$-$C_6$ alkyl;

$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;

optionally, $R_1$ and $R_2$ are directly linked to form a ring;

B is a base or a base analog; and

W is a leaving group.

In some embodiments, W is MMTr or DMTr.

In some embodiments, the compound described above is not

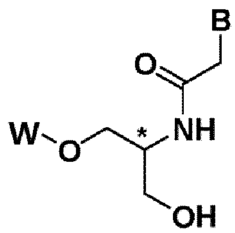

wherein W and B are as defined above.

In some embodiments, when X is NH-CO, $R_1$ is not H.

In some embodiments, the compound described above is not one or more of the following compounds:

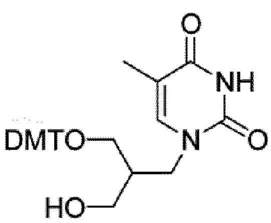
,
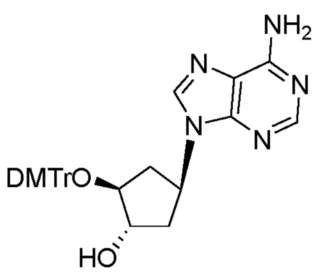
,
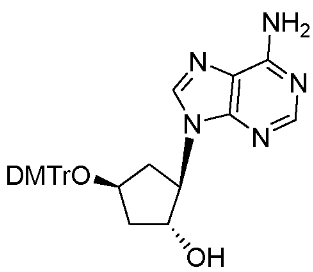

and

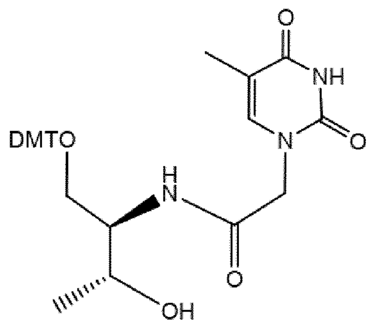

.

In some embodiments, the compound of formula (III) or the tautomer thereof is specifically a compound of formula (III-1) or a tautomer thereof,

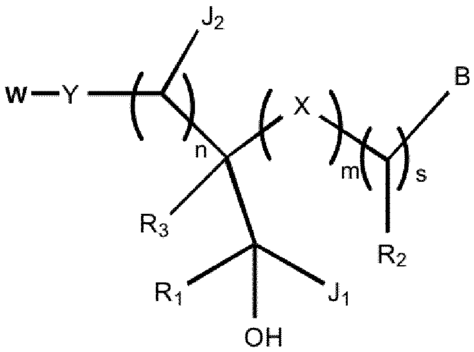

(III-1)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
each $J_1$ and each $J_2$ is independently H or $C_1$-$C_6$ alkyl;
n = 0, 1 or 2; m = 0, 1 or 2; s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is a base or a base analog; and
W is a leaving group.

In some embodiments, W is MMTr or DMTr.

In some embodiments, the compound described above is not

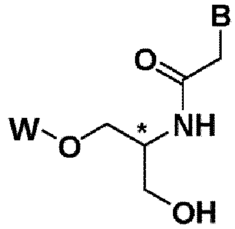

,

wherein W and B are as defined above.

In some embodiments, when X is NH-CO, $R_1$ is not H.

In some embodiments, the compound of formula (III) is not one or more of the following compounds:

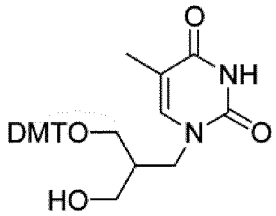
,
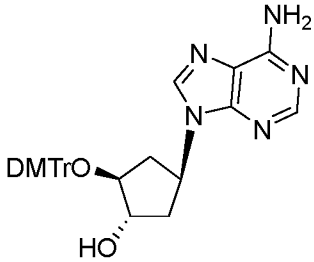
,
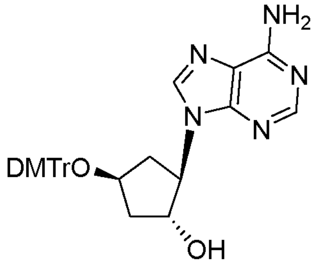

and

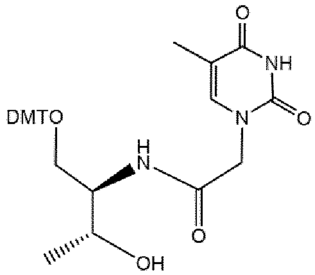
.

In some embodiments, the compound of formula (III) or the tautomer thereof is specifically a compound of formula (III-2) or a tautomer thereof,

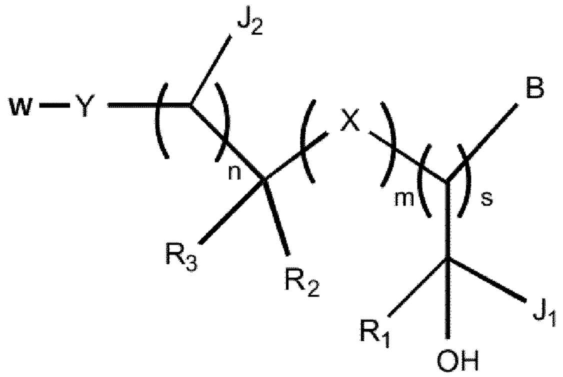

(III-2)

wherein Y is selected from the group consisting of O, NH and S;

each X is independently selected from the group consisting of $CR_4(R_4')$, S, $NR_5$ and NH-CO, wherein $R_4$, $R_4'$ and $R_5$ are each independently H or $C_1$-$C_6$ alkyl;

n = 0, 1 or 2; m = 0, 1 or 2; s = 0 or 1;

each $J_1$ and each $J_2$ is independently H or $C_1$-$C_6$ alkyl;

$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;

$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-

alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is a base or a base analog; and
W is a leaving group.

In some embodiments, W is MMTr or DMTr.

In some embodiments, the compound described above is not

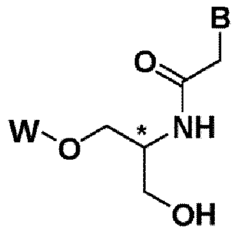
,

wherein W and B are as defined above.

In some embodiments, when X is NH-CO, $R_1$ is not H.

In some embodiments, the compound described above is not one or more of the following compounds:

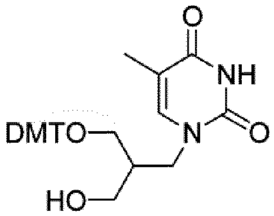
,
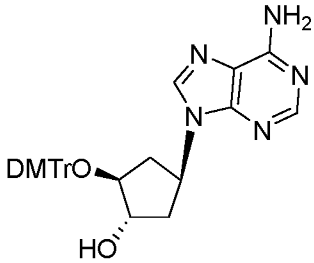
,
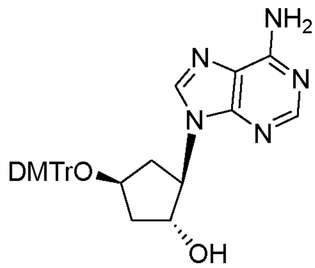

and

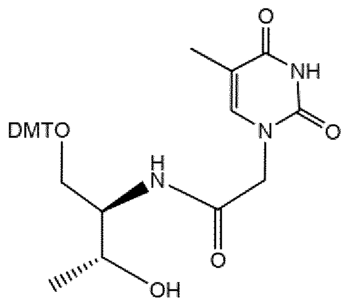
.

In some embodiments, each X is independently selected from the group consisting of $CR_4(R_4')$, S, $NR_5$ and NH-CO, wherein $R_4$, $R_4'$ and $R_5$ are each independently H or $C_1$-$C_3$ alkyl;

n = 0, 1 or 2; m = 0, 1 or 2; s = 0 or 1;
each $J_1$ and each $J_2$ is independently H or $C_1$-$C_3$ alkyl;
$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, $S$-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_3$ alkyl, $C_1$-$C_3$ alkoxy, $C_2$-$C_4$ alkenyl, $C_2$-$C_4$ alkynyl and $(CH_2)_qR_7$, wherein $R_7$ is selected from the group consisting of OH, halogen, methoxy, ethoxy, $N_3$, $C_2$-$C_4$ alkenyl and $C_2$-$C_4$ alkynyl, and q = 1, 2 or 3;
$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$

alkynyl, $S\text{-}CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_4$ alkenyl and $C_2$-$C_4$ alkynyl, and r = 1, 2 or 3;
optionally, $R_1$ and $R_2$ are directly linked to form a ring;
B is selected from the group consisting of purine bases, pyrimidine bases, indole, 5-nitroindole and 3-nitropyrrole.

In some embodiments, each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;

n = 0, 1 or 2; m = 0, 1 or 2; s = 0 or 1;
each $J_1$ and each $J_2$ is independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring;
B is selected from the group consisting of purine, adenine, guanine, isoguanine, hypoxanthine, xanthine, C2-modified purine, N8-modified purine, 2,6-diaminopurine, 6-dimethylaminopurine, 2-aminopurine, N6-alkyladenine, O6-alkylguanine, 7-deazapurine, cytosine, 5-methylcytosine, isocytosine, pseudocytosine, uracil, pseudouracil, 2-thiouridine, 4-thiouridine, C5-modified pyrimidine, thymine, indole, 5-nitroindole and 3-nitropyrrole.

In some embodiments, each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;

n = 0, 1 or 2; m = 0, 1 or 2; s = 0 or 1;
each $J_1$ and each $J_2$ is independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, $S\text{-}CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring;
B is selected from the group consisting of purine, adenine, guanine, isoguanine, hypoxanthine, xanthine, 2,6-diaminopurine, 6-dimethylaminopurine, 2-aminopurine, 7-deazapurine, cytosine, 5-methylcytosine, isocytosine, pseudocytosine, uracil, pseudouracil, 2-thiouridine, 4-thiouridine, thymine, indole, 5-nitroindole and 3-nitropyrrole.

In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and CHzOH;
$R_2$ is selected from the group consisting of H, OH, $NH_2$, methyl and CHzOH;
$R_3$ is selected from the group consisting of H, OH, $NH_2$, methyl and CHzOH;
optionally, $R_1$ and $R_2$ are directly linked to form a ring;
B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In some embodiments, Y is O or NH; each X is independently selected from the group consisting of NH-CO, $CH_2$ and NH;

n = 0 or1; m = 0 or1; s = 0 or 1;
each $J_1$ and each $J_2$ is independently H;
$R_1$ is selected from the group consisting of H, methyl and CHzOH;
$R_2$ is selected from the group consisting of H, methyl and CHzOH;
$R_3$ is selected from the group consisting of H, OH, $NH_2$, methyl and CHzOH;
optionally, $R_1$ and $R_2$ are directly linked to form a ring;
B is selected from the group consisting of purine, adenine, guanine, 2,6-diaminopurine, 6-dimethylaminopurine, 2-aminopurine, cytosine, uracil, thymine, indole, 5-nitroindole and 3-nitropyrrole.

In other embodiments, B is selected from the group consisting of adenine, guanine, cytosine, uracil and thymine.

In some embodiments, the compound described above includes, but is not limited to:

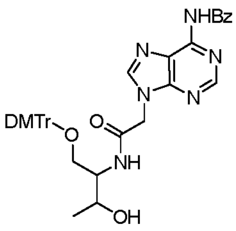
,
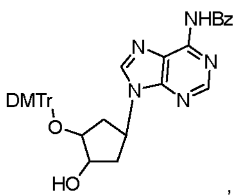
,
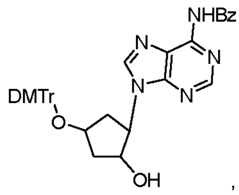
,
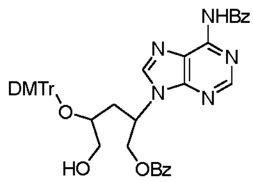

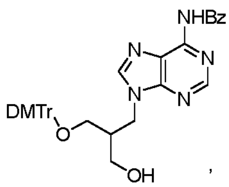
,

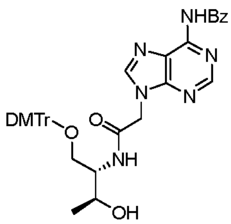
,
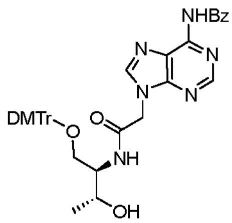
,
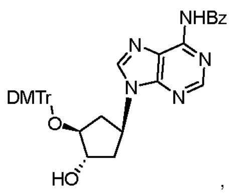
,
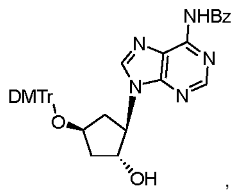
,

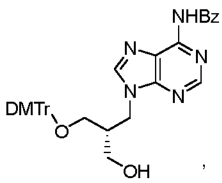
,
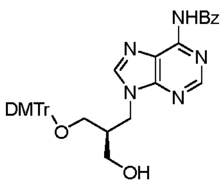
,
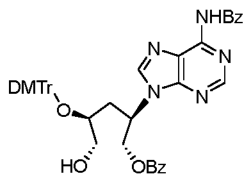
,

and those compounds where adenine is replaced with guanine, cytosine, uracil or thymine, and

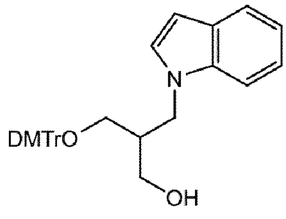
,
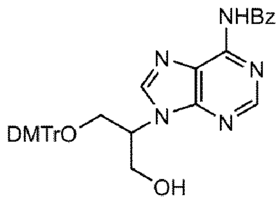
,
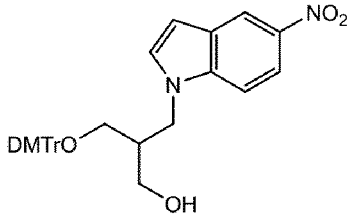
,

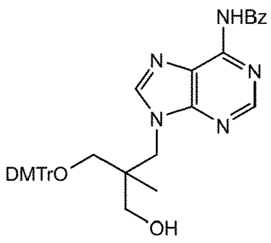
,
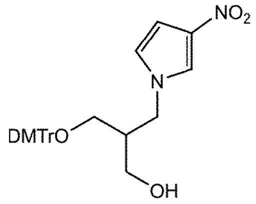
,
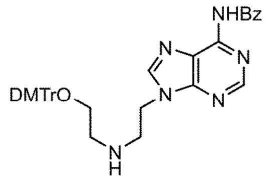
,

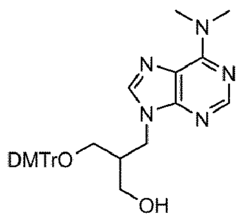
,
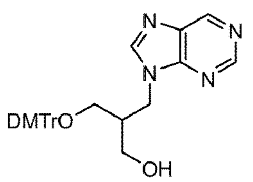
,
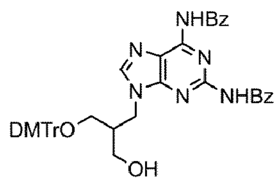
,

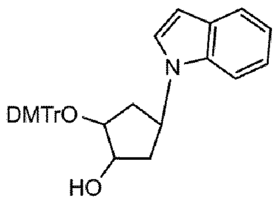
,
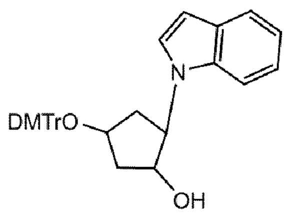
,
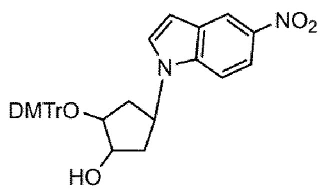
,

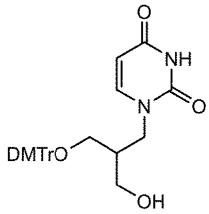
,
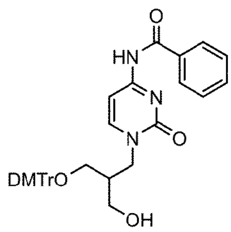
,
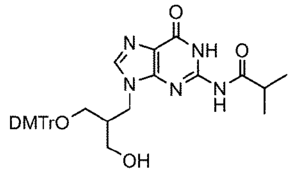
,

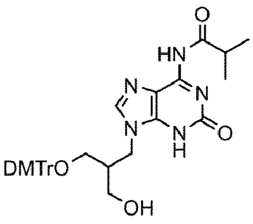
,
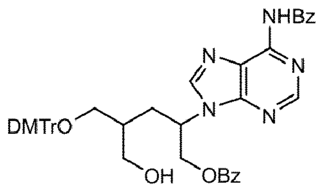
,
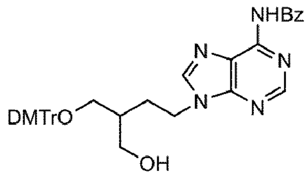
,
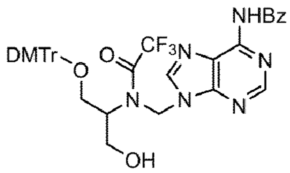
,
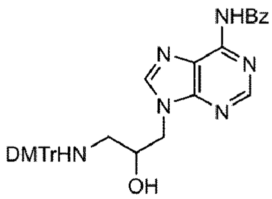
,
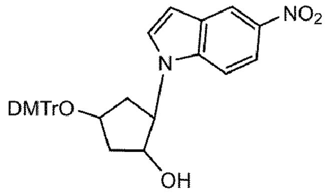

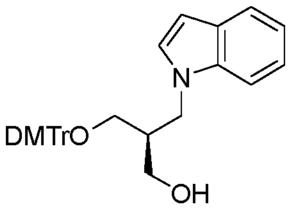
,
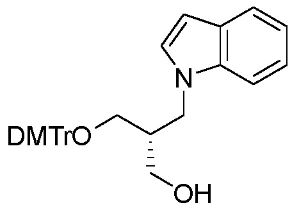
,
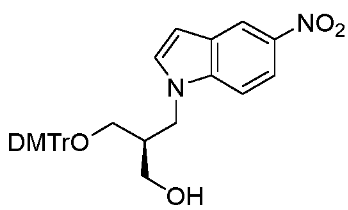
,
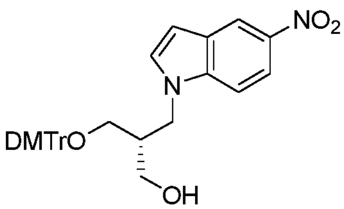
,
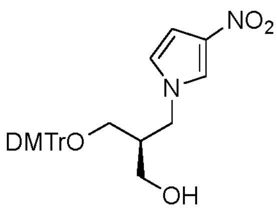
,
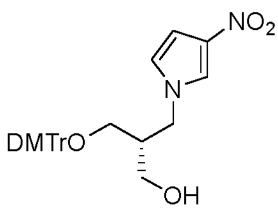
,
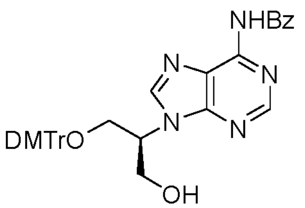
,
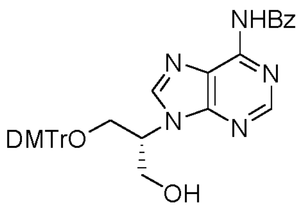
,
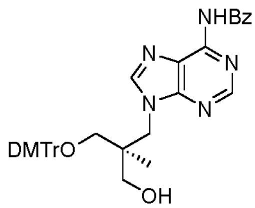
,
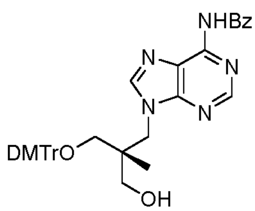
,
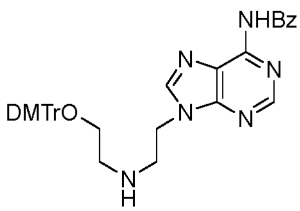
,
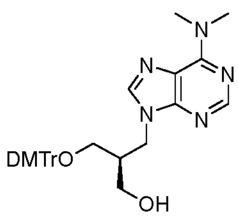
,
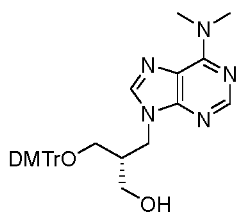
,

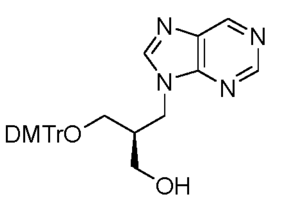 , 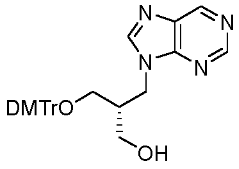 , 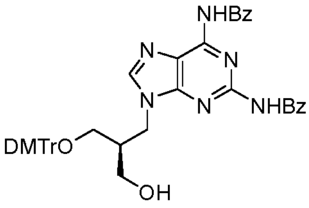 ,

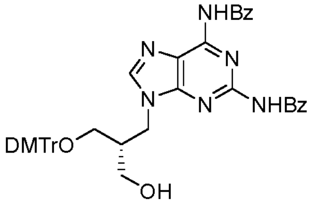 , 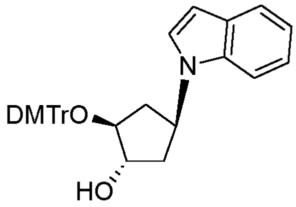 , 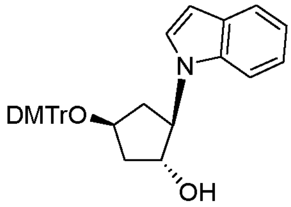 ,

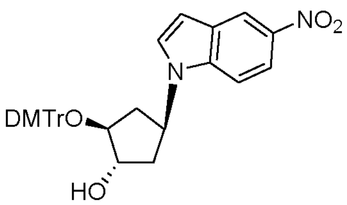 , 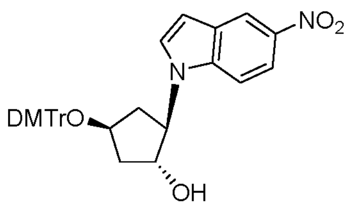 , 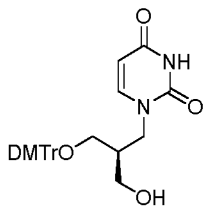 ,

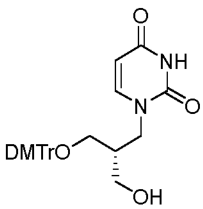 , 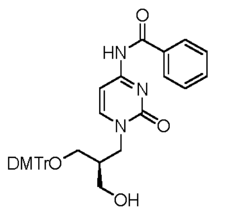 , 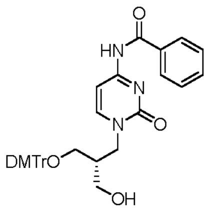 , 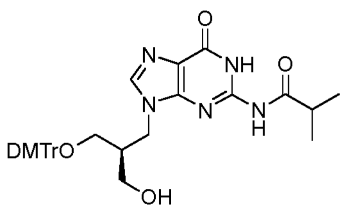 ,

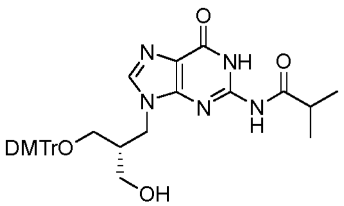 , 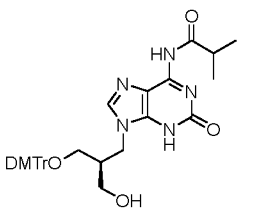 , 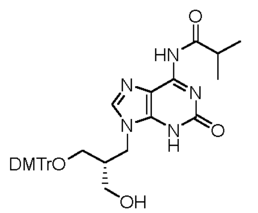 ,

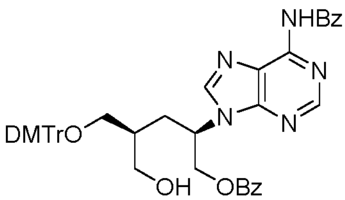 , 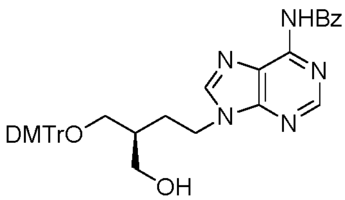 , 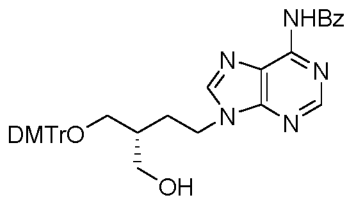 ,

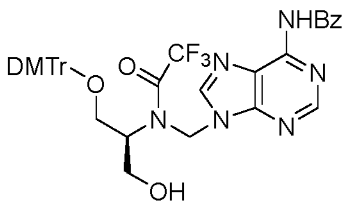 , 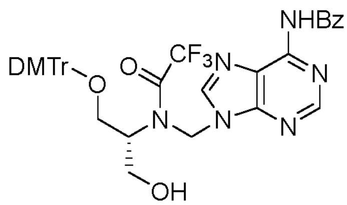 ,

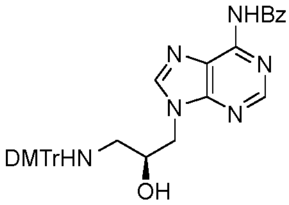 , 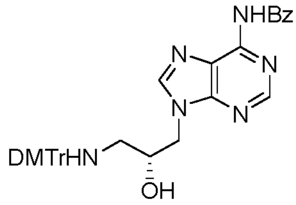 ,

and those compounds where bases or base analogs are replaced with purine, adenine, guanine, 2,6-diaminopurine, 6-dimethylaminopurine, 2-aminopurine, cytosine, uracil, thymine, indole, 5-nitroindole or 3-nitropyrrole.

The present disclosure also provides an siRNA or an siRNA conjugate, wherein the chemical modification of formula (I) or the tautomer modification thereof in the antisense strand of any siRNA or siRNA conjugate of the present disclosure is replaced with a 2'-methoxy modification.

The present disclosure also provides an siRNA or an siRNA conjugate, wherein the chemical modification of formula (I) comprised in the antisense strand of any siRNA or siRNA conjugate of the present disclosure is a 2'-methoxy modification. The present disclosure also provides an siRNA or an siRNA conjugate, wherein the antisense strand comprises a modification in at least one of nucleotide positions 2 to 8 of the 5' region thereof, and the modification is a chemical modification of formula (I) or a tautomer modification.

The present disclosure also provides an siRNA or an siRNA conjugate, wherein one or more bases U, e.g., 1, 2, 3, 3, 5, 6, 7, 8, 9 or 10 bases U, of any siRNA or siRNA conjugate of the present disclosure are replaced with bases T.

The present disclosure also provides a method for preparing the aforementioned siRNA or siRNA conjugate, which comprises the following steps: (1) synthesizing a compound of formula (II) or a tautomer thereof; and (2) synthesizing the siRNA or siRNA conjugate using the compound or the tautomer thereof of step (1).

In some embodiments, the compound of formula (II) or the tautomer thereof is synthesized using a compound of formula (III) or a tautomer thereof.

The present disclosure also provides use of the compound of formula (II) or the tautomer thereof described above in inhibiting or reducing the off-target activity of an siRNA.

The present disclosure also provides use of the compound of formula (II) or the tautomer thereof described above in preparing an siRNA.

## Terms

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. Where the configuration is not specified, the compounds of the present disclosure can be present in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure.

The compounds and intermediates of the present disclosure may also exist in different tautomeric forms, and all such forms are included within the scope of the present disclosure.

The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

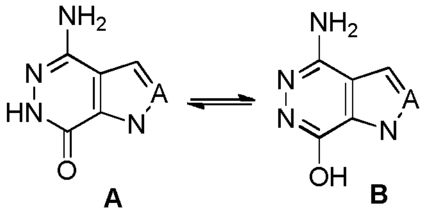

A B .

All compounds in the present disclosure can be drawn as form A or form B. All tautomeric forms are within the

scope of the present disclosure. The nomenclature of the compounds does not exclude any tautomers.

The compounds of the present disclosure may be asymmetric; for example, the compounds have one or more stereoisomers. Where the configuration is not specified, all stereoisomers include, for example, enantiomers and diastereomers. The compounds of the present disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers, and D- and L-isomers can be prepared by chiral synthesis, chiral reagents or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. Furthermore, separation of enantiomers and diastereomers is typically accomplished by chromatography using a chiral stationary phase, optionally in combination with chemical derivatization (e.g., carbamate formation from amines).

The present disclosure also comprises isotopically-labeled compounds which are identical to those recited herein but have one or more atoms replaced by an atom having an atomic mass or mass number different from the atomic mass or mass number usually found in nature. Examples of isotopes that can be incorporated into the compound of the present disclosure include isotopes of hydrogen, carbon, nitrogen, oxygen, phosphorus, sulfur, fluorine, iodine, and chlorine, such as $^{2}H$ , $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$.

Unless otherwise stated, when a position is specifically designated as deuterium (D), that position shall be understood to be deuterium having an abundance that is at least 1000 times greater than the natural abundance of deuterium (which is 0.015%) (i.e., incorporating at least 10% deuterium). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also comprises various deuterated forms of the compound of formula I. Each available hydrogen atom connected to a carbon atom may be independently replaced with a deuterium atom. Those skilled in the art are able to synthesize the deuterated forms of the compound of general formula I with reference to the relevant literature. Commercially available deuterated starting materials can be used in preparing the deuterated forms of the compound of formula I, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

The term "optionally" or "optional" means that the event or circumstance subsequently described may, but not necessarily, occur, and that the description includes instances where the event or circumstance occurs or does not occur. For example, "$C_{1-6}$ alkyl optionally substituted with halogen or cyano" means that halogen or cyano may, but not necessarily, be present, and the description includes the instance where alkyl is substituted with halogen or cyano and the instance where alkyl is not substituted with halogen and cyano.

In the chemical structure of the compound of the present disclosure, a bond "/""" represents an unspecified configuration, namely if chiral isomers exist in the chemical structure, the bond "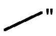" may be "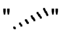" or "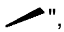", or contains both the configurations of "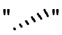" and "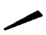". Although all of the above structural formulae are drawn as certain isomeric forms for the sake of simplicity, the present disclosure may include all isomers, such as tautomers, rotamers, geometric isomers, diastereomers, racemates and enantiomers. In the chemical structure of the compound of the present disclosure, a bond "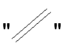" does not specify a configuration-that is, the configuration for the bond "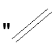" can be an E configuration or a Z configuration, or includes both the E configuration and the Z configuration.

Unless otherwise specified, "chemical modification", "compound", "ligand", "conjugate" and "nucleic acid" of the present disclosure can each independently exist in the form of a salt, mixed salts, or a non-salt (e.g., a free acid or free base). When existing in the form of a salt or mixed salts, it can be a pharmaceutically acceptable salt. The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic acid or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, methanesulfonates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts and magnesium salts; sodium salts are preferred. Salts derived from organic bases include, but are not limited to, salts of the following: primary, secondary and tertiary amines, substituted amines including naturally occurring substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

As used herein, in the context of RNA-mediated gene silencing, the sense strand (also referred to as SS or SS strand) of an siRNA refers to a strand that comprises a sequence that is identical or substantially identical to a target mRNA sequence; the antisense strand (also referred to as AS or AS strand) of an siRNA refers to a strand having a sequence complementary to a target mRNA sequence.

As used herein, the terms "complementary" and "reverse complementary" are used interchangeably and have the meaning well known to those skilled in the art-that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (C) is always paired with the pyrimidine base cytosine (G). Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases in the corresponding positions are not paired in a complementary manner.

The term "base" encompasses any known DNA and RNA bases and base analogs such as purines or pyrimidines, which also include the natural compounds adenine, thymine, guanine, cytosine, uracil, inosine and natural analogs.

In the present disclosure, base analogs are typically purine or pyrimidine bases, excluding the common bases: guanine (G), cytosine (C), adenine (A), thymine (T) and uracil (U). Non-limiting examples of bases include hypoxanthine (I), xanthine (X), 3β-D-ribofuranosyl-(2,6-diaminopyrimidine) (K), 3-β-D-ribofuranosyl-(1-methyl-pyrazolo[4,3-d]pyrimidine-5,7(4H,6H)-dione) (P), isocytosine (iso-C), isoguanine (iso-G), 1-β-D-ribofuranosyl-(5-nitroindole), 1-β-D-ribofuranosyl-(3-nitropyrrole), 5-bromouracil, 2-aminopurine, 4-thio-dT, 7-(2-thienyl)-imidazo[4,5-b]pyridine (Ds) and pyrrole-2-carbaldehyde (Pa), 2-amino-6-(2-thienyl)purine (S), 2-oxopyridine (Y), difluorotolyl, 4-fluoro-6-methylbenzimidazole, 4-methylbenzimidazole, 3-methylhydroxyisoquinolyl, 5-methylhydroxyisoquinolyl and 3-methyl-7-propynyl hydroxyisoquinolyl, 7-azaindolyl, 6-methyl-7-azaindolyl, imidazopyridinyl, 9-methyl-imidazopyridinyl, pyrrolopyrazinyl, hydroxyisoquinolyl, 7-propynyl hydroxyisoquinolyl, propynyl-7-azaindolyl, 2,4,5-trimethylphenyl, 4-methylindolyl, 4,6-dimethylindolyl, phenyl, naphthyl, anthracenyl, phenanthrenyl, pyrenyl, stilbenzyl, tetracenyl, pentacenyl and structural derivatives thereof. Base analogs can also be universal bases.

As used herein, "universal base" refers to a heterocyclic moiety located in the 1' position of a nucleotide sugar moiety in a modified nucleotide, or the equivalent position in a nucleotide sugar moiety substitution, and the heterocyclic moiety, when present in a nucleic acid duplex, can be positioned opposite more than one type of base without altering the double helical structure (e.g., the structure of the phosphate backbone). In addition, the universal base does not destroy the ability of the single-stranded nucleic acid in which it resides to form a duplex with a target nucleic acid. The ability of a single-stranded nucleic acid containing a universal base to form a duplex with a target nucleic can be determined using methods apparent to those skilled in the art (e.g., UV absorbance, circular dichroism, gel shift, and single-stranded nuclease sensitivity). In addition, conditions under which duplex formation is observed can be changed to determine duplex stability or formation, e.g., temperature, such as melting temperature (Tm), related to the stability of nucleic acid duplexes. Compared to a reference single-stranded nucleic acid that is exactly complementary to a target nucleic acid,

the single-stranded nucleic acid containing a universal base forms a duplex with the target nucleic acid that has a lower Tm than a duplex formed with the complementary nucleic acid. However, compared to a reference single-stranded nucleic acid in which the universal base has been replaced with a base to generate a single mismatch, the single-stranded nucleic acid containing the universal base forms a duplex with the target nucleic acid that has a higher Tm than a duplex formed with the nucleic acid having the mismatched base.

Some universal bases are capable of base pairing by forming hydrogen bonds between the universal base and all of the bases guanine (G), cytosine (C), adenine (A), thymine (T) and uracil (U) under base pairing conditions. A universal base is not a base that forms a base pair with only one single complementary base. In a duplex, a universal base can form no hydrogen bond, one hydrogen bond, or more than one hydrogen bond with each of G, C, A, T and U opposite to it on the opposite strand of the duplex. Preferably, the universal base does not interact with the base opposite to it on the opposite strand of the duplex. In a duplex, base pairing with a universal base will not alter the double helical structure of the phosphate backbone. A universal base may also interact with bases in adjacent nucleotides on the same nucleic acid strand by stacking interactions. Such stacking interactions can stabilize the duplex, particularly in cases where the universal base does not form any hydrogen bond with the base positioned opposite to it on the opposite strand of the duplex. Non-limiting examples of universal binding nucleotides include inosine, 1-β-D-ribofuranosyl-5-nitroindole, and/or 1-β-D-ribofuranosyl-3-nitropyrrole.

As used herein, "chemical modification" or "modification" includes all changes made to a nucleotide by chemical means, such as the addition or removal of a chemical moiety, or the substitution of one chemical moiety for another.

In the chemical structural formulas of the present disclosure, the wavy lines "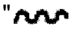" represent linking sites, and asterisks "*" indicate chiral centers.

In the context of the present disclosure, the

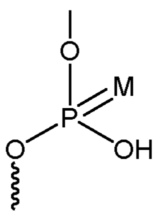

moiety in the group

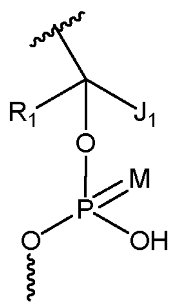

can be replaced with any group capable of linking to an adjacent nucleotide.

The term "alkyl" refers to a saturated aliphatic hydrocarbyl group which is a linear or branched chain group containing 1 to 20 carbon atoms, for example, an alkyl group containing 1 to 12 carbon atoms, or an alkyl group containing 1 to 6 carbon atoms. Non-limiting examples of alkyl include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, or 2,3-dimethylbutyl.

The term "alkoxy" refers to -O-alkyl, wherein the alkyl is as defined above. Non-limiting examples of alkoxy include, but are not limited to, methoxy, ethoxy, propoxy, butoxy, cyclopropyloxy, cyclobutoxy, cyclopentyloxy and cyclohexyloxy. $C_1$-$C_6$ alkoxy may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

The term "alkenyl" refers to a hydrocarbyl group containing at least one double bond. Non-limiting examples

of alkenyl include, but are not limited to, ethenyl, 1-propenyl, 2-propenyl, 1-butenyl or 2-butenyl and various branched chain isomers thereof.

The term "alkynyl" refers to a hydrocarbyl group containing at least one triple bond. Non-limiting examples of alkynyl include, but are not limited to, ethynyl, 1-propynyl, 2-propynyl, 1-butynyl or 2-butynyl and various branched chain isomers thereof.

The term "halogen" refers to fluorine, chlorine, bromine or iodine.

In the present disclosure, the "ring" in "Ri and $R_2$ are directly linked to form a ring" can be "cycloalkyl" or "heterocycloalkyl".

The term "cycloalkyl" can be referred to as "carbocycle", and refers to a saturated or partially unsaturated monocyclic or polycyclic cyclohydrocarbon substituent. The cycloalkyl ring contains 3 to 20 carbon atoms, in some embodiments 3 to 7 carbon atoms, and in some embodiments 5 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, etc. Polycyclic cycloalkyl includes spiro cycloalkyl, fused cycloalkyl, and bridged cycloalkyl. Cycloalkyl may be substituted or unsubstituted, and when it is substituted, the substituent can be substituted at any available linking site; in some embodiments, the substituent is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano.

The cycloalkyl ring may be fused to an aryl or heteroaryl ring, wherein the ring attached to the parent structure is cycloalkyl. Non-limiting examples of cycloalkyl ring include indanyl, tetrahydronaphthyl, benzocycloheptyl, etc. Cycloalkyl may be optionally substituted or unsubstituted, and when it is substituted, the substituent, in some embodiments, is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano.

The term "heterocycloalkyl", also known as "heterocycle" or "heterocyclyl", refers to a saturated or partially unsaturated monocyclic or polycyclic cyclohydrocarbon substituent containing 3 to 20 ring atoms, wherein one or more of the ring atoms are heteroatoms selected from the group consisting of nitrogen, oxygen and $S(O)_m$ (where m is an integer from 0 to 2), excluding a cyclic moiety of -O-O-, -O-S- or -S-S-, and the remaining ring atoms are carbon atoms. In some embodiments, heterocycloalkyl contains 3 to 12 ring atoms, 1-4 of which are heteroatoms; in some embodiments, heterocycloalkyl contains 3 to 7 ring atoms. Non-limiting examples of monocyclic heterocycloalkyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, etc. The polycyclic heterocycloalkyl includes spiro heterocyclyl, fused heterocyclyl, and bridged heterocycloalkyl. Non-limiting examples of "heterocycloalkyl" include:

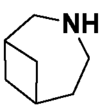 , 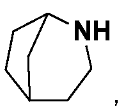 , 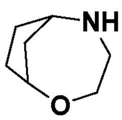 , 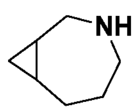 , 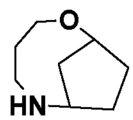 , 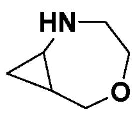 ,

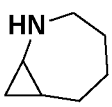 , 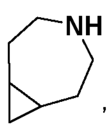 , 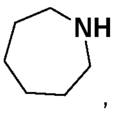 , 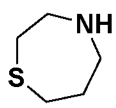 , 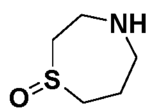 , 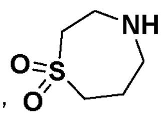 ,

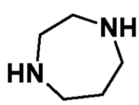 , 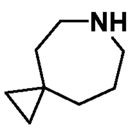 , 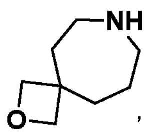 , 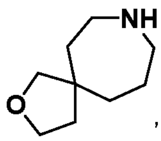 , 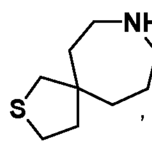 , 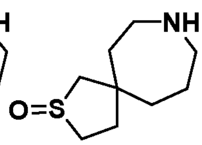 ,

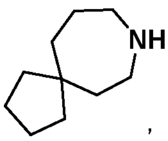, 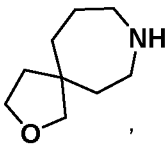, 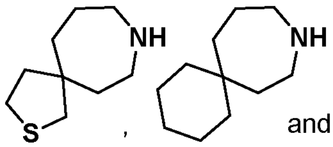, and 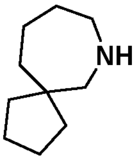,

etc.

The heterocycloalkyl ring may be fused to an aryl or heteroaryl ring, wherein the ring attached to the parent structure is heterocycloalkyl. Non-limiting examples of the heterocycloalkyl ring include, but are not limited to:

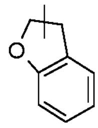 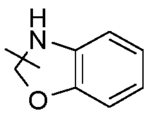 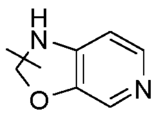 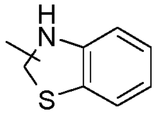,

etc.

Heterocycloalkyl may be optionally substituted or unsubstituted, and when it is substituted, the substituent, in some embodiments, is selected from the group consisting of one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano.

In the context of the present disclosure, Bz represents a benzoyl protecting group; MMTr represents methoxyphenyl benzhydryl; DMTr represents a dimethoxytrityl protecting group.

Unless otherwise stated, in the context of the present disclosure, the uppercase letters C, G, U, A and T represent base components of a nucleotide; the lowercase letter d indicates that the right nucleotide adjacent to the letter d is a deoxyribonucleotide; the lowercase letter m indicates that the left nucleotide adjacent to the letter m is a methoxy-modified nucleotide; the lowercase letter f indicates that the left nucleotide adjacent to the letter f is a fluoro-modified nucleotide; the lowercase letter s indicates that the two nucleotides adjacent to the letter s is linked by a phosphorothioate group. As used herein, the term "fluoro-modified nucleotide" refers to a nucleotide in which the hydroxy group in the 2' position of the ribosyl group of the nucleotide is substituted with fluorine, and "non-fluoro-modified nucleotide" refers to a nucleotide or a nucleotide analog in which the hydroxy group at the 2' position of the ribosyl group of the nucleotide is substituted with a non-fluorine group. "Nucleotide analog" refers to a group that can replace a nucleotide in a nucleic acid but has a structure different from adenine ribonucleotide, guanine ribonucleotide, cytosine ribonucleotide, uracil ribonucleotide, or thymine deoxyribonucleotide, e.g., an isonucleotide, a bridged nucleic acid (BNA for short) or an acyclic nucleotide. The methoxy-modified nucleotide refers to a nucleotide in which the 2'-hydroxy group of the ribosyl group is substituted with a methoxy group. An isonucleotide refers to a compound formed by changing the position of a base on the ribose ring in a nucleotide. In some embodiments, the isonucleotide can be a compound formed by moving a base from the 1'-position to the 2'-position or 3'-position of the ribose ring. BNA refers to a constrained or inaccessible nucleotide. BNA may contain five-membered, six-membered, or seven-membered ring bridged structure with a "fixed" C3'-endo sugar puckering. The bridge is typically incorporated at the 2'-, 4'-position of the ribose to afford a 2',4'-BNA nucleotide. In some embodiments, BNA may be LNA, ENA, cET BNA, etc. Acyclic nucleotides are a class of nucleotides in which the sugar ring of the nucleotide is opened. In some embodiments, the acyclic nucleotide can be an unlocked nucleic acid (UNA) or a glycerol nucleic acid (GNA).

As used herein, the term "inhibit" is used interchangeably with "decrease", "silence", "down-regulate", "repress" and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from a similar untreated or control (e.g., buffer only control or inert agent control) treated subject, cell, or sample. For example, the remaining expression level of mRNA can be used to characterize the degree of inhibition of target gene expression by the siRNA; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition of target gene expression can be measured using Dual-Glo® Luciferase Assay System: the Firefly chemiluminescence value and the Renilla chemiluminescence value are each read, and the relative value Ratio = Ren/Fir and inhibition (%) = 1-(Ratio + siRNA/Ratioreporter only) × 100% are calculated; in the present disclosure, the proportion of remaining expression of mRNA (or remaining activity%) = 100% - inhibition (%).

"Effective amount" or "effective dose" refers to the amount of a drug, a compound or a pharmaceutical composition necessary to obtain any one or more beneficial or desired therapeutic results. For preventive use, the beneficial or desired results include elimination or reduction of risk, reduction of severity or delay of the onset of a condition, including the biochemistry, histology and/or behavioral symptoms of the condition, complications thereof and intermediate pathological phenotypes that appear during the progression of the condition. For therapeutic applications, the beneficial or desired results include clinical results, such as reducing the incidence of various conditions related to the target gene, target mRNA or target protein of the present disclosure or alleviating one or more symptoms of the condition, reducing the dosage of other agents required to treat the condition, enhancing the therapeutic effect of another agent, and/or delaying the progression of conditions related to the target gene, target mRNA or target protein of the present disclosure in the patient.

As used herein, the term "angiopoietin-like protein-3" (also known as "ANGPTL3" or "ANGPTL3") can refer to any nucleic acid or protein of ANGPTL3. The sequence of human ANGPTL3 is under accession number NP_055310. "ANGPTL3 expression" refers to the level of mRNA transcribed from a gene encoding ANGPTL3 or the level of protein translated from the mRNA.

As used herein, the term "transthyretin" ("TTR"), also known as ATTR, HsT2651, PALB, prealbumin, TBPA and transthyretin (prealbumin, amyloidosis type I), can refer to any nucleic acid or protein of TTR. The sequence of the mRNA transcript of human TTR is under accession number NM_000371. "TTR expression" refers to the level of mRNA transcribed from a gene encoding TTR or the level of protein translated from the mRNA.

"Pharmaceutical composition" comprises the siRNA or siRNA conjugate of the present disclosure and a pharmaceutically acceptable auxiliary material and/or adjuvant; the auxiliary material can be one or more of various formulations or compounds conventionally used in the art. For example, the pharmaceutically acceptable auxiliary material can include at least one of a pH buffer, a protective agent, and an osmotic pressure regulator.

As used herein, "patient", "subject" and "individual" are used interchangeably and include human or non-human animals, e.g., mammals, e.g., humans or monkeys. Various delivery systems are known and can be used for the siRNA or siRNA conjugate of the present disclosure, e.g., encapsulation in liposomes, microparticles, microcapsules, recombinant cells capable of expressing the compound, receptor-mediated endocytosis, and construction of a nucleic acid as part of a retroviral or other vectors.

The siRNA provided by the present disclosure can be obtained using a preparation method conventional in the art (e.g., solid-phase synthesis and liquid-phase synthesis). Solid phase synthesis has been commercially available as customization service. A modified nucleotide group can be introduced into the siRNA of the present disclosure using a nucleoside monomer with a corresponding modification. Methods of preparing a nucleoside monomer with a corresponding modification and introducing a modified nucleotide group into an siRNA are also well known to those skilled in the art.

## BRIEF DESCRIPTION OF THE DRAWINGS

FIGs. 1A to 1L show the experimental results of the off-target activity of siRNA comprising different test compounds.

FIGs. 2A to 2G show the experimental results of the off-target activity of siRNA2 comprising different test compounds.

FIGs. 3A to 3G show the experimental results of the off-target activity of siRNA3 comprising test compounds.

FIG. 4 shows the inhibitory activity of galactosamine molecule cluster-conjugated siRNAs against the mTTR gene in murine primary hepatocytes.

FIG. 5 shows the *in vivo* inhibitory activity of galactosamine molecule cluster-conjugated siRNAs against the mouse mTTR gene.

FIG. 6 shows the *in vivo* long-term inhibitory activity of galactosamine molecule cluster-conjugated siRNAs against the mouse mTTR gene.

FIG. 7 shows the effect of siRNA agents on the total cholesterol level in Apoc3 transgenic mice.

FIG. 8 shows the effect of siRNA agents on the triglyceride level in Apoc3 transgenic mice.

FIG. 9 shows the effect of siRNA agents on the Apoc3 protein level in Apoc3 transgenic mice.

## DETAILED DESCRIPTION

The present disclosure is further described below with reference to examples, which are not intended to limit the scope of the present disclosure. Experimental procedures without conditions specified in the examples of the present disclosure are generally conducted according to conventional conditions, or according to conditions recommended by

the manufacturers of the starting materials or commercial products. If the source of a reagent is not shown, the reagent is obtained from any molecular biology reagent supplier in quality/purity for molecular biology applications.

**I. Preparation of Chemical Modifications and Activity Evaluation**

**Example 1. Preparation of Chemical Modifications**

**1.1. Synthesis of compound 1-1a and compound 1-1b**

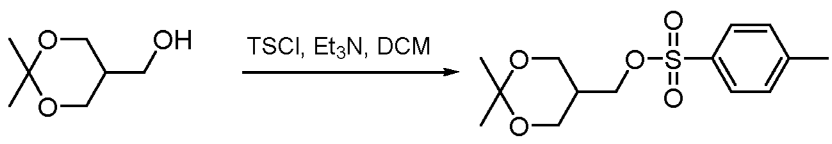


1 2

Compound 1 (500 mg, 3.42 mmol) and triethylamine ($Et_3N$, 692 mg, 6.84 mmol, 0.95 mL) were dissolved in dichloromethane (DCM, 10 mL). A solution of 4-toluenesulfonyl chloride (TsCl, 717 mg, 3.76 mmol) in dichloromethane (10 mL) was added dropwise under ice bath conditions. After the dropwise addition was complete, the reaction mixture was stirred at room temperature overnight. After the reaction was complete, the mixture was quenched with water. The aqueous phase was extracted three times with dichloromethane (15 mL). The organic phases were combined, washed first with saturated aqueous sodium bicarbonate solution (10 mL) and then with saturated brine (20 mL), and then concentrated under reduced pressure to evaporate the solvent to give crude 2 (820 mg, 80%), which was directly used in the next step. MS m/z: $C_{14}H_{21}O_5S$, $[M+H]^+$ calculated: 301.10, found: 301.2.

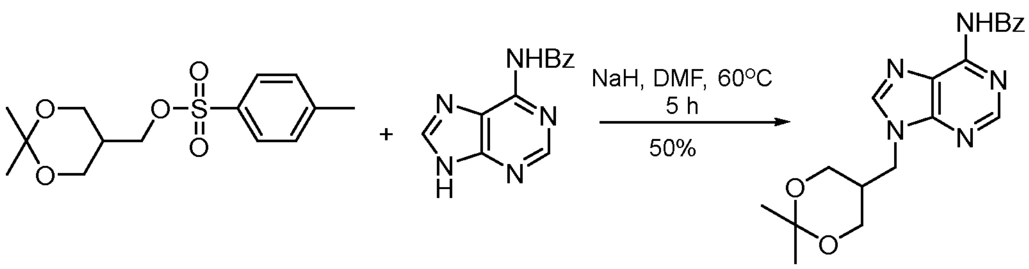


2 3 4

Compound 3 (239 mg, 1.22 mmol) was dissolved in dimethylformamide (DMF, 10 mL). A solution of NaH (60% in mineral oil, 93 mg, 2.33 mmol) was added under ice bath conditions. The reaction mixture was stirred for 30 min, and then compound 2 (350 mg, 1.16 mmol) was added dropwise. After the dropwise addition was complete, the reaction mixture was stirred at 60 °C for 5 h. After the reaction was complete, the mixture was quenched with water. The aqueous phase was extracted with ethyl acetate (15 mL) three times. The combined organic phases were washed first with water (10 mL) three times and then with saturated brine (10 mL), then concentrated under reduced pressure to evaporate the solvent, purified by reversed-phase preparative HPLC ($C^{18}$, conditions: 5-50% (A: HzO, B: $CH_3CN$), flow rate: 70 mL/min), and lyophilized to give compound 4 (220 mg). MS m/z: $C_{19}H_{21}N_5O_3Na$, $[M+Na]^+$ calculated: 390.16, found: 390.3.

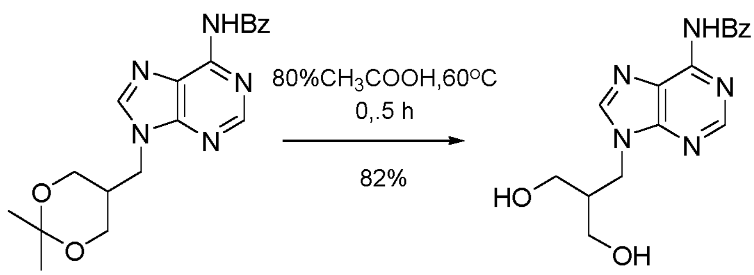


4 5

Compound 4 (1.50 g, 4.08 mmol) was dissolved in 20 mL of a mixed solution of acetic acid and water (4:1) at room temperature. The mixture was stirred at 60 °C for 30 min. After the reaction was complete, the mixture was concentrated under reduced pressure to evaporate the solvent, purified by reversed-phase preparative HPLC ($C^{18}$, conditions: 5-25% (A: HzO, B: $CH_3CN$), flow rate: 70 mL/min), and lyophilized to give compound 5 (1.10 g). MS m/z: $C_{16}H_{18}N_5O_3$, $[M+H]^+$ calculated: 328.13, found: 328.4.

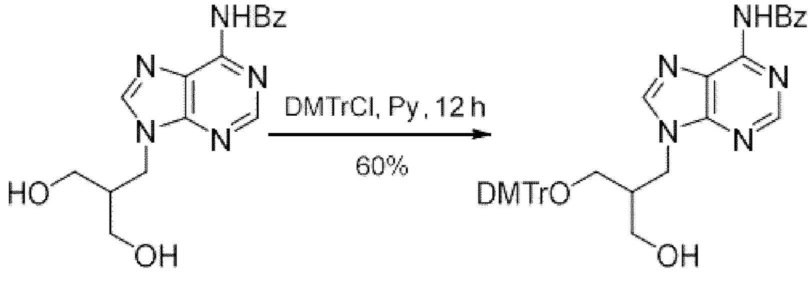


5 6

Compound 5 (1.00 g, 3.05 mmol) was dissolved in pyridine (Py, 10 mL). A solution of 4,4'-dimethoxytrityl chloride (DMTrCl, 1.50 g, 4.58 mmol) in pyridine (5 mL) was added dropwise under ice bath conditions. After the dropwise addition was complete, the reaction mixture was stirred at room temperature overnight. After the reaction was complete, the mixture was quenched with water, concentrated under reduced pressure to evaporate the solvent, purified by reversed-phase preparative HPLC ($C^{18}$, conditions: 5-80% (A: HzO, B: $CH_3CN$), flow rate: 70 mL/min), and lyophilized to give compound 6 (1.00 g). MS m/z: $C_{37}H_{36}N_5O_5$, $[M-H]^+$ calculated: 630.26, found: 630.5. The racemate compound 6 was resolved using a chiral column (Daicel CHIRALPAK® IE 250 × 4.6 mm, 5 μm, A: n-hexane, B: ethanol) into 6A(-) (410 mg) and 6B(+) (435 mg).

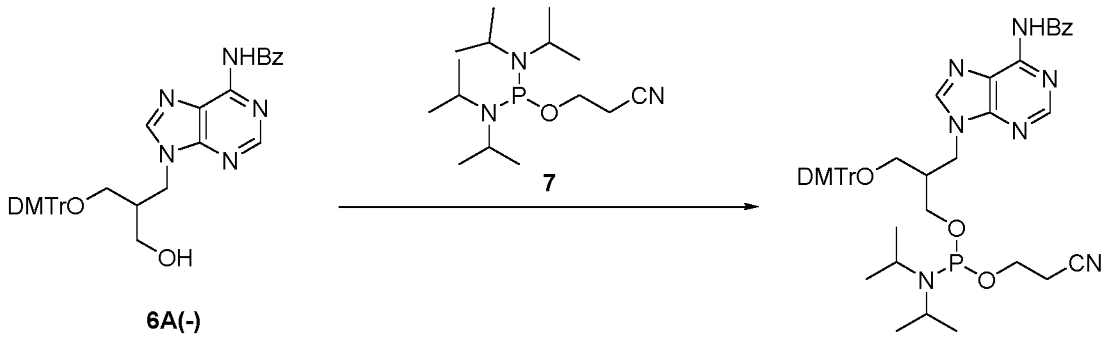

**6A(-)** **7** **1-1a**

Compound **6A(-)** (200 mg, 0.32 mmol), tetrazole (11 mg, 0.16 mmol), N-methylimidazole (5 mg, 0.06 mmol) and 3A molecular sieve (500 mg) were dissolved in 10 mL of acetonitrile. Compound 7 (144 mg, 0.48 mmol) was added at room temperature. The mixture was stirred at room temperature overnight. After the reaction was complete, the molecular sieve was filtered out, and dichloromethane (30 mL) was added. The mixture was washed with saturated aqueous sodium bicarbonate solution (10 mL) three times and then with saturated brine (20 mL). The filtrate was concentrated by rotary evaporation, purified by reversed-phase preparative HPLC ($C^{18}$, conditions: 5-100% (A: water, B: $CH_3CN$), flow rate: 70 mL/min), and lyophilized to give compound **1-1a** (200 mg). MS m/z: $C_{40}H_{39}N_6O_7P$, [M-diisopropyl+OH]$^+$ calculated: 747.26, found: 747.6. 1H NMR (400 MHz, acetonitrile-$d_3$) δ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

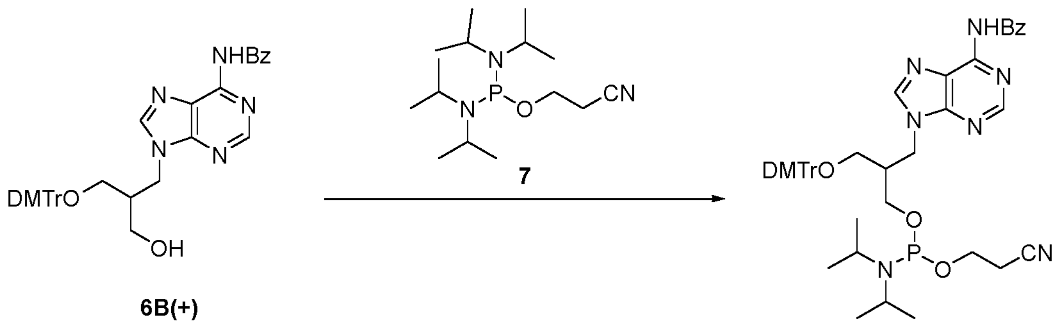

7

6B(+)

1-1b

Compound **6B(+)** (200 mg, 0.32 mmol), tetrazole (11 mg, 0.16 mmol), N-methylimidazole (5 mg, 0.06 mmol) and 3A molecular sieve (500 mg) were dissolved in 10 mL of acetonitrile. Compound 7 (144 mg, 0.48 mmol) was added at room temperature. The mixture was stirred at room temperature overnight. After the reaction was complete, the molecular sieve was filtered out, and dichloromethane (30 mL) was added. The mixture was washed with saturated aqueous sodium bicarbonate solution (10 mL) three times and then with saturated brine (20 mL). The filtrate was concentrated by rotary evaporation, purified by reversed-phase preparative HPLC ($C^{18}$, conditions: 5-100% (A: water, B: $CH_3CN$), flow rate: 70 mL/min), and lyophilized to give compound **1-1b** (200 mg). MS m/z: $C_{40}H_{39}N6O_7P$, [M-diisopropyl+OH]$^+$ calculated: 747.26, found: 747.5.

**1.2. Synthesis of compound 1-2**

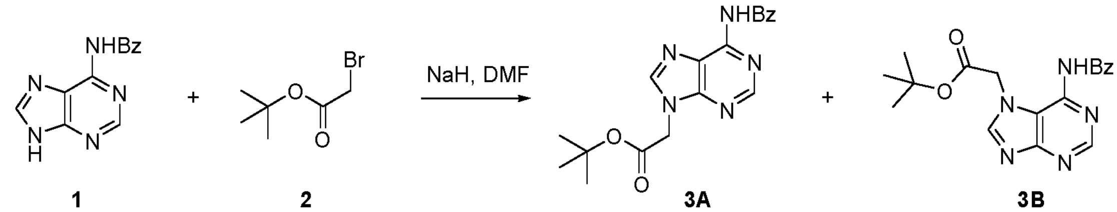


1 2 3A 3B

Compound **1** (2 g, 8.36 mmol) was dissolved in DMF (20 mL). NaH (0.37 g, 9.2 mmol, 60% in mineral oil) was added slowly under argon at room temperature. After 2 h of stirring at room temperature, compound 2 (3.3 g, 16.72 mmol) was added to the reaction mixture. After 12 h of stirring at room temperature, the reaction mixture was concentrated. The residue was recrystallized from ethanol (EtOH, 50 mL) to give the target product **3A** (1.3 g, yield: 44.0%) (dichloromethane:ethyl acetate = 2:1, Rf = 0.2) and the target product **3B** (0.6 g, a mixture of compound 1) (dichloromethane:ethyl acetate = 2:1, Rf = 0.18).

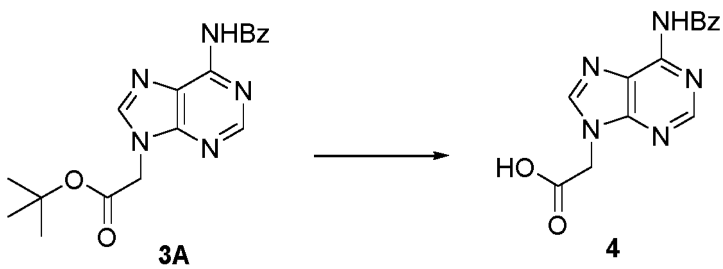

3A 4

Compound **3A** (1.3 g, 3.68 mmol) was dissolved in a mixture of trifluoroacetic acid (TFA, 4 mL) and DCM (20 mL), and then the reaction mixture was stirred at room temperature for 12 h and concentrated. The resulting residue was purified using a reversed-phase column ($C^{18}$, $H_2O$ + acetonitrile) to give the target product **4** (1 g, yield: 91.44%). MS m/z: $C_{39}H_{38}N_6O_6$, [M+H]$^+$: 687.5.

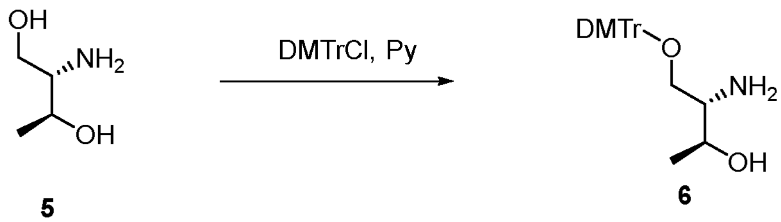


5 6

The compound (D-Threonol **5,** 1.2 g, 11.4 mmol) was dissolved in pyridine (10 mL), and then a solution of DMTrCl (4.64 g, 13.70 mmol) in pyridine (15 mL) was slowly added. After 16 h of stirring at room temperature, the reaction mixture was quenched with $H_2O$ (10 mL) and concentrated. The reaction mixture was concentrated. The resulting residue was purified using a reversed-phase column ($C^{18}$, $H_2O$ + acetonitrile) to give the target product **6** (4.0 g, yield: 86.0%). MS m/z: $C_{25}H_{29}NO_4$, $[M+Na]^+$: 430.4.

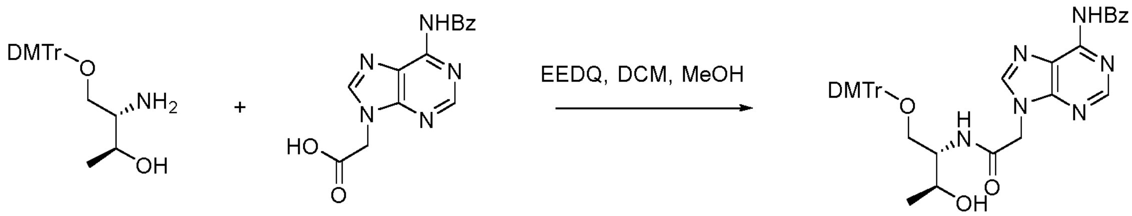


6 4 7

Compound **6** (600 mg, 2.02 mmol), compound **4** (822.5 mg, 2.02 mmol) and dihydroquinoline (EEDQ, 998.2 mg, 4.04 mmol) were dissolved in DCM (10 mL) and methanol (MeOH, 5 mL). After the mixture was stirred at room temperature for 16 h, the solid was filtered out and the filtrate was diluted with DCM (100 mL). The organic phase was washed three times with $H_2O$ (30 mL), dried over anhydrous $Na_2SO_4$, filtered and concentrated. The resulting residue was purified using a reversed-phase column ($C^{18}$, $H_2O$ + acetonitrile) to give the target product 7 (780 mg, yield: 56.3%). MS m/z: $C_{39}H_{38}N_6O_6$, $[M+H]^+$: 687.5.

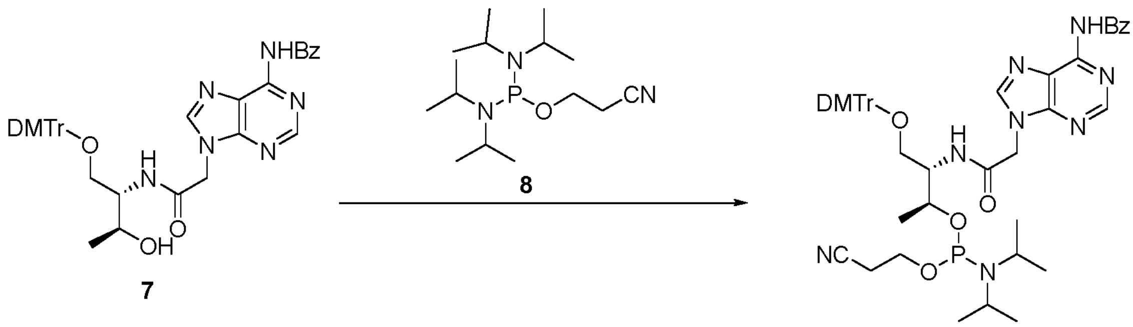

7 8 1-2

Compound **7** (780 mg, 1.13 mmol), tetrazole (39.8 mg, 0.57 mmol) and N-methylimidazole (18.7 mg, 0.23 mmol) were dissolved in $CH_3CN$ (10 mL). 3A molecular sieve (700 mg) was added. After 5 min of stirring at room temperature under argon, compound **8** (513.5 mg, 1.70 mmol) was added. After 1 h of stirring at room temperature, the molecular sieve was filtered out and the solid was rinsed three times with DCM (30 mL). The filtrate was washed successively with saturated aqueous $NaHCO_3$ solution (30 mL × 4) and $H_2O$ (30 mL × 4). The organic phase was concentrated at 30 °C. The resulting residue was purified using a reversed-phase column ($C^{18}$, $H_2O$ + acetonitrile, acetonitrile 90%) and lyophilized to give the target compound **1-2** (700 mg, yield: 69.5%). MS m/z: $C_{48}H_{55}N_8O_7P$, [M-cyanoethyl-diisopropyl+OH]$^-$: 749.3.

**1.3. Synthesis of compound 1-3**

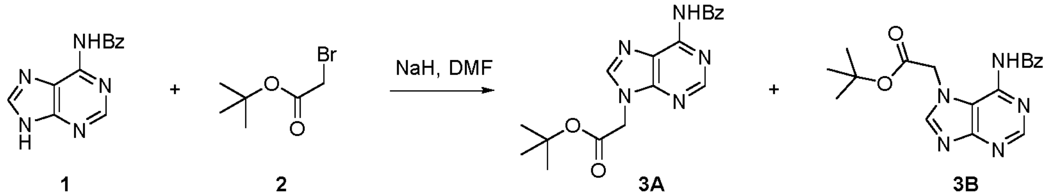


1 2 3A 3B

Compound **1** (2 g, 8.36 mmol) was dissolved in DMF (20 mL). NaH (0.37 g, 9.2 mmol, 60% in mineral oil) was added slowly under argon at room temperature. After 2 h of stirring at room temperature, compound **2** (3.3 g, 16.72 mmol) was added to the reaction mixture. After 12 h of stirring at room temperature, the reaction mixture was concentrated. The residue was recrystallized from EtOH (50 mL) to give the target product **3A** (1.3 g, yield: 44.0%) (dichloromethane:ethyl acetate = 2:1, Rf = 0.2) and the target product **3B** (0.6 g, a mixture of compound 1) (dichloromethane:ethyl acetate = 2:1, Rf = 0.18).

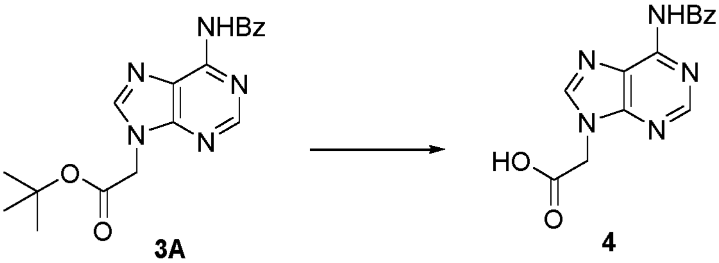

3A 4

Compound **3A** (1.3 g, 3.68 mmol) was dissolved in a mixture of TFA (4 mL) and DCM (20 mL), and then the reaction mixture was stirred at room temperature for 12 h and concentrated. The resulting residue was purified using a reversed-phase column ($C^{18}$, $H_2O$ + acetonitrile) to give the target product **4** (1 g, yield: 91.44%). MS m/z: $C_{39}H_{38}N_6O_6$, $[M+H]^+$: 687.5.

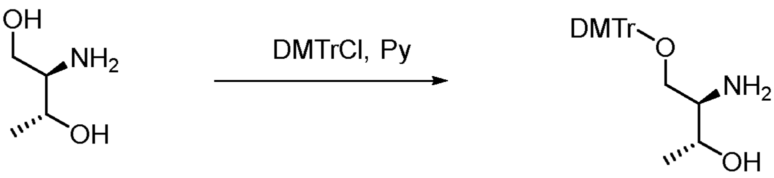


5 6

The compound L-Threoninol **5** (1.2 g, 11.4 mmol) was dissolved in pyridine (10 mL), and then a solution of DMTrCl (4.64 g, 13.70 mmol) in pyridine (15 mL) was slowly added. After 16 h of stirring at room temperature, the reaction mixture was quenched with $H_2O$ (10 mL) and concentrated. The reaction mixture was concentrated. The resulting residue was purified using a reversed-phase column ($C^{18}$, $H_2O$ + acetonitrile) to give the target product **6** (4.0 g, yield: 86.0%). MS m/z: $C_{25}H_{29}NO_4$, $[M+Na]^+$: 430.4.

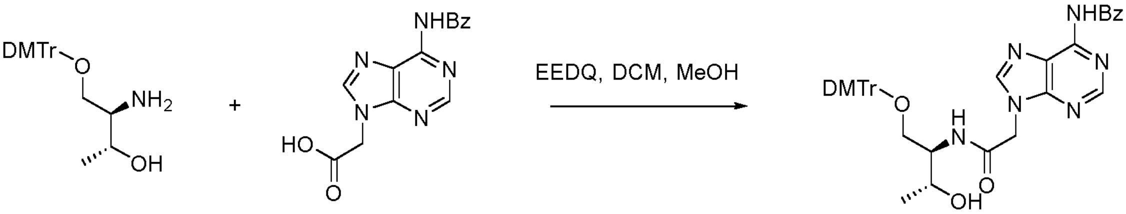


6 4 7

Compound **6** (600 mg, 2.02 mmol), compound **4** (822.5 mg, 2.02 mmol), tetramethyluronium hexafluorophosphate (HATU, 1.15 g, 3.03 mmol) and diisopropylethylamine (DIEA, 1 mL, 6.05 mmol) were dissolved in DMF (10 mL). After 16 h of stirring at room temperature, the reaction mixture was filtered and the filtrate was diluted with DCM (100 mL). The organic phase was washed three times with $H_2O$ (30 mL), dried over anhydrous $Na_2SO_4$, filtered and concentrated. The resulting residue was purified using a reversed-phase column ($C^{18}$, $H_2O$ + acetonitrile, acetonitrile 60%) and

lyophilized to give the target compound **7** (1.0 g, yield: 72.1%). MS m/z: $C_{39}H_{38}N_6O_6$, $[M+H]^+$: 687.5.

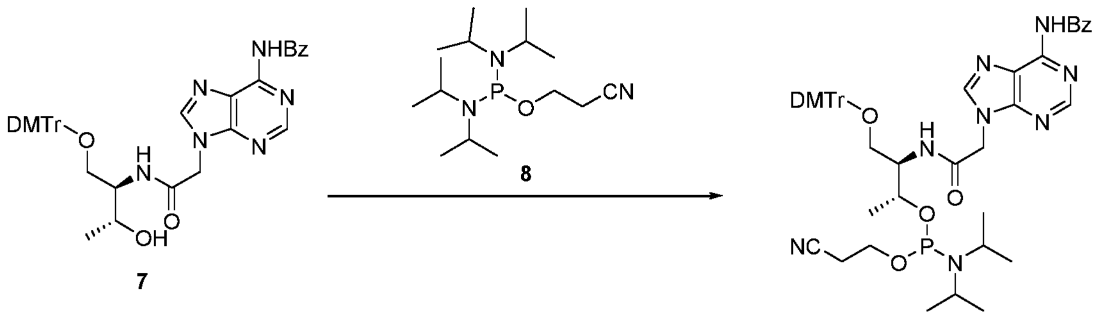

7 8 1-3

Compound **7** (1.2 g, 1.75 mmol), tetrazole (61.2 mg, 0.87 mmol) and N-methylimidazole (28.7 mg, 0.35 mmol) were dissolved in $CH_3CN$ (10 mL). 3A molecular sieve (700 mg) was added. After 5 min of stirring at room temperature under argon, compound **8** (0.79 g, 2.62 mmol) was added. After 1 h of stirring at room temperature, the molecular sieve was filtered out and the solid was rinsed three times with DCM (30 mL). The filtrate was washed successively with saturated aqueous $NaHCO_3$ solution (30 mL × 4) and $H_2O$ (30 mL × 4). The organic phase was concentrated at 30 °C. The resulting residue was purified using a reversed-phase column ($C^{18}$, $H_2O$ + acetonitrile, acetonitrile 90%) and lyophilized to give the target compound **1-3** (1.2 g, yield: 77.4%). MS m/z: $C_{48}H_{55}N_8O_7P$, [M-cyanoethyl-diisopropyl+OH]$^-$: 749.3.

### 1.4. Synthesis of compound 1-4a and compound 1-4b

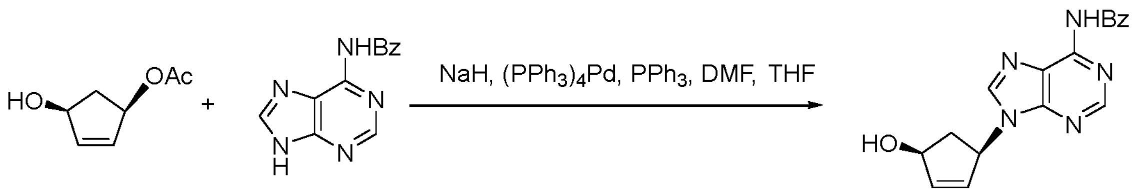


1 1A 2

Compound **1A** (6.73 g, 28.14 mmol) was dissolved in dry DMF (80 mL). NaH (60%, 1.24 g, 30.95 mmol) was added slowly under argon. After the mixture was stirred at room temperature for 30 min, the reaction mixture was added to a solution of tetrakis(triphenylphosphine)palladium(0) ($Pd(PPh_3)_4$, 1.95 g, 1.69 mmol), triphenylphosphine ($PPh_3$, 0.74 g, 2.81 mmol) and compound **1** (4.0 g, 28.14 mmol) in tetrahydrofuran (THF, 60 mL). After the reaction mixture was stirred at 55 °C for 16 h, the solid was filtered out and washed three times with DCM (60 mL). The filtrate was concentrated. The resulting residue was purified using a normal phase column (elution first with ethyl acetate and then with ethyl acetate: methanol (12:1)) to give the target product **2** (7 g, crude).

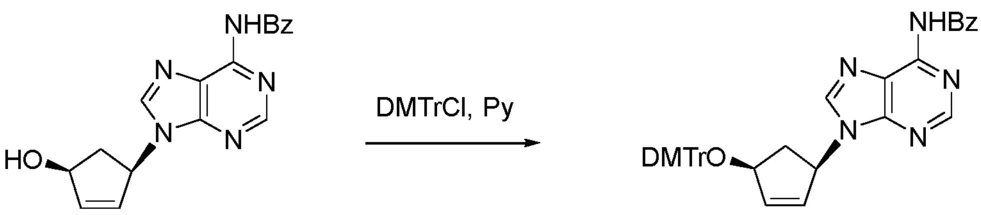


2 3

Compound **2** (8 g, crude) and DMTrCl (12.65 g, 37.34 mmol) were dissolved in pyridine (10 mL). The mixture was stirred at room temperature for 16 h, then quenched with water (80 mL) and concentrated. The resulting residue was purified using a reversed-phase column ($C^{18}$, water + acetonitrile) and lyophilized to give the target compound **3** (13 g, yield: 83.7%).

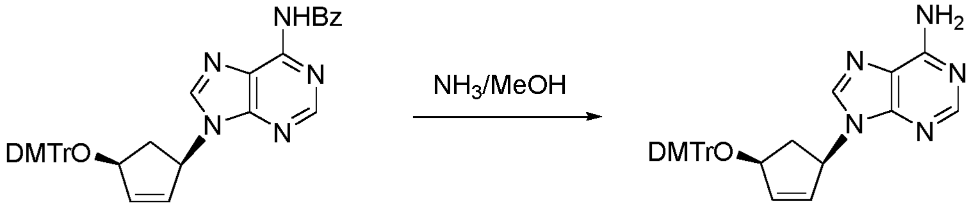


3 4

Compound **3** (5 g, 8.02 mmol) was dissolved in methanol (MeOH, 20 mL) and ammonia water (6 mL). After the mixture was stirred at room temperature for 16 h, the reaction mixture was concentrated. The resulting residue was purified using a normal phase column (DCM:MeOH = 20:1) to give the target compound **4** (4 g, yield: 96.0%).

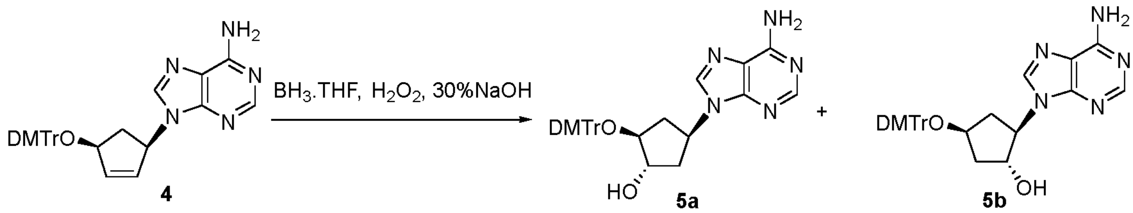


4 5a + 5b

A solution of borane ($BH_3$) in tetrahydrofuran (1.0 M in THF, 38.54 mL, 38.54 mmol) was added dropwise to a solution of compound **4** (4.00 g, 7.71 mmol) in THF (12 mL) at 0 °C under argon. After the compound was stirred at 0 °C under argon for 6 h, $H_2O$ (27 mL) was added dropwise. Then, after 3 M aqueous NaOH solution (52 mL, 156 mmol) was added dropwise to the reaction mixture at 0 °C, 30% aqueous HzOz (106 mL) was added dropwise to the reaction mixture, and EtOH (10 mL) was added. After the reaction mixture was stirred at room temperature for 48 h, saturated $Na_2S_2O_3$ was added slowly at 0 °C until no bubbles were formed. $H_2O$ (300 mL) was added to the reaction mixture, and the mixture was extracted with DCM (4 × 200 mL). The organic phase was dried over anhydrous $Na_2SO_4$, filtered and concentrated. The resulting residue was purified using a reversed-phase column ($C^{18}$, acetonitrile + H2O, 50%) and lyophilized to give the target product **5a** (730 mg, yield: 17.6%) and the target product **5b** (1.1 g, 26.6%).

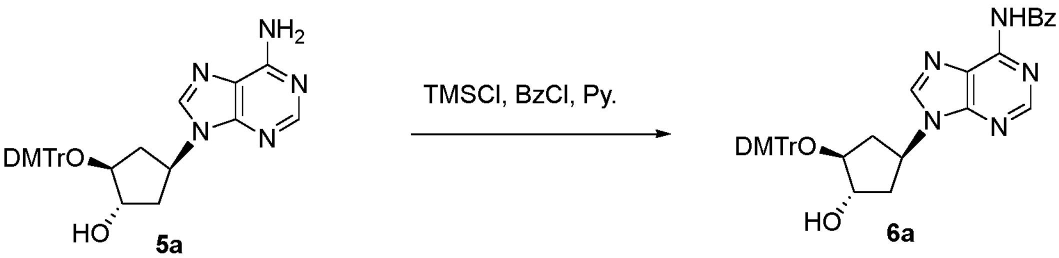


5a 6a

Compound **5a** (730 mg, 1.36 mmol) was dissolved in pyridine (8 mL). TMSCl (0.67 g, 6.14 mmol) was added under argon at room temperature. After 1 h of stirring at room temperature, BzCl (0.29 mL, 2.46 mmol) was added to the reaction mixture. After 16 h of stirring at room temperature, the reaction mixture was quenched with $H_2O$ (10 mL) and concentrated. The resulting residue was dissolved in THF (30 mL). Tetrabutylammonium fluoride (TBAF, 1 mL) was added. After 1 h of stirring at room temperature, ammonia water (0.5 mL) was added. The mixture was stirred at room temperature for 5 h. The reaction mixture was diluted with ethanol (EA, 100 mL) and washed five times with saturated brine (30 mL). The organic phase was concentrated. The resulting residue was purified using a reversed-phase column (C18, $H_2O$ + acetonitrile, acetonitrile 60%) and lyophilized to give the target product **6a** (480 mg, yield: 74.8%). MS m/z: $C_{38}H_{35}N_5O_5$, $[M+H]^+$: 642.6.

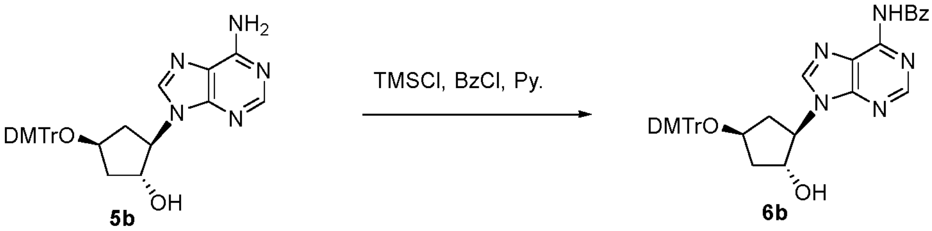


5b 6b

Compound **5b** (1.1 g, 2.05 mmol) was dissolved in pyridine (20 mL). TMSCl (1.34 g, 1.28 mmol) was added under argon at room temperature. After 1 h of stirring at room temperature, benzoyl chloride (BzCl, 0.59 mL, 5.92 mmol) was added to the reaction mixture. After 16 h of stirring at room temperature, the reaction mixture was quenched with $H_2O$ (10 mL) and concentrated. The resulting residue was dissolved in THF (30 mL). TBAF (2 mL) was added. After 1 h of stirring at room temperature, ammonia water (0.5 mL) was added. The mixture was stirred at room temperature for 5 h. The reaction mixture was diluted with EA (100 mL) and washed five times with saturated brine (30 mL). The organic phase was concentrated. The resulting residue was purified using a reversed-phase column (C18, $H_2O$ + acetonitrile, acetonitrile 60%) and lyophilized to give the target product **6b** (1.4 g, yield: 82.1%). MS m/z: $C_{38}H_{35}N_5O_5$, $[M+H]^+$: 642.5.

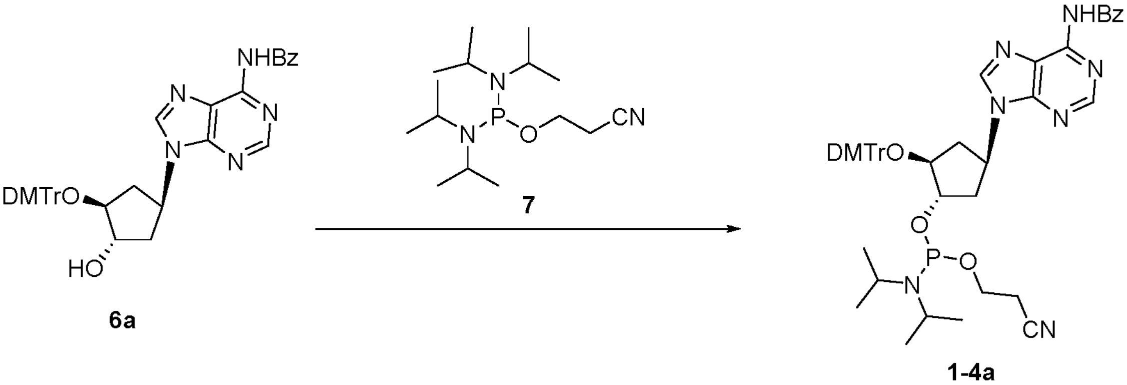

6a 7 1-4a

Compound **6a** (700 mg, 1.04 mmol), tetrazole (26.2 mg, 0.37 mmol) and N-methylimidazole were dissolved in $CH_3CN$ (10 mL). 3A molecular sieve (500 mg) was added. After 5 min of stirring at room temperature under argon, compound 7 (470.4 mg, 1.56 mmol) was added. After 1 h of stirring at room temperature, the molecular sieve was filtered out and the solid was rinsed three times with DCM (50 mL). The filtrate was washed successively with saturated aqueous $NaHCO_3$ solution (50 mL × 4) and $H_2O$ (50 mL × 4). The organic phase was concentrated at 30 °C. The resulting residue was purified using a reversed-phase column ($C^{18}$, $H_2O$ + acetonitrile, acetonitrile 90%) and lyophilized to give the target compound **1-4A** (600 mg, yield: 66.1%). MS m/z: $C_{47}H_{52}N_7O_6P$, [M-cyanoethyl-diisopropyl+OH]$^-$: 704.3.

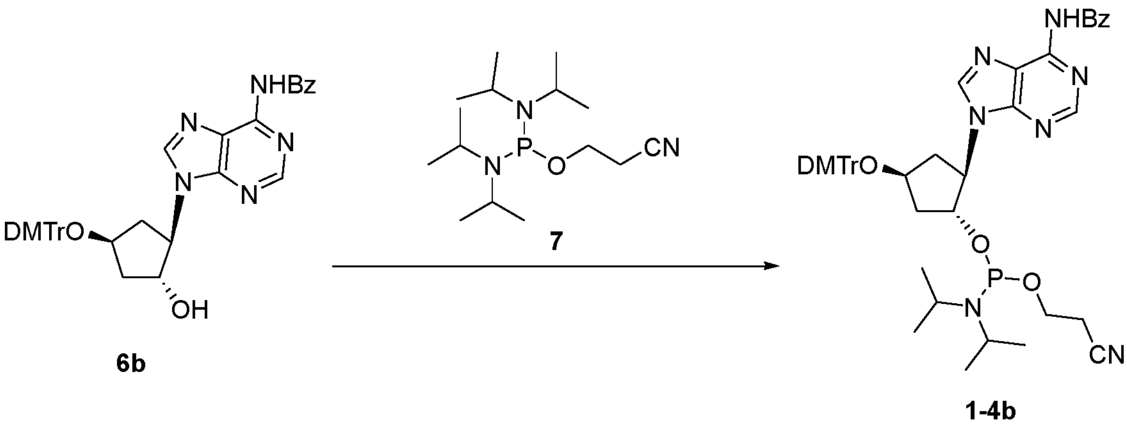

6b 7 1-4b

Compound **6b** (1.3 g, 2.03 mmol), tetrazole (71.0 mg, 1.01 mmol) and N-methylimidazole (33.3 mg, 0.41 mmol) were dissolved in $CH_3CN$ (20 mL). 3A molecular sieve (700 mg) was added. After 5 min of stirring at room temperature under argon, compound 7 (0.92 g, 3.04 mmol) was added. After 1 h of stirring at room temperature, the molecular sieve

was filtered out and the solid was rinsed three times with DCM (50 mL). The filtrate was washed successively with saturated aqueous $NaHCO_3$ solution (50 mL × 4) and $H_2O$ (50 mL × 4). The organic phase was concentrated at 30 °C. The resulting residue was purified using a reversed-phase column ($C^{18}$, $H_2O$ + acetonitrile, acetonitrile 90%) and lyophilized to give the target compound **1-4b** (1.4 g, yield: 82.1%). MS m/z: $C_{47}H_{52}N_7O_6P$, [M-cyanoethyl-diisopropyl]$^-$: 704.3.

**1.5. Synthesis of compound 1-5**

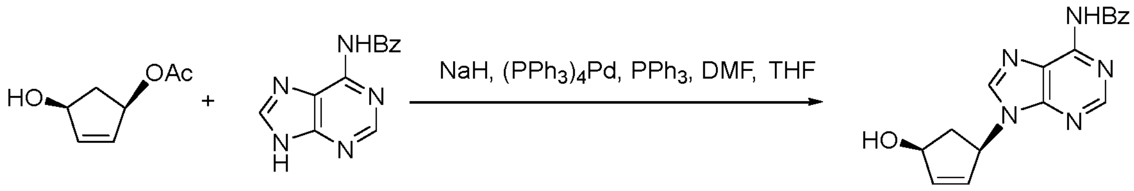


**1** **1A** **2**

Compound **1A** (6.73 g, 28.14 mmol) was dissolved in dry DMF (80 mL). NaH (60%, 1.24 g, 30.95 mmol) was added slowly under argon. After the mixture was stirred at room temperature for 30 min, the reaction mixture was added to a solution of $Pd(PPh_3)_4$ (1.95 g, 1.69 mmol), $PPh_3$ (0.74 g, 2.81 mmol) and compound **1** (4.0 g, 28.14 mmol) in THF (60 mL). After the reaction mixture was stirred at 55 °C for 16 h, the solid was filtered out and washed three times with DCM (60 mL). The filtrate was concentrated. The resulting residue was purified using a normal phase column (elution first with ethyl acetate and then with ethyl acetate: methanol (12:1)) to give the target solid **2** (7 g, crude).

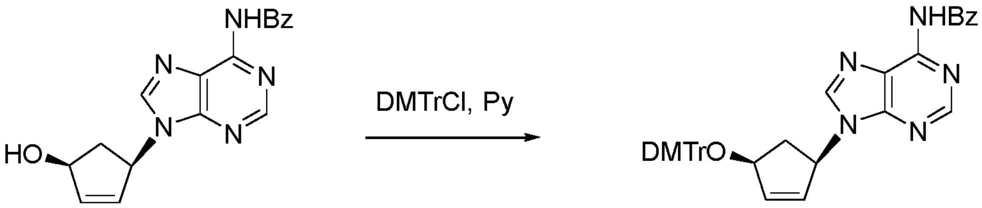


**2** **3**

Compound **2** (8 g, crude) and DMTrCl (12.65 g, 37.34 mmol) were dissolved in pyridine (10 mL). The mixture was stirred at room temperature for 16 h, then quenched with water (80 mL) and concentrated. The resulting residue was purified using a reversed-phase column ($C^{18}$, water + acetonitrile) and lyophilized to give the target compound **3** (13 g, yield: 83.7%).

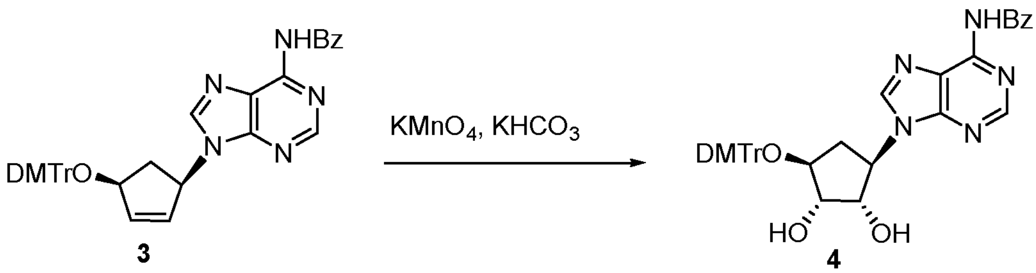


**3** **4**

Compound **3** (1 g, 1.60 mmol), $KHCO_3$ (0.48 g, 4.81 mmol) and ethylene glycol (0.40 g, 6.41 mmol) were dissolved in acetone (50 mL). $KMnO_4$ (40% in water, 0.67 g, 1.68 mmol) was slowly added at -30 °C. After 1 h of stirring at -30 °C, the reaction mixture was quenched with saturated aqueous sodium thiosulfate solution (30 mL). The mixture was extracted four times with DCM (30 mL). The organic phase was dried over anhydrous $Na_2SO_4$, filtered and concentrated. The residue was purified using a reversed-phase column (C18, $H_2O$ + acetonitrile, acetonitrile 60%) and lyophilized to give the target product **4** (600 mg, yield: 56.9%). MS m/z: $C_{38}H_{35}N_5O_6$, $[M+H]^+$: 658.5.

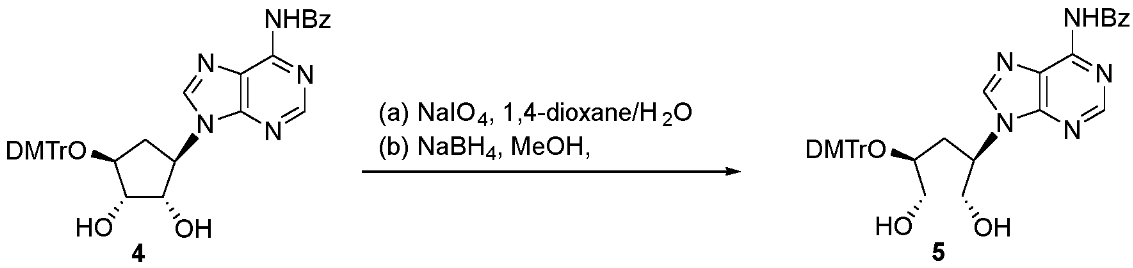


To a 250 mL round-bottom flask were added reactant **4** (5.0 g, 7.601 mmol), $NaIO_4$ and 1,4-dioxane/water (50 mL/5 mL). The mixture was reacted at room temperature for 2 h and then concentrated under reduced pressure to remove the solvent to give a white solid (6.0 g). Then the solid was dissolved in methanol (50 mL), and sodium borohydride (1.62 g, 38 mmol) was added. After the mixture was stirred at room temperature for 2 h, 10% ammonium chloride solution (10 mL) was added. After removal of the solvent under reduced pressure, the residue was purified by C18 column chromatography (water/acetonitrile: 5%-95%) to give product P1 as a colorless oil **5** (2.0 g, 3.0315 mmol, 39%), LCMS, MS+, $[M+H]^+$: 660.

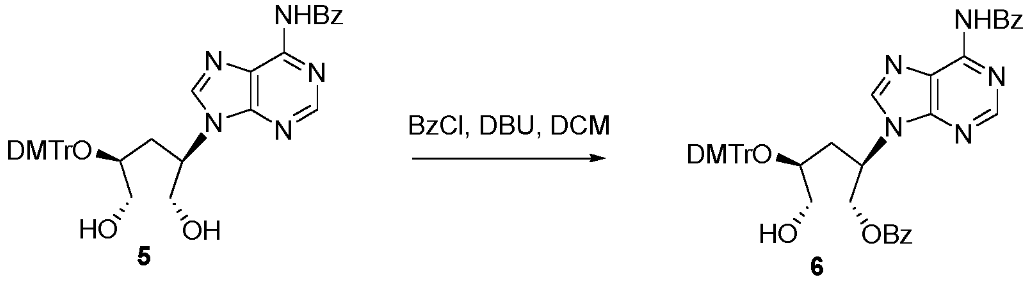


Compound **5** (1.7 g, 2.58 mmol) and DBU (0.77 mL, 5.15 mmol) were dissolved in DCM (20 mL). BzCl (0.5 M in DCM, 0.8 mL) was added dropwise to the reaction at - 70 °C under argon. The reaction mixture was let stand at -70 °C for 1 h and quenched with ethanol (5 mL). The quenched reaction mixture was diluted with DCM (100 mL) and washed three times with water (30 mL). The organic phase was dried over anhydrous $Na_2SO_4$, filtered and concentrated. The resulting residue was purified using a normal phase column (DCM:EA = 1:1) to give **6** as a white solid (80 mg, yield: 4.14%). MS m/z: $C_{45}H_{41}N_5O_7$, $[M+H]^+$: 764.5.

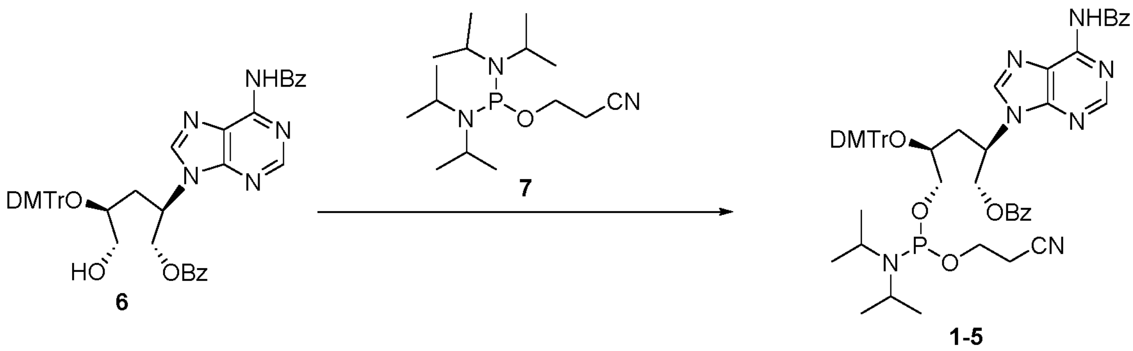

Compound **4** (380 mg, 0.50 mmol), tetrazole (17.43 mg, 0.25 mmol) and N-methylimidazole (8.17 mg, 0.10 mmol) were dissolved in $CH_3CN$ (10 mL). 3A molecular sieve (500 mg) was added. After 5 min of stirring at room temperature under argon, compound 7 (224.95 mg, 0.75 mmol) was added. After 1 h of stirring at room temperature, the molecular sieve was filtered out and the solid was rinsed three times with DCM (50 mL). The filtrate was washed successively with saturated aqueous $NaHCO_3$ solution (50 mL × 4) and $H_2O$ (50 mL × 4). The organic phase was concentrated at 30 °C. The resulting residue was purified using a reversed-phase column ($C^{18}$, $H_2O$ + acetonitrile, acetonitrile 90%) and lyophilized to give the target product **1-5** (330 mg, yield: 68.8%). MS m/z: $C_{54}H_{58}N_7O_8P$, [M-cyanoethyl-diisopropyl]$^-$: 826.3.

**1.6. Synthesis of compound 1-6a**

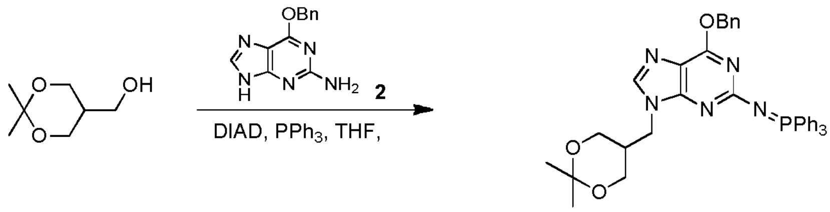


1 3

Compound 1 (10 g, 68.404 mmol), compound 2 (15 g, 62.186 mmol) and triphenylphosphine (32.62 g, 124.371 mmol) were dissolved in dry THF (30 mL). DIAD (24.656 mL, 124.371 mmol) was slowly added dropwise at 0 °C. The reaction mixture was reacted at 25 °C for 12 h, and LCMS showed the reaction had been complete. The reaction mixture was extracted with ethyl acetate (200 mL) and water (200 mL). The organic phase was dried. The filtrate was concentrated. The resulting residue was purified using a normal phase column (DCM/MeOH = 10/1) to give the target product 3 (20 g).

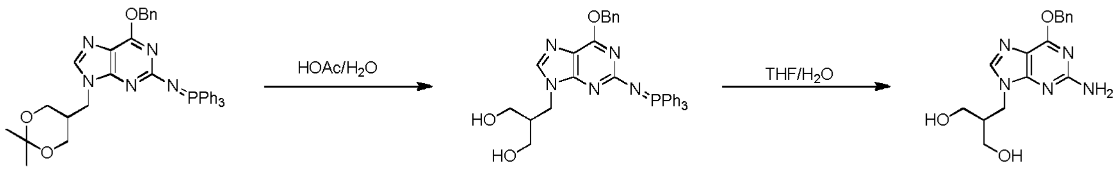


3 4 5

Compound **3** (20 g, 28.585 mmol) was dissolved in acetic acid (24 mL, 426.016 mmol) and H2O (12 mL). The mixture was stirred at 60 °C for 1 h. Then the reaction mixture was concentrated to dryness by rotary evaporation. THF (12 mL) and H2O (12 mL) were added. The mixture was stirred at 80 °C for 7 h. LCMS showed the reaction had been complete. The reaction mixture was extracted with ethyl acetate (200 mL) and water (100 mL). Solid sodium carbonate was added to the aqueous phase until a large amount of solid precipitated out of the aqueous phase. The solid was collected by filtration and washed with water. The filter cake was dried with an oil pump to give the target compound **5** (9 g).

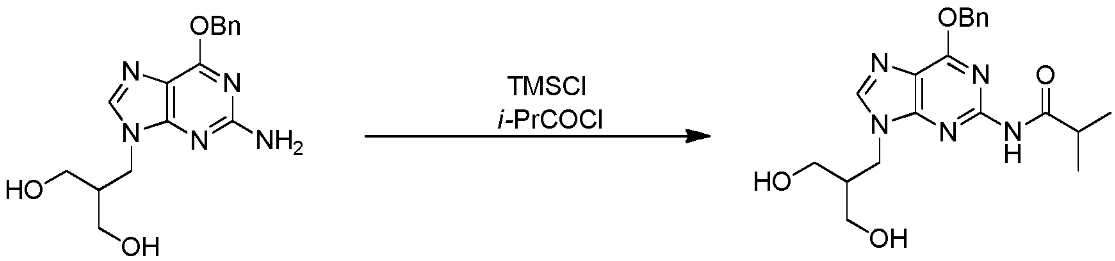


**5** **6**

Compound 5 (6.8 g, 18.581 mmol) was dissolved in pyridine (80 mL) under nitrogen. TMSCl (14.250 mL, 111.489 mmol) was slowly added at 0 °C. The mixture was stirred for 2 h. Isobutyryl chloride (2.044 mL, 19.511 mmol) was then added at 0 °C. The mixture was stirred at 25 °C for 1 h, and LCMS showed the reaction had been complete. The mixture was extracted with dichloromethane (200 mL) and water (200 mL). After the organic phase was dried and concentrated to dryness by rotary evaporation, a sample to be purified was prepared. The sample was purified using a normal phase column (elution with DCM:MeOH = 10:1, peak at 4.8%) to give compound 6 as a yellow oil (12 g).

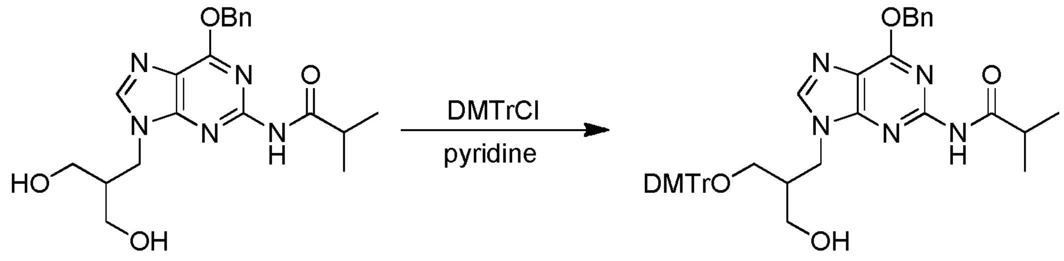


6 7

Compound 6 (5.5 g, 12.392 mmol) was dissolved in pyridine (30 mL) under nitrogen. MOLECULAR SIEVE 4A 1/16 (7 g, 12.392 mmol) was added, and then solid DMTrCl (5.04 g, 14.870 mmol) was added in batches at 0 °C. The mixture was reacted at 25 °C for 2 h, and TLC (PE:EtOAc = 1:1, Rf = 0.69) showed the reaction had been complete. The reaction mixture and TJN200879-040-P1 were combined and treated together. The reaction mixture was extracted with ethyl acetate (200 mL) and water (200 mL). After the organic phase was dried and concentrated to dryness by rotary evaporation, a sample to be purified was prepared. The sample was purified using a normal phase column (elution with PE:EtOAc, peak at 84%) to give compound 7 as a yellow oil (12 g).

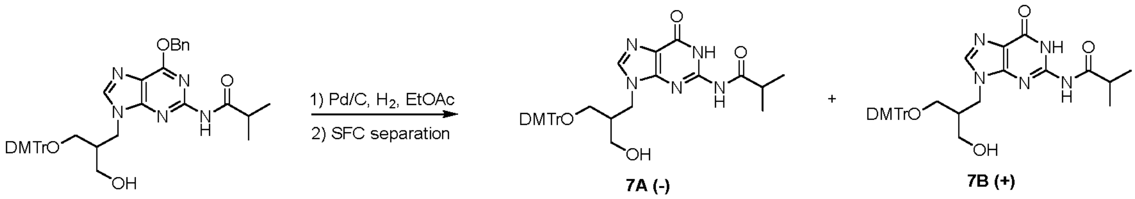


7A (-) + 7B (+)

7

Compound 7 (12 g, 15.389 mmol) was dissolved in EtOAc (140 mL). Wet palladium on carbon Pd/C (7 g, 15.389 mmol) was added. The reaction mixture was reacted at 25 °C under hydrogen (15 Psi) for 2 h. TLC (PE:EtOAc = 0:1, Rf = 0.09) showed the reaction had been complete. The reaction mixture was filtered. After the filter cake was rinsed three times with ethyl acetate (30 mL), the filtrate was collected. After the filtrate was concentrated to dryness by rotary evaporation, 50 mL of dichloromethane and 2 mL of triethylamine were added to prepare a sample to be purified. The sample was purified using a normal phase column (elution with DCM:MeOH = 10:1, peak at 0.5%) to give 9 g (yellow foamy solid). The resulting racemic compound was resolved by SFC into the target compound 7A(-) (3.9 g) and the target compound 7B(+) 3.8 g).

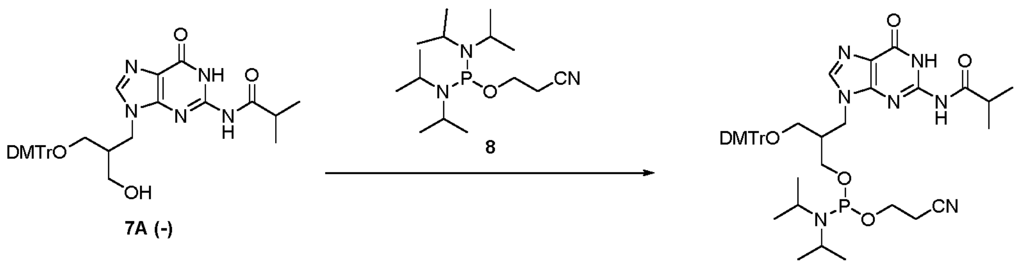

7A (-) 8

1-6a

Compound 7A(-) (3.30 g, 5.40 mmol), tetrazole (190 mg, 2.70 mmol), 1-methylimidazole (90 mg, 1.10 mmol) and 3A molecular sieve (500 mg) were dissolved in 30 mL of acetonitrile. Compound 8 (2.50 g, 8.10 mmol) was added at room temperature. The mixture was stirred at room temperature for 2 h. After the reaction was complete, the molecular sieve was filtered out, and DCM (150 mL) was added. The mixture was washed with saturated aqueous sodium bicarbonate solution (30 mL × 3) and then with saturated brine (30 mL). The filtrate was concentrated by rotary evaporation, purified by reversed-phase preparative HPLC (C18, conditions: 5-100% (A: water, B: CH3CN), flow rate: 70 mL/min), and lyophilized to give compound **1-6a** (2.9 g, 66%). MS m/z: C43H55N7O7P [M+H]+, calculated: 812.38, found: 812.5. 1H NMR (400 MHz, acetonitrile-d3) δ 7.56, 7.54 (2s, 1H), 7.36-7.27 (m, 2H), 7.24-7.21 (m, 7H), 6.83-6.80 (m, 4H), 4.12-4.10 (m, 2H), 3.75-3.68 (m, 10H), 3.20-2.80 (m, 2H), 2.68-2.54 (m, 4H), 1.22-1.04 (m, 18H).

**1.7. Synthesis of compound 1-7a**

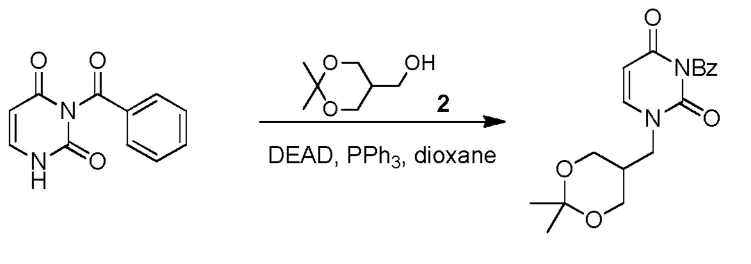


1 3

Compound 1 (5 g, 23.1272 mmol), compound 2 (6.76 g, 46.254 mmol) and triphenylphosphine (7.28 g, 27.753 mmol) were dissolved in 30 mL of dioxane under nitrogen. DEAD (5.502 mL, 27.753 mmol) was slowly added dropwise at 0 °C. After the dropwise addition was complete, the reaction mixture was slowly warmed to 25 °C and reacted for another hour. The reaction mixture was extracted with 100 mL of H2O and 100 mL of EtOAc. After the organic phases were combined, dried, filtered and concentrated, a sample to be purified was prepared. The sample was purified using a normal phase column (elution with PE:EtOAc = 1:1) to give the target product (4 g).

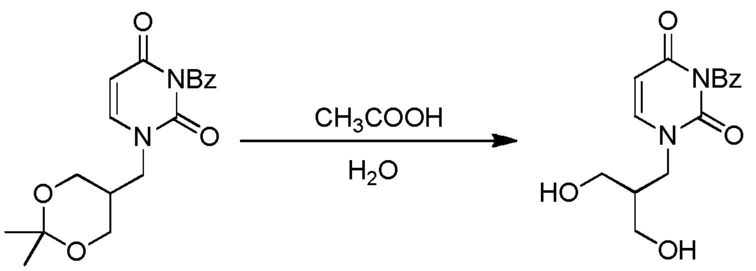


3 4

Compound 3 (3.3 g) was dissolved in HOAc (16 mL) and H2O (4 mL). The reaction mixture was heated in an oil bath at 60 °C for 0.5 h and concentrated to dryness by rotary evaporation. The resulting residue was purified using a normal phase column (elution with PE:EtOAc = 0:1) to give the target product 4 (3 g).

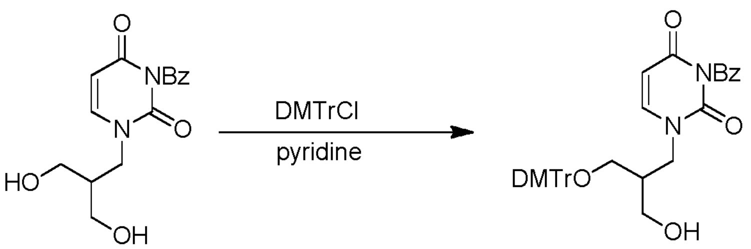


4 5

Compound 4 (3 g, 8.873 mmol) was dissolved in 5 mL of pyridine. A solution of DMTrCl (3.91 g, 11.535 mmol) in 10 mL of pyridine was slowly added dropwise at 0 °C under nitrogen. After the dropwise addition was complete, the reaction mixture was warmed to 25 °C and reacted for another hour. The reaction mixture was extracted with 50 mL of water and 100 mL of ethyl acetate. The aqueous phase was extracted three more times with 100 mL of ethyl acetate. The organic phases were combined, dried, filtered, concentrated, and purified using a normal phase column (with PE:EtOAc = 2:1) to give the target product 5 (4 g).

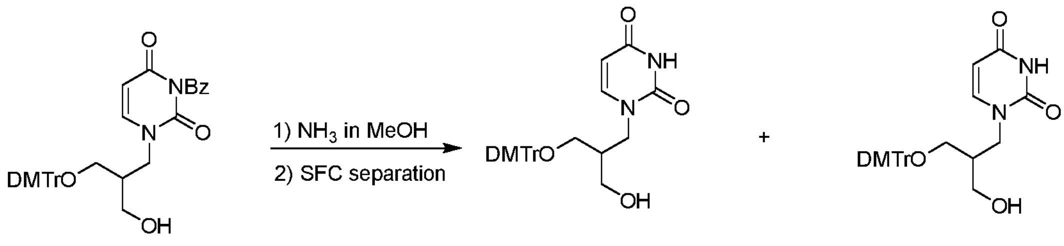


5

6A (-)

6B (+)

Compound 5 (4 g, 5.769 mmol) was dissolved in methanol (10 mL). A saturated solution of NH3 in methanol (40 mL) was added. The mixture was reacted at 0 °C for 6 h. The reaction mixture was concentrated to dryness by rotary evaporation and purified using a normal phase column (PE:EtOAc = 0:1) to give a racemic compound (2.4 g). The compound was resolved by SFC into the target product 6A (750 mg, 100% purity) and the target product 6B (400 mg, 99.16% purity).

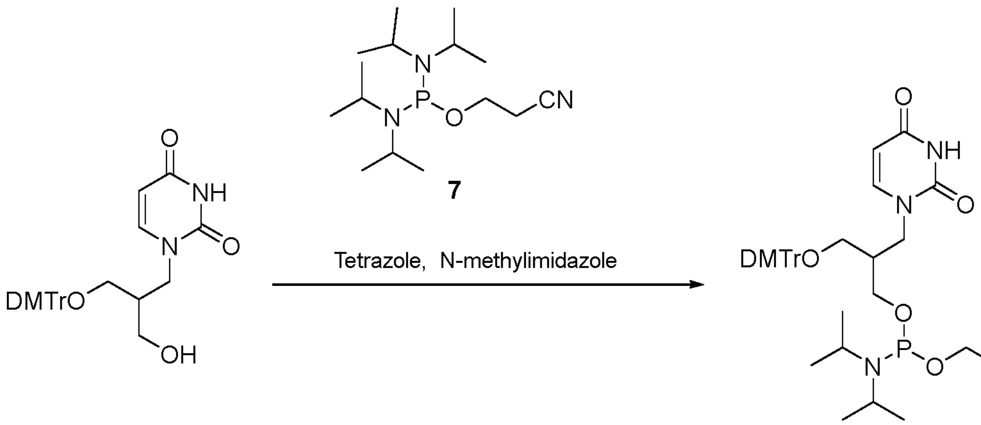


7

6A (-)

1-7a

Compound 6A(-) (700 mg, 1.40 mmol), tetrazole (50 mg, 0.70 mmol), 1-methylimidazole (23 mg, 0.28 mmol) and 3A molecular sieve (500 mg) were dissolved in 10 mL of acetonitrile. Compound 7 (630 mg, 2.10 mmol) was added at room temperature. The mixture was stirred at room temperature for 2 h. After the reaction was complete, the molecular sieve was filtered out, and DCM (50 mL) was added. The mixture was washed with saturated aqueous sodium bicarbonate solution (10 mL × 3) and then with saturated brine (20 mL). The filtrate was concentrated by rotary evaporation, purified by reversed-phase preparative HPLC (C18, conditions: 5-100% (A: water, B: CH3CN), flow rate: 70 mL/min), and lyophilized to give compound **1-7a** (700 mg, 72%). MS m/z: C38H47N4O7PNa [M+Na]+, calculated: 725.32, found: 725.5.

**1.8. Synthesis of compound 1-8a**

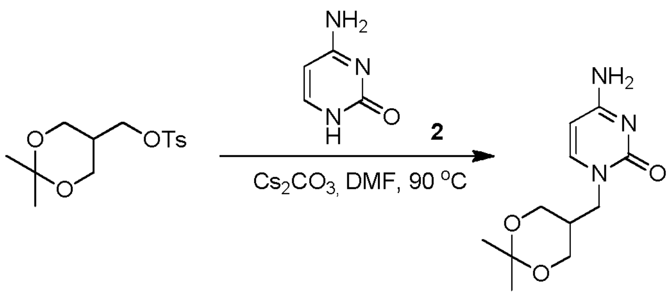


2

1

3

Compound 1 (8.5 g, 76.508 mmol) and compound 2 (30.64 g, 91.809 mmol) were dissolved in DMF (150 mL). CS2CO3 (29.91 g, 91.809 mmol) was added. The reaction mixture was reacted under nitrogen at 90 °C for 12 h. LCMS detection showed the reaction had been complete. The reaction mixture was filtered, concentrated to dryness by rotary evaporation using an oil pump, and separated and purified using a normal phase column (80 g, DCM/MeOH = 10/1 to 5/1) to give the target product 3 (13.5 g, 80% purity).

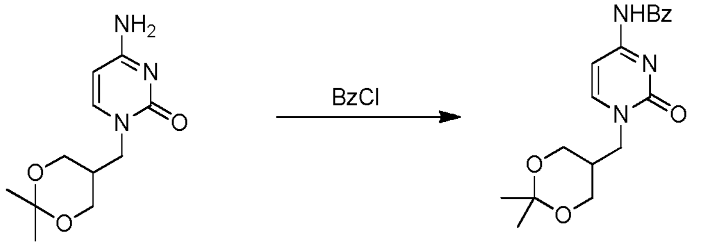


3 4

Compound 3 (10.5 g, 35.105 mmol) was dissolved in pyridine (65 mL) and CH3CN (65 mL). BzCl (4.894 mL, 42.126 mmol) was added dropwise to the solution. The mixture was reacted at 25 °C for 2 h. LCMS detection showed starting materials were mostly reacted. The mixture was quenched with H2O (100 mL) and extracted with EtOAc (100 mL × 3). The extract was dried, concentrated to dryness by rotary evaporation, and separated (combined with TJN200872-101) and purified by column chromatography (80 g, PE/EtOAc = 10/1 to 0/1, DCM/MeOH = 10/1) to give the target product 4 (14 g, 90% purity).

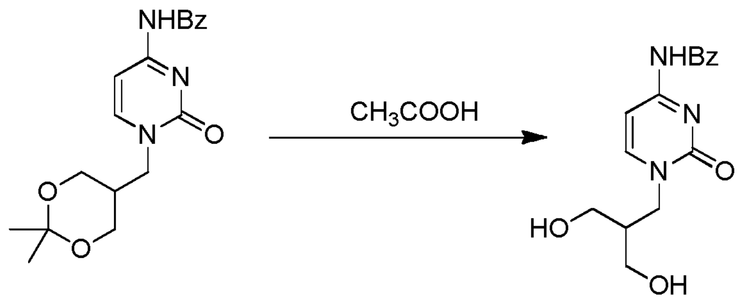


4 5

Compound 4 (14 g, 36.694 mmol) was dissolved in HOAc (56 mL, 314.796 mmol) and H2O (14 mL). The mixture was reacted at 60 °C for 2 h, and LCMS showed the reaction had been complete. The mixture was concentrated using an oil pump and separated using a normal phase column (40 g, DCM/MeOH = 1/0 to 5/1) to give the target product 5 (8.4 g, 90% purity & 2.4 g, 80% purity).

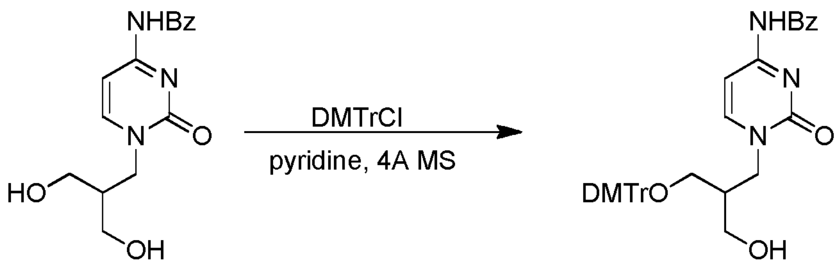


5 6

Compound 5 (7.4 g, 21.957 mmol), DMAP (0.54 g, 4.391 mmol) and MOLECULAR SIEVE 4A (11.1 g, 2.967 mmol) were dissolved in pyridine (60 mL). The mixture was stirred under ice bath conditions for 10 min, and then DMTrCl (8.93 g, 26.348 mmol) was added. The reaction mixture was stirred for 1.8 h, and LCMS detection showed about 19% of the starting material remained and about 60% was target MS. The mixture was combined with TJN200872-105&106

and purified together. H2O (50 mL) was added to the reaction mixture. The mixture was extracted with DCM (50 mL × 3), dried, concentrated to dryness by rotary evaporation, and separated by column chromatography (120 g, PE/(EA:DCM:TEA = 1:1:0.05) = 1/0 to 0/1 to DCM/MeOH = 10/1) to give compound 6 as a yellow solid (11 g, 89% purity, TJN200872-105&106&107). The starting material was recovered (3.0 g, 70% purity).

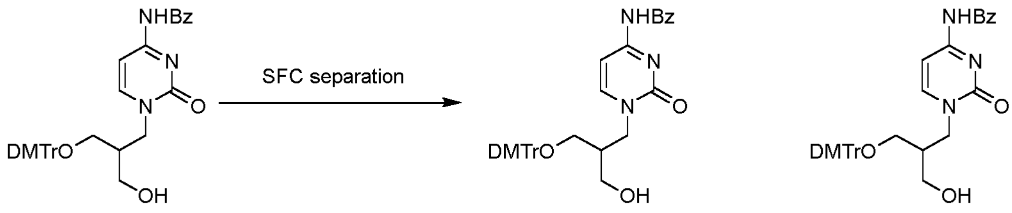


**6** **6A (+)** **6B (-)**

Compound 6 (15 g, 22.041 mmol) was resolved by SFC (DAICEL CHIRALPAK AD (250 mm × 50 mm,10 μm); 0.1% NH3H2O EtOH, B: 45% to 45%; 200 mL/min) into the target product 6A (5.33 g, 94.29% purity) and the target product 6B (6.14 g, 97.91% purity). 1.0 g of compound 6 was recovered.

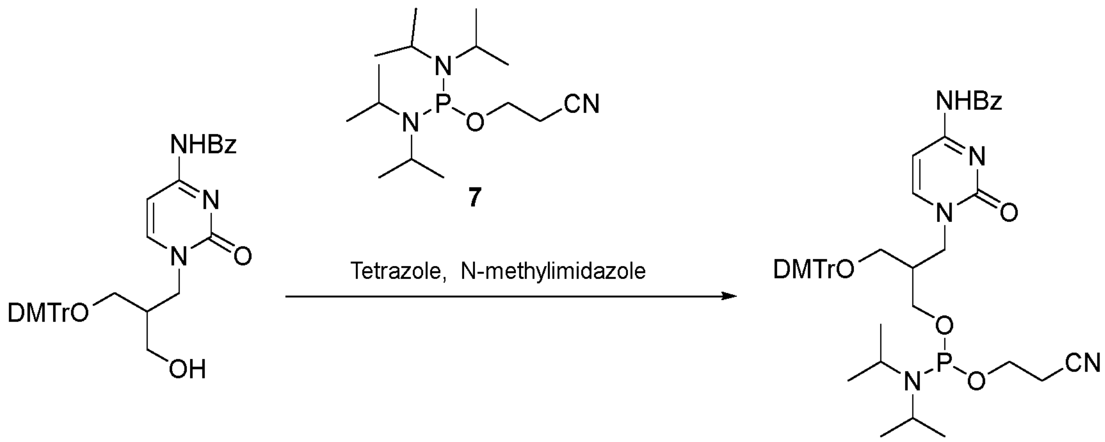


**7**

**6B (-)** **1-8a**

Compound 6B(-) (5.4 g, 8.92 mmol), tetrazole (312 mg, 4.46 mmol), 1-methylimidazole (146 mg, 1.78 mmol) and 3A molecular sieve (500 mg) were dissolved in 40 mL of acetonitrile. Compound 7 (4 g, 13.4 mmol) was added at room temperature. The mixture was stirred at room temperature for 2 h. After the reaction was complete, the molecular sieve was filtered out, and DCM (200 mL) was added. The mixture was washed with saturated aqueous sodium bicarbonate solution (30 mL × 3) and then with saturated brine (50 mL). The filtrate was concentrated by rotary evaporation, purified by reversed-phase preparative HPLC (C18, conditions: 5-100% (A: water, B: CH3CN), flow rate: 70 mL/min), and lyophilized to give compound **1-8a** (5.8 g, 80%). MS m/z: C45H51N5O7P, [M+H]+, calculated: 804.36, found: 804.4.

**Example 2. Synthesis of siRNA**

The siRNA synthesis was the same as the conventional phosphoramidite solid-phase synthesis. In synthesizing the modified nucleotide in 5' position 7 of the AS strand, the original nucleotide of the parent sequence was replaced with the phosphoramidite monomer synthesized above.

The synthesis process is briefly described below: Nucleoside phosphoramidite monomers were linked one by one according to the synthesis program on a Dr. Oligo48 synthesizer (Biolytic), starting at a Universal CPG support. Other than the phosphoramidite monomer in 5' position 7 of the AS strand described above, the other nucleoside monomer materials 2'-F RNA, 2'-O-methyl RNA, and other nucleoside phosphoramidite monomers were purchased from Hongene, Shanghai or Genepharma, Suzhou. 5-Ethylthio-1H-tetrazole (ETT) was used as an activator (a 0.6 M solution in acetonitrile), a 0.22 M solution of PADS in acetonitrile and collidine (1:1 by volume) (Kroma, Suzhou) as a sulfurizing agent, and iodopyridine/water solution (Kroma) as an oxidant.

After completion of solid phase synthesis, oligoribonucleotides were cleaved from the solid support and soaked in a solution of 28% ammonia water and ethanol (3:1) at 50 °C for 16 h. The mixture was centrifuged, and the supernatant

was transferred to another centrifuge tube. After the supernatant was concentrated to dryness by evaporation, the residue was purified by C18 reversed-phase chromatography using 0.1 M TEAA and acetonitrile as the mobile phase, and DMTr was removed using 3% trifluoroacetic acid solution. The target oligonucleotides were collected, then lyophilized, identified as the target products by LC-MS, and quantified by UV (260 nm).

The resulting single-stranded oligonucleotides were paired in an equimolar ratio in a complementary manner and annealed. The final double-stranded siRNA was dissolved in 1× PBS, and the solution was adjusted to the concentration required for the experiment so it was ready to be used.

## Example 3. psiCHECK Activity Screening

### 3.1. Experimental materials and instruments

The synthesis of siRNA samples is as described before. The plasmids were obtained from Sangon Biotech (Shanghai) Co., Ltd. The consumables, reagents and instruments for the psiCHECK assay are shown in Table 1 and Table 2.

Table 1. Consumables and reagents for the psiCHECK assay

| **Reagent consumables** | | | | |
|---|---|---|---|---|
| **Name** | **Company** | **Catalog number/model** | **Batch No.** | **Shelf life** |
| Huh 7 cells | Cobioer, Nanjing | ATCC-Cobioer/CBP60202 | / | / |
| Dual-Glo® Luciferase Assay System | Promega | E2940 | 0000363099 | 2020/5/13 |
| Lipofectamine® 2000 | Invitrogen | 11668-019 | / | 2021/6/14 |

Table 2. Instruments for the psiCHECK assay

| **Instruments** | | |
|---|---|---|
| **Name** | **Company** | **Catalog number/model** |
| Nanodrop | Thermo | Nanodrop One |
| Microplate reader | PerkinElmer | EnVision2105 |
| Graphing software | / | Graph Prism 5 |

### 3.2. Procedure of psiCHECK activity screening

Cell plating and cell transfection were carried out. The specific amounts for preparing the transfection complex are shown in Table 3.

Table 3. Amounts required for transfection complex in each well of a 96-well plate

| | Amount/well | Opti-MEM |
|---|---|---|
| Plasmid Mix | 0.05 μL | 10 μL |
| Lipofectamine 2000 | 0.2 μL | 10 μL |
| Note: Lipo: 0.2 μL/well; Plasmid: 0.05 μL/well; Opti-MEM: 10 μL/well. | | |

Dilutions with different concentrations were prepared as working solutions for later use to meet different experimental requirements according to Table 4.

Table 4. Multi-concentration-point dilution protocol for siRNAs

| Final concentration (nM) | Added water and siRNA |
|---|---|
| / | / |
| 40 | 4 µL siRNA (20 µM) + 96 µL H2O |
| 13.33333333 | 30 µL siRNA + 60 µL H2O |
| 4.444444444 | 30 µL siRNA + 60 µL H2O |
| 1.481481481 | 30 µL siRNA + 60 µL H2O |
| 0.49382716 | 30 µL siRNA + 60 µL H2O |
| 0.164609053 | 30 µL siRNA + 60 µL H2O |
| 0.054869684 | 30 µL siRNA + 60 µL H2O |
| 0.018289895 | 30 µL siRNA + 60 µL H2O |
| 0.006096632 | 30 µL siRNA + 60 µL H2O |
| 0.002032211 | 30 µL siRNA + 60 µL H2O |
| 0.000677404 | 30 µL siRNA + 60 µL H2O |

24 h after transfection, assays were carried out according to the instructions of the Dual-Glo® Luciferase Assay System kit. The Dual-Glo® Luciferase Assay System assays were carried out using the dual luciferase reporter gene assay kit (Promega, cat.E2940), and the Firefly chemiluminescence values and Renilla chemiluminescence values were read. The relative values were calculated as Ren/Fir, and the inhibition (%) was calculated as 1 - (Ratio + siRNA / Ratioreporter only) × 100%.

In the present disclosure, the proportion of remaining expression of mRNA = 100% - inhibition (%).

**Example 4. On-Target and Off-Target Activity Experiments of siRNAs Comprising Different Chemical Modifications**

The following siRNAs were synthesized using the compounds of Example 1 and the method of Example 2, and the on-target activity and off-target activity of each siRNA were verified using the method of Example 3. The siRNAs had identical sense strands and comprised the following modified nucleotides/chemical modifications, respectively, in position 7 of the 5' end of the antisense strand:

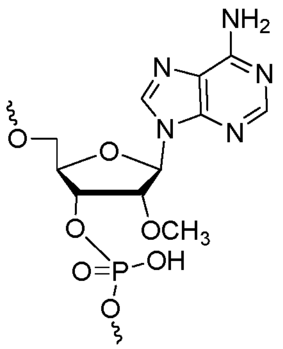

**Am**

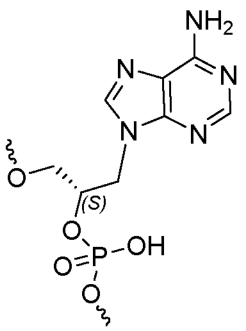

**GNA (A)**

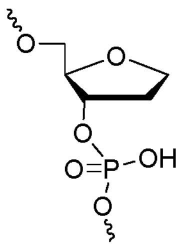

**Abasic**

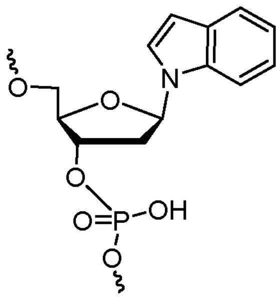

**Id**

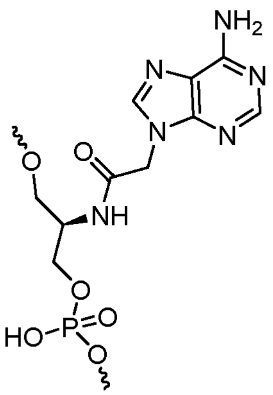

TJ-NA009(A)

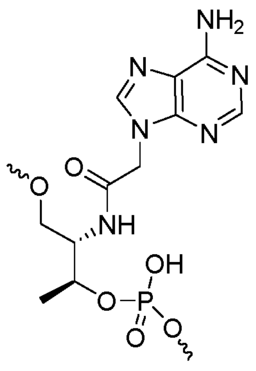

D-aTNA
TJ-NA019(A)

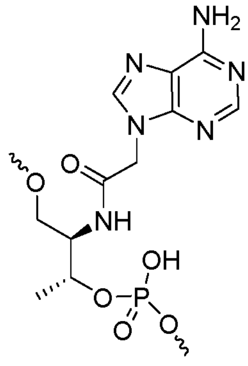

L-aTNA
TJ-NA020(A)

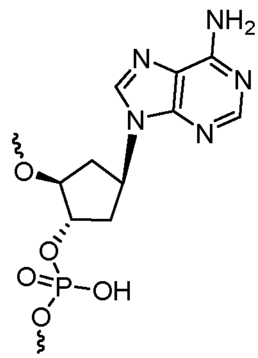

TJ-NA026(A)

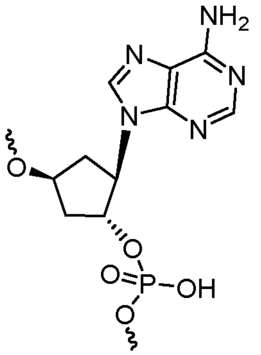

TJ-NA027(A)

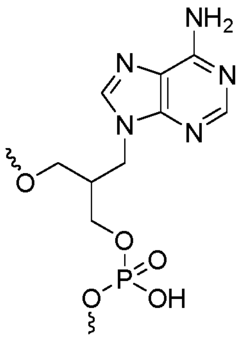

(-)hmpNA(A)

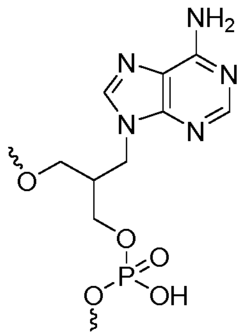

(+)hmpNA(A)

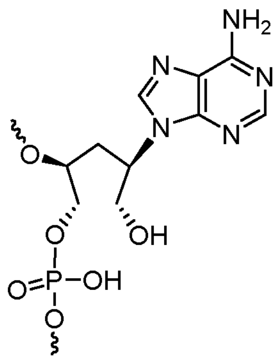

TJ-NA038(A)

wherein the nucleotide synthesized using 2-hydroxymethyl-1,3-propanediol as the starting material was defined as hmpNA;

TJ-NA019(A) was obtained by solid-phase synthesis using the nucleoside phosphoramidite monomer 1-2 of example section 1.1;

TJ-NA020(A) was obtained by solid-phase synthesis using the nucleoside phosphoramidite monomer 1-3 of example section 1.1;

TJ-NA026(A) was obtained by solid-phase synthesis using the nucleoside phosphoramidite monomer 1-4a of example section 1.1;

TJ-NA027(A) was obtained by solid-phase synthesis using the nucleoside phosphoramidite monomer 1-4b of example section 1.1;

TJ-NA038(A) was obtained by solid-phase synthesis using the nucleoside phosphoramidite monomer 1-5 of example section 1.1;

(+)hmpNA(A) was obtained by solid-phase synthesis using the nucleoside phosphoramidite monomer 1-1b of example section 1.1, and its absolute configuration was (S)-hmpNA(A);

(-)hmpNA(A) was obtained by solid-phase synthesis using the nucleoside phosphoramidite monomer 1-1a of example section 1.1, and its absolute configuration was (R)-hmpNA(A).

Similarly, the following structures were obtained by solid-phase synthesis and by changing the base species of hmpNA, and their absolute configurations were determined:

(+)hmpNA(G), with the absolute configuration (S)-hmpNA(G);

(-)hmpNA(G), with the absolute configuration (R)-hmpNA(G), obtained by solid-phase synthesis using the nucleoside phosphoramidite monomer 1-6a of example section 1.6;

(+)hmpNA(C), with the absolute configuration (S)-hmpNA(C);

(-)hmpNA(C), with the absolute configuration (R)-hmpNA(C), obtained by solid-phase synthesis using the nucleoside phosphoramidite monomer 1-8a of example section 1.8;

(+)hmpNA(U), with the absolute configuration (R)-hmpNA(U); and
(-)hmpNA(U), with the absolute configuration (S)-hmpNA(U), obtained by solid-phase synthesis using the nucleoside phosphoramidite monomer 1-7a of example section 1.7.

The absolute configurations (S)-hmpNA(G), (R)-hmpNA(G), (S)-hmpNA(C), (R)-hmpNA(C), (S)-hmpNA(U) and (R)-hmpNA(U) are determined from their intermediate or derivative by X-Ray diffraction.

The structure of the intermediate or derivative is:

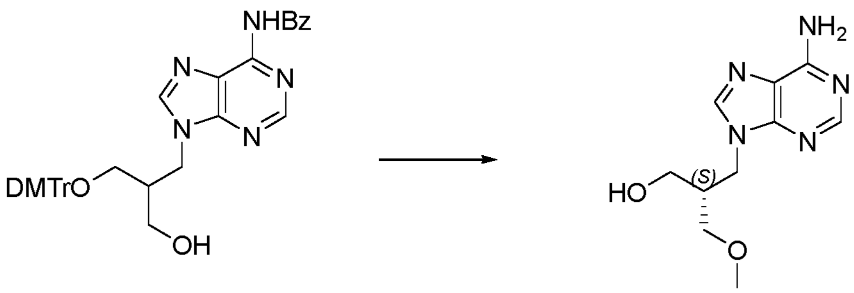

**15.1 6B(+)** **TJ-NA067**

**TJ-NA067:** determined as a colorless massive crystal (0.30 × 0.10 × 0.04 mm3), belonging to the monoclinic crystal system with a P21 space group. Lattice parameter a = 16.0496(5) Å, b = 4.86260(10) Å, c = 16.4686(5) Å, $\alpha$ = 90°, $\beta$ = 118.015(4)°, $\gamma$ = 90°, V = 1134.65(7) Å3, Z = 4. Calculated density Dc = 1.389 g/cm3; the number of electrons in a unit cell F(000) = 504.0; linear absorption coefficient of a unit cell $\mu$ (Cu K$\alpha$) = 0.840 mm-1; diffraction experiment temperature T = 150.00(11) K.

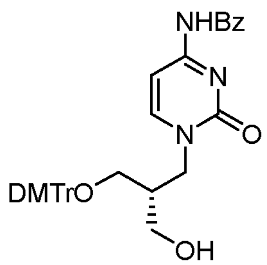

**15.8 6A (+)**

**6A(+)**: determined as a colorless massive crystal (0.30 × 0.20 × 0.10 mm3), belonging to the monoclinic crystal system with a P21 space group. Lattice parameter a = 22.6688(7) Å, b = 8.5595(2) Å, c = 23.3578(5) Å, $\alpha$ = 90°, $\beta$ = 113.876(3)°, $\gamma$ = 90°, V = 4144.3(2) Å3, Z = 2. Calculated density Dc = 0.999 g/cm3; the number of electrons in a unit cell F(000) = 1318.0; linear absorption coefficient of a unit cell $\mu$ (Cu K$\alpha$) = 0.570 mm-1; diffraction experiment temperature T = 100.01(18) K.

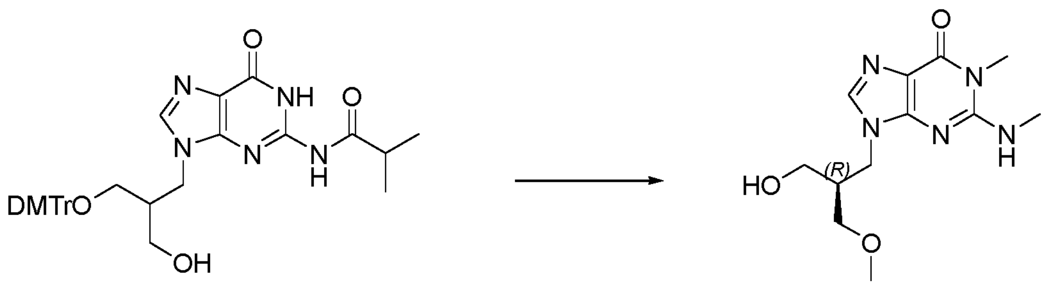

**15.6 7A (-)** **TJ-NA068**

**TJ-NA048:** determined as a colorless acicular crystal (0.30 × 0.04 × 0.04 mm3), belonging to the monoclinic crystal system with a P1 space group. Lattice parameter a = 7.6165(4) Å, b = 11.3423(5) Å, c = 17.3991(8) Å, $\alpha$ =

85.007(4)°, $\beta$ = 88.052(4)°, $\gamma$ = 70.532(4)°, V = 1411.75(12) Å3, Z = 2. Calculated density Dc = 1.366 g/cm3; the number of electrons in a unit cell F(000) = 620.0; linear absorption coefficient of a unit cell $\mu$ (Cu K$\alpha$) = 0.856 mm-1; diffraction experiment temperature T = 150.00(13) K.

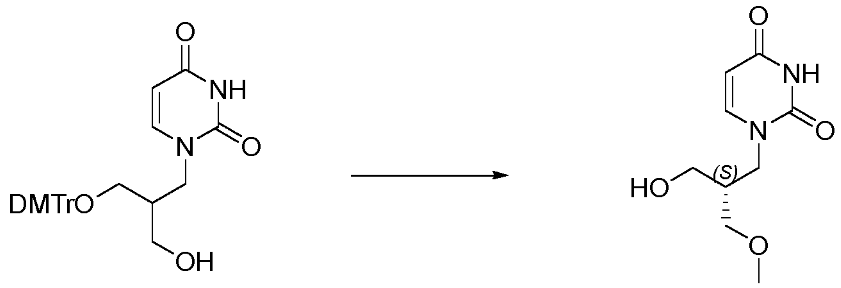

**15.7 6A (-)** **TJ-NA092**

**TJ-NA092:** determined as a colorless prismatic crystal (0.30 × 0.10 × 0.10 mm3), belonging to the triclinic crystal system with a P1 space group. Lattice parameter a = 5.17960(10) Å, b = 8.0667(2) Å, c = 12.4077(2) Å, $\alpha$ = 93.146(2)°, $\beta$ = 101.266(2)°, $\gamma$ = 96.134(2)°, V = 503.993(18) Å3, Z = 2. Calculated density Dc = 1.412 g/cm3; the number of electrons in a unit cell *F*(000) = 228.0; linear absorption coefficient of a unit cell $\mu$ (Cu K$\alpha$) = 0.945 mm-1; diffraction experiment temperature T = 100.00(10) K.

Table 5. HBV-S-targeting siRNA sequences and modifications

| | SEQ ID NO | SS strand 5'-3' |
|---|---|---|
| | SEQ ID NO:1 | UmsGmsAmCmAfAmGfAfAfUmCmCmUmCmAmCmAmAmUm |
| Double strand code | | AS strand 5'-3' |
| TRD4389 parent sequence | SEQ ID NO:2 | AmsUfsUmGmUmGfAmGmGmAmUmUmCmUfUmGfUmCmAmsAmsCm |
| TRD5252 | SEQ ID NO:3 | AmsUfsUmGmUmGf**GNA(A)**GmGmAmUmUmCmUfUmGfUmCmAmsAmsCm |
| TRD5812 | SEQ ID NO:4 | AmsUfsUmGmUmGf**Abasic**GmGmAmUmUmCmUfUmGfUmCmAmsAmsCm |
| TRD5813 | SEQ ID NO:5 | AmsUfsUmGmUmGf**Id**GmGmAmUmUmCmUfUmGfUmCmAmsAmsCm |
| TRD5816 | SEQ ID NO:6 | AmsUfsUmGmUmGf**TJ-NA009(A)**GmGmAmUmUmCmUfUmGfUmCmAmsAmsCm |
| TRD5817 | SEQ ID NO:7 | AmsUfsUmGmUmGf**TJ-NA019(A)**GmGmAmUmUmCmUfUmGfUmCmAmsAmsCm |
| TRD5818 | SEQ ID NO:8 | AmsUfsUmGmUmGf**TJ-NA020(A)**GmGmAmUmUmCmUfUmGfUmCmAmsAmsCm |
| TRD5821 | SEQ ID NO:9 | AmsUfsUmGmUmGf**TJ-NA027(A)**GmGmAmUmUmCmUfUmGfUmCmAmsAmsCm |
| TRD5822 | SEQ ID NO:10 | AmsUfsUmGmUmGf**(+)hmpNA(A)**GmGmAmUmUmCmUfUmGfUmCmAmsAmsCm |
| TRD5823 | SEQ ID NO:11 | AmsUfsUmGmUmGf**(-)hmpNA(A)**GmGmAmUmUmCmUfUmGfUmCmAmsAmsCm |
| TRD5825 | SEQ ID NO:12 | AmsUfsUmGmUmGf**TJ-NA038(A)**GmGmAmUmUmCmUfUmGfUmCmAmsAmsCm |

The experimental results of on-target activity are shown in Table 6, and the experimental results of off-target activity are shown in Table 7 and FIGs. 1A-1L. The test sequences with the compounds of the current experiment all showed activity similar to or slightly better than that of the parent sequence, which indicates that the modifications did not affect on-target activity. The siRNAs comprising GNA/Abasic/Id, TJ-NA019**(A)**, TJ-NA020**(A)**, TJ-NA026**(A)**, **(+)hmpNA(A)** and **(-)hmpNA(A)** had the best activity. In addition, the parent sequence had significant off-target activity, and all the modifications showed significant inhibitory effects against off-target activity. Particularly, in the siRNAs comprising TJ-NA027**(A)**, **(+)hmpNA(A)** and **(-)hmpNA(A),** no off-target activity was observed.

Table 6. On-target activity results of HBV-S-targeting siRNAs

| Double strand code | Remaining percentage of target gene's mRNA (on-target activity) ex pression (mean) | | | | | | | | | | | |
|---|---|---|---|---|---|---|---|---|---|---|---|---|
| | 40nM | 13.3 nM | 4.44 nM | 1.48 nM | 0.493 nM | 0.16 4nM | 0.05 4nM | 0.018 2nM | 0.006 09nM | 0.002 03nM | 0.000 67nM | $IC_{50}$ value (nM) |
| TRD 4389 | 5.4% | 4.1% | 4.8% | 4.8% | 8.4% | 21.7% | 53.0% | 82.5% | 104.9% | 99.4% | 95.2% | 0.0589 |
| TRD 5252 | 3.4% | 3.1% | 3.1% | 3.6% | 5.7% | 11.1% | 22.1% | 44.7% | 72.8% | 92.2% | 86.6% | 0.0162 |
| TRD 5812 | 3.8% | 3.0% | 3.4% | 3.6% | 7.3% | 9.8% | 23.5% | 44.8% | 63.9% | 90.4% | 81.4% | 0.0158 |
| TRD 5813 | 5.1% | 3.8% | 4.4% | 4.3% | 6.1% | 13.2% | 33.5% | 53.8% | 74.5% | 80.8% | 96.4% | 0.0214 |
| TRD 5816 | 3.9% | 3.8% | 3.4% | 4.9% | 6.9% | 16.1% | 39.8% | 71.8% | 96.3% | 92.9% | 108.1% | 0.0389 |
| TRD 5817 | 4.8% | 4.2% | 4.5% | 3.7% | 6.6% | 13.7% | 31.0% | 61.0% | 81.8% | 92.9% | 103.7% | 0.0251 |
| TRD 5818 | 3.7% | 3.3% | 3.1% | 3.7% | 6.1% | 10.9% | 26.3% | 55.8% | 69.1% | 87.4% | 88.8% | 0.0195 |
| TRD 5820 | 4.4% | 3.8% | 3.5% | 3.7% | 4.2% | 9.5% | 22.8% | 49.2% | 79.4% | 93.2% | 91.7% | 0.0191 |
| TRD 5821 | 6.8% | 5.2% | 5.7% | 6.1% | 8.7% | 19.7% | 39.3% | 69.9% | 102.8% | 92.9% | 97.9% | 0.0398 |
| TRD 5822 | 4.4% | 4.5% | 4.1% | 3.7% | 5.3% | 13.2% | 24.6% | 51.2% | 82.3% | 84.9% | 101.9% | 0.0200 |
| TRD 5823 | 3.6% | 3.8% | 3.4% | 3.4% | 5.2% | 11.0% | 29.7% | 58.3% | 71.4% | 84.7% | 100.7% | 0.0200 |
| TRD 5825 | 4.3% | 3.6% | 3.4% | 4.2% | 7.1% | 18.0% | 32.7% | 66.0% | 88.7% | 93.8% | 103.2% | 0.0302 |

Table 7. Off-target activity results of HBV-S-targeting siRNAs

| Double strand code | Remaining percentage of target gene's mRNA (off-target activity) expression (mean) | | | | | | | | | | |
|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM |
| TRD 4389 | 57.4% | 55.9% | 65.5% | 73.3% | 89.2% | 92.8% | 105.3% | 102.4% | 107.6% | 96.0% | 101.2% |
| TRD 5252 | 97.3% | 100.4% | 104.0% | 108.1% | 107.3% | 102.9% | 108.7% | 94.9% | 101.2% | 101.8% | 97.7% |
| TRD 5812 | 98.2% | 107.0% | 99.1% | 100.7% | 110.1% | 125.2% | 113.7% | 105.3% | 105.5% | 99.5% | 93.8% |
| TRD 5813 | 100.5% | 105.2% | 95.9% | 112.1% | 102.3% | 104.3% | 101.5% | 97.2% | 110.7% | 100.6% | 93.6% |
| TRD 5816 | 108.3% | 101.5% | 97.2% | 109.5% | 116.7% | 122.8% | 108.5% | 113.2% | 121.6% | 112.9% | 106.8% |
| TRD 5817 | 104.5% | 106.7% | 110.0% | 109.3% | 119.4% | 120.9% | 127.3% | 113.6% | 117.7% | 112.2% | 105.0% |
| TRD 5818 | 83.7% | 89.7% | 83.0% | 91.0% | 117.5% | 79.4% | 99.1% | 103.4% | 89.2% | 92.9% | 98.7% |
| TRD 5820 | 92.1% | 100.3% | 104.3% | 98.9% | 103.6% | 103.8% | 106.2% | 108.3% | 105.8% | 100.3% | 97.7% |
| TRD 5821 | 102.9% | 99.3% | 98.3% | 99.6% | 106.8% | 106.4% | 108.7% | 108.1% | 104.5% | 95.4% | 107.8% |
| TRD 5822 | 106.1% | 93.8% | 81.6% | 100.4% | 100.4% | 96.9% | 105.3% | 101.9% | 94.6% | 101.4% | 94.0% |
| TRD 5823 | 91.8% | 89.1% | 92.9% | 99.8% | 97.8% | 101.1% | 90.7% | 92.6% | 97.9% | 95.9% | 87.1% |
| TRD 5825 | 84.9% | 89.7% | 97.7% | 106.7% | 103.9% | 104.7% | 100.0% | 100.9% | 90.2% | 112.7% | 98.3% |

**Example 5. Sequence-Dependence Experiment of siRNAs Comprising Different Chemical Modifications**

The Abasic modification is known to be siRNA sequence-dependent, so the inventors tested the test compounds of the present disclosure on multiple different sequences. siRNAs targeting the mRNAs of four different genes (ANGPTL3, HBV-S, HBV-X and TTR) (their sequences are shown in Table 8) were used and modified in position 7 of the 5' end of the AS strand with the compounds of Example 1: TJ-NA020(A), TJ-NA027**(A)**, **(+)hmpNA(A), (-)hmpNA(A),** GNA**(A)** (as a control), and Id compound (the sequences are shown in Table 9), and were compared to the parent sequences with respect of on-target activity and off-target activity.

Table 8. Sequences of siRNAs targeting different genes

| **siRNA target gene** | SEQ ID NO | SS strand 5'-3' | SEQ ID NO | AS strand 5'-3' |
|---|---|---|---|---|
| ANGPTL3 (siRNA1) | SEQ ID NO: 13 | GmsAmsAmCmUfAmCfUfCfCmCmUmUmUmCmUmUmCmAm | SEQ ID NO: 14 | UmsGfsAmAfGmAfAmAmGfGmGmAfGmUfAmGfUmUfCmsUmsUm |
| HBV-S (siRNA2) | SEQ ID NO: 15 | CmsCmsAmUmUfUmGfUfUfCmAmGmUmGmGmUmUmCmsGm | SEQ ID NO: 16 | UmsGfsAmAmCmCfAmCmUmGmAmAmCmAfAmAfUmGmGmsCmsAm |
| HBV-X (siRNA3) | SEQ ID NO: 17 | CmsAmsCmCmUfCmUfGfCfAmCmGmUmCmGmCmAmUmsGm | SEQ ID NO: 18 | UmsAfsUmGfCmGfAmCmGfUmGmCfAmGfAmGfGmUfGmsAmsAm |
| TTR (siRNA4) | SEQ ID NO: 19 | CmsAmsGmUmGfUmUfCfUfUmGmCmUmCmUmAmUmAmAm | SEQ ID NO: 20 | UmsUfsAmUfAmGfAmGmCfAmAmGfAmAfCmAfCmUfGmsUmsUm |

Table 9. Sequences of siRNAs targeting different genes and comprising chemical modifications

| Target mRNA | siRNA | SEQ ID NO | AS strand modification |
|---|---|---|---|
| ANGPT L3 | TRD5840 | SEQ ID NO:21 | UmsGfsAmAfGmAfAmAmGfGmGmAfGmUfAmGfUmUfCmsUmsUm |
| | TRD5841 | SEQ ID NO:22 | UmsGfsAmAfGmAfGNA(A)AmGfGmGmAfGmUfAmGfUmUfCmsUmsUm |
| | TRD5842 | SEQ ID NO:23 | UmsGfsAmAfGmAfIdAmGfGmGmAfGmUfAmGfUmUfCmsUmsUm |
| | TRD5843 | SEQ ID NO:24 | UmsGfsAmAfGmAfTJ-020**(A)**AmGfGmGmAfGmUfAmGfUmUfCmsUmsUm |
| | TRD5844 | SEQ ID NO:25 | UmsGfsAmAfGmAfTJ-027**(A)**AmGfGmGmAfGmUfAmGfUmUfCmsUmsUm |
| | TRD5845 | SEQ ID NO:26 | UmsGfsAmAfGmAf**(+)hmpNA(A)**AmGfGmGmAfGmUfAmGfUmUfCmsUmsUm |
| | TRD5846 | SEQ ID NO:27 | UmsGfsAmAfGmAf**(-)hmpNA(A)**AmGfGmGmAfGmUfAmGfUmUfCmsUmsUm |

(continued)

| Target mRNA | siRNA | SEQ ID NO | AS strand modification |
|---|---|---|---|
| HBV-S | TRD5847 | SEQ ID NO:28 | UmsGfsAmAmCmCfAmCmUmGmAmAmCmAfAmAfUmGmGmsCmsAm |
| | TRD5848 | SEQ ID NO:29 | UmsGfsAmAmCmCfGNA(A)CmUmGmAmAmCmAfAmAfUmGmGmsCmsAm |
| | TRD5849 | SEQ ID NO:30 | UmsGfsAmAmCmCfIdCmUmGmAmAmCmAfAmAfUmGmGmsCmsAm |
| | TRD5850 | SEQ ID NO:31 | UmsGfsAmAmCmCfTJ-020**(A)**CmUmGmAmAmCmAfAmAfUmGmGmsCmsAm |
| | TRD5851 | SEQ ID NO:32 | UmsGfsAmAmCmCfTJ-027**(A)**CmUmGmAmAmCmAfAmAfUmGmGmsCmsAm |
| | TRD5852 | SEQ ID NO:33 | UmsGfsAmAmCmCf**(+)hmpNA(A)**CmUmGmAmAmCmAfAmAfUmGmGmsCmsAm |
| | TRD5853 | SEQ ID NO:34 | UmsGfsAmAmCmCf**(-)hmpNA(A)**CmUmGmAmAmCmAfAmAfUmGmGmsCmsAm |
| HBV-X | TRD5854 | SEQ ID NO:35 | UmsAfsUmGfCmGfAmCmGfUmGmCfAmGfAmGfGmUfGmsAmsAm |
| | TRD5855 | SEQ ID NO:36 | UmsAfsUmGfCmGfGNA(A)CmGfUmGmCfAmGfAmGfGmUfGmsAmsAm |
| | TRD5856 | SEQ ID NO:37 | UmsAfsUmGfCmGfIdCmGfUmGmCfAmGfAmGfGmUfGmsAmsAm |
| | TRD5857 | SEQ ID NO:38 | UmsAfsUmGfCmGfTJ-020**(A)**CmGfUmGmCfAmGfAmGfGmUfGmsAmsAm |
| | TRD5858 | SEQ ID NO:39 | UmsAfsUmGfCmGfTJ-027**(A)**CmGfUmGmCfAmGfAmGfGmUfGmsAmsAm |
| | TRD5859 | SEQ ID NO:40 | UmsAfsUmGfCmGf**(+)hmpNA(A)**CmGfUmGmCfAmGfAmGfGmUfGmsAmsAm |
| | TRD5860 | SEQ ID NO:41 | UmsAfsUmGfCmGf**(-)hmpNA(A)**CmGfUmGmCfAmGfAmGfGmUfGmsAmsAm |

(continued)

| Target mRNA | siRNA | SEQ ID NO | AS strand modification |
|---|---|---|---|
| TTR | TRD5861 | SEQ ID NO:42 | UmsUfsAmUfAmGfAmGmCfAmAmGfAmAfCmAfCmUfGmsUmsUm |
| | TRD5862 | SEQ ID NO:43 | UmsUfsAmUfAmGfGNA(A)GmCfAmAmGfAmAfCmAfCmUfGmsUmsUm |
| | TRD5863 | SEQ ID NO:44 | UmsUfsAmUfAmGfIdGmCfAmAmGfAmAfCmAfCmUfGmsUmsUm |
| | TRD5864 | SEQ ID NO:45 | UmsUfsAmUfAmGfTJ-020**(A)**GmCfAmAmGfAmAfCmAfCmUfGmsUmsUm |
| | TRD5865 | SEQ ID NO:46 | UmsUfsAmUfAmGfTJ-027**(A)**GmCfAmAmGfAmAfCmAfCmUfGmsUmsUm |
| | TRD5866 | SEQ ID NO:47 | UmsUfsAmUfAmGf**(+)hmpNA(A)**GmCfAmAmGfAmAfCmAfCmUfGmsUmsUm ( |
| | TRD5867 | SEQ ID NO:48 | UmsUfsAmUfAmGf**(-)hmpNA(A)**GmCfAmAmGfAmAfCmAfCmUfGmsUmsUm |

The results of the on-target activity experiment are shown in Table 10. GNA**(A)** showed significant sequence dependence, and different sequences had significantly different on-target activity. The test compounds of the present disclosure did not show significant sequence dependence, which indicates that they are more universally applicable. Moreover, making only a 2'-F modification in position 9 of the 5' end of the AS strand and only a 2'-OMe modification in position 10 resulted in similar activity-that is, the test compounds of the present disclosure did not show significant sequence dependence.

Table 10. On-target activity results of siRNAs for different target sequences

| Double strand code | Remaining percentage of target gene's mRNA (on-target activity) ex pression (mean) | | | | | | | | | | | |
|---|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM | IC50 value (nM) |
| TRD 5840 | 28.0% | 24.2% | 30.9% | 52.8% | 48.9% | 86.7% | 92.7% | 89.8% | 92.4% | 95.8% | 102.4% | 0.8710 |
| TRD 5841 | 57.5% | 51.1% | 55.5% | 68.3% | 76.5% | 85.9% | 82.9% | 87.8% | 81.5% | 64.0% | 97.8% | >40 |
| TRD 5842 | 39.4% | 43.7% | 41.7% | 70.8% | 82.5% | 99.1% | 99.1% | 92.1% | 98.8% | 95.6% | 92.7% | 3.1623 |
| TRD 5843 | 28.7% | 30.0% | 33.3% | 50.4% | 78.8% | 75.0% | 76.0% | 107.4% | 95.7% | 92.0% | 94.0% | 1.6218 |
| TRD 5844 | 38.6% | 36.3% | 44.9% | 59.8% | 76.2% | 104.5% | 111.5% | 105.4% | 110.6% | 103.6% | 114.3% | 2.3442 |
| TRD 5845 | 28.2% | 30.5% | 41.7% | 55.0% | 63.9% | 78.0% | 77.1% | 84.1% | 95.8% | 83.2% | 91.9% | 1.9953 |
| TRD 5846 | 31.6% | 26.8% | 34.1% | 59.1% | 84.8% | 102.1% | 97.2% | 108.9% | 95.6% | 107.2% | 102.1% | 1.9055 |
| TRD 5847 | 9.3% | 7.2% | 6.3% | 8.5% | 17.9% | 47.2% | 80.6% | 94.7% | 100.5% | 106.1% | 110.6% | 0.1380 |
| TRD 5848 | 46.5% | 35.1% | 26.6% | 36.0% | 67.3% | 76.3% | 88.4% | 104.1% | 91.6% | 95.1% | 98.1% | 0.7943 |
| TRD 5849 | 24.8% | 16.7% | 13.7% | 20.9% | 41.0% | 71.6% | 95.5% | 98.2% | 93.1% | 104.3% | 113.3% | 0.3311 |
| TRD 5850 | 19.7% | 14.2% | 12.8% | 15.5% | 29.3% | 54.3% | 84.2% | 87.6% | 86.6% | 90.0% | 95.2% | 0.2042 |
| TRD 5851 | 22.9% | 15.5% | 12.6% | 20.2% | 38.6% | 70.0% | 88.4% | 102.3% | 106.6% | 101.0% | 101.9% | 0.3020 |
| TRD 5852 | 24.7% | 17.5% | 13.1% | 21.1% | 40.5% | 64.1% | 84.3% | 94.5% | 88.4% | 100.2% | 95.1% | 0.2951 |
| TRD 5853 | 17.5% | 11.5% | 9.9% | 13.5% | 30.3% | 54.5% | 74.6% | 86.3% | 90.3% | 91.0% | 84.1% | 0.1905 |
| TRD 5854 | 37.9% | 32.4% | 35.3% | 50.3% | 70.6% | 89.7% | 98.8% | 101.1% | 106.1% | 99.6% | 114.7% | 1.3804 |
| TRD 5855 | 41.3% | 40.7% | 36.9% | 73.6% | 71.7% | 87.0% | 89.0% | 85.8% | 94.9% | 104.4% | 101.6% | 4.2658 |
| TRD 5856 | 38.6% | 37.8% | 35.8% | 59.5% | 72.7% | 92.3% | 92.5% | 85.2% | 102.1% | 93.1% | 102.1% | 2.0417 |
| TRD 5857 | 38.5% | 34.4% | 35.6% | 45.6% | 66.8% | 81.4% | 82.7% | 84.7% | 85.6% | 95.0% | 103.3% | 1.1749 |
| TRD 5858 | 25.0% | 24.3% | 26.0% | 38.1% | 59.3% | 75.4% | 86.5% | 104.8% | 93.8% | 92.4% | 94.7% | 0.7244 |
| TRD 5860 | 43.5% | 37.1% | 34.1% | 50.8% | 77.6% | 88.5% | 86.6% | 100.0% | 95.1% | 97.8% | 110.8% | 1.5488 |
| TRD 5861 | 3.8% | 2.5% | 1.7% | 2.2% | 5.5% | 20.6% | 43.0% | 64.4% | 96.7% | 105.0% | 92.9% | 0.0407 |
| TRD 5862 | 1.2% | 1.3% | 1.1% | 1.3% | 3.8% | 12.7% | 36.6% | 85.4% | 101.8% | 97.8% | 117.2% | 0.0417 |
| TRD 5863 | 1.7% | 1.4% | 1.2% | 1.7% | 5.1% | 18.9% | 45.7% | 75.5% | 92.5% | 106.3% | 106.7% | 0.0447 |
| TRD 5864 | 1.1% | 1.2% | 0.9% | 1.4% | 2.3% | 7.2% | 27.9% | 52.4% | 84.7% | 90.8% | 100.0% | 0.0219 |

(continued)

| Double strand code | Remaining percentage of target gene's mRNA (on-target activity) ex pression (mean) | | | | | | | | | | | |
|---|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM | IC50 value (nM) |
| TRD 5865 | 3.2% | 2.3% | 1.7% | 1.9% | 3.1% | 11.9% | 36.3% | 64.4% | 96.0% | 97.2% | 93.2% | 0.0331 |
| TRD 5866 | 1.2% | 1.2% | 1.3% | 1.4% | 3.4% | 12.7% | 32.2% | 67.7% | 87.7% | 97.4% | 103.4% | 0.0309 |
| TRD 5867 | 1.8% | 1.5% | 1.3% | 1.3% | 2.2% | 8.8% | 27.3% | 56.0% | 85.3% | 86.9% | 108.5% | 0.0224 |

The experimental results of the off-target activity of siRNA2 and siRNA3 are shown in Table 11, FIGs. 2A-2G (targeting HBV-S) and FIGs. 3A-3G (targeting HBV-X). It can be seen that the test compounds of the present disclosure significantly reduced the off-target activity of siRNA relative to the parent sequences. Moreover, making only a 2'-F modification in position 9 of the 5' end of the AS strand and only a 2'-OMe modification in position 10 resulted in similar off-target activity-that is, the modifications can similarly reduce the off-target activity of siRNA significantly.

Table 11. Off-target activity results of siRNAs for different target sequences

| Double strand code | Remaining percentage of target gene's mRNA (off-target activity) expression (mean) | | | | | | | | | | |
|---|---|---|---|---|---|---|---|---|---|---|---|
| | 40nM | 13.3 nM | 4.44 nM | 1.48 nM | 0.493 nM | 0.164 nM | 0.054 nM | 0.0182 nM | 0.006 09nM | 0.002 03nM | 0.000 67nM |
| TRD 5847 | 51.2% | 47.6% | 47.5% | 66.7% | 77.8% | 81.8% | 93.2% | 93.3% | 93.1% | 96.5% | 85.7% |
| TRD 5848 | 99.9% | 96.7% | 101.6% | 100.6% | 91.6% | 107.0% | 96.7% | 100.7% | 95.4% | 101.9% | 113.0% |
| TRD 5849 | 77.3% | 77.6% | 69.3% | 87.2% | 90.7% | 83.1% | 85.4% | 95.2% | 94.1% | 94.0% | 108.0% |
| TRD 5850 | 86.3% | 90.2% | 92.1% | 92.9% | 89.8% | 99.3% | 98.6% | 96.0% | 95.8% | 98.0% | 103.5% |
| TRD 5851 | 84.9% | 85.0% | 87.7% | 84.8% | 86.8% | 88.7% | 92.1% | 83.2% | 91.5% | 84.8% | 104.1% |
| TRD 5852 | 81.8% | 83.1% | 79.0% | 89.9% | 91.3% | 98.2% | 99.3% | 96.7% | 109.6% | 94.0% | 99.8% |
| TRD 5853 | 86.4% | 87.2% | 91.4% | 92.9% | 91.9% | 99.7% | 87.0% | 81.0% | 89.0% | 86.8% | 91.3% |
| TRD 5854 | 36.9% | 32.7% | 36.1% | 39.8% | 62.9% | 81.3% | 87.6% | 87.0% | 95.8% | 93.6% | 99.8% |
| TRD 5855 | 71.1% | 78.2% | 81.6% | 92.0% | 91.0% | 94.1% | 87.3% | 93.6% | 99.4% | 119.9% | 96.6% |
| TRD 5856 | 89.7% | 100.1% | 96.5% | 106.1% | 112.7% | 124.4% | 117.5% | 122.3% | 117.5% | 120.1% | 112.6% |
| TRD 5857 | 84.9% | 69.5% | 86.0% | 79.6% | 87.1% | 91.1% | 96.1% | 87.8% | 104.8% | 95.1% | 95.2% |
| TRD 5858 | 73.9% | 82.8% | 92.5% | 95.4% | 107.5% | 97.5% | 99.1% | 96.1% | 94.1% | 101.8% | 99.8% |
| TRD 5859 | 79.8% | 81.0% | 86.0% | 96.4% | 101.9% | 98.8% | 99.8% | 118.4% | 101.3% | 93.3% | 103.2% |
| TRD 5860 | 78.4% | 75.6% | 80.6% | 86.1% | 83.2% | 95.9% | 91.6% | 91.5% | 95.6% | 97.3% | 98.6% |

## II. Preparation and Activity Evaluation of Targeting Ligands

Table 12. Main instrument models and sources of starting materials for preparing targeting ligands

| Main instrument models and sources of starting materials | | |
|---|---|---|
| Name | Company | Catalog number/model |
| Solid-phase synthesizer | Dr.Oligo 48 | Biolytic |
| HPLC | Agilent 1260 Infinity II | Agilent |
| Mass spectrometer | Waters Acquity UPLC | Waters |
| Nucleoside phosphoramidite monomer starting material | | Hongene Biotech |

### Example 6. Galactosamine Compound 1-t Linked to Solid-Phase Support

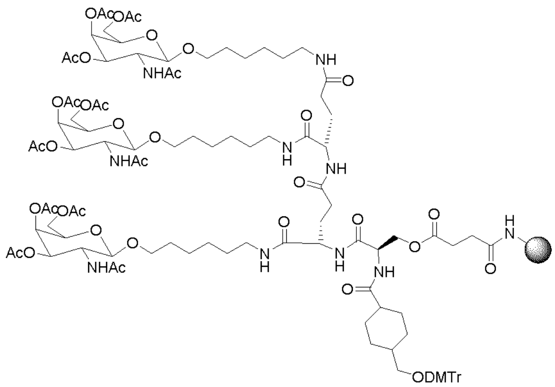

The synthesis schemes are as follows:

1) Scheme of synthesis of compound **1-g**

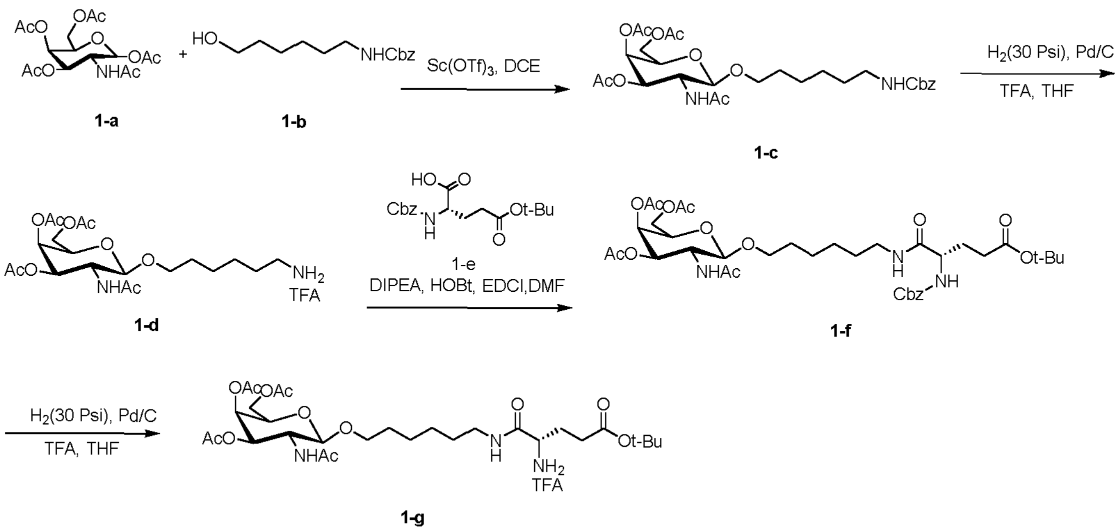


1-a 1-b 1-c 1-d 1-f 1-g

2) Scheme of synthesis of compound **1-h**

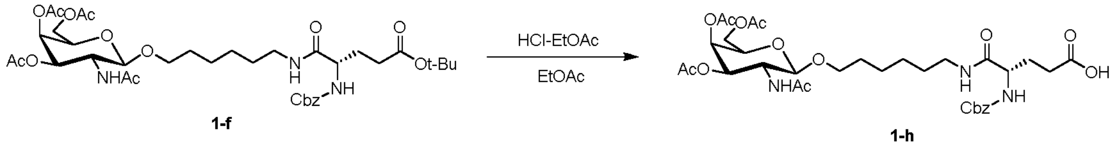


1-f 1-h

3) Scheme of synthesis of compound **1-1**

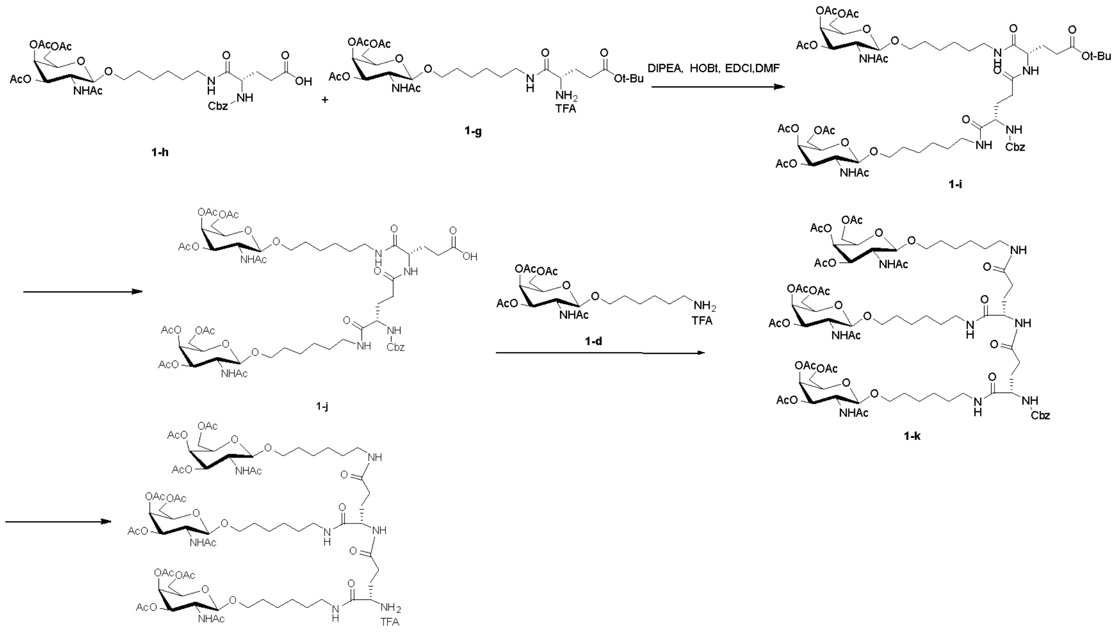


1-h 1-g 1-i 1-j 1-d 1-k 1-l

4) Synthesis of compound **1-q**

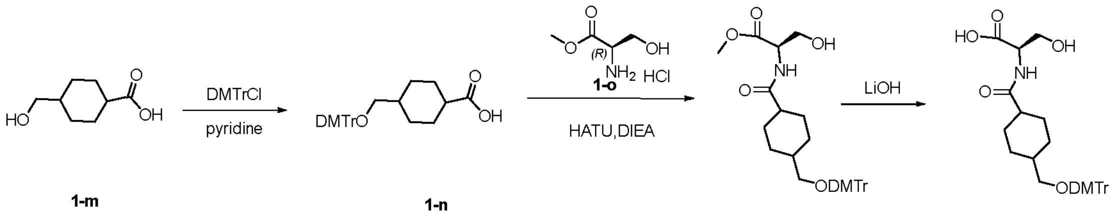


1-m 1-n 1-o 1-p 1-q

5) Synthesis of galactosamine compound 1-t linked to solid-phase support

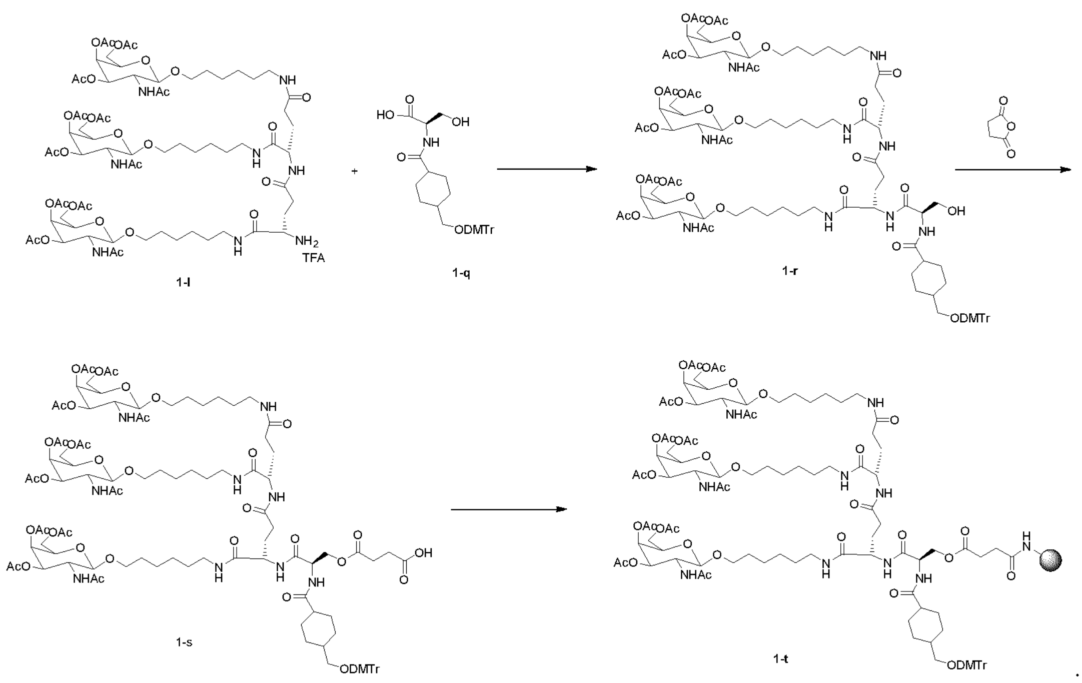

1-l 1-q 1-r 1-s 1-t

Step 1

The starting material **1-a** (297 g, 763 mmol) and the starting material **1-b** (160 g, 636 mmol) were dissolved in 960 mL of DCE. $Sc(OTf)_3$ (15.6 g, 31.8 mmol) was added at 15 °C. Then the reaction mixture was heated to 85 °C and stirred for 2 h. After the reaction was complete, 1.5 L of saturated $NaHCO_3$ was added to terminate the reaction. The organic phase was separated, washed with 1.5 L of saturated brine, dried over anhydrous $Na_2SO_4$ and filtered. The filtrate was distilled under reduced pressure and purified by silica gel column chromatography (petroleum ether:ethyl acetate = 5:1 to 0:1) to give product **1-c** as a light yellow oil (328 g, 544 mmol, yield: 85.5%, purity: 96.4%).

$^1$HNMR:(400 MHz, $CDCl_3$) $\delta$ 7.44-7.29 (m, 5H), 5.83 (d, J = 8.8 Hz, 1H), 5.40-5.23 (m, 2H), 5.18-5.06 (m, 2H), 4.86 (s, 1H), 4.66 (d, J = 8.4 Hz, 1H), 4.21-4.07 (m, 2H), 4.04-3.77 (m, 3H), 3.51-3.45 (m, 1H), 3.31-3.11 (m, 2H), 2.18 (d, J = 2.0 Hz, 1H), 2.14 (s, 3H), 2.06 (s, 3H), 2.03-1.99 (m, 3H), 1.95 (s, 3H), 1.64-1.46 (m, 4H), 1.43-1.29 (m, 4H).

MS, $C_{28}H_{40}N_2O^{11}$, found: $M^+$ 581.3.

Step 2

The compound obtained in step 1 was divided into two parts for parallel reactions, each of which was carried out as follows: Compound **1-c** (72.0 g, 124 mmol) was added to 432 mL of THF. Pd/C (20.0 g, 10% purity) was added under argon, and then TFA (14.1 g, 124 mmol, 9.18 mL) was added. Hydrogen gas was introduced into the reaction solution, and the gas pressure was maintained at 30 Psi. The reaction solution was heated to 30 °C and stirred for 16 h. After the reaction was complete, the two reactions carried out in parallel were combined. The reaction mixture was filtered, and the filtrate was concentrated under reduced pressure. The residue was diluted with dichloromethane and concentrated under reduced pressure; the process was repeated three times. The residue was dried under reduced pressure to give the target compound **1-d** (139 g).

$^1$HNMR(400 MHz, DMSO-$d_6$)$\delta$ 7.85 (d, $J$ = 9.2 Hz, 1H), 7.74 (s, 3H), 5.21 (d, $J$ = 3.6 Hz, 1H), 4.97 (dd, $J$ = 2.8, 10.8 Hz, 1H), 4.48 (d, $J$ = 8.8 Hz, 1H), 4.06-3.98 (m, 3H), 3.93-3.82 (m, 1H), 3.73-3.68 (m, 1H), 3.63-3.56 (m, 1H), 3.43-3.38 (m, 1H), 2.82-2.71 (m, 2H), 2.13-2.09 (m, 3H), 2.01-1.97 (m, 3H), 1.91-1.87 (m, 3H), 1.77 (s, 3H), 1.76-1.73 (m, 1H), 1.52-1.44 (m, 4H), 1.28 (s, 4H).

Step 3

Compound **1-d** (139 g, 247 mmol) and compound **1-e** (75.3 g, 223 mmol) were added to DMF solution (834 mL), and then DIPEA (41.6 g, 322 mmol, 56.1 mL), HOBt (36.8 g, 272 mmol) and EDCI (52.2 g, 272 mmol) were added at 0 °C. The reaction mixture was stirred at 15 °C for 16 h. After the reaction was complete, the reaction mixture was diluted with dichloromethane (400 mL) and then washed successively with saturated ammonium chloride solution (1 L), saturated $NaHCO_3$ (1.00 L) and saturated brine. The organic phase was separated, dried over anhydrous sodium sulfate,

filtered and distilled under reduced pressure to remove the solvent. The residue was purified by silica gel column chromatography (petroleum ether:ethyl acetate = 5:1 to 0:1) to give the target compound **1-f** (108 g, yield: 56.8%).

$^1$HNMR(40 (400 MHz, DMSO-$d_6$) δ 7.89-7.78 (m, 2H), 7.41-7.27 (m, 6H), 5.21 (d, *J* = 3.2 Hz, 1H), 5.08-4.92 (m, 3H), 4.48 (d, *J* = 8.4 Hz, 1H), 4.07-3.99 (m, 3H), 3.97-3.81 (m, 2H), 3.75-3.64 (m, 1H), 3.42-3.37 (m, 1H), 3.13-2.93 (m, 2H), 2.20 (t, *J* = 8.0 Hz, 2H), 2.10 (s, 3H), 1.99 (s, 3H), 1.89 (s, 3H), 1.87-1.79 (m, 1H), 1.76 (s, 3H), 1.74-1.64 (m, 1H), 1.48-1.41 (m, 2H), 1.38 (s, 12H), 1.29-1.20 (m, 4H), 1.19-1.14 (m, 1H).

MS, $C_{37}H_{55}N_3O_{14}$, found: $M^+$766.4.

Step 4

The compound **1-f** obtained above was divided into two parts for parallel reactions, each of which was carried out as follows: Compound **6** (47.0 g, 61.3 mmol) was added to 280 mL of THF. Pd/C (15.0 g, 10% purity) was added under argon, and then TFA (7.00 g, 61.3 mmol, 4.54 mL) was added. Hydrogen gas was introduced into the reaction solution, and the gas pressure was maintained at 30 Psi. The reaction solution was heated to 30 °C and stirred for 16 h. After the reaction was complete, the two reactions carried out in parallel were combined. The reaction mixture was filtered, and the filtrate was concentrated under reduced pressure. The residue was diluted with dichloromethane and concentrated under reduced pressure; the process was repeated three times. The residue was dried under reduced pressure to give the target compound 1-g (94.0 g, crude).

$^1$HNMR(400 MHz, DMSO-d6)δ 8.38 (s, 1H), 8.10 (s, 3H), 7.83 (d, J = 9.2 Hz, 1H), 5.21 (d, J = 3.2 Hz, 1H), 4.96 (dd, J = 3.6, 11.2 Hz, 1H), 4.47 (d, J = 8.4 Hz, 1H), 4.06-3.98 (m, 3H), 3.92-3.82 (m, 1H), 3.75-3.67 (m, 2H), 3.60 (s, 1H), 3.43-3.37 (m, 1H), 3.18-3.04 (m, 2H), 2.30-2.24 (m, 2H), 2.10 (s, 3H), 2.00 (s, 3H), 1.95-1.90 (m, 2H), 1.89 (s, 3H), 1.78-1.75 (m, 3H), 1.49-1.41 (m, 3H), 1.40 (s, 9H), 1.26 (s, 4H).

Step 5

The compound **1-f** obtained above was divided into two parts for parallel reactions, each of which was carried out as follows: Compound **1-f** (46.0 g, 60 mmol) was added to HCl-EtOAc (2.00 M, 276 mL), and the reaction mixture was stirred at 15 °C for 16 h. After the reaction was complete, the two reaction solutions were combined and concentrated by distillation under reduced pressure. The residue was diluted with dichloromethane and concentrated under reduced pressure; the process was repeated three times. The residue was dried under reduced pressure to give a light red compound **1-h** (91.0 g, crude).

$^1$HNMR(400 MHz, DMSO-$d_6$)δ 7.91-7.80 (m, 2H), 7.42-7.26 (m, 6H), 5.21 (d, *J* = 3.2 Hz, 1H), 5.07-4.92 (m, 4H), 4.48 (d, *J* = 8.4 Hz, 1H), 4.06-3.98 (m, 3H), 3.98-3.82 (m, 3H), 3.73-3.65 (m, 1H), 3.44-3.35 (m, 1H), 3.12-2.94 (m, 2H), 2.22 (t, J = 8.0 Hz, 2H), 2.10 (s, 3H), 2.01-1.97 (m, 4H), 1.94-1.90 (m, 1H), 1.89 (s, 3H), 1.87-1.79 (m, 2H), 1.76 (s, 3H), 1.74-1.67 (m, 1H), 1.49-1.40 (m, 2H), 1.40-1.32 (m, 2H), 1.24 (d, *J*= 4.0 Hz, 4H), 1.19-1.13 (m, 1H).

MS, $C_{33}H_{47}N_3O_{14}$, found: $M^+$710.3.

Step 6

Two reactions were carried in parallel as follows: Compound **1-g** (45.0 g, 60.3 mmol) and compound **1-h** (38.5 g, 54.3 mmol) were added to 270 mL of DMF, then DIPEA (10.1 g, 78.4 mmol, 13.6 mL) was added at 0 °C, and then HOBt (8.97 g, 66.3 mmol) and EDCI (12.7 g, 66.3 mmol) were added. The reaction mixture was stirred at 15 °C for 16 h. After the reaction was complete, the two reaction solutions were combined, diluted with 300 mL of DCM, and washed successively with saturated ammonium chloride (800 mL), saturated $NaHCO_3$ (800 mL) and saturated brine (800 mL). The organic phase was dried over anhydrous $Na_2SO_4$. After filtration, the filtrate was concentrated by evaporation under increased pressure. The residue was purified by silica gel column chromatography (petroleum ether:ethyl acetate = 5:1 to 0:1) to give a white compound **1-i** (66.0 g, 47.4 mmol, yield: 39.3%, purity 95.1%).

$^1$HNMR(400 MHz, DMSO-$d_6$) δ 7.96-7.78 (m, 5H), 7.41-7.25 (m, 6H), 5.21 (d, *J*= 3.6 Hz, 2H), 5.05-4.92 (m, 4H), 4.48 (d, *J* = 8.8 Hz, 2H), 4.22-4.12 (m, 1H), 4.02 (s, 6H), 3.94-3.80 (m, 3H), 3.74-3.64 (m, 2H), 3.45-3.35 (m, 2H), 3.11-2.92 (m, 4H), 2.20-2.12 (m, 4H), 2.10 (s, 6H), 1.99 (s, 6H), 1.89 (s, 6H), 1.82-1.79 (m, 2H), 1.76 (s, 6H), 1.74-1.63 (m, 2H), 1.44 (d, *J* = 6.0 Hz, 4H), 1.37 (s, 12H), 1.24 (s, 9H).

MS: $C_{62}H_{94}N_6O_{25}$, found: m/z 1323.8.

Step 7

This step was performed in 11 reactions, each of which was carried out as follows: Compound **1-i** (5.00 g, 3.78 mmol) and toluene (300 mL) were added, and silica gel (45.0 g) was added. The reaction mixture was stirred at 100 °C for 40 h. After the 11 reactions were complete, the reaction mixtures were combined. After the solvent was distilled off

under reduced pressure, isopropanol and dichloromethane were added to the residue, and the mixture was stirred for 20 min. Insoluble matter was removed by filtration, and the filter cake was washed with isopropanol until no product was dissolved in isopropanol. The resulting solution was concentrated to remove the solvent and dried under reduced pressure to give a light yellow compound **1-j** (43.2 g, 34.0 mmol, yield: 82.0%).

$^1$HNMR: (400 MHz, DMSO-d6)δ 8.01 (d, J = 7.6 Hz, 1H), 7.93-7.79 (m, 2H), 7.39-7.27 (m, 3H), 5.21 (d, J = 3.2 Hz, 1H), 5.06-4.91 (m, 2H), 4.48 (d, J = 8.0 Hz, 1H), 4.07-3.97 (m, 3H), 3.94-3.82 (m, 2H), 3.73-3.65 (m, 1H), 3.45-3.36 (m, 2H), 3.10-2.94 (m, 2H), 2.15 (d, J = 7.6 Hz, 2H), 2.10 (s, 3H), 1.99 (s, 3H), 1.89 (s, 3H), 1.86-1.79 (m, 1H), 1.77 (s, 3H), 1.74-1.65 (m, 1H), 1.44 (s, 2H), 1.37 (d, J = 5.2 Hz, 2H), 1.24 (s, 4H).

MS: $C_{58}H_{86}N_6O_{25}$, found: m/z = 1267.8.

Step 8

This step was performed in two parallel reactions, each of which was carried out as follows: Compound **1-d** (11.8 g, 21.0 mmol) and compound **1-j** (21.3 g, 16.8 mmol) were added to 70 mL of DMF, then DIPEA (3.54 g, 27.3 mmol, 4.77 mL) was added at 0 °C, and then HOBt (3.13 g, 23.1 mmol) and EDCI (4.44 g, 23.1 mmol) were added. The reaction mixture was stirred at 15 °C for 16 h. After the reaction was complete, the two reaction solutions were combined, diluted with 500 mL of DCM, and washed successively with saturated ammonium chloride (1.5 L), saturated $NaHCO_3$ (1.5 mL) and saturated brine (1.5 mL). The organic phase was dried over anhydrous $Na_2SO_4$. After filtration, the filtrate was concentrated by evaporation under increased pressure. The residue was purified by silica gel column chromatography (dichloromethane:methanol = 50:1 to 10:1) to give a light yellow compound **1-k** (54.0 g, 31.8 mmol, yield: 75.6%).

$^1$HNMR(400 MHz, DMSO-$d_6$) δ 7.91 (d, J = 7.6 Hz, 1H), 7.87-7.78 (m, 5H), 7.73 (t, J = 5.2 Hz, 1H), 7.42-7.24 (m, 6H), 5.21 (d, J = 3.6 Hz, 3H), 5.06-4.92 (m, 5H), 4.48 (d, J = 8.4 Hz, 3H), 4.19-4.09 (m, 2H), 4.07-3.97 (m, 10H), 3.94-3.80 (m, 4H), 3.76-3.64 (m, 3H), 3.42-3.37 (m, 4H), 3.08-2.94 (m, 6H), 2.20-2.12 (m, 2H), 2.10 (s, 9H), 2.08-2.01 (m, 2H), 1.99 (s, 9H), 1.89 (s, 9H), 1.87-1.79 (m, 2H), 1.77 (s, 9H), 1.74-1.63 (m, 2H), 1.44 (d, J = 5.6 Hz, 6H), 1.40-1.31 (m, 6H), 1.24 (s, 13H).

MS: $C_{78}H_{118}N_8O_{33}$, found: m/z = 1696.1.

Step 9

This step was performed in 3 parallel reactions, each of which was carried out as follows: Compound **1-k** (17.0 g, 10.0 mmol) and THF (100 mL) were added. Pd/C (5.0 g, 10% purity) was added under argon, and then TFA (1.14 g, 10.0 mmol, 742 μL) was added. Hydrogen gas was introduced into the reaction solution, and the gas pressure was maintained at 15 Psi. The reaction solution was heated to 30 °C and stirred for 4 h. After the reaction was complete, the 3 reactions carried out in parallel were combined. The reaction mixture was filtered, and the filtrate was concentrated under reduced pressure. The residue was diluted with dichloromethane and concentrated under reduced pressure; the process was repeated three times. The residue was purified by preparative liquid chromatography (C18, mobile phase A 0.1% TFA-water, mobile phase B: 10-40% CAN, 20 min) to give a white compound **1-1** (17.3 g, 10.2 mmol, yield: 34.0%).

$^1$HNMR: (400 MHz, DMSO-$d_6$) δ 8.45 (t, *J* = 5.2 Hz, 1H), 8.14 (d, *J* = 5.2 Hz, 3H), 7.97 (t, *J* = 5.2 Hz, 1H), 7.90-7.77 (m, 4H), 5.21 (d, *J* = 2.8 Hz, 3H), 4.96 (dd, *J* = 3.2, 11.6 Hz, 3H), 4.47 (d, *J* = 8.4 Hz, 3H), 4.20-4.10 (m, 1H), 4.02 (s, 8H), 3.87 (q, *J* = 9.6 Hz, 3H), 3.75-3.61 (m, 4H), 3.46-3.34 (m, 3H), 3.21-2.93 (m, 6H), 2.21 (s, 2H), 2.14-2.02 (m, 11H), 1.99 (s, 9H), 1.96-1.82 (m, 12H), 1.80-1.65 (m, 10H), 1.44 (d, *J* = 5.6 Hz, 8H), 1.36 (d, *J* = 6.4 Hz, 4H), 1.30-1.17 (m, 12H)

MS: $C_{70}H_{112}N_8O_{31}$, found: m/2z = 781.8.

Step 10

Compound 1-m (2 g, 12.64 mmol) was dissolved in pyridine (10 mL). A solution of DMTrCl (4.71 g, 13.90 mmol) in pyridine (10 mL) was added dropwise at room temperature. The reaction mixture was stirred at room temperature for 5 h. After the reaction was complete, the reaction mixture was quenched with methanol and concentrated under reduced pressure to give a crude product. The crude product was purified using silica gel (elution with petroleum ether:ethyl acetate = 10:1). The product eluate was collected and concentrated under reduced pressure to evaporate the solvent to give compound **1-n** (4 g).

MS m/z: $C_{29}H_{32}O_5$, $[M+H]^+$ found: 461.3.

Step 11

Compound **1-n** (2 g, 4.34 mmol), N,N-diisopropylethylamine (DIEA, 1.43 mL, 8.68 mmol) and HATU (2.47 g, 6.51 mmol) were dissolved in DMF (10 mL). A solution of compound **1-o** in DMF (5 mL) was added at room temperature.

The reaction mixture was stirred at room temperature for 8 h. After the reaction was complete, water was added to quench the reaction. The aqueous phase was extracted with ethyl acetate. The combined organic phases were washed first with water and then with saturated brine (20 mL), then concentrated under reduced pressure to evaporate the solvent, purified by reversed-phase preparative HPLC (column: Boston Green ODS 150 × 30 mm × 5 μm, conditions: 25-80% (A: water 0.075% $NH_3 \cdot H_2O$, B: $CH_3CN$), flow rate: 55 mL/min), and lyophilized to give compound **1-p** (2.4 g).

MS m/z: $C_{33}H_{39}NO_7$, $[M+H]^+$ found: 562.4.

Step 12

Compound **1-p** (2.4 g, 4.27 mmol) was dissolved in 15 mL of a mixed solution of methanol and water (2:1). LiOH (0.36 g, 8.54 mmol) was added at room temperature. The mixture was stirred overnight. After the reaction was complete, the mixture was concentrated under reduced pressure to evaporate the solvent, purified by reversed-phase preparative HPLC (column: Boston Green ODS 150 × 30 mm × 5 μm, conditions: 25-75% (A: water 0.075% $NH_3 \cdot H_2O$, B: $CH_3CN$), flow rate: 55 mL/min), and lyophilized to give compound **1-p** (2 g).

MS m/z: $C_{32}H_{37}NO_7$, $[M+H]^+$ found: 548.6.

Step 13

Compound **1-q** (0.37 g, 0.69 mmol), DIEA (0.19 mL, 1.15 mmol) and HATU (0.32 g, 0.86 mmol) were dissolved in 2 mL of DMF. A solution of compound **1-1** (0.9 g, 0.69 mmol) in DMF (2 mL) was added at room temperature. The mixture was stirred at room temperature overnight. After the reaction was complete, the reaction mixture was diluted with dichloromethane (10 mL) and washed successively with saturated $NaHCO_3$ (20 mL) and saturated brine (20 mL). The organic phase was dried over anhydrous $Na_2SO_4$, filtered and then concentrated under reduced pressure. The residue was purified by reversed-phase preparative HPLC (column: Boston Green ODS 150 × 30 mm × 5 μm, conditions: 25-65% (A: water 0.075% $NH_3 \cdot H_2O$, B: $CH_3CN$), flow rate: 45 mL/min) and lyophilized to give compound **1-r** (0.5 g).

MS m/z: $C_{102}H_{147}N_9O_{37}$, $[M-H]^+$ found: 2088.5.

Step 14

Compound **1-r** (300 mg, 0.14 mmol) and succinic anhydride (28.70 mg, 0.28 mmol) were dissolved in tetrahydrofuran. DMAP (3.50 mg, 0.028 mmol) was added to the reaction mixture, and the mixture was stirred at 40 °C overnight. After the reaction was complete, methanol (18.8 mg) was added. The reaction mixture was stirred for 10 min, then diluted with dichloromethane (3 mL) and washed twice with saturated NaHCO3 (5 mL). The organic phase was concentrated to dryness under reduced pressure and purified by reversed-phase preparative HPLC (column: Boston Green ODS 150 × 30 mm × 5 μm, conditions: 25-65% (A: water 0.075% $NH_3 \cdot H_2O$, B: $CH_3CN$), flow rate: 35 mL/min) and lyophilized to give compound **1-s** (140 mg).

MS m/z: $C_{106}H_{151}N_9O_{40}$, $[M-H]^+$ found: 2189.4.

Step 15

The compound **1-r** (140 mg, 64 μmmol) obtained in the previous step was added to acetonitrile (5 mL). Then HBTU (48.7 mg, 128 μmol) was added, a surface aminomodified solid-phase support (CPG-NH2, 2.3 g) was added, and DIEA (41.5 mg, 320 μmol, 55 μL) was added. The mixture was reacted with shaking at 30 °C for 16 h. After the reaction was complete, the mixture was filtered and washed successively with methanol (8 mL × 4) and dichloromethane (8 mL × 4). The solid was added to pyridine:acetic anhydride (v:v = 4:1, 10.0 mL), and the mixture was reacted with shaking at 30 °C for another 16 h. After the reaction was complete, the mixture was filtered and washed successively with methanol (8 mL × 4) and dichloromethane (8 mL × 4) to give compound **1-t** linked to the solid-phase support (2.1 g).

**Example 7. Galactosamine Compound 2-e Linked to Solid-Phase Support**

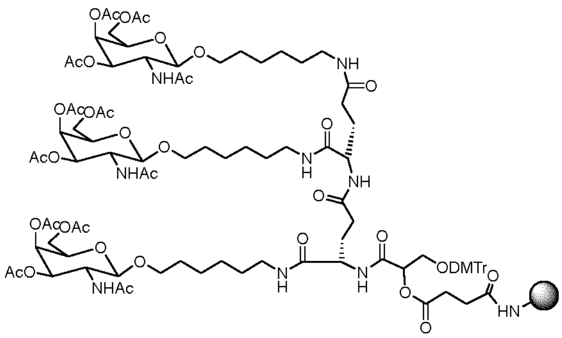

The synthesis schemes are as follows:

1) Synthesis of compound **2-b**

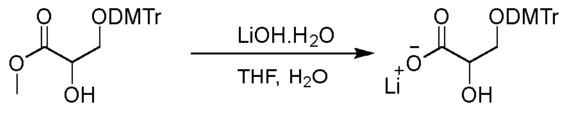


**2-a** **2-b**

2) Synthesis of compound **2-e**

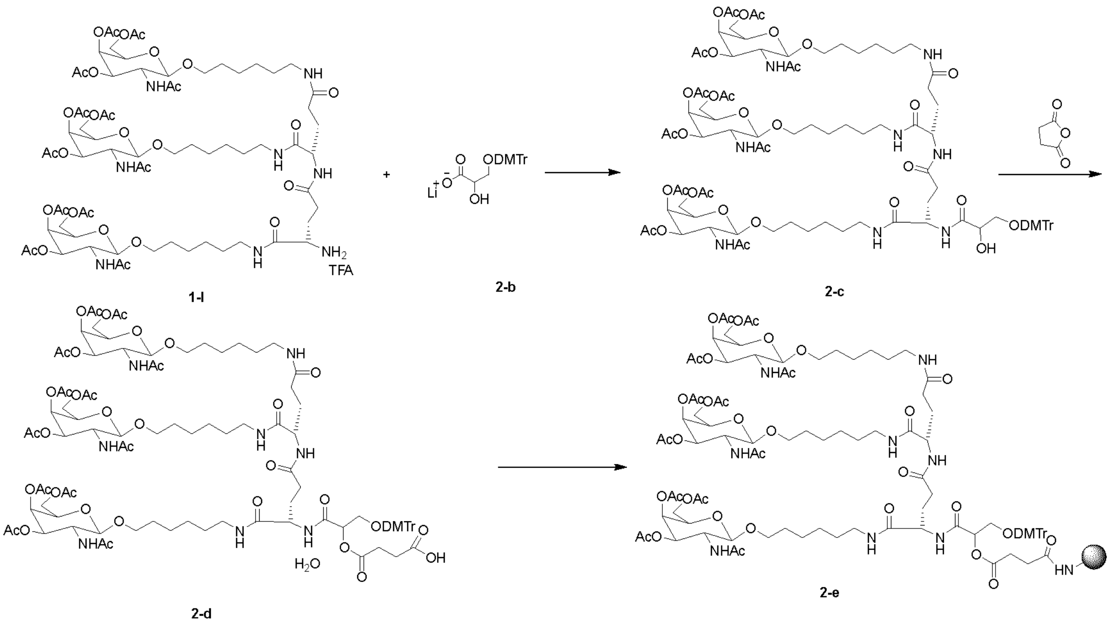

1-l 2-b 2-c 2-d 2-e

Step 1

Compound **2-a** (1.00 g, 2.37 mmol) was added to THF (7.5 mL) and $H_2O$ (7.5 mL), and then LiOH.$H_2O$ (109 mg, 2.60 mmol) was added. The reaction mixture was stirred at 16 °C for 16 h. After the reaction was complete, the solvent was removed by evaporation under reduced pressure. The residue was further lyophilized to give a white compound **2-b** (960 mg, 2.32 mmol, yield: 97.8%).

$^1$HNMR: (400 MHz, DMSO-d6) δ 7.44 (d, J = 8.4 Hz, 2H), 7.34-7.23 (m, 6H), 7.22-7.15 (m, 1H), 6.86 (d, J = 8.0 Hz, 4H), 3.73 (s, 6H), 3.66 (d, J = 6.4 Hz, 1H), 3.32 (d, J = 12.0 Hz, 1H), 3.11 (dd, J = 2.0, 9.2 Hz, 1H), 2.85 (t, J = 8.8 Hz, 1H).

MS m/z: $C_{24}H_{24}O_6$, found: m/z: 407.2.

Step 2

Compound **1-1** (500 mg, 0.30 mmol) was added to dichloromethane (3 mL), then compound **2-b** (0.14 g, 0.34 mmol) was added at 15 °C to the reaction, and HBTU (142 mg, 375 μmol) and DIEA (115 mg, 895 μmol) were added at 0 °C. The mixture was reacted at 15 °C for 16 h. After the reaction was complete, the reaction mixture was diluted with dichloromethane (10 mL) and washed successively with saturated $NaHCO_3$ (20 mL) and saturated brine (20 mL). The organic phase was dried over anhydrous Na2SO4, filtered and then concentrated under reduced pressure. The residue was purified by preparative liquid chromatography (column: Welch Xtimate C18 250 × 70 mm #10 μm; mobile phase: [water-ACN]; B%: 40% to 66%,18 min) to give compound **2-c.**

MS m/z: $C_{94}H_{134}N_8O_{36}$, [M-H]+ found: 1952.1.

Step 3

Compound **2-c** (230 mg, 0.12 mmol) and succinic anhydride (23.5 mg, 0.26 mmol) were dissolved in a dichloromethane solution (2 mL). DMAP (43.1 mg, 0.35 mmol) was added to the reaction mixture. The mixture was stirred at 15 °C for 16 h. After the reaction was complete, methanol (18.8 mg) was added. The reaction mixture was stirred for 10 min, then diluted with dichloromethane (3 mL) and washed twice with saturated $NaHCO_3$. The reaction mixture was concentrated to dryness under reduced pressure to give compound **2-d** (240 mg, crude).

MS m/z: C106H151N9O40, [M-H]+ found: m/2z: 2070.2

Step 4

The compound **2-d** (240 mg, 116 μmmol) obtained in the previous step was added to acetonitrile (8 mL). Then HBTU (88.7 mg, 233 μmol) was added, a surface aminomodified solid-phase support (CPG-NH2, 4 g) was added, and DIEA (75.5 mg, 584 μmol, 101 μL) was added. The mixture was reacted with shaking at 30 °C for 16 h. After the reaction was complete, the mixture was filtered and washed successively with methanol (8 mL × 4) and dichloromethane (8 mL × 4). The solid was added to pyridine:acetic anhydride (v:v = 4:1, 10.0 mL), and the mixture was reacted with shaking at 30 °C for another 16 h. After the reaction was complete, the mixture was filtered and washed successively with methanol (8 mL × 4) and dichloromethane (8 mL × 4) to give the target product compound **2-e** linked to the solid-phase support (3.7 g).

**Example 8. Galactosamine Compound 3-n Linked to Solid-Phase Support**

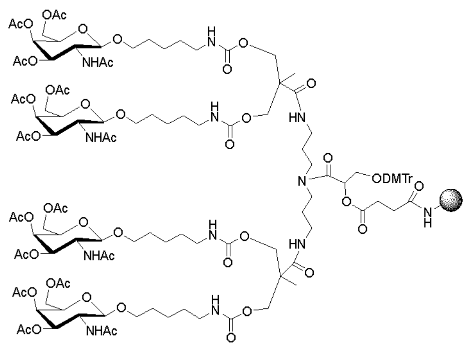

The synthesis schemes are as follows:

1) Synthesis of compound **3-d**

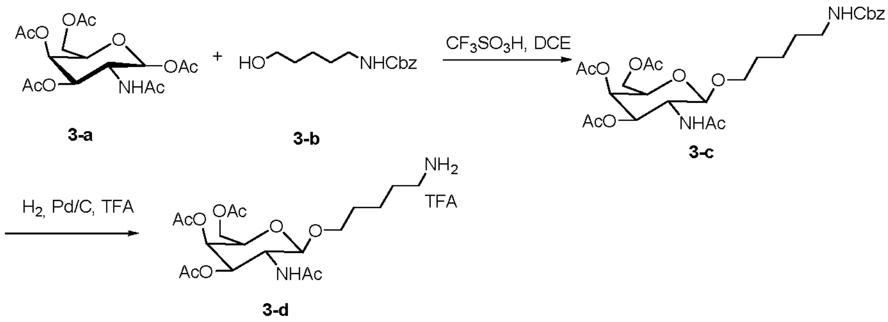


3-a 3-b 3-c 3-d

2) Synthesis of compound **3-g**

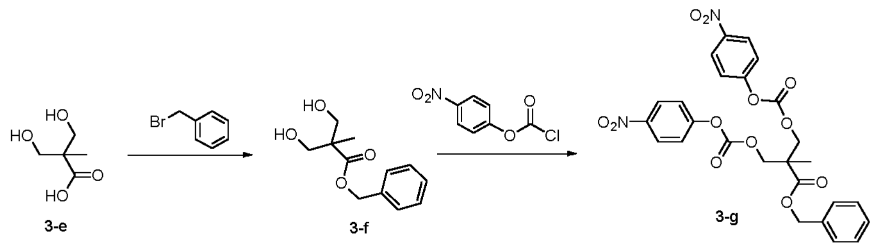

3-e 3-f 3-g

3) Synthesis of compound **3-n**

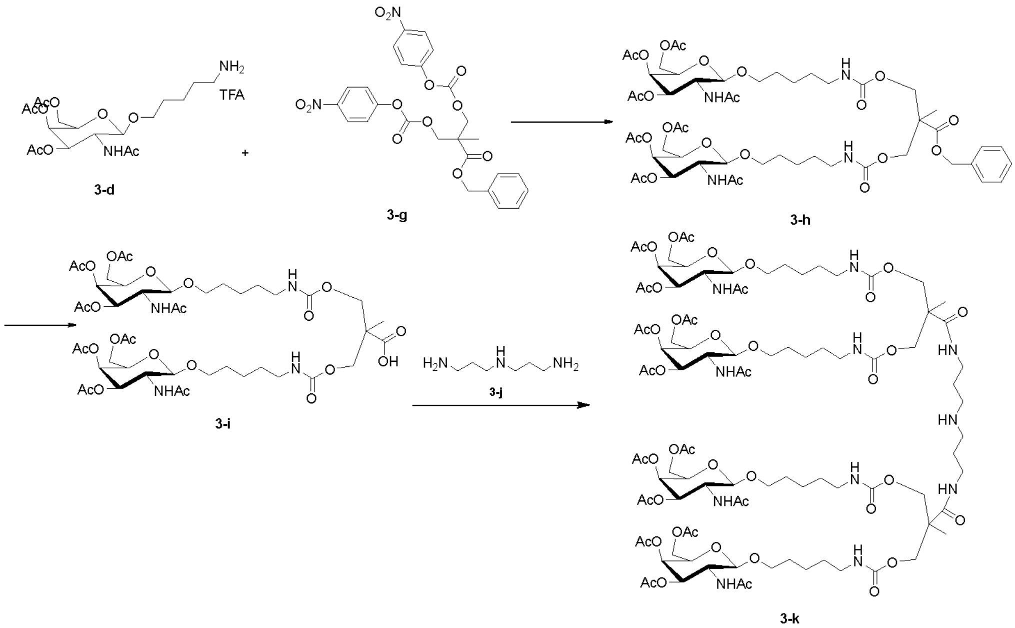

3-d 3-g 3-h 3-i 3-j 3-k

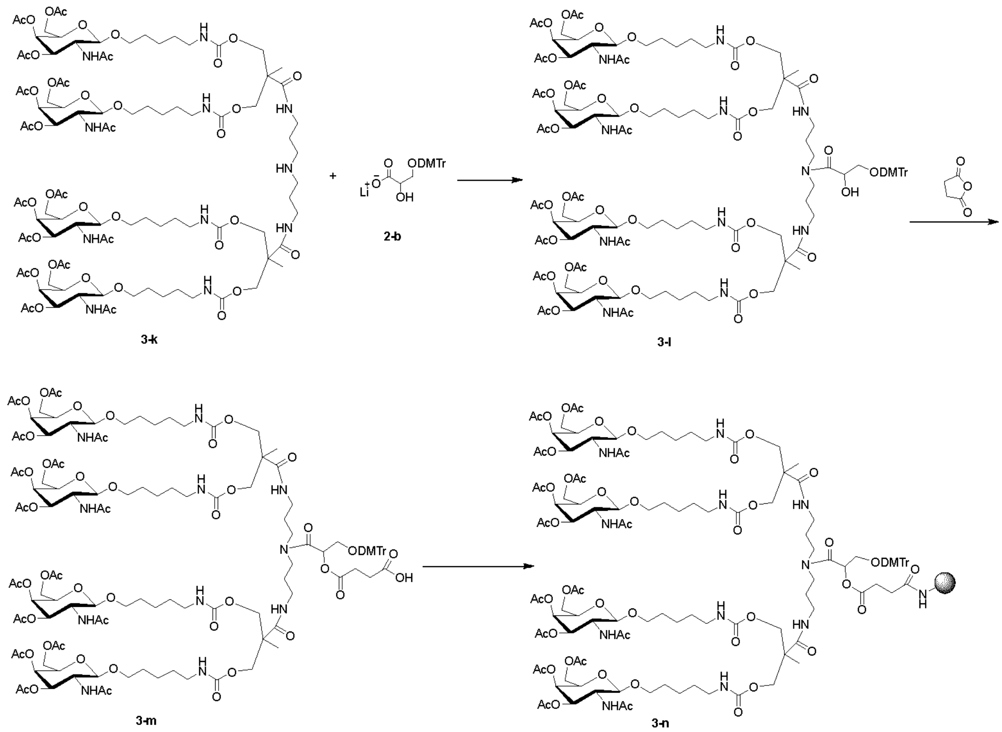

3-k 2-b 3-l 3-m 3-n

Step 1

The starting material **3-a** (78.8 g, 202 mmol) and the starting material **3-b** (40 g, 168 mmol) were dissolved in DCE (250 mL). $CF_3SO_3H$ (4.15 g, 8.43 mmol) was added at 15 °C. Then the reaction mixture was heated to 75 °C and stirred for 2 h. After the reaction was complete, 1 L of saturated $NaHCO_3$ was added to terminate the reaction. The organic phase was separated, washed with 1 L of saturated brine, dried over anhydrous $Na_2SO_4$ and filtered. The filtrate was distilled under reduced pressure and purified by silica gel column chromatography (petroleum ether:ethyl acetate = 5:1 to 0:1) to give the target product **3-c** (63.2 g, 107 mmol, yield: 63.5%).

$^1$HNMR:(400 MHz, $CDCl_3$) δ 7.35-7.26 (m, 5H), 5.88 (s, 1H), 5.34-5.25 (m, 2H), 4.65 (d, *J* = 8.4 Hz, 1H), 4.16-4.13 (m, 2H), 3.92-3.87 (m, 3H), 3.18-3.17 (m, 1H), 3.15-3.14 (m, 2H), 2.16-1.91 (m, 15H), 1.58-1.50 (m, 5H), 1.49-1.36 (m, 2H).

MS m/z: $C_{24}H_{40}N_2O_{11}$, found: m/z: 567.4.

Step 2

The compound 3-c (60.0 g, 106 mmol) obtained above was added to 360 mL of THF. Pd/C (15.0 g, 10% purity) was added under argon, and then TFA (12.1 g, 106 mmol, 7.84 mL) was added. Hydrogen gas was introduced into the reaction solution, and the gas pressure was maintained at 30 Psi. The reaction solution was heated to 30 °C and stirred for 16 h. After the reaction was complete, the reaction mixture was filtered, and the filtrate was concentrated under reduced pressure. The residue was diluted with dichloromethane and concentrated under reduced pressure; the process was repeated three times (500 mL × 3). The residue was dried under reduced pressure to give a light yellow compound **3-d** (44 g, 102 mmol, yield: 96.1%).

Step 3

Compound **3-e** (60.0 g, 447 mmol) was dissolved in DMF (300 mL). $K_2CO_3$ (92.7 g, 671 mmol) was added, and BnBr (115 g, 671 mmol, 79.7 mL) was added dropwise at 0 °C. The reaction mixture was stirred at 25 °C for 6 h. The reaction mixture was poured into crushed ice and then extracted with ethyl acetate (100 mL × 6). The organic phase was washed successively with water (100 mL × 2) and saturated brine (100 mL × 3). The organic phase was dried over anhydrous sodium sulfate and distilled under reduced pressure to remove the solvent. The residue was purified by silica gel column chromatography (petroleum ether:ethyl acetate = 2:1 to 0:1) to give compound **3-f** as a white solid (60.3 g, 269 mmol, yield: 60.1%).

$^1$HNMR: (400 MHz, $CDCl_3$) δ 7.37-7.26 (m, 5H), 5.18 (d, J = 4.4 Hz, 2H), 3.95-3.90 (m, 2H), 3.75-3.71 (m, 2H),

1.08 (s, 1H).
MS m/z: $C_{12}H_{16}O_4$, found: m/z: 223.5.

Step 4

Compound **3-f** (50.0 g, 223 mmol) was dissolved in dichloromethane (300 mL). Pyridine (73.5 g, 929 mmol, 75 mL) and a solution of p-nitrophenyl chloroformate (180 g, 892 mmol) in dichloromethane (50 mL) were added. The reaction mixture was stirred at 25 °C under nitrogen for 24 h. After the reaction was complete, the mixture was diluted with dichloromethane (250 mL) and washed successively with a $NaHSO_4$ solution (30 mL × 3) and saturated brine (30 mL × 2). The organic phase was dried over $MgSO_4$, filtered and concentrated under reduced pressure to evaporate the solvent. The resulting crude product was purified by silica gel column chromatography (petroleum ether:ethyl acetate = 3:1) to give the target compound **3-g** (37.0 g, 66.7 mmol, yield: 29.9%).
MS m/z: $C_{26}H_{22}N_2O_{12}$, found: m/z: 553.4.

Step 5

Compound **3-g** (22.0 g, 39.7 mmol) was added to acetonitrile (120 mL), and triethylamine (24.1 g, 238 mmol, 33,1 mL) was added under nitrogen. The reaction mixture was cooled to 0 °C, and a solution of compound **3-d** (42.1 g, 40 mmol) in acetonitrile (120 mL) was added dropwise. The reaction mixture was warmed to 25 °C and stirred for 1 h. After the reaction was complete, the mixture was concentrated under reduced pressure to remove the solvent and then purified by silica gel column chromatography (petroleum ether:ethyl acetate = 2:1) to give the target compound **3-h** (37.0 g, 12.0 mmol, yield: 30.2%).
MS m/z: $C_{52}H_{76}N_4O_{24}$, found: m/z: 1141.8.

Step 6

Compound **3-h** (11.0 g, 9.64 mmol) was dissolved in ethyl acetate (60 mL). Pd/C (2.00 g, 10% purity) was added. Hydrogen gas was introduced into the reaction solution, and the gas pressure was maintained at 40 Psi. The reaction solution was stirred at 25 °C for 8 h. After the reaction was complete, the mixture was filtered and concentrated to dryness by evaporation under reduced pressure to give the target compound **3-i** (10.0 g, 9.42 mmol, yield: 97.7%).
$^1$HNMR: (400 MHz, DMSO-$d_6$) δ 7.79 (d, J = 9.2 Hz, 2H), 7.10 (s, 2H), 5.74 (t, J = 1.6 Hz, 2H), 5.21 (d, J = 3.6 Hz, 2H), 4.98-4.95 (m, 2H), 4.48 (d, J = 8.4 Hz, 2H), 4.02 (d, J = 4.8 Hz, 11H), 3.87-3.84 (m, 2H), 3.69-3.67 (m, 2H), 3.41-3.39 (m, 2H), 2.94-2.90 (m, 4H), 2.10 (s, 5H), 1.99 (s, 7H), 1.89 (s, 6H), 1.77 (s, 6H), 1.47-1.35 (m, 8H), 1.26-1.24 (m, 4H), 1.23-1.08 (m, 3H).
MS m/z: $C_{45}H_{70}N_4O_{24}$, found: m/z: 1051.4.

Step 7

Compound **3-i** (5.00 g, 4.76 mmol) was added to a mixed solvent of dichloromethane (30 mL) and DMF (30 mL), then compound 33 (312 mg, 2.38 mmol) was added, and HBTU (1.80 g, 4.76 mmol) and DIEA (615 mg, 4.76 mmol) were added. The reaction mixture was stirred at 25 °C for 12 h. After the reaction was complete, the reaction mixture was poured into ethyl acetate (100 mL), then washed with saturated brine, dried over anhydrous $Na_2SO_4$, filtered and concentrated under reduced pressure to evaporate the solvent. The residue was purified by preparative HPLC to give the target compound **3-k** (2.1 g, 956 μmol, yield: 20.1%).
$^1$HNMR: (400 MHz, DMSO-$d_6$) δ 7.84-7.81 (m, 5H), 7.12-7.07 (m, 3H), 5.21 (d, J = 3.6 Hz, 4H), 4.99-4.96 (m, 4H), 4.49 (d, J = 8.4 Hz, 4H), 4.06-4.00 (m, 24H), 3.88-3.86 (m, 4H), 3.55-3.52 (m, 4H), 3.49-3.43 (m, 4H), 3.25-3.05 (m, 4H), 2.94-2.93 (m, 8H), 2.11 (s, 12H), 2.00 (s, 16H), 1.90 (s, 12H), 1.78 (s, 12H), 1.46-1.44 (m, 8H), 1.38-1.35 (m, 8H), 1.26-1.24 (m, 8H), 1.18-1.16 (m, 6H), 1.09-0.99 (m, 2H).
MS m/z: $C_{96}H_{153}N_{11}O_{46}$, found: m/z: 2197.5.

Step 8

Compound **3-k** (100 mg, 45.5 μmol) was added to DMF (1 mL), then compound **2-b** (21.1 mg, 54 μmol) was added to the reaction, and HBTU (21.8 mg, 57.3 μmol) and DIEA (17.7 mg, 136 μmol) were added. The mixture was reacted at 15 °C for 16 h. After the reaction was complete, the reaction mixture was diluted with dichloromethane (10 mL) and washed successively with saturated $NaHCO_3$ and saturated brine. The organic phase was dried over anhydrous $Na_2SO_4$, filtered and then concentrated under reduced pressure. The residue was purified by preparative liquid chromatography (column: Phenomenex Gemini-NX 150 × 30 mm × 5 μm; mobile phase: [water-ACN]; B%: 35% to 75%,12

min) to give compound **3-l.** MS m/z: $C_{120}H_{175}N_{11}O_{51}$, found: 2586.9.

Step 9

Compound **3-l** (14 mg, 5.4 μmol) and succinic anhydride (1.08 mg,10.8 μmol) were dissolved in a dichloromethane solution (1 mL). DMAP (2.0 mg, 16 μmol) and TEA (1.1 mg,10.8 μmol, 1.5 μL) were added to the reaction mixture. The mixture was stirred at 15 °C for 16 h. After the reaction was complete, methanol (0.9 mg) was added. The reaction mixture was stirred for 10 min, then diluted with dichloromethane and washed twice with saturated $NaHCO_3$. The reaction mixture was concentrated to dryness under reduced pressure to give compound **3-m** (18 mg).

MS m/z: $C_{124}H_{179}N_{11}O^{54}$, found: 2687.2.

Step 10

The compound 3-m (18 mg, 6.7 μmmol) obtained in the previous step was added to acetonitrile (3 mL). Then HBTU (5.1 mg, 13.4 μmol) was added, a surface aminomodified solid-phase support (CPG-NH2, 200 mg) was added, and DIEA (4.3 mg, 33.5 μmol, 5.8 μL) was added. The mixture was reacted with shaking at 30 °C for 16 h. After the reaction was complete, the mixture was filtered and washed successively with methanol (2 mL × 4) and dichloromethane (2 mL × 4). The solid was added to pyridine:acetic anhydride (v:v = 4:1, 2 mL), and the mixture was reacted with shaking at 30 °C for another 16 h. After the reaction was complete, the mixture was filtered and washed successively with methanol and dichloromethane to give the target product compound **3-n** linked to the solid-phase support (200 mg).

**Example 9. Galactosamine Compound 4-c Linked to Solid-Phase Support**

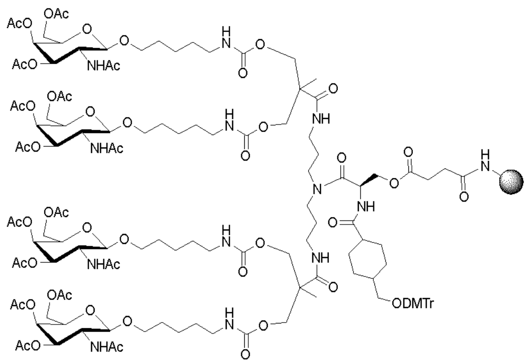

The synthesis schemes are as follows:

1) Synthesis of compound **4-c**

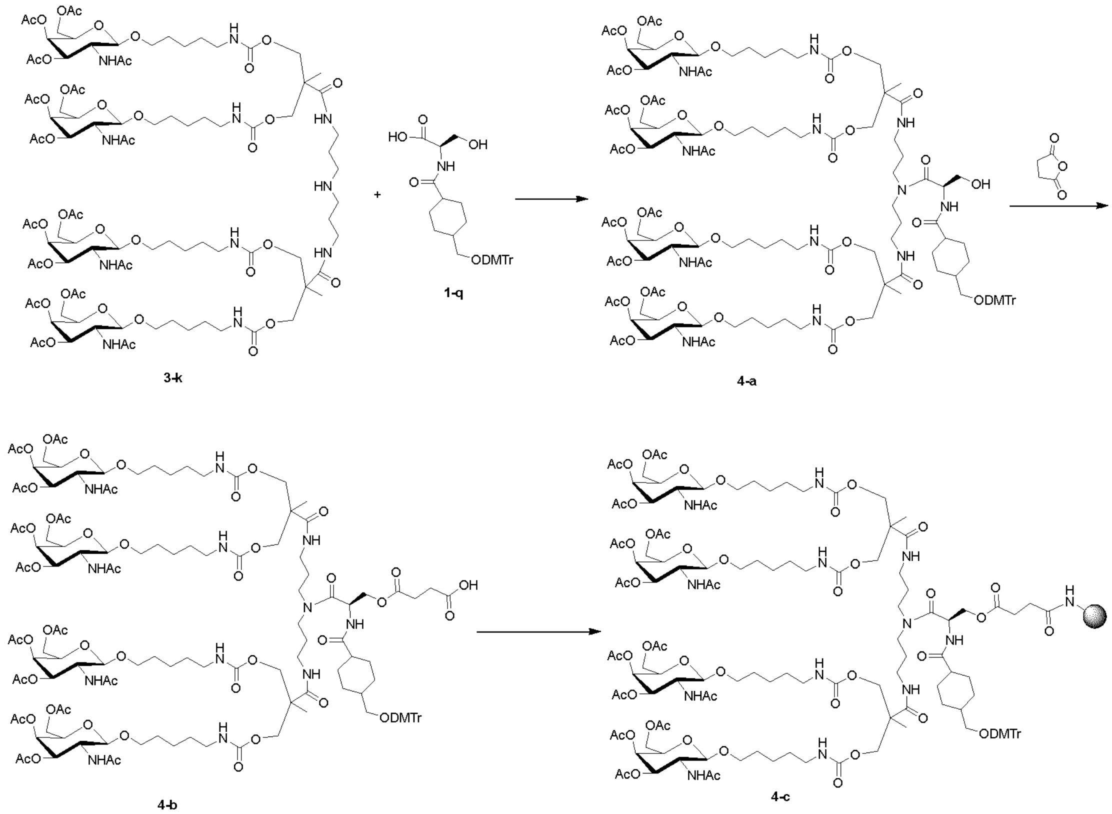

3-k 1-q 4-a 4-b 4-c

Step 1

Compound **3-k** (149.5 mg, 68 μmmol), DIEA (141.0 mg, 1.09 mmol), 3A molecular sieve (500 mg) and DEPBT (163.4 mg, 0.55 mmol) were dissolved in 5 mL of DCM. Compound **1-q** (400 mg, 0.18 mmol) was added at room temperature. The mixture was stirred at room temperature overnight. After the reaction was complete, the molecular sieve was filtered out. The filtrate was concentrated to dryness by rotary evaporation, purified by reversed-phase preparative HPLC (column: Boston Green ODS 150 × 30 mm × 5 μm, conditions: 5-50% (A: water, B: CH3CN), flow rate: 45 mL/min), and lyophilized to give compound **4-a** (118 mg, 32 μmmol, yield: 62.6%).

MS m/z: $C_{128}H_{188}N_{12}O_{52}$, found: [M+HCOO$^-$] = 2770.6.

Step 2

Compound **4-a** (110 mg, 4.0 μmol), DMAP (7.4 mg, 40 μmol), 3A molecular sieve (100 mg) and succinic anhydride (11.9 mg, 120 μmol) were dissolved in 5 mL THF. The mixture was stirred at 40 °C under argon for 4 h. After the reaction was complete, the molecular sieve was filtered out. The filtrate was concentrated to dryness by rotary evaporation, purified by reversed-phase preparative HPLC (column: Boston Green ODS 150 × 30 mm × 5 μm, conditions: 5-50% (A: water, B: $CH_3CN$), flow rate: 45 mL/min), and lyophilized to give compound **4-b** (80 mg, 28.3 μmmol, yield: 70.8%).

MS m/z: $C_{132}H_{192}N_{12}O_{55}$, [M-H]$^+$ found: 2824.6.

Step 3

The compound 38 (71 mg, 25 μmmol) obtained in the previous step was added to acetonitrile (5 mL). Then HBTU (19.0 mg, 50 μmol) was added, a surface aminomodified solid-phase support (CPG-NH2, 0.86 g) was added, and DIEA (16.2 mg, 125 μmol, 21.6 μL) was added. The mixture was reacted with shaking at 30 °C for 16 h. After the reaction was complete, the mixture was filtered and washed successively with methanol (5 mL × 4) and dichloromethane (5 mL × 4). The solid was added to pyridine:acetic anhydride (v:v = 4:1, 6.0 mL), and the mixture was reacted with shaking at 30 °C for another 16 h. After the reaction was complete, the mixture was filtered and washed successively with methanol and dichloromethane to give compound **4-c** linked to the solid-phase support (0.74 g).

**Example 10. Preparation of Control Compound L96**

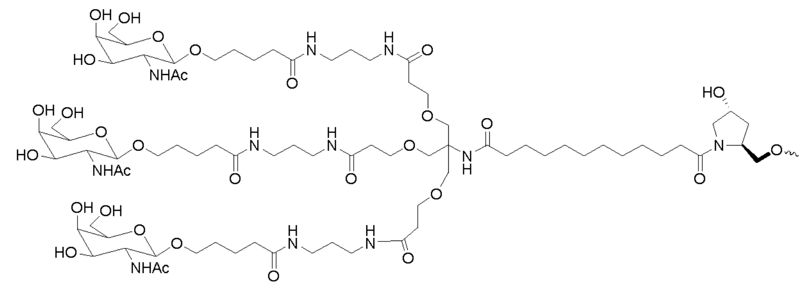

The control compound L96 was prepared using the method described in the patent WO2014025805A1.

**Example 11. Synthesis of Galactosamine Molecule Cluster-Conjugated siRNAs**

An siRNA used for testing, the siRNA targeting the mRNA of the mouse TTR gene (Molecular Therapy Vol. 26 No 3 March 2018), is shown below. A galactosamine molecule cluster M was linked to the 3' end of the SS strand by a covalent bond.

SS strand (5'-3'): CmsAmsGmUmGfUmUfCfUfUmGmCmUmCmUmAmUmAm Am-M
AS strand (5'-3'): UmsUfsAmUmAmGfAmGmCmAmAmGmAmAfCm AfCmUmGmsUmsUm

Reference was made to the aforementioned phosphoramidite solid-phase synthesis method, and the difference was that in synthesizing the SS strand, a CPG support to which a galactosamine cluster was linked was used in place of the Universal-CPG support.

The synthesis is briefly described below: Nucleoside phosphoramidite monomers were linked one by one according to the synthesis program on a Dr. Oligo48 synthesizer (Biolytic), starting at the synthesized CPG support to which a galactosamine cluster was linked described above. The nucleoside monomer materials 2'-F RNA, 2'-O-methyl RNA, and other nucleoside phosphoramidite monomers were purchased from Hongene, Shanghai or Genepharma, Suzhou. 5-Ethylthio-1H-tetrazole (ETT) was used as an activator (a 0.6 M solution in acetonitrile), a 0.22 M solution of PADS in acetonitrile and collidine (1: 1 by volume) (Kroma, Suzhou) as a sulfurizing agent, and iodopyridine/water solution (Kroma) as an oxidant.

After completion of solid phase synthesis, oligoribonucleotides were cleaved from the solid support and soaked in a solution of 28% ammonia water and ethanol (3:1) at 50 °C for 16 h. The mixture was centrifuged, and the supernatant was transferred to another centrifuge tube. After the supernatant was concentrated to dryness by evaporation, the residue was purified by C18 reversed-phase chromatography using 0.1 M TEAA and acetonitrile as the mobile phase, and DMTr was removed using 3% trifluoroacetic acid solution. The target oligonucleotides were collected, then lyophilized, identified as the target products by LC-MS, and quantified by UV (260 nm).

The resulting single-stranded oligonucleotides were paired in an equimolar ratio in a complementary manner and annealed with the AS strand. The final double-stranded siRNA was dissolved in 1× PBS, and the solution was adjusted to the concentration required for the experiment.

The galactosamine cluster-conjugated siRNAs were synthesized. The siRNAs used in the experiment target the mouse TTR mRNA. M =

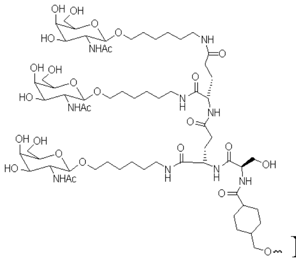 NAG1 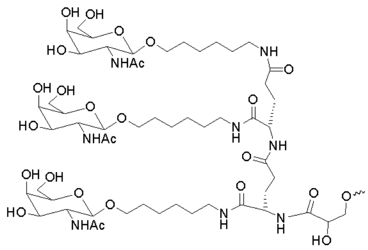 NAG2

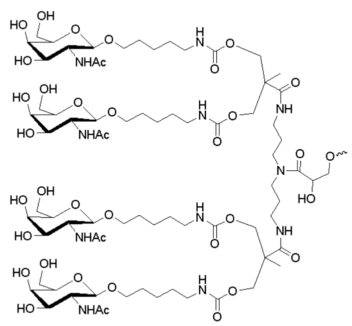

NAG3

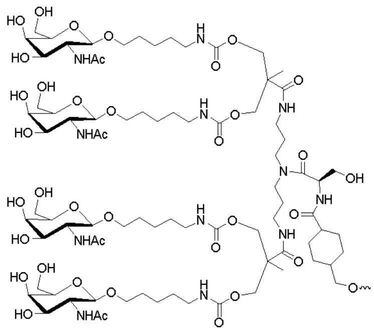

NAG4

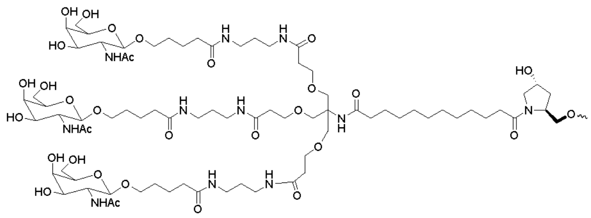

L96 (control compound).

Table 13. Targeting ligand activity evaluation siRNA numbering and sequences

| siRNA compound No. | SEQ ID NO | SS strand (5'-3') | SEQ ID NO | AS strand (5'-3') |
|---|---|---|---|---|
| S-1 | SEQ ID NO:49 | CmsAmsGmUmGfUmUfCfUfUmGmCmUmCmUmAmUmAmAm–NAG1 | SEQ ID NO:50 | UmsUfsAmUmAmGfAmGmCmAmAmGmAmAfCmAfCmUmGmsUmsUm |
| S-2 | SEQ ID NO:51 | CmsAmsGmUmGfUmUfCfUfUmGmCmUmCmUmAmUmAmAm–NAG2 | SEQ ID NO:52 | UmsUfsAmUmAmGfAmGmCmAmAmGmAmAfCmAfCmUmGmsUmsUm |
| S-3 | SEQ ID NO:53 | CmsAmsGmUmGfUmUfCfUfUmGmCmUmCmUmAmUmAmAm–NAG3 | SEQ ID NO:54 | UmsUfsAmUmAmGfAmGmCmAmAmGmAmAfCmAfCmUmGmsUmsUm |
| S-4 | SEQ ID NO:55 | CmsAmsGmUmGfUmUfCfUfUmGmCmUmCmUmAmUmAmAm–NAG4 | SEQ ID NO:56 | UmsUfsAmUmAmGfAmGmCmAmAmGmAmAfCmAfCmUmGmsUmsUm |
| S-L96 | SEQ ID NO:57 | CmsAmsGmUmGfUmUfCfUfUmGmCmUmCmUmAmUmAmAm-L96 | SEQ ID NO:58 | UmsUfsAmUmAmGfAmGmCmAmAmGmAmAfCmAfCmUmGmsUmsUm |
| S-1-2 | SEQ ID NO:59 | CmsAmsGmUmGfUmUfCfUfUmGmCmUmCmUmAmUmAmsAms–NAG1 | SEQ ID NO:60 | UmsUfsAmUmAmGfAmGmCmAmAmGmAmAfCmAfCmUmGmsUmsUm |
| S-L96-2 | SEQ ID NO:61 | CmsAmsGmUmGfUmUfCfUfUmGmCmUmCmUmAmUmAmAm-L96 | SEQ ID NO:62 | UmsUfsAmUmAmGfAmGmCmAmAmGmAmAfCmAfCmUmGmsUmsUm |

**Example 12. Inhibition of mRNA Expression in Primary Hepatocytes by Galactosamine Molecule Cluster-Conjugated siRNAs**

Fresh primary hepatocytes were isolated from mice using the method reported by Severgini et al. (Cytotechnology. 2012;64(2):187-195).

After being isolated, the primary hepatocytes were inoculated into a 24-well plate at 100 thousand cells per well. The test siRNAs were added at final concentrations of 50 nM, 10 nM, 2 nM, 0.4 nM, 0.08 nM, 0.016 nM, 0.0032 nM and 0.00064 nM. Subsequently, the primary hepatocytes were cultured at 37 °C with 5% $CO_2$ for 24 h. After 24 h, the mTTR's mRNA expression level was determined using the qPCR method.
As shown in FIG. 4, S-1, S-2, S-3 and S-4 all exhibited excellent inhibition efficiency against mTTR gene expression. The IC50 values of S-1 and S-4 are lower than those of the other two groups. The IC50 value of the control group S-L96 is 0.280 nM, while the IC50 value of S-1 is 0.131 nM and that of S-4 is 0.135 nM, which indicates that siRNAs conjugated with the S-1 and S-4 compounds have better efficiency of being taken by primary hepatocytes *in vitro* than the control group, and that the S-1 and S-4 compounds can more efficiently mediate the entry of siRNA into primary hepatocytes.

**Example 13. *In Vivo* Inhibition of mRNA Expression by Galactosamine Molecule Cluster-Conjugated siRNAs**

8-week-old C57BL/6 mice (Joinnbio, SPF, female) were injected subcutaneously with the siRNAs described above. On day 1, 100 μL of solution containing PBS or a dose (1 mg/kg (mpk) or 0.2 mpk) of a corresponding siRNA (S-L96, S-3, S-2, S-4 or S-1) formulated in PBS was injected subcutaneously into the loose skin on the neck and shoulder of the mice. In each group, 6 mice were given injections.
Three days after administration, the mice were sacrificed by cervical dislocation, and the mTTR's mRNA expression levels in the liver tissues of the mice were determined by qPCR.
As shown in FIG. 5, S-1, S-2, S-3 and S-4 all exhibited excellent inhibition efficiency against mTTR gene expression. When administered at 1 mpk and 0.2 mpk, S-2, S-3, S-4 and the control group S-L96 showed similar activity. S-1 showed better activity than the control group S-L96 when administered at 1 mpk and 0.2 mpk.

**Example 14. Long-Term Effectiveness Experiment for *In Vivo* Inhibition of mRNA Expression by Galactosamine Molecule Cluster-Conjugated siRNAs**

Two siRNA compounds **S-1-2** and **S-L96-2** (see Table 13 for their SS and AS strands) were synthesized using the synthesis method in Example 11 and used for *in vivo* administration to mice. 8-week-old C57BL/6 mice (Joinnbio, SPF, female) were injected subcutaneously with the galactosamine molecule cluster-conjugated siRNAs described above. On day 0, 100 μL of solution containing PBS (referred to as the Mock group, i.e., the blank control group) or a dose (1 mg/kg (mpk)) of a corresponding galactosamine molecule cluster-conjugated siRNA (S-1 or S-L96) formulated in PBS was injected subcutaneously into the loose skin on the neck and shoulder of the mice. In each group, 9 mice were given injections.
Three mice were sacrificed by cervical dislocation 7 days, 14 days, and 28 days after administration. Two samples of liver tissue were collected from each mouse, and the mTTR's mRNA expression levels in the liver tissues of the mice were determined by qPCR.
7 days, 14 days and 28 days after administration, the mRNA ratios of **S-1-2** relative to the PBS group were 0.13, 0.12 and 0.21, respectively, and the mRNA ratios of **S-L96-2** relative to the PBS group were 0.17, 0.13 and 0.29, respectively.
FIG. 6 also shows the mRNA expression levels in mouse liver tissue 7 days, 14 days and 28 days after administration of compounds **S-1-2** and **S-L96-2.**
The results show that the siRNA administered still showed efficient mRNA inhibition on day 28, and that **S-1-2** had higher inhibition than the control group **S-L96-2.**

**III. Activity Verification**

**Example 15. Synthesis of siRNA Conjugates**

Nucleoside monomers were linked one by one in the 3'-5' direction in the order in which the nucleotides were arranged using the solid-phase phosphoramidite method. Each time a nucleoside monomer was linked, four reactions-deprotection, coupling, capping, oxidation and sulfurization-were involved. The sense strand and the antisense strand were synthesized under identical conditions.
Oligonucleotide synthesis instrument models: a Biolytic Dr. Oligo 48 oligonucleotide solid-phase synthesizer and a GE oligo pilot100 oligonucleotide solid-phase synthesizer.

Table 14. Reagents used in the synthesis of siRNA conjugates

| Reagent name | Reagent composition | Specification | Use | Manufacturer |
|---|---|---|---|---|
| ACT | 0.6M ETT in ACN | 4L | Catalyst | Kroma |
| Cap A | N-methylimidazole:acetonitrile 2:8 | 4L | Capping reagent | Kroma |
| Cap B1 | Acetic anhydride:acetonitrile 40:60 | 4L | | Kroma |
| Cap B2 | Pyridine:acetonitrile 60:40 | 4L | | Kroma |

Detection method: The purity of the sense and antisense strands described above was determined and the molecular weights were analyzed using Waters Acquity UPLC-SQD2 LCMS (column: ACQUITY UPLC BEH C18). The found values agreed with the calculated values, which indicates that what had been synthesized were sense strands conjugated by molecules at the 3' end and antisense strands. The siRNAs had the sense and antisense strands shown in Table 15.

Table 15. Synthesis of siRNA and siRNA conjugate sequences

| Double strand No. | SEQ ID NO | Sense strand 5'-3' | SEQ ID NO | Antisense strand 5'-3 |
|---|---|---|---|---|
| Naked sequence 1 | SEQ ID NO:63 | GUG UGC ACU UCG CUU CACC | SEQ ID NO:64 | AGU GAA GCG AAG UGC ACA CGG |
| TRD006890 | SEQ ID NO:65 | GmsUmsGm UmGfCm AfCfUf UmCmGm CmUmUm CmAmCms Cms-NAG1 | SEQ ID NO:66 | AmsGfsUm GfAmAf (-)hmpNA(G)CmGm AfAmGf UmGfCm AfCmAf CmsGmsGm |
| TRD006924 | SEQ ID NO:67 | GmsUmsGm UmGmCm AfCfUf UmCmGm CmUmUm CmAmCms Cms-NAG1 | SEQ ID NO:68 | AmsGfsUm GfAmAf (-)hmpNA(G)CmGm AfAmGf UmGfCm AfCmAf CmsGmsGm |
| Naked sequence 2 | SEQ ID NO:69 | CUU UUG UCU UUG GGU AUA U | SEQ ID NO:70 | AUA UAC CCA AAG ACA AAA GAA |
| TRD006896 | SEQ ID | CmsUmsUm UmUfGm | SEQ ID | AmsUfsAm UfAmCf (- |
| | NO:71 | UfCfUf UmUmGm GmGmUm AmUmAms Ums-NAG1 | NO:72 | )hmpNA(C)CmAm AfAmGf AmCfAm AfAmAf GmsAmsAm |
| Naked sequence 3 | SEQ ID NO:73 | UUA CCA AUU UUC UUU UGU U | SEQ ID NO:74 | AAC AAA AGA AAA UUG GUA ACA |
| TRD006897 | SEQ ID NO:75 | UmsUmsAm CmCfAm AfUfUf UmUmCm UmUmUm UmGmUms Ums-NAG1 | SEQ ID NO:76 | AmsAfsCm AfAmAf (-)hmpNA(A)GmAm AfAmAf UmUfGm GfUmAf AmsCmsAm |
| Naked sequence 4 | SEQ ID NO:77 | CGU GUG CAC UUC GCU UCA C | SEQ ID NO:78 | AUG AAG CGA AGU GCA CAC GGU |
| TRD006905 | SEQ ID NO:79 | CmsGmsUm GmUfGm CfAfCf UmUmCm GmCmUm UmCmAms Cms-NAG1 | SEQ ID NO:80 | AmsUfsGm AfAmGf (-)hmpNA(C)GmAm AfGmUf GmCfAm CfAmCf GmsGmsUm |
| Naked sequence 5 | SEQ ID NO:81 | UGU CUU UGG GUA UAC AUU U | SEQ ID NO:82 | AAA UGU AUA CCC AAA GAC AAA |

(continued)

| Double strand No. | SEQ ID NO | Sense strand 5'-3' | SEQ ID NO | Antisense strand 5'-3 |
|---|---|---|---|---|
| TRD006894 | SEQ ID NO:83 | UmsGmsUm CmUfUm UfGfGf GmUmAm UmAmCm AmUmUms Ums-NAG1 | SEQ ID NO:84 | AmsAfsAm UfGmUf (-)hmpNA(A)UmAm CfCmCf AmAfAm GfAmCf AmsAmsAm |
| Naked sequence 6 | SEQ ID NO:85 | CUU UUG UCU UUG GGU AUA C | SEQ ID NO:86 | AUA UAC CCA AAG ACA AAA GAA |
| TRD006895 | SEQ ID NO:87 | CmsUmsUm UmUfGm UfCfUf UmUmGm GmGmUm AmUmAms Cms-NAG1 | SEQ ID NO:88 | AmsUfsAm UfAmCf (-)hmpNA(C)CmAm AfAmGf AmCfAm AfAmAf GmsAmsAm |
| Naked sequence 7 | SEQ ID NO:89 | CAU CUU CUU GUU GGU UCU U | SEQ ID NO:90 | AAG AAC CAA CAA GAA GAU GAG |
| TRD006899 | SEQ ID NO:91 | CmsAmsUm CmUfUm CfUfUf GmUmUm GmGmUm UmCmUms Ums-NAG1 | SEQ ID NO:92 | AmsAfsGm AfAmCf (-)hmpNA(C)AmAm CfAmAf GmAfAm GfAmUf GmsAmsGm |
| Naked sequence 8 | SEQ ID NO:93 | UGU CUG CGG CGU UUU AUC A | SEQ ID NO:94 | UGA UAA AAC GCC GCA GAC ACA |
| TRD006900 | SEQ ID NO:95 | UmsGmsUm CmUfGm CfGfGf CmGmUm UmUmUm AmUmCms Ams-NAG1 | SEQ ID NO:96 | UmsGfsAm UfAmAf (-)hmpNA(A)AmCm GfCmCf GmCfAm GfAmCf AmsCmsAm |
| Naked sequence 9 | SEQ ID NO:97 | UGC ACU UCG CUU CAC CUC U | SEQ ID NO:98 | AGA GGU GAA GCG AAG UGC ACA |
| TRD006906 | SEQ ID NO:99 | UmsGmsCm AmCfUm UfCfGf CmUmUm CmAmCm CmUmCms Ums-NAG1 | SEQ ID NO:100 | AmsGfsAm GfGmUf (-)hmpNA(G)AmAm GfCmGf AmAfGm UfGmCf AmsCmsAm |
| Naked sequence 10 | SEQ ID NO:101 | GCA CUU CGC UUC ACC UCU G | SEQ ID NO:102 | UAG AGG UGA AGC GAA GUG CAC |
| TRD006907 | SEQ ID NO:103 | GmsCmsAm CmUfUm CfGfCf UmUmCm AmCmCm UmCmUms Gms-NAG1 | SEQ ID NO:104 | UmsAfsGm AfGmGf (-)hmpNA(U)GmAm AfGmCf GmAfAm GfUmGf CmsAmsCm |
| Naked sequence 11 | SEQ ID NO:105 | GGC GCU GAA UCC UGC GGA C | SEQ ID NO:106 | AUC CGC AGG AUU CAG CGC CGA |
| TRD006908 | SEQ ID NO:107 | GmsGmsCm GmCfUm GfAfAf UmCmCm UmGmCm GmGmAms Cms-NAG1 | SEQ ID NO:108 | AmsUfsCm CfGmCf (-)hmpNA(A)GmGm AfUmUf CmAfGm CfGmCf CmsGmsAm |
| AD66810 | SEQ ID NO:109 | GmsUmsGm UmGfCm AfCfUf UmCmGm CmUmUm CmAmCm Am-L96 | SEQ ID NO:110 | UmsGfsUm GmAmAf GmCfGf AmAmGm UmGfCm AfCmAm CmsUmsUm |

(continued)

| Double strand No. | SEQ ID NO | Sense strand 5'-3' | SEQ ID NO | Antisense strand 5'-3 |
|---|---|---|---|---|
| AD81890 | SEQ ID NO:111 | GmsUmsGm UmGfCm AfCfUf UmCmGm CmUmUm CmAmCm Am-L96 | SEQ ID NO:112 | UmsGfsUm GmAmAGNA(A) GmCfGf AmAmGm UmGfCm AfCmAm CmsUmsUm |
| TRD006912 | SEQ ID NO:113 | GmsUmsGm UmGfCm AfCfUf UmCmGm CmUmUm CmAmCm Am-L96 | SEQ ID NO:114 | UmsGfsUm GmAmAf G(GNA)CfGf AmAmGm UmGfCm AfCmAm CmsUmsUm |

wherein the nucleotide synthesized using 2-hydroxymethyl-1,3-propanediol as the starting material was defined as hmpNA;

(-)hmpNA(A) was obtained by solid-phase synthesis using the nucleoside phosphoramidite monomer 1-1a of example section 1.1;
(-)hmpNA(G) was obtained by solid-phase synthesis using the nucleoside phosphoramidite monomer 1-6a of example section 1.6;
(-)hmpNA(C) was obtained by solid-phase synthesis using the nucleoside phosphoramidite monomer 1-8a of example section 1.8;
(-)hmpNA(U) was obtained by solid-phase synthesis using the nucleoside phosphoramidite monomer 1-7a of example section 1.7.

In Table 15, the structure of NAG1 is as shown in Example 11.

**Example 16. siRNA Activity and Off-Target Level Validation**

*In vitro* molecular level simulation on-target and off-target level screening was performed on the compounds of the present disclosure in HEK293A cells.

On-target sequences and off-target sequences corresponding to the siRNA sequences were constructed and inserted into psiCHECK-2 plasmids. The plasmids contained the renilla luciferase gene and the firefly luciferase gene. The plasmids were dual reporter gene systems. The target sequence of siRNA was inserted into the 3' UTR region of the renilla luciferase gene. The activity of siRNA for the target sequence was reflected by measuring the renilla luciferase expression after calibration with firefly luciferase. The measurement used Dual-Luciferase Reporter Assay System (Promega, E2940). HEK293A cells were cultured at 37 °C with 5% $CO_2$ in a DMEM high glucose medium containing 10% fetal bovine serum. 24 h prior to transfection, the HEK293A cells were inoculated into a 96-well plate at a density of 10 thousand cells per well. Each well contained 100 μL of medium.

The cells were co-transfected with siRNA and the corresponding plasmid using Lipofectamine2000 (ThermoFisher, 11668019) according to the instructions. 0.2 μL of Lipofectamine2000 was used for each well. The transfection amount of plasmid was 10 ng per well. For the on-target sequence plasmids and the off-target sequence plasmids, a total of 5 concentration points or 11 concentration points of siRNA were set up. In cases where 5 concentration points were set up, the highest concentration point in transfection was 10 nM, and 10-fold serial dilution was carried out. In cases where 11 concentration points were set up, the highest concentration point final concentration in transfection was 20 nM, and 3-fold serial dilution was carried out. 24 h after transfection, the off-target levels were determined using Dual-Luciferase Reporter Assay System (Promega, E2940).

The results in Table 16 to Table 19 show that the compounds TRD006890 and TRD006924 of the present disclosure have low off-target activity while having high on-target activity, and are both significantly better than the positive control AD81890.

The results in Table 20 show that the GNA modification was significantly sequence sitedependent. When the GNA modification site was in 5' position 7 (TRD006912) or position 6 (AD81890) of the AS strand, off-target activity was higher, which indicates greater toxicity. Further, TRD006912 saw a further decrease in on-target activity (from 0.68 to 0.96) compared to AD81890.

Table 16. TRD006890 activity and off-target IC50 value

| TRD006890 | Remaining percentage of target gene's mRNA expression (mean) | | | | | | | | | | | |
|---|---|---|---|---|---|---|---|---|---|---|---|---|
| Transfection concentration nM | 20.000 | 6.67 | 2.22 | 0.74 | 0.25 | 0.082 | 0.027 | 0.0091 | 0.0030 | 0.0010 | 0.0003 | IC50 value (nM) |
| On-target activity | 0.28 | 0.21 | 0.14 | 0.16 | 0.24 | 0.44 | 0.73 | 0.95 | 0.99 | 1.02 | 1.00 | 0.08 |
| Off-target activity | 1.05 | 0.92 | 0.97 | 1.07 | 1.11 | 1.06 | 1.07 | 1.10 | 1.05 | 1.13 | 1.05 | >3000 |

Table 17. TRD006924 activity IC50 values

| TRD006924 | Remaining percentage of target gene's mRNA expression (mean) | | | | | | | | | | | |
|---|---|---|---|---|---|---|---|---|---|---|---|---|
| Transfection concentration nM | 20.000 | 6.67 | 2.22 | 0.74 | 0.25 | 0.082 | 0.027 | 0.0091 | 0.0030 | 0.0010 | 0.0003 | IC50 value (nM) |
| On-target activity | 0.40 | 0.25 | 0.18 | 0.17 | 0.21 | 0.40 | 0.66 | 0.83 | 0.92 | 0.99 | 1.00 | 0.060 |
| Off-target activity | 0.88 | 0.83 | 0.92 | 1.06 | 1.04 | 1.04 | 1.01 | 1.02 | 1.04 | 0.98 | 1.00 | 133.4 |

Table 18. AD81890 activity and off-target IC50 value

| AD81890 | Remaining percentage of target gene's mRNA expression (mean) | | | | | | | | | | |
|---|---|---|---|---|---|---|---|---|---|---|---|
| Transfection concentration nM | 20.0 | 6.67 | 2.22 | 0.74 | 0.25 | 0.082 | 0.027 | 0.0091 | 0.0030 | 0.0010 | 0.0003 | IC50 value (nM) |
| On-target activity | 0.12 | 0.11 | 0.23 | 0.41 | 0.75 | 0.93 | 1.00 | 1.05 | 1.02 | 1.04 | 1.03 | 0.68 |
| Off-target activity | 0.23 | 0.36 | 0.60 | 0.87 | 0.95 | 0.95 | 0.89 | 0.92 | 0.95 | 0.95 | 0.98 | 3.93 |

Table 19. AD66810 activity and off-target IC50 value

| AD66810 | Remaining percentage of target gene's mRNA expression (mean) | | | | | | | | | | | |
|---|---|---|---|---|---|---|---|---|---|---|---|---|
| Transfection concentration nM | 20.000 | 6.67 | 2.22 | 0.74 | 0.25 | 0.082 | 0.027 | 0.0091 | 0.0030 | 0.0010 | 0.0003 | IC50 value (nM) |
| On-target activity | 0.07 | 0.05 | 0.09 | 0.17 | 0.46 | 0.73 | 0.92 | 0.91 | 1.01 | 1.02 | 1.02 | 0.2 |
| Off-target activity | 0.05 | 0.06 | 0.14 | 0.30 | 0.63 | 0.88 | 0.96 | 0.96 | 1.03 | 1.07 | 1.12 | 0.4 |

Table 20. TRD006912 activity and off-target IC50 value

| TRD006912 | Remaining percentage of target gene's mRNA expression (mean) | | | | | | | | | | | |
|---|---|---|---|---|---|---|---|---|---|---|---|---|
| Transfection concentration nM | 20.000 | 6.67 | 2.22 | 0.74 | 0.25 | 0.082 | 0.027 | 0.0091 | 0.0030 | 0.0010 | 0.0003 | IC50 value (nM) |
| On-target activity | 0.26 | 0.22 | 0.31 | 0.51 | 0.75 | 0.91 | 0.95 | 0.99 | 1.05 | 1.02 | 0.98 | 0.96 |
| Off-target activity | 0.82 | 0.82 | 0.93 | 0.91 | 0.90 | 0.94 | 0.86 | 0.93 | 0.94 | 1.01 | 1.04 | 64.46 |

**Example 17. Evaluation of siRNA Compounds' *In Vitro* Anti-HBV Activity Using HepG2.2.15 Cells**

On day 1, HepG2.2.15 cells were inoculated into a 96-well plate at 20 thousand cells per well. While the cells were inoculated, the HepG2.2.15 cells were transfected with different concentrations of siRNA using RNAiMax. On day 4, the cell culture supernatant was collected and tested for HBsAg by ELISA (the remaining supernatant was frozen for later use). Finally, the cells were collected, and the RNA was extracted from the cells. The total HBV RNA (including 3.5kb+2.4kb+2.1kb+0.7kb RNA) and 3.5kb HBV RNA (including pgRNA+preCore RNA) were measured by RT-PCR, and meanwhile the GAPDH gene's RNA was measured as an internal reference. Five concentration points were set for the test compounds, and 2 replicate wells were assayed in parallel. The final concentration of DMSO in the culture was 0.5%.

Percent inhibition was calculated using the formulas below:

% HBsAg inhibition = (1 - HBsAg content of sample/HBsAg content of DMSO control group) × 100

% HBV RNA inhibition = (1 - HBV's RNA content of sample/HBV's RNA content of DMSO control group) × 100

% cell viability = (absorbance of sample - absorbance of culture control)/(absorbance of DMSO control - absorbance of culture control) × 100.

$EC_{50}$ values were calculated by analysis using Graphpad Prism software (four parameter logistic equations).

Table 21. Antiviral activity of compounds in HepG2.2.15

| Compound No. | HBsAg $EC_{50}$ (nM) | pgRNA IC50 (nM) | Total RNA IC50 (nM) |
|---|---|---|---|
| TRD006890 | 0.003 | 0.575 | 0.612 |
| TRD006924 | 0.008 | 0.010 | 0.068 |
| AD81890 | 0.053 | 0.620 | 0.207 |
| AD66810 | 0.092 | 0.163 | 1.639 |
| TRD006894 | 0.027 | 0.409 | 0.752 |
| TRD006895 | 0.1693 | 0.707 | 0.790 |
| TRD006896 | 0.002 | 0.373 | 0.584 |
| TRD006897 | 0.008 | 0.122 | 0.211 |
| TRD006899 | NA | 2.805 | NA |
| TRD006900 | 0.142 | NA | 1.640 |
| TRD006905 | NA | 0.228 | 0.845 |
| TRD006906 | 0.034 | 0.576 | 0.521 |
| TRD006907 | 0.020 | 0.836 | 0.847 |
| TRD006908 | 0.022 | 0.331 | 0.311 |
| NA: undetectable. | | | |

As shown in Table 21, referring to the control compounds AD66810 and AD81890 and the test indicators for antiviral activity, the test compounds TRD006890, TRD006894, TRD006895, TRD006896, TRD006897, TRD006899, TRD006900, TRD006905, TRD006906, TRD006907 and TRD006908 exhibited excellent antiviral activity on HepG2.2.15 cells.

**Example 18. Evaluation of siRNA Compounds' *In Vitro* Anti-HBV Infection Activity Using Primary Human Hepatocytes**

On day 0, primary human hepatocytes were inoculated into a 48-well plate at 120 thousand cells per well. While the cells were inoculated, test compounds were added. siRNA was transferred into primary human hepatocytes in a free uptake manner. siRNA was 5-fold diluted from a starting concentration of 200 nM to 7 concentrations. On day 1, the type D HBV was added to infect the primary human hepatocytes. On day 2, day 4 and day 6, the media were replaced with fresh media. The final concentration of DMSO in the cultures was 2%. On day 8, the cell culture supernatant was collected and tested for HBV DNA by qPCR, and for HBeAg and HBsAg by ELISA. Seven concentration points were set for the test compounds and the control compound, and 2 replicate wells were assayed in parallel.

Table 22. Antiviral activity of compounds in primary human hepatocytes

| **Compound** | **Concentration** (nM) | **HBsAg inhibition** (%) | **HBeAg inhibition (%)** | **HBV DNA inhibition (%)** |
|---|---|---|---|---|
| AD81890 | 200 | 87.60±0.00 | 81.95±0.92 | 88.48±2.13 |
| TRD006924 | | 97.55±0.07* | 96.05±0.21* | 96.98±0.08* |
| AD81890 | 40 | 87.55±1.48 | 80.95±1.20 | 88.78±2.21 |
| TRD006924 | | 95.80±0.42* | 92.55±0.64* | 95.51±0.76 |
| AD81890 | 8 | 77.10±2.12 | 68.90±2.26 | 75.47±2.49 |
| TRD006924 | | 89.35±0.78* | 82.80±0.28* | 90.34±1.27* |
| AD81890 | 1.6 | 56.70±0.57 | 43.55±3.32 | 56.74±4.33 |
| TRD006924 | | 66.30±2.26* | 59.20±0.71* | 74.37±3.09* |

* indicates that there was a significant difference ($p < 0.05$) in the results of the same test indicator between TRD006924 and AD81890 when they were at the same concentration.

As shown in table 22, referring to the control compound AD81890 and the test indicators for antiviral activity, the test compound TRD006894 exhibited significantly better antiviral activity on primary human hepatocytes.

**Example 19. *In Vivo* Anti-HBV Activity of siRNA Compounds**

On day 28, mice (C57BL/6, male) were injected with rAAV8-1.3HBV via tail vein. On day 14 and day 21 after virus injection, blood was collected from the submandibular veins of all the experimental mice so as to collect plasma. The HBV DNA content, the HBeAg content and the HBsAg content of the plasma were measured.

On day 28 after virus injection, the mice were randomized into groups based on the test results of the plasma samples on day 14 and day 21 after virus injection.

All the mice were dosed subcutaneously once at 3 mg/kg on day 28 after virus injection. Before administration, submandibular blood was collected from all the mice so as to collect plasma, which was then tested for HBV DNA, HBeAg, HBsAg and ALT. The day of administration was day 0. On day 7, day 14 and day 21 after administration, submandibular blood was collected from all the mice so as to collect plasma for tests.

HBV DNA in the plasma was quantified by qPCR. HBeAg and HBsAg in the plasma were quantified by ELISA.

On day 7, compared to the control compound AD81890, the test compound TRD006894 exhibited excellent antiviral activity in mice. The test compound TRD006894 can maintain the activity *in vivo* for a long time: it can effectively inhibit the virus activity on both day 14 and day 21.

Table 23. *In vivo* anti-HBV activity of compounds

| **Compound** | **Time (days)** | **HBsAg inhibition (%)** | **HBeAg inhibition (%)** | **HBV DNA inhibition (%)** |
|---|---|---|---|---|
| PBS | 7 | 62.2±16.3 | 92.1±9.3 | 66.0±22.0 |
| AD81890 | | | | 20.8±8.5 |
| TRD006924 | | 7.3±4.6 # | 33.4±5.7 # | 7.8±6.2 *# |
| PBS | 14 | 157.8±73.2 | 199.7±88.4 | 94.8±38.0 |
| TRD006924 | | 11.3±6.3 # | 58.8±12.1 # | 24.5±16.9 # |

(continued)

| Compound | Time (days) | HBsAg inhibition (%) | HBeAg inhibition (%) | HBV DNA inhibition (%) |
|---|---|---|---|---|
| PBS | 21 | 93.8±47.4 | 93.2±9.8 | 143.2±57.9 |
| TRD006924 | | 15.9±12.0 # | 58.3±13.2 # | 39.2±33.6 # |
| * indicates that there was a significant difference ($p < 0.05$) in the results of the same test indicator on the same day of testing between TRD006924 and AD81890 when they were at the same concentration. # indicates that there was a significant difference ($p < 0.05$) in the results of the same test indicator on the same day of testing between TRD006924 and PBS. | | | | |

**Example 20. Design and Synthesis of Human ApoC3 siRNAs**

1) **siRNA design:** the human ApoC3 gene (NM_000040.3) was used as the target gene to meet the general rules for active siRNA to design 19/21nt siRNAs. The sequences of the unmodified sense strand and antisense strand are detailed in Table 14, wherein the SS strand and the AS strand of the unmodified siRNA are both unmodified.
2) **siRNA synthesis:** siRNAs were synthesized on a Dr.Oligo48 synthesizer (Biolytic) in a specification of 200 nmol using universal solid support (Biocomma, Shenzhen)-mediated phosphoramidite chemistry. The target oligonucleotides were collected, then lyophilized, identified as the target products by LC-MS, and quantified by UV (260 nm).

In synthesizing the modified nucleotide in 5' position 7 of the AS strand, the original nucleotide of the parent sequence was replaced with the phosphoramidite monomers synthesized in Example 1. The sequences of the antisense strands modified in 5' position 7 are detailed in Table 14, wherein W' is selected from the group consisting of

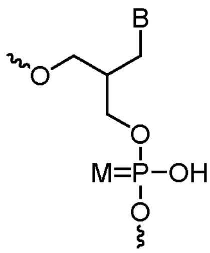 , 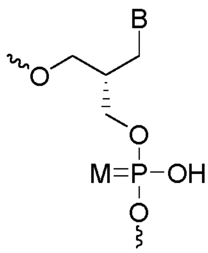 and 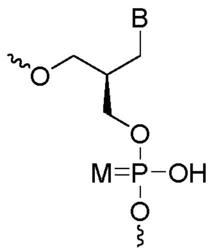 ;

wherein M is O or S; wherein B is selected from a natural base in the corresponding position in Table 24.

The sequences of the sense strands and the antisense strands of ApoC3 siRNAs modified by 2'-fluoro, 2'-methoxy, etc. are detailed in Table 25, and the sequences of the sense strands and the antisense strands of ApoC3 siRNA conjugates are detailed in Table 26.

The sense strands and the antisense strands were synthesized by following the steps described above and were annealed in an equimolar ratio to form double-stranded structures by hydrogen bonding. Finally, the resulting double-stranded siRNAs were dissolved in 1× PBS, and the solutions were adjusted to the concentrations required for the experiment.

Table 24. Sense strans and antisense strands of human ApoC3 siRNAs

| SEQ ID NO | SS strand (5'-3') | SEQ ID NO | Unmodified AS strand (5'-3') | SEQ ID NO | AS strand with chemical modification in position 7 (5'-3') |
|---|---|---|---|---|---|
| SEQ ID NO:115 | GCCUCUGCCCG AGCUUCAA | SEQ ID NO:116 | UUGAAGCUCGG GCAGAGGCCA | SEQ ID NO:117 | UUGAAGW'UCGG GCAGAGGCCA |
| SEQ ID NO:118 | GCUUCAUGCA GGGUUACAU | SEQ ID NO:119 | AUGUAACCCUG CAUGAAGCUG | SEQ ID NO:120 | AUGUAAW'CCUG CAUGAAGCUG |
| SEQ ID NO:121 | UGAGCAGCGU GCAGGAGUU | SEQ ID NO:122 | AACUCCUGCAC GCUGCUCAGU | SEQ ID NO:123 | AACUCCW'GCAC GCUGCUCAGU |

(continued)

| SEQ ID NO | SS strand (5'-3') | SEQ ID NO | Unmodified AS strand (5'-3') | SEQ ID NO | AS strand with chemical modification in position 7 (5'-3') |
|---|---|---|---|---|---|
| SEQ ID NO:124 | CAGUUCCCUG AAAGACUAU | SEQ ID NO:125 | AUAGUCUUUCA GGGAACUGAA | SEQ ID NO:126 | AUAGUCW'UUCA GGGAACUGAA |
| SEQ ID NO:127 | AAGUCCACCU GCCUAUCCA | SEQ ID NO:128 | UGGAUAGGCAG GUGGACUUGG | SEQ ID NO:129 | UGGAUAW'GCAG GUGGACUUGG |
| SEQ ID NO:130 | UCUCAGUGCU CUCCUACCU | SEQ ID NO:131 | AGGUAGGAGAG CACUGAGAAU | SEQ ID NO:132 | AGGUAGW'AGAG CACUGAGAAU |
| SEQ ID NO:133 | GGCAUGCUGG CCUCCCAAU | SEQ ID NO:134 | AUUGGGAGGCC AGCAUGCCUG | SEQ ID NO:135 | AUUGGGW'GGCC AGCAUGCCUG |
| SEQ ID NO:136 | GCAUGCUGGC CUCCCAAUA | SEQ ID NO:137 | UAUUGGGAGGC CAGCAUGCCU | SEQ ID NO:138 | UAUUGGW'AGGC CAGCAUGCCU |
| SEQ ID NO:139 | CUGGCCUCCCA AUAAAGCU | SEQ ID NO:140 | AGCUUUAUUGG GAGGCCAGCA | SEQ ID NO:141 | AGCUUUW'UUGG GAGGCCAGCA |
| SEQ ID NO:142 | GGCCUCCCAAU AAAGCUGA | SEQ ID NO:143 | UCAGCUUUAUU GGGAGGCCAG | SEQ ID NO:144 | UCAGCUW'UAUU GGGAGGCCAG |
| SEQ ID NO:145 | UAAAGCUGGA CAAGAAGCU | SEQ ID NO:146 | AGCUUCUUGUC CAGCUUUAUU | SEQ ID NO:147 | AGCUUCW'UGUC CAGCUUUAUU |
| SEQ ID NO:148 | UAUUCUCAGU GCUCUCCUA | SEQ ID NO:149 | UAGGAGAGCAC UGAGAAUACU | SEQ ID NO:150 | UAGGAGW'GCAC UGAGAAUACU |
| SEQ ID NO:151 | CCGUUAAGGA CAAGUUCUU | SEQ ID NO:152 | AAGAACUUGUC CUUAACGGUG | SEQ ID NO:153 | AAGAACW'UGUC CUUAACGGUG |
| SEQ ID NO:154 | CUGCGAGCUCC UUGGGUCU | SEQ ID NO:155 | AGACCCAAGGA GCUCGCAGGA | SEQ ID NO:156 | AGACCCW'AGGA GCUCGCAGGA |
| SEQ ID NO:157 | ACAGUAUUCU CAGUGCUCU | SEQ ID NO:158 | AGAGCACUGAG AAUACUGUCC | SEQ ID NO:159 | AGAGCAW'UGAG AAUACUGUCC |
| SEQ ID NO:160 | UUCUCAGUGC UCUCCUACU | SEQ ID NO:161 | AGUAGGAGAGC ACUGAGAAUA | SEQ ID NO:162 | AGUAGGW'GAGC ACUGAGAAUA |
| SEQ ID NO:163 | AAGGGACAGU AUUCUCAGU | SEQ ID NO:164 | ACUGAGAAUAC UGUCCCUUUU | SEQ ID NO:165 | ACUGAGW'AUAC UGUCCCUUUU |
| SEQ ID NO:166 | AAUAAAGCUG GACAAGAAA | SEQ ID NO:167 | UUUCUUGUCCA GCUUUAUUGG | SEQ ID NO:168 | UUUCUUW'UCCA GCUUUAUUGG |
| SEQ ID NO:169 | GACAAGUUCU CUGAGUUCU | SEQ ID NO:170 | AGAACUCAGAG AACUUGUCCU | SEQ ID NO:171 | AGAACUW'AGAG AACUUGUCCU |
| SEQ ID NO:172 | CGAGGAUGCC UCCCUUCUU | SEQ ID NO:173 | AAGAAGGGAGG CAUCCUCGGC | SEQ ID NO:174 | AAGAAGW'GAGG CAUCCUCGGC |
| SEQ ID NO:175 | ACUACUGGAG CACCGUUAA | SEQ ID NO:176 | UUAACGGUGCU CCAGUAGUCU | SEQ ID NO:177 | UUAACGW'UGCU CCAGUAGUCU |
| SEQ ID NO:178 | AUAAAGCUGG ACAAGAAGU | SEQ ID NO:179 | ACUUCUUGUCC AGCUUUAUUG | SEQ ID NO:180 | ACUUCUW'GUCC AGCUUUAUUG |
| SEQ ID NO:181 | AGGGACAGUA UUCUCAGUA | SEQ ID NO:182 | UACUGAGAAUA CUGUCCCUUU | SEQ ID NO:183 | UACUGAW'AAUA CUGUCCCUUU |

(continued)

| SEQ ID NO | SS strand (5'-3') | SEQ ID NO | Unmodified AS strand (5'-3') | SEQ ID NO | AS strand with chemical modification in position 7 (5'-3') |
|---|---|---|---|---|---|
| SEQ ID NO:184 | GCCUCCCAAUA AAGCUGGA | SEQ ID NO:185 | UCCAGCUUUAU UGGGAGGCCA | SEQ ID NO:186 | UCCAGCW'UUAU UGGGAGGCCA |
| SEQ ID NO:187 | UGCUGGCCUCC CAAUAAAA | SEQ ID NO:188 | UUUUAUUGGGA GGCCAGCAUG | SEQ ID NO:189 | UUUUAUW'GGGA GGCCAGCAUG |
| SEQ ID NO:190 | AUUCUCAGUG CUCUCCUAU | SEQ ID NO:191 | AUAGGAGAGCA CUGAGAAUAC | SEQ ID NO:192 | AUAGGAW'AGCA CUGAGAAUAC |
| SEQ ID NO:193 | UUCAGUUCCC UGAAAGACU | SEQ ID NO:194 | AGUCUUUCAGG GAACUGAAGC | SEQ ID NO:195 | AGUCUUW'CAGG GAACUGAAGC |
| SEQ ID NO:196 | CAUGCUGGCC UCCCAAUAA | SEQ ID NO:197 | UUAUUGGGAGG CCAGCAUGCC | SEQ ID NO:198 | UUAUUGW'GAGG CCAGCAUGCC |
| SEQ ID NO:199 | UAUUCUCAGU GCUCUCCUU | SEQ ID NO:200 | UAGGAGAGCAC UGAGAAUACU | SEQ ID NO:201 | UAGGAGW'GCAC UGAGAAUACU |
| SEQ ID NO:202 | UAUUCUCAGU GCUCUCCUC | SEQ ID NO:203 | UAGGAGAGCAC UGAGAAUACU | SEQ ID NO:204 | UAGGAGW'GCAC UGAGAAUACU |
| SEQ ID NO:205 | UAUUCUCAGU GCUCUCCUG | SEQ ID NO:206 | UAGGAGAGCAC UGAGAAUACU | SEQ ID NO:207 | UAGGAGW'GCAC UGAGAAUACU |
| SEQ ID NO:208 | CCGUUAAGGA CAAGUUCUA | SEQ ID NO:209 | AAGAACUUGUC CUUAACGGUG | SEQ ID NO:210 | AAGAACW'UGUC CUUAACGGUG |
| SEQ ID NO:211 | CCGUUAAGGA CAAGUUCUC | SEQ ID NO:212 | AAGAACUUGUC CUUAACGGUG | SEQ ID NO:213 | AAGAACW'UGUC CUUAACGGUG |
| SEQ ID NO:214 | CCGUUAAGGA CAAGUUCUG | SEQ ID NO:215 | AAGAACUUGUC CUUAACGGUG | SEQ ID NO:216 | AAGAACW'UGUC CUUAACGGUG |
| SEQ ID NO:217 | AAUAAAGCUG GACAAGAAU | SEQ ID NO:218 | UUUCUUGUCCA GCUUUAUUGG | SEQ ID NO:219 | UUUCUUW'UCCA GCUUUAUUGG |
| SEQ ID NO 220 | AAUAAAGCUG GACAAGAAC | SEQ ID NO:221 | UUUCUUGUCCA GCUUUAUUGG | SEQ ID NO:222 | UUUCUUW'UCCA GCUUUAUUGG |
| SEQ ID NO:223 | AAUAAAGCUG GACAAGAAG | SEQ ID NO:224 | UUUCUUGUCCA GCUUUAUUGG | SEQ ID NO:225 | UUUCUUW'UCCA GCUUUAUUGG |
| SEQ ID NO 226 | GCACCGUUAA GGACAAGUA | SEQ ID NO:227 | AACUUGUCCUU AACGGUGCUC | SEQ ID NO:228 | AACUUGW'CCUU AACGGUGCUC |
| SEQ ID NO:229 | GCACCGUUAA GGACAAGUC | SEQ ID NO:230 | AACUUGUCCUU AACGGUGCUC | SEQ ID NO:231 | AACUUGW'CCUU AACGGUGCUC |
| SEQ ID NO:232 | GCACCGUUAA GGACAAGUG | SEQ ID NO:233 | AACUUGUCCUU AACGGUGCUC | SEQ ID NO:234 | AACUUGW'CCUU AACGGUGCUC |
| SEQ ID NO:235 | GACAAGUUCU CUGAGUUCA | SEQ ID NO:236 | AGAACUCAGAG AACUUGUCCU | SEQ ID NO:237 | AGAACUW'AGAG AACUUGUCCU |
| SEQ ID NO:238 | GACAAGUUCU CUGAGUUCC | SEQ ID NO:239 | AGAACUCAGAG AACUUGUCCU | SEQ ID NO:240 | AGAACUW'AGAG AACUUGUCCU |
| SEQ ID NO:241 | GACAAGUUCU CUGAGUUCG | SEQ ID NO:242 | AGAACUCAGAG AACUUGUCCU | SEQ ID NO:243 | AGAACUW'AGAG AACUUGUCCU |

(continued)

| SEQ ID NO | SS strand (5'-3') | SEQ ID NO | Unmodified AS strand (5'-3') | SEQ ID NO | AS strand with chemical modification in position 7 (5'-3') |
|---|---|---|---|---|---|
| SEQ ID NO:244 | AUUCUCAGUG CUCUCCUAA | SEQ ID NO:245 | AUAGGAGAGCA CUGAGAAUAC | SEQ ID NO:246 | AUAGGAW'AGCA CUGAGAAUAC |
| SEQ ID NO:247 | AUUCUCAGUG CUCUCCUAC | SEQ ID NO:248 | AUAGGAGAGCA CUGAGAAUAC | SEQ ID NO:249 | AUAGGAW'AGCA CUGAGAAUAC |
| SEQ ID NO:250 | AUUCUCAGUG CUCUCCUAG | SEQ ID NO:251 | AUAGGAGAGCA CUGAGAAUAC | SEQ ID NO:252 | AUAGGAW'AGCA CUGAGAAUAC |
| SEQ ID NO:253 | GCACCGUUAA GGACAAGUU | SEQ ID NO:254 | AACUUGUCCUU AACGGUGCUC | SEQ ID NO:255 | AACUUGW'CCUU AACGGUGCUC |
| SEQ ID NO:256 | CCGUUAAGGA CAAGUUCUU | SEQ ID NO:257 | AAGAACUUGUC CUUAACGGUG | SEQ ID NO:258 | AAGAACW'UGUC CUUAACGGUG |
| SEQ ID NO:259 | AUUCUCAGUG CUCUCCUAU | SEQ ID NO:260 | AUAGGAGAGCA CUGAGAAUAC | SEQ ID NO:261 | AUAGGAW'AGCA CUGAGAAUAC |
| SEQ ID NO:262 | AAUAAAGCUG GACAAGAAA | SEQ ID NO:263 | UUUCUUGUCCA GCUUUAUUGG | SEQ ID NO:264 | UUUCUUW'UCCA GCUUUAUUGG |
| SEQ ID NO:265 | GACAAGUUCU CUGAGUUCU | SEQ ID NO:266 | AGAACUCAGAG AACUUGUCCU | SEQ ID NO:267 | AGAACUW'AGAG AACUUGUCCU |
| SEQ ID NO:268 | UAUUCUCAGU GCUCUCCUA | SEQ ID NO:269 | UAGGAGAGCAC UGAGAAUACU | SEQ ID NO 270 | UAGGAGW'GCAC UGAGAAUACU |
| SEQ ID NO:271 | AUUCUCAGUG CUCUCCUAU | SEQ ID NO:272 | AUAGGAGAGCA CUGAGAAUAC | SEQ ID NO:273 | AUAGGAW'AGCA CUGAGAAUAC |
| SEQ ID NO:274 | UAUUCUCAGU GCUCUCCUG | SEQ ID NO:275 | UAGGAGAGCAC UGAGAAUACU | SEQ ID NO:276 | UAGGAGW'GCAC UGAGAAUACU |
| SEQ ID NO:277 | GACAAGUUCU CUGAGUUCC | SEQ ID NO:278 | AGAACUCAGAG AACUUGUCCU | SEQ ID NO:279 | AGAACUW'AGAG AACUUGUCCU |
| SEQ ID NO:280 | GCACCGUUAA GGACAAGUC | SEQ ID NO:281 | AACUUGUCCUU AACGGUGCUC | SEQ ID NO:282 | AACUUGW'CCUU AACGGUGCUC |

Table 25. Modified sense strands and antisense strands of human ApoC3 siRNAs

| Double strand No. | SEQ ID NO | SS strand (5'-3') | SEQ ID NO | AS strand (5'-3') |
|---|---|---|---|---|
| TRD005077 | SEQ ID NO:283 | GmsCmsCmUmCfUmGf CfCfCmGmAmGmCmU mUmCmAmAm | SEQ ID NO:284 | UmsUfsGmAmAmGfCmUmC mGmGmGmCmAfGmAfGmG mCmsCmsAm |
| TRD005088 | SEQ ID NO:285 | CmsGmsAmGmGfAmUf GfCfCmUmCmCmCmU mUmCmUmUm | SEQ ID NO:286 | AmsAfsGmAmAmGfGmGmA mGmGmCmAmUfCmCfUmC mGmsGmsCm |
| TRD005092 | SEQ ID NO:287 | GmsCmsUmUmCfAmUf GfCfAmGmGmGmUmU mAmCmAmUm | SEQ ID NO:288 | AmsUfsGmUmAmAfCmCmC mUmGmCmAmUfGmAfAmG mCmsUmsGm |

(continued)

| Double strand No. | SEQ ID NO | SS strand (5'-3') | SEQ ID NO | AS strand (5'-3') |
|---|---|---|---|---|
| TRD005112 | SEQ ID NO:289 | UmsGmsAmGmCfAmGfCfGfUmGmCmAmGmGmAmGmUmUm | SEQ ID NO:290 | AmsAfsCmUmCmCfUmGmCmAmCmGmCmUfGmCfUmCmAmsGmsUm |
| TRD005124 | SEQ ID NO:291 | UmsUmsCmAmGfUmUfCfCfCmUmGmAmAmAmGmAmCmUm | SEQ ID NO:292 | AmsGfsUmCmUmUfUmCmAmGmGmGmAmAfCmUfGmAmAmsGmsCm |
| TRD005126 | SEQ ID NO:293 | CmsAmsGmUmUfCmCfCfUfGmAmAmAmGmAmCmUmAmUm | SEQ ID NO:294 | AmsUfsAmGmUmCfUmUmUmCmAmGmGmGfAmAfCmUmGmsAmsAm |
| TRD005131 | SEQ ID NO:295 | AmsCmsUmAmCfUmGfGfAfGmCmAmCmCmGmUmUmAmAm | SEQ ID NO:296 | UmsUfsAmAmCmGfGmUmGmCmUmCmCmAfGmUfAmGmUmsCmsUm |
| TRD005140 | SEQ ID NO:297 | GmsCmsAmCmCfGmUfUfAfAmGmGmAmCmAmAmGmUmUm | SEQ ID NO:298 | AmsAfsCmUmUmGfUmCmCmUmUmAmAmCfGmGfUmGmCmsUmsCm |
| TRD005143 | SEQ ID NO:299 | CmsCmsGmUmUfAmAfGfGfAmCmAmAmGmUmUmCmUmUm | SEQ ID NO:300 | AmsAfsGmAmAmCfUmUmGmUmCmCmUmUfAmAfCmGmGmsUmsGm |
| TRD005151 | SEQ ID NO:301 | GmsAmsCmAmAfGmUfUfCfUmCmUmGmAmGmUmUmCmUm | SEQ ID NO:302 | AmsGfsAmAmCmUfCmAmGmAmGmAmAmCfUmUfGmUmCmsCmsUm |
| TRD005171 | SEQ ID NO:303 | AmsAmsGmUmCfCmAfCfCfUmGmCmCmUmAmUmCmCmAm | SEQ ID NO:304 | UmsGfsGmAmUmAfGmGmCmAmGmGmUmGfGmAfCmUmUmsGmsGm |
| TRD005181 | SEQ ID NO:305 | CmsUmsGmCmGfAmGfCfUfCmCmUmUmGmGmGmUmCmUm | SEQ ID NO:306 | AmsGfsAmCmCmCfAmAmGmGmAmGmCmUfCmGfCmAmGmsGmsAm |
| TRD005197 | SEQ ID NO:307 | AmsAmsGmGmGfAmCfAfGfUmAmUmUmCmUmCmAmGmUm | SEQ ID NO:308 | AmsCfsUmGmAmGfAmAmUmAmCmUmGmUfCmCfCmUmUmsUmsUm |
| TRD005198 | SEQ ID NO:309 | AmsGmsGmGmAfCmAfGfUfAmUmUmCmUmCmAmGmUmAm | SEQ ID NO:310 | UmsAfsCmUmGmAfGmAmAmUmAmCmUmGfUmCfCmCmUmsUmsUm |
| TRD005202 | SEQ ID NO:311 | AmsCmsAmGmUfAmUfUfCfUmCmAmGmUmGmCmUmCmUm | SEQ ID NO:312 | AmsGfsAmGmCmAfCmUmGmAmGmAmAmUfAmCfUmGmUmsCmsCm |
| TRD005204 | SEQ ID NO:313 | UmsAmsUmUmCfUmCfAfGfUmGmCmUmCmUmCmCmUmAm | SEQ ID NO:314 | UmsAfsGmGmAmGfAmGmCmAmCmUmGmAfGmAfAmUmAmsCmsUm |
| TRD005205 | SEQ ID NO:315 | AmsUmsUmCmUfCmAfGfUfGmCmUmCmUmCmCmUmAmUm | SEQ ID NO:316 | AmsUfsAmGmGmAfGmAmGmCmAmCmUmGfAmGfAmAmUmsAmsCm |

(continued)

| Double strand No. | SEQ ID NO | SS strand (5'-3') | SEQ ID NO | AS strand (5'-3') |
|---|---|---|---|---|
| TRD005206 | SEQ ID NO:317 | UmsUmsCmUmCfAmGfUfGfCmUmCmUmCmCmUmAmCmUm | SEQ ID NO:318 | AmsGfsUmAmGmGfAmGmAmGmCmAmCmUfGmAfGmAmAmsUmsAm |
| TRD005207 | SEQ ID NO:319 | UmsCmsUmCmAfGmUfGfCfUmCmUmCmCmUmAmCmCmUm | SEQ ID NO:320 | AmsGfsGmUmAmGfGmAmGmAmGmCmAmCfUmGfAmGmAmsAmsUm |
| TRD005208 | SEQ ID NO:321 | GmsGmsCmAmUfGmCfUfGfGmCmCmUmCmCmCmAmAmUm | SEQ ID NO:322 | AmsUfsUmGmGmGfAmGmGmCmCmAmGmCfAmUfGmCmCmsUmsGm |
| TRD005209 | SEQ ID NO:323 | GmsCmsAmUmGfCmUfGfGfCmCmUmCmCmCmAmAmUmAm | SEQ ID NO:324 | UmsAfsUmUmGmGfGmAmGmGmCmCmAmGfCmAfUmGmCmsCmsUm |
| TRD005210 | SEQ ID NO:325 | CmsAmsUmGmCfUmGfGfCfCmUmCmCmCmAmAmUmAmAm | SEQ ID NO:326 | UmsUfsAmUmUmGfGmGmAmGmGmCmCmAfGmCfAmUmGmsCmsCm |
| TRD005212 | SEQ ID NO:327 | UmsGmsCmUmGfGmCfCfUfCmCmCmAmAmUmAmAmAmAm | SEQ ID NO:328 | UmsUfsUmUmAmUfUmGmGmGmAmGmGmCfCmAfGmCmAmsUmsGm |
| TRD005214 | SEQ ID NO:329 | CmsUmsGmGmCfCmUfCfCfCmAmAmUmAmAmAmGmCmUm | SEQ ID NO:330 | AmsGfsCmUmUmUfAmUmUmGmGmGmAmGfGmCfCmAmGmsCmsAm |
| TRD005216 | SEQ ID NO:331 | GmsGmsCmCmUfCmCfCfAfAmUmAmAmAmGmCmUmGmAm | SEQ ID NO:332 | UmsCfsAmGmCmUfUmUmAmUmUmGmGmGfAmGfGmCmCmsAmsGm |
| TRD005217 | SEQ ID NO:333 | GmsCmsCmUmCfCmCfAfAfUmAmAmAmGmCmUmGmGmAm | SEQ ID NO:334 | UmsCfsCmAmGmCfUmUmUmAmUmUmGmGfGmAfGmGmCmsCmsAm |
| TRD005219 | SEQ ID NO:335 | AmsAmsUmAmAfAmGfCfUfGmGmAmCmAmAmGmAmAmAm | SEQ ID NO:336 | UmsUfsUmCmUmUfGmUmCmCmAmGmCmUfUmUfAmUmUmsGmsGm |
| TRD005220 | SEQ ID NO:337 | AmsUmsAmAmAfGmCfUfGfGmAmCmAmAmGmAmAmGmUm | SEQ ID NO:338 | AmsCfsUmUmCmUfUmGmUmCmCmAmGmCfUmUfUmAmUmsUmsGm |
| TRD005221 | SEQ ID NO:339 | UmsAmsAmAmGfCmUfGfGfAmCmAmAmGmAmAmGmCmUm | SEQ ID NO:340 | AmsGfsCmUmUmCfUmUmGmUmCmCmAmGfCmUfUmUmAmsUmsUm |

Table 26. Modified sense strands and antisense strands of human ApoC3 siRNA conjugates

| Double strand No. | SEQ ID NO | SS strand (5'-3') | SEQ ID NO | AS strand (5'-3') |
|---|---|---|---|---|
| TRD005874 | SEQ ID NO:341 | UmsAmsUmUmCfUmCfAfGfUmGmCmUmCmUmCmCmUmAm-NAG1 | SEQ ID NO:342 | UmsAfsGmGfAmGf(-)hmpNA(A)GmCfAmCmUfGmAfGmAfAmUfAmsCmsUm |
| TRD005875 | SEQ ID NO:343 | CmsCmsGmUmUfAmAfGfGfAmCmAmAmGmUmUmCmUmUm-NAG1 | SEQ ID NO:344 | AmsAfsGmAfAmCf(-)hmpNA(U)UmGfUmCmCfUmUfAmAfCmGfGmsUmsGm |
| TRD005876 | SEQ ID NO:345 | CmsUmsGmCmGfAmGfCfUfCmCmUmUmGmGmGmUmCmUm-NAG1 | SEQ ID NO:346 | AmsGfsAmCfCmCf(-)hmpNA(A)AmGfGmAmGfCmUfCmGfCmAfGmsGmsAm |
| TRD005877 | SEQ ID NO:347 | AmsCmsAmGmUfAmUfUfCfUmCmAmGmUmGmCmUmCmUm-NAG1 | SEQ ID NO:348 | AmsGfsAmGfCmAf(-)hmpNA(C)UmGfAmGmAfAmUfAmCfUmGfUmsCmsCm |
| TRD005878 | SEQ ID NO:349 | UmsUmsCmUmCfAmGfUfGfCmUmCmUmCmCmUmAmCmUm-NAG1 | SEQ ID NO:350 | AmsGfsUmAfGmGf(-)hmpNA(A)GmAfGmCmAfCmUfGmAfGmAfAmsUmsAm |
| TRD005879 | SEQ ID NO:351 | AmsAmsGmGmGfAmCfAfGfUmAmUmUmCmUmCmAmGmUm-NAG1 | SEQ ID NO:352 | AmsCfsUmGfAmGf(-)hmpNA(A)AmUfAmCmUfGmUfCmCfCmUfUmsUmsUm |
| TRD005882 | SEQ ID NO:353 | AmsAmsUmAmAfAmGfCfUfGmGmAmCmAmAmGmAmAmAm-NAG1 | SEQ ID NO:354 | UmsUfsUmCfUmUf(-)hmpNA(G)UmCfCmAmGfCmUfUmUfAmUfUmsGmsGm |
| TRD005884 | SEQ ID NO:355 | GmsAmsCmAmAfGmUfUfCfUmCmUmGmAmGmUmUmCmUm-NAG1 | SEQ ID NO:356 | AmsGfsAmAfCmUf(-)hmpNA(C)AmGfAmGmAfAmCfUmUfGmUfCmsCmsUm |
| TRD005885 | SEQ ID NO:357 | CmsGmsAmGmGfAmUfGfCfCmUmCmCmCmUmUmCmUmUm-NAG1 | SEQ ID NO:358 | AmsAfsGmAfAmGf(-)hmpNA(G)GmAfGmGmCfAmUfCmCfUmCfGmsGmsCm |
| TRD005886 | SEQ ID NO:359 | AmsCmsUmAmCfUmGfGfAfGmCmAmCmCmGmUmUmAmAm-NAG1 | SEQ ID NO:360 | UmsUfsAmAfCmGf(-)hmpNA(G)UmGfCmUmCfCmAfGmUfAmGfUmsCmsUm |
| TRD005887 | SEQ ID NO:361 | AmsUmsAmAmAfGmCfUfGfGmAmCmAmAmGmAmAmGmUm-NAG1 | SEQ ID NO:362 | AmsCfsUmUfCmUf(-)hmpNA(U)GmUfCmCmAfGmCfUmUfUmAfUmsUmsGm |
| TRD005888 | SEQ ID NO:363 | AmsGmsGmGmAfCmAfGfUfAmUmUmCmUmCmAmGmUmAm-NAG1 | SEQ ID NO:364 | UmsAfsCmUfGmAf(-)hmpNA(G)AmAfUmAmCfUmGfUmCfCmCfUmsUmsUm |
| TRD005889 | SEQ ID NO:365 | GmsCmsCmUmCfCmCfAfAfUmAmAmAmGmCmUmGmGmAm-NAG1 | SEQ ID NO:366 | UmsCfsCmAfGmCf(-)hmpNA(U)UmUfAmUmUfGmGfGmAfGmGfCmsCmsAm |
| TRD005890 | SEQ ID NO:367 | UmsGmsCmUmGfGmCfCfUfCmCmCmAmAmUmAmAmAmAm-NAG1 | SEQ ID NO:368 | UmsUfsUmUfAmUf(-)hmpNA(U)GmGfGmAmGfGmCfCmAfGmCfAmsUmsGm |
| TRD005891 | SEQ ID NO:369 | AmsUmsUmCmUfCmAfGfUfGmCmUmCmUmCmCmUmAmUm-NAG1 | SEQ ID NO:370 | AmsUfsAmGfGmAf(-)hmpNA(G)AmGfCmAmCfUmGfAmGfAmAfUmsAmsCm |

(continued)

| Double strand No. | SEQ ID NO | SS strand (5'-3') | SEQ ID NO | AS strand (5'-3') |
|---|---|---|---|---|
| TRD005892 | SEQ ID NO:371 | UmsUmsCmAmGfUmUfCfCfCmUmGmAmAmAmGmAmCmUm-NAG1 | SEQ ID NO:372 | AmsGfsUmCfUmUf(-)hmpNA(U)CmAfGmGmGfAmAfCmUfGmAfAmsGmsCm |
| TRD005893 | SEQ ID NO:373 | CmsAmsUmGmCfUmGfGfCfCmUmCmCmCmAmAmUmAmAm-NAG1 | SEQ ID NO:374 | UmsUfsAmUfUmGf(-)hmpNA(G)GmAfGmGmCfCmAfGmCfAmUfGmsCmsCm |
| TRD006925 | SEQ ID NO:375 | AmsUmsUmCmUfCmAfGfUfGmCmUmCmUmCmCmUmAmsUms-NAG1 | SEQ ID NO:376 | AmsUfsAmGfGmAfGmAmGmCfAmCfUmGfAmGfAmAfUmsAmsCm |
| TRD006926 | SEQ ID NO:377 | UmsAmsUmUmCfUmCfAfGfUmGmCmUmCmUmCmCmUmUm-NAG1 | SEQ ID NO:378 | UmsAfsGmGfAmGf(-)hmpNA(A)GmCmAfCmUfGmAfGmAfAmUfAmsCmsUm |
| TRD006927 | SEQ ID NO:379 | UmsAmsUmUmCfUmCfAfGfUmGmCmUmCmUmCmCmUmCm-NAG1 | SEQ ID NO:380 | UmsAfsGmGfAmGf(-)hmpNA(A)GmCmAfCmUfGmAfGmAfAmUfAmsCmsUm |
| TRD006928 | SEQ ID NO:381 | UmsAmsUmUmCfUmCfAfGfUmGmCmUmCmUmCmCmUmGm-NAG1 | SEQ ID NO:382 | UmsAfsGmGfAmGf(-)hmpNA(A)GmCmAfCmUfGmAfGmAfAmUfAmsCmsUm |
| TRD006929 | SEQ ID NO:383 | CmsCmsGmUmUfAmAfGfGfAmCmAmAmGmUmUmCmUmAm-NAG1 | SEQ ID NO:384 | AmsAfsGmAfAmCf(-)hmpNA(U)UmGmUfCmCfUmUfAmAfCmGfGmsUmsGm |
| TRD006930 | SEQ ID NO:385 | CmsCmsGmUmUfAmAfGfGfAmCmAmAmGmUmUmCmUmCm-NAG1 | SEQ ID NO:386 | AmsAfsGmAfAmCf(-)hmpNA(U)UmGmUfCmCfUmUfAmAfCmGfGmsUmsGm |
| TRD006931 | SEQ ID NO:387 | CmsCmsGmUmUfAmAfGfGfAmCmAmAmGmUmUmCmUmGm-NAG1 | SEQ ID NO.388 | AmsAfsGmAfAmCf(-)hmpNA(U)UmGmUfCmCfUmUfAmAfCmGfGmsUmsGm |
| TRD006932 | SEQ ID NO:389 | AmsAmsUmAmAfAmGfCfUfGmGmAmCmAmAmGmAmAmUm-NAG1 | SEQ ID NO:390 | UmsUfsUmCfUmUf(-)hmpNA(G)UmCmCfAmGfCmUfUmUfAmUfUmsGmsGm |
| TRD006933 | SEQ ID NO:391 | AmsAmsUmAmAfAmGfCfUfGmGmAmCmAmAmGmAmAmCm-NAG1 | SEQ ID NO:392 | UmsUfsUmCfUmUf(-)hmpNA(G)UmCmCfAmGfCmUfUmUfAmUfUmsGmsGm |
| TRD006934 | SEQ ID NO:393 | AmsAmsUmAmAfAmGfCfUfGmGmAmCmAmAmGmAmAmGm-NAG1 | SEQ ID NO:394 | UmsUfsUmCfUmUf(-)hmpNA(G)UmCmCfAmGfCmUfUmUfAmUfUmsGmsGm |
| TRD006935 | SEQ ID NO:395 | GmsAmsCmAmAfGmUfUfCfUmCmUmGmAmGmUmUmCmAm-NAG1 | SEQ ID NO:396 | AmsGfsAmAfCmUf(-)hmpNA(C)AmGmAfGmAfAmCfUmUfGmUfCmsCmsUm |
| TRD006936 | SEQ ID NO:397 | GmsAmsCmAmAfGmUfUfCfUmCmUmGmAmGmUmUmCmCm-NAG1 | SEQ ID NO:398 | AmsGfsAmAfCmUf(-)hmpNA(C)AmGmAfGmAfAmCfUmUfGmUfCmsCmsUm |

(continued)

| Double strand No. | SEQ ID NO | SS strand (5'-3') | SEQ ID NO | AS strand (5'-3') |
|---|---|---|---|---|
| TRD006937 | SEQ ID NO:399 | GmsAmsCmAmAfGmUfUfCfUmCmUmGmAmGmUmUmCmGm-NAG1 | SEQ ID NO:400 | AmsGfsAmAfCmUf(-)hmpNA(C)AmGmAfGmAfAmCfUmUfGmUfCmsCmsUm |
| TRD006938 | SEQ ID NO:401 | AmsUmsUmCmUfCmAfGfUfGmCmUmCmUmCmCmUmAmAm-NAG1 | SEQ ID NO:402 | AmsUfsAmGfGmAf(-)hmpNA(G)AmGmCfAmCfUmGfAmGfAmAfUmsAmsCm |
| TRD006939 | SEQ ID NO:403 | AmsUmsUmCmUfCmAfGfUfGmCmUmCmUmCmCmUmAmCm-NAG1 | SEQ ID NO:404 | AmsUfsAmGfGmAf(-)hmpNA(G)AmGmCfAmCfUmGfAmGfAmAfUmsAmsCm |
| TRD006940 | SEQ ID NO:405 | AmsUmsUmCmUfCmAfGfUfGmCmUmCmUmCmCmUmAmGm-NAG1 | SEQ ID NO:406 | AmsUfsAmGfGmAf(-)hmpNA(G)AmGmCfAmCfUmGfAmGfAmAfUmsAmsCm |
| TRD006963 | SEQ ID NO:407 | GmsCmsAmCmCfGmUfUfAfAmGmGmAmCmAmAmGmUmAm-NAG1 | SEQ ID NO:408 | AmsAfsCmUfUmGf(-)hmpNA(U)CmCmUfUmAfAmCfGmGfUmGfCmsUmsCm |
| TRD006964 | SEQ ID NO:409 | GmsCmsAmCmCfGmUfUfAfAmGmGmAmCmAmAmGmUmCm-NAG1 | SEQ ID NO:410 | AmsAfsCmUfUmGf(-)hmpNA(U)CmCmUfUmAfAmCfGmGfUmGfCmsUmsCm |
| TRD006965 | SEQ ID NO:411 | GmsCmsAmCmCfGmUfUfAfAmGmGmAmCmAmAmGmUmGm-NAG1 | SEQ ID NO:412 | AmsAfsCmUfUmGf(-)hmpNA(U)CmCmUfUmAfAmCfGmGfUmGfCmsUmsCm |
| TRD006966 | SEQ ID NO:413 | GmsCmsAmCmCfGmUfUfAfAmGmGmAmCmAmAmGmUmUm-NAG1 | SEQ ID NO:414 | AmsAfsCmUfUmGf(-)hmpNA(U)CmCmUfUmAfAmCfGmGfUmGfCmsUmsCm |
| TRD006884 | SEQ ID NO:415 | CmsCmsGmUmUfAmAfGfGfAmCmAmAmGmUmUmCmUmsUms-NAG1 | SEQ ID NO:416 | AmsAfsGmAfAmCf(-)hmpNA(U)UmGmUfCmCfUmUfAmAfCmGfGmsUmsGm |
| TRD006885 | SEQ ID NO:417 | AmsUmsUmCmUfCmAfGfUfGmCmUmCmUmCmCmUmAmsUms-NAG1 | SEQ ID NO:418 | AmsUfsAmGfGmAf(-)hmpNA(G)AmGmCfAmCfUmGfAmGfAmAfUmsAmsCm |
| TRD006886 | SEQ ID NO:419 | AmsAmsUmAmAfAmGfCfUfGmGmAmCmAmAmGmAmAmsAms-NAG1 | SEQ ID NO:420 | UmsUfsUmCfUmUf(-)hmpNA(G)UmCmCfAmGfCmUfUmUfAmUfUmsGmsGm |
| TRD006887 | SEQ ID NO:421 | GmsAmsCmAmAfGmUfUfCfUmCmUmGmAmGmUmUmCmsUms-NAG1 | SEQ ID NO:422 | AmsGfsAmAfCmUf(-)hmpNA(C)AmGmAfGmAfAmCfUmUfGmUfCmsCmsUm |
| TRD006888 | SEQ ID NO:423 | UmsAmsUmUmCfUmCfAfGfUmGmCmUmCmUmCmCmUmsAms-NAG1 | SEQ ID NO:424 | UmsAfsGmGfAmGf(-)hmpNA(A)GmCmAfCmUfGmAfGmAfAmUfAmsCmsUm |
| TRD006971 | SEQ ID NO:425 | UmsAmsUmUmCfUmCfAfGfUmGmCmUmCmUmCmCmUmsGms-NAG1 | SEQ ID NO:426 | UmsAfsGmGfAmGf(-)hmpNA(A)GmCmAfCmUfGmAfGmAfAmUfAmsCmsUm |

(continued)

| Double strand No. | SEQ ID NO | SS strand (5'-3') | SEQ ID NO | AS strand (5'-3') |
|---|---|---|---|---|
| TRD006972 | SEQ ID NO:427 | GmsAmsCmAmAfGmUfUfCfUmCmUmGmAmGmUmUmCmsCms-NAG1 | SEQ ID NO:428 | AmsGfsAmAfCmUf(-)hmpNA(C)AmGmAfGmAfAmCfUmUfGmUfCmsCmsUm |
| TRD006975 | SEQ ID NO:429 | GmsCmsAmCmCfGmUfUfAfAmGmGmAmCmAmAmGmUmsCms-NAG1 | SEQ ID NO:430 | AmsAfsCmUfUmGf(-)hmpNA(U)CmCmUfUmAfAmCfGmGfUmGfCmsUmsCm |
| TRD006976 | SEQ ID NO:431 | GmsCmsAmCmCfGmUfUfAfAmGmGmAmCmAmAmGmUmsGms-NAG1 | SEQ ID NO:432 | AmsAfsCmUfUmGf(-)hmpNA(U)CmCmUfUmAfAmCfGmGfUmGfCmsUmsCm |

In Table 25 to Table 26, the nucleotide synthesized using 2-hydroxymethyl-1,3-propanediol as the starting material is defined as hmpNA; hmpNA is a racemic structure;

(-)hmpNA(A) was obtained by solid-phase synthesis using the nucleoside phosphoramidite monomer 1-1a of example section 1.1; (+)hmpNA(A) is an optical isomer;
(-)hmpNA(G) was obtained by solid-phase synthesis using the nucleoside phosphoramidite monomer 1-3a of example section 1.6; (+)hmpNA(G) is an optical isomer;
(-)hmpNA(C) was obtained by solid-phase synthesis using the nucleoside phosphoramidite monomer 1-8a of example section 1.8; (+)hmpNA(C) is an optical isomer;
(-)hmpNA(U) was obtained by solid-phase synthesis using the nucleoside phosphoramidite monomer 1-7a of example section 1.7; (+)hmpNA(U) is an optical isomer.

The lowercase letter m indicates that the left nucleotide adjacent to the letter m is a 2'-methoxy-modified nucleotide; the lowercase letter f indicates that the left nucleotide adjacent to the letter f is a 2'-fluoro-modified nucleotide;

the lowercase letter s, when present between uppercase letters, indicates that the two nucleotides adjacent to the letter s are linked by a phosphorothioate group;
the lowercase letter s, when being the first at the 3' end, indicates that the left nucleotide adjacent to the letter s ends in a phosphorothioate group.

In Table 26, the structure of NAG1 is as shown in Example 11.

**Example 21. Inhibition of Human ApoC3 in Huh7 Cells by siRNAs - Single Concentration Point Inhibitory Activity Screening**

The effects of siRNAs targeting human ApoC3 on the human ApoC3 mRNA expression level were tested *in vitro.* Huh7 cells were cultured at 37 °C with 5% $CO_2$ in a DMEM high glucose medium containing 10% fetal bovine serum. 24 h prior to transfection, the Huh7 cells were inoculated into a 96-well plate at a density of 10 thousand cells per well. Each well contained 100 μL of medium.

The cells were transfected with siRNAs at a final concentration of 10 nM using Lipofectamine RNAiMAX (ThermoFisher, 13778150) according to the instructions of the product. 24 h after treatment, the cells were lysed using TaqMan™ Fast Advanced Cells-to-CT™ Kit (ThermoFisher, A35378), and one-step reverse transcription and quantitative real-time PCR detection were carried out. The human ApoC3 mRNA level was measured and corrected based on the ACTIN internal reference gene level. Experimental materials and instruments for cell viability screening (Cells-to-CT) in a 96-well plate are shown in Table 1 and Table 2 in example section 3.1.

Experimental procedure of cell viability screening (Cells-to-CT) in a 96-well plate:

(I) Cell transfection. The amounts of the components of the transfection complex are shown in Table 27:

Table 27. Amounts required for transfection complex in each well of a 96-well plate

| | Amount | Opti-MEM |
|---|---|---|
| siRNA | 10 nM (final concentration in 96-well plate) | 15 μL |

(continued)

| | Amount | Opti-MEM |
|---|---|---|
| RNAiMAX | 0.9 μL | 15 μL |

(II) Extraction of cellular RNA using Cell-to-CT method and cell RNA reverse transcription. The reverse transcription reaction system is shown in Table 29, and the reaction conditions are shown in Table 30.

Table 28. Cells-to-CT kit components and storage conditions

| Reagent name | Brand | Cat. No. | Components | Storage conditions |
|---|---|---|---|---|
| TaqMan™ Fast Advanced Master Mix | Thermo | 4444964 | TaqMan™ Fast Advanced Master Mix | 4 °C |
| Cells-to-CT Bulk Lysis Reagents | Thermo | 4391851C | Lysis Solution | 4 °C |
| | | | Stop Solution | -20 °C |
| | | | Dnase I | -20 °C |
| Cells-to-CT Bulk Fast Advanced RT Reagents | Thermo | A39110 | 20×RT Fast Advanced Enzyme Mix | -20 °C |
| | | | 2×Fast Advanced RT Buffer | 4 °C |

Table 29. Cellular RNA reverse transcription reaction system

| Reagent | Amount (μL) |
|---|---|
| 20×RT Fast Advanced Enzyme Mix | 25 |
| 2×Fast Advanced RT Buffer | 2.5 |
| RNA (Lysis Mix) | 22.5 |
| Total amount | 50 |

Table 30. Reverse transcription reaction conditions

| Reverse transcription reaction program | | | | |
|---|---|---|---|---|
| Step | Phase | Cycle | Temperature | Time |
| Reverse transcription | 1 | 1 | 37 °C | 30 min |
| Reverse transcriptase inactivation | 2 | 1 | 95 °C | 5 min |
| Holding | 3 | 1 | 4 °C | Long-term |

After the reverse transcription was complete, the samples could be stored in a refrigerator at 4 °C before use in Taqman Q-PCR or stored in a freezer at -40 °C (6 months).

(III) Taqman probe Q-PCR detection

1. Reaction kit (ThermoFisher TaqMan Fast Advanced Master Mix (4444964); the shelf life of the kit was checked; the kit components were stored in a freezer at -40 °C, and stored in a refrigerator at 4 °C after dissolution and use);

2. The following reaction mixtures (Table 32) were prepared in Microtubes. The working concentration of the primers was 10 μM.

Table 31. Taqman probe primers

| Primer name | SEQ ID NO | Primer sequence |
|---|---|---|
| hApoc3-PF | SEQ ID NO:433 | TGCCTCCCTTCTCAGCTTCA |

(continued)

| Primer name | SEQ ID NO | Primer sequence |
|---|---|---|
| hApoc3-PR | SEQ ID NO:434 | GGGAACTGAAGCCATCGGTC |
| hApoc3-P | SEQ ID NO:435 | 5`6-FAM-ATGAAGCACGCCACCAAGACCGCCA-3`BHQ1 |
| hACTB-PF | SEQ ID NO:436 | ACGTGGACATCCGCAAAGAC |
| hACTB-PR | SEQ ID NO:437 | TCTTCATTGTGCTGGGTGCC |
| hACTB-P | SEQ ID NO:438 | 5`TET-AACACAGTGCTGTCTGGCGGCACCA-3`BHQ2 |

Table 32. Detection solutions for Taqman probe Q-PCR detection reaction

| Reagent | Amount (μL) |
|---|---|
| TaqMan™ Fast Advanced Master Mix | 10 |
| Target gene-probe-F | 0.4 |
| Target gene-probe-R | 0.4 |
| Target gene-probe | 0.2 |
| Internal reference gene-probe-F | 0.4 |
| Internal reference gene-probe-R | 0.4 |
| Internal reference gene-probe | 0.2 |
| cDNA (RT Mix) | 8 |
| Total amount | 20 |

The samples were placed in an RT-PCR instrument and reacted according to the reaction program in Table 33 (40 cycles of reaction).

Table 33. RT-PCR reaction program

| RT-PCR instrument reaction program | | | | |
|---|---|---|---|---|
| Step | Phase | Cycle | Temperature | Time |
| UDG activation | 1 | 1 | 50 °C | 2 min |
| Enzyme activation | 2 | 1 | 95 °C | 20 s |
| PCR | 3 | 40 | 95 °C | 1 s |
| | | | 60 °C | 24 s |
| Note: TaqMan® Fast Advanced Master Mix includes ROX™ reference dye. | | | | |

3. Result analysis method

After the Taqman probe Q-PCR detection was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene, and is also referred to as $2^{-\triangle\triangle Ct}$ $\triangle\triangle Ct$ = [(target gene of Ct experimental group - internal reference of Ct experimental group) - (target gene of Ct control group - internal reference of Ct control group)]. Inhibition (%) = (1 - remaining amount of target gene expression) × 100%. The experimental results are expressed relative to the remaining percentage of human ApoC3 mRNA expression in cells treated with the control siRNA. The results are shown in Table 34.

Table 34. Single concentration point screening results of inhibition of human ApoC3 in Huh7 cells by siRNAs

| Compound No. | Remaining mRNA expression level | SD | Compound No. | Remaining mRNA expression level | SD |
|---|---|---|---|---|---|
| TRD005077 | 19.1% | 6.8% | TRD005151 | 5.4% | 3.1% |
| TRD005088 | 7.2% | 4.9% | TRD005157 | 7.6% | 0.5% |
| TRD005089 | 6.4% | 4.5% | TRD005163 | 18.9% | 2.6% |
| TRD005092 | 6.7% | 3.4% | TRD005171 | 4.3% | 0.5% |
| TRD005110 | 19.0% | 12.3% | TRD005173 | 10.8% | 2.7% |
| TRD005112 | 10.2% | 3.4% | TRD005181 | 16.5% | 3.2% |
| TRD005115 | 19.2% | 5.9% | TRD005197 | 5.3% | 0.9% |
| TRD005117 | 19.6% | 2.5% | TRD005198 | 10.8% | 2.2% |
| TRD005118 | 16.3% | 0.8% | TRD005202 | 5.1% | 1.6% |
| TRD005119 | 10.1% | 2.6% | TRD005203 | 19.6% | 2.0% |
| TRD005124 | 9.0% | 1.7% | TRD005204 | 2.9% | 0.6% |
| TRD005125 | 16.8% | 2.9% | TRD005205 | 12.6% | 2.1% |
| TRD005126 | 15.9% | 3.6% | TRD005206 | 8.1% | 1.5% |
| TRD005131 | 7.4% | 1.5% | TRD005207 | 7.7% | 2.6% |
| TRD005135 | 10.0% | 0.7% | TRD005208 | 19.5% | 9.4% |
| TRD005140 | 4.7% | 0.6% | TRD005209 | 10.9% | 4.3% |
| TRD005141 | 16.7% | 2.2% | TRD005210 | 10.8% | 3.0% |
| TRD005142 | 17.4% | 2.3% | TRD005211 | 14.4% | 2.6% |
| TRD005143 | 3.5% | 0.2% | TRD005212 | 10.8% | 3.8% |
| TRD005144 | 15.3% | 4.6% | TRD005214 | 15.0% | 3.5% |
| TRD005146 | 11.5% | 3.7% | TRD005216 | 9.1% | 2.2% |
| TRD005220 | 17.8% | 2.1% | TRD005217 | 19.1% | 7.0% |
| TRD005221 | 19.7% | 3.6% | TRD005219 | 16.4% | 1.7% |

**Example 22. Inhibition of Human ApoC3 in Huh7 Cells by siRNAs - Five Concentration Point Inhibitory Activity**

Screening was performed in Huh7 cells using siRNAs in 5 concentration gradients. Each siRNA sample for transfection was serially diluted 10-fold from the starting final concentration 10 nM to five concentration points.

Huh7 cells were cultured at 37 °C with 5% $CO_2$ in a DMEM high glucose medium containing 10% fetal bovine serum. 24 h prior to transfection, the Huh7 cells were inoculated into a 96-well plate at a density of 10 thousand cells per well. Each well contained 100 μL of medium.

The cells were transfected with siRNAs at final concentrations of 10 nM, 1 nM, 0.1 nM, 0.01 nM and 0.001 nM using Lipofectamine RNAiMAX (ThermoFisher, 13778150) according to the instructions of the product. 24 h after treatment, the cells were lysed using TaqMan™ Fast Advanced Cells-to-CT™ Kit (ThermoFisher, A35378), and one-step reverse transcription and quantitative real-time PCR detection were carried out. The human ApoC3 mRNA level was measured and corrected based on the ACTIN internal reference gene level.

The results are expressed relative to the remaining percentage of human ApoC3 mRNA expression in cells treated with the control siRNA. The IC50 results of inhibition are shown in Table 35.

The experiment was carried out with reference to the cell viability screening (Cells-to-CT) in a 96-well plate in Example 21.

Table 35. Multi-dose inhibitory activity of siRNAs against human ApoC3 in Huh7 cells

| siRNA sample | Remaining percentage of target gene's mRNA expression (mean) | | | | | IC50 value (nM) |
|---|---|---|---|---|---|---|
| | 10nM | 1nM | 0.1nM | 0.01nM | 0.001nM | |
| TRD005077 | 18.9% | 29.4% | 73.5% | 85.0% | 104.8% | 0.2951 |
| TRD005088 | 7.4% | 8.1% | 21.6% | 57.6% | 101.4% | 0.0148 |
| TRD005092 | 17.1% | 19.0% | 62.1% | 71.3% | 79.4% | 0.1660 |
| TRD005112 | 14.3% | 19.6% | 88.6% | 105.5% | 98.1% | 0.3020 |
| TRD005124 | 59.5% | 105.6% | 125.1% | 144.7% | 136.9% | 0.1023 |
| TRD005126 | 17.0% | 22.8% | 64.5% | 98.4% | 104.8% | 0.1738 |
| TRD005131 | 12.9% | 25.5% | 68.6% | 90.3% | 105.2% | 0.2399 |
| TRD005140 | 9.0% | 23.5% | 70.2% | 107.4% | 101.7% | 0.2344 |
| TRD005143 | 6.2% | 10.1% | 38.3% | 92.9% | 112.1% | 0.0631 |
| TRD005151 | 5.7% | 11.2% | 53.4% | 87.5% | 122.0% | 0.0891 |
| TRD005171 | 12.7% | 65.1% | 101.7% | 114.1% | 147.4% | 0.3090 |
| TRD005181 | 9.0% | 17.8% | 55.7% | 74.6% | 83.3% | 0.1288 |
| TRD005197 | 4.8% | 11.8% | 58.9% | 72.5% | 98.4% | 0.1047 |
| TRD005198 | 6.3% | 21.1% | 67.8% | 103.6% | 110.0% | 0.2042 |
| TRD005202 | 4.3% | 6.8% | 15.4% | 56.7% | 102.1% | 0.0135 |
| TRD005204 | 5.9% | 7.7% | 11.8% | 45.4% | 87.3% | 0.0083 |
| TRD005205 | 11.9% | 18.3% | 45.3% | 110.3% | 114.3% | 0.0871 |
| TRD005206 | 7.3% | 7.8% | 16.3% | 52.2% | 106.0% | 0.0112 |
| TRD005207 | 11.6% | 22.0% | 72.6% | 88.3% | 108.8% | 0.2399 |
| TRD005208 | 20.7% | 25.3% | 64.2% | 96.0% | 107.3% | 0.1862 |
| TRD005209 | 13.2% | 16.1% | 32.8% | 76.8% | 105.2% | 0.0363 |
| TRD005210 | 25.4% | 31.6% | 58.3% | 95.0% | 122.3% | 0.1738 |
| TRD005212 | 21.0% | 30.2% | 62.3% | 85.5% | 111.2% | 0.1862 |
| TRD005214 | 16.9% | 38.0% | 61.7% | 106.0% | 99.3% | 0.2630 |
| TRD005216 | 14.5% | 30.7% | 74.7% | 105.0% | 93.5% | 0.3311 |
| TRD005217 | 8.1% | 18.4% | 54.7% | 89.0% | 108.7% | 0.1175 |
| TRD005219 | 7.1% | 11.6% | 32.8% | 69.3% | 107.8% | 0.0295 |
| TRD005220 | 9.7% | 12.7% | 34.9% | 63.9% | 94.2% | 0.0269 |
| TRD005221 | 12.2% | 19.7% | 45.2% | 75.6% | 104.8% | 0.0631 |

**Example 23. siRNAs' On-Target Activity and Off-Target Level Validation by psiCHECK**

*In vitro* molecular level simulation on-target and off-target level screening was performed on siRNAs in Huh 7 cells using 11 concentration gradients. The results show that the siRNAs of the present disclosure have low off-target activity while having high activity.

Huh 7 cells were cultured at 37 °C with 5% $CO_2$ in a DMEM high glucose medium containing 10% fetal bovine serum. 24 h prior to transfection, the Huh7 cells were inoculated into a 96-well plate at a density of 10 thousand cells per well. Each well contained 100 μL of medium.

The cells were co-transfected with siRNA and the corresponding plasmid using Lipofectamine2000 (ThermoFisher, 11668019) according to the instructions. 0.2 μL of Lipofectamine2000 was used for each well. The transfection

amount of plasmid was 10 ng per well. For the on-target and off-target plasmids, a total of 11 concentration points of siRNA were set up. The highest concentration point final concentration was 40 nM, and 3-fold serial dilution was carried out (40 nM, 13.3 nM, 4.44 nM, 1.48 nM, 0.494 nM, 0.165 nM, 0.0549 nM, 0.0183 nM, 0.00609 nM, 0.00203 nM and 0.000677 nM). 24 h after transfection, the off-target levels were determined using Dual-Luciferase Reporter Assay System (Promega, E2940). The results are shown in Table 37 to Table 40.

In the Huh7 cell line, the on-target/off-target activity of siRNAs in Table 35 with good activity was determined by performing psi-CHECK screening.

The Psi-CHECK plasmids were purchased from Synbio Technologies (Suzhou) Co., Ltd. and Sangon Biotech (Shanghai) Co., Ltd.

The experimental materials and instruments are detailed in Table 1 and Table 2 in Example 3.1, and the experimental results are detailed in Table 37 to Table 40. See Example 3.2 for the experimental procedure of psiCHECK activity screening, wherein the multi-concentration dilution protocol for siRNA samples is shown in Table 36. The results are shown in Table 37 to Table 40.

Table 36. Multi-concentration dilution protocol for siRNA samples

| siRNA concentration (μM) | Final concentration (nM) | Added water and siRNA |
|---|---|---|
| 20 | / | / |
| 4 | 40 | 4 μL siRNA + 16 μL $H_2O$ |
| 1.333333 | 13.33333 | 20 μL siRNA + 40 μL $H_2O$ |
| 0.444444 | 4.444444 | 20 μL siRNA + 40 μL $H_2O$ |
| 0.148148 | 1.481481 | 20 μL siRNA + 40 μL $H_2O$ |
| 0.049383 | 0.493827 | 20 μL siRNA + 40 μL $H_2O$ |
| 0.016461 | 0.164609 | 20 μL siRNA + 40 μL $H_2O$ |
| 0.005487 | 0.05487 | 20 μL siRNA + 40 μL $H_2O$ |
| 0.001829 | 0.01829 | 20 μL siRNA + 40 μL $H_2O$ |
| 0.00061 | 0.006097 | 20 μL siRNA + 40 μL $H_2O$ |
| 0.000203 | 0.002032 | 20 μL siRNA + 40 μL $H_2O$ |
| 6.77E-05 | 0.000677 | 20 μL siRNA + 40 μL $H_2O$ |

Table 37. Results of psiCHECK on-target activity screening of siRNAs (GSCM)

| Double strand No. | 40 nM | 13.3 nM | 4.44 nM | 1.48 nM | 0.494 nM | 0.165 nM | 0.0549 nM | 0.0183 nM | 0.00609 nM | 0.00203 nM | 0.000677 nM | IC50 value (nM) |
|---|---|---|---|---|---|---|---|---|---|---|---|---|
| TRD005088 | 0.21 | 0.19 | 0.20 | 0.25 | 0.38 | 0.53 | 0.76 | 0.84 | 0.85 | 0.93 | 1.04 | 0.1950 |
| TRD005092 | 0.28 | 0.25 | 0.24 | 0.28 | 0.34 | 0.44 | 0.73 | 0.84 | 0.96 | 1.07 | 1.09 | 0.1349 |
| TRD005126 | 0.36 | 0.29 | 0.24 | 0.28 | 0.34 | 0.48 | 0.68 | 0.79 | 0.83 | 0.96 | 0.95 | 0.1349 |
| TRD005131 | 0.25 | 0.25 | 0.28 | 0.29 | 0.38 | 0.55 | 0.72 | 0.83 | 0.98 | 0.96 | 0.94 | 0.1995 |
| TRD005140 | 0.19 | 0.16 | 0.16 | 0.24 | 0.32 | 0.52 | 0.69 | 0.84 | 0.98 | 0.95 | 1.00 | 0.1660 |
| TRD005143 | 0.13 | 0.13 | 0.13 | 0.13 | 0.18 | 0.26 | 0.38 | 0.56 | 0.75 | 0.88 | 0.94 | 0.0263 |
| TRD005151 | 0.11 | 0.11 | 0.11 | 0.20 | 0.33 | 0.52 | 0.69 | 0.75 | 0.85 | 0.94 | 0.90 | 0.1738 |
| TRD005181 | 0.23 | 0.25 | 0.23 | 0.23 | 0.22 | 0.32 | 0.49 | 0.66 | 0.85 | 0.91 | 0.92 | 0.0447 |
| TRD005197 | 0.06 | 0.07 | 0.08 | 0.12 | 0.20 | 0.34 | 0.60 | 0.68 | 0.85 | 0.94 | 1.00 | 0.0692 |
| TRD005198 | 0.31 | 0.33 | 0.27 | 0.33 | 0.42 | 0.61 | 0.67 | 0.79 | 0.90 | 0.85 | 0.91 | 0.2630 |
| TRD005202 | 0.11 | 0.10 | 0.12 | 0.13 | 0.21 | 0.35 | 0.49 | 0.73 | 0.95 | 0.97 | 0.98 | 0.0603 |
| TRD005204 | 0.06 | 0.05 | 0.05 | 0.06 | 0.09 | 0.14 | 0.18 | 0.31 | 0.55 | 0.77 | 0.93 | 0.0074 |
| TRD005206 | 0.06 | 0.05 | 0.05 | 0.08 | 0.13 | 0.28 | 0.54 | 0.78 | 0.90 | 0.88 | 0.94 | 0.0676 |
| TRD005219 | 0.13 | 0.12 | 0.12 | 0.15 | 0.27 | 0.45 | 0.73 | 0.85 | 0.90 | 0.97 | 0.92 | 0.1413 |
| TRD005220 | 0.16 | 0.15 | 0.15 | 0.23 | 0.39 | 0.61 | 0.72 | 0.88 | 0.92 | 0.88 | 0.92 | 0.2570 |

Table 38. Results of psiCHECK off-target activity screening of the seed regions of the AS strands of siRNAs (GSSM)

| Double strand No. | 40 nM | 13.3 nM | 4.44 nM | 1.48 nM | 0.494 nM | 0.165 nM | 0.0549 nM | 0.0183 nM | 0.00609 nM | 0.00203 nM | 0.000677 nM | IC50 value (nM) |
|---|---|---|---|---|---|---|---|---|---|---|---|---|
| TRD005077 | 0.98 | 0.94 | 0.94 | 0.99 | 0.95 | 1.03 | 1.01 | 0.93 | 1.08 | 1.04 | 0.96 | >40nM |
| TRD005088 | 1.17 | 1.14 | 1.13 | 1.12 | 1.19 | 1.15 | 1.20 | 1.08 | 1.25 | 1.00 | 1.15 | >40nM |
| TRD005092 | 0.89 | 0.96 | 0.94 | 1.05 | 1.04 | 1.03 | 0.94 | 1.10 | 1.05 | 1.10 | 1.21 | >40nM |
| TRD005112 | 0.72 | 0.69 | 0.77 | 0.85 | 0.92 | 0.89 | 1.06 | 0.96 | 1.12 | 1.02 | 0.98 | >40nM |
| TRD005124 | 0.99 | 0.95 | 0.96 | 1.02 | 1.08 | 0.97 | 0.99 | 1.04 | 1.03 | 0.95 | 1.12 | >40nM |
| TRD005126 | 0.88 | 1.01 | 1.03 | 0.96 | 0.90 | 0.96 | 1.05 | 0.98 | 0.98 | 1.03 | 1.04 | >40nM |
| TRD005140 | 0.95 | 0.99 | 0.92 | 0.93 | 0.94 | 0.92 | 0.99 | 1.01 | 1.01 | 1.01 | 0.95 | >40nM |
| TRD005143 | 1.08 | 1.21 | 1.00 | 1.11 | 1.00 | 0.89 | 1.06 | 1.00 | 0.90 | 1.08 | 1.13 | >40nM |
| TRD005151 | 0.94 | 0.95 | 0.96 | 0.89 | 0.93 | 0.92 | 0.86 | 0.89 | 0.89 | 0.90 | 0.77 | >40nM |
| TRD005171 | 0.94 | 1.16 | 1.01 | 1.00 | 1.18 | 0.94 | 1.10 | 1.17 | 1.07 | 1.03 | 1.03 | >40nM |
| TRD005181 | 1.01 | 0.99 | 0.98 | 1.04 | 1.09 | 1.07 | 1.25 | 1.02 | 1.07 | 0.98 | 1.09 | >40nM |
| TRD005197 | 0.70 | 0.87 | 0.89 | 1.05 | 0.93 | 0.94 | 0.98 | 0.89 | 0.90 | 0.88 | 0.85 | >40nM |
| TRD005198 | 0.94 | 0.91 | 0.92 | 0.99 | 1.04 | 1.01 | 1.21 | 0.86 | 0.97 | 1.02 | 1.01 | >40nM |
| TRD005202 | 0.53 | 0.44 | 0.50 | 0.67 | 0.75 | 0.81 | 0.85 | 0.83 | 0.87 | 0.93 | 0.99 | 39.81 |
| TRD005204 | 0.75 | 0.73 | 0.83 | 0.89 | 0.85 | 1.03 | 0.95 | 0.85 | 0.78 | 1.01 | 0.99 | >40nM |
| TRD005205 | 0.89 | 0.88 | 0.75 | 0.98 | 0.83 | 0.91 | 0.88 | 0.94 | 0.83 | 0.82 | 0.83 | >40nM |
| TRD005206 | 0.64 | 0.64 | 0.64 | 0.92 | 0.86 | 0.98 | 0.86 | 1.06 | 1.12 | 0.97 | 1.02 | >40nM |
| TRD005207 | 0.67 | 0.89 | 0.84 | 0.97 | 1.01 | 1.04 | 0.99 | 0.99 | 1.01 | 0.97 | 1.09 | >40nM |
| TRD005208 | 0.72 | 0.71 | 0.69 | 0.83 | 0.95 | 0.84 | 0.97 | 1.01 | 1.04 | 0.94 | 1.04 | >40nM |
| TRD005209 | 0.73 | 0.72 | 0.79 | 0.96 | 1.02 | 1.01 | 0.99 | 1.11 | 1.06 | 1.05 | 1.23 | >40nM |
| TRD005210 | 0.76 | 0.85 | 0.77 | 0.86 | 1.04 | 0.99 | 0.99 | 1.07 | 1.04 | 1.01 | 1.09 | >40nM |
| TRD005212 | 0.79 | 0.89 | 0.91 | 0.96 | 0.93 | 0.96 | 0.95 | 1.04 | 0.98 | 0.88 | 1.01 | >40nM |
| TRD005214 | 0.92 | 0.89 | 0.99 | 1.00 | 0.92 | 1.06 | 1.02 | 0.98 | 1.11 | 0.99 | 1.04 | >40nM |
| TRD005216 | 0.88 | 0.87 | 0.83 | 0.92 | 0.91 | 0.83 | 0.86 | 0.81 | 0.84 | 0.92 | 0.98 | >40nM |
| TRD005217 | 0.91 | 0.93 | 0.96 | 0.85 | 0.92 | 0.85 | 0.82 | 0.84 | 0.90 | 0.80 | 0.83 | >40nM |

(continued)

| Double strand No. | 40 nM | 13.3 nM | 4.44 nM | 1.48 nM | 0.494 nM | 0.165 nM | 0.0549 nM | 0.0183 nM | 0.00609 nM | 0.00203 nM | 0.000677 nM | IC50 value (nM) |
|---|---|---|---|---|---|---|---|---|---|---|---|---|
| TRD005219 | 0.70 | 0.68 | 0.80 | 0.84 | 0.76 | 0.93 | 0.88 | 0.81 | 0.87 | 0.89 | 0.90 | >40nM |
| TRD005220 | 0.98 | 1.03 | 0.93 | 1.08 | 1.02 | 1.05 | 0.98 | 1.15 | 1.13 | 1.04 | 1.01 | >40nM |
| TRD005221 | 0.74 | 0.74 | 0.87 | 0.99 | 1.02 | 0.90 | 0.99 | 0.97 | 1.01 | 0.88 | 1.09 | >40nM |

Table 39. Results of off-target activity screening (PSCM)

| Double strand No. | 40nM | 13.3nM | 4.44nM | 1.48nM | 0.494nM | 0.165nM | 0.0549nM | 0.0183nM | 0.00609nM | 0.00203nM | 0.000677nM | IC50 value (nM) |
|---|---|---|---|---|---|---|---|---|---|---|---|---|
| TRD005088 | 0.98 | 1.00 | 1.00 | 0.93 | 0.95 | 0.97 | 0.98 | 0.94 | 1.01 | 1.00 | 0.97 | >40nM |
| TRD005092 | 1.01 | 1.03 | 0.98 | 1.05 | 1.03 | 1.08 | 1.08 | 0.96 | 1.09 | 1.02 | 0.92 | >40nM |
| TRD005126 | 0.99 | 0.93 | 1.03 | 0.98 | 1.00 | 1.01 | 0.95 | 0.99 | 0.97 | 1.04 | 1.03 | >40nM |
| TRD005140 | 1.02 | 0.97 | 1.03 | 1.12 | 1.07 | 1.07 | 1.05 | 1.08 | 1.16 | 0.98 | 0.98 | >40nM |
| TRD005143 | 1.01 | 0.90 | 0.97 | 0.96 | 1.04 | 0.98 | 1.01 | 0.95 | 0.91 | 1.03 | 0.95 | >40nM |
| TRD005151 | 0.96 | 0.94 | 0.90 | 0.92 | 0.90 | 0.99 | 0.95 | 0.97 | 0.93 | 0.88 | 0.96 | >40nM |
| TRD005181 | 0.96 | 0.99 | 0.84 | 0.91 | 0.84 | 0.86 | 0.84 | 0.86 | 0.89 | 0.93 | 0.95 | >40nM |
| TRD005197 | 0.84 | 0.78 | 0.83 | 0.91 | 0.92 | 0.87 | 0.83 | 0.88 | 0.84 | 0.88 | 1.01 | >40nM |
| TRD005198 | 0.84 | 0.89 | 0.99 | 0.90 | 0.99 | 0.98 | 1.02 | 1.02 | 1.01 | 0.91 | 0.92 | >40nM |
| TRD005202 | 0.95 | 0.83 | 0.85 | 0.94 | 0.99 | 0.93 | 0.91 | 0.95 | 0.98 | 0.74 | 0.84 | >40nM |
| TRD005204 | 0.92 | 0.85 | 0.86 | 0.99 | 1.01 | 1.00 | 1.02 | 1.03 | 0.92 | 1.13 | 0.95 | >40nM |
| TRD005206 | 1.03 | 1.02 | 1.03 | 1.07 | 1.17 | 1.14 | 1.19 | 1.14 | 0.98 | 1.17 | 1.07 | >40nM |
| TRD005219 | 1.15 | 1.09 | 0.99 | 0.97 | 0.96 | 1.05 | 1.00 | 1.06 | 1.02 | 0.89 | 1.12 | >40nM |
| TRD005220 | 0.90 | 1.04 | 0.94 | 0.93 | 1.09 | 0.94 | 0.90 | 0.95 | 1.04 | 0.86 | 1.02 | >40nM |

Table 39. Results of off-target activity screening (PSSM)

| Double strand No. | 40nM | 13.3nM | 4.44nM | 1.48nM | 0.494nM | 0.165nM | 0.0549nM | 0.0183nM | 0.00609nM | 0.00203nM | 0.000677nM | IC50 value (nM) |
|---|---|---|---|---|---|---|---|---|---|---|---|---|
| TRD005088 | 0.98 | 1.00 | 1.00 | 0.93 | 0.95 | 0.97 | 0.98 | 0.94 | 1.01 | 1.00 | 0.97 | >40nM |
| TRD005092 | 1.01 | 1.03 | 0.98 | 1.05 | 1.03 | 1.08 | 1.08 | 0.96 | 1.09 | 1.02 | 0.92 | >40nM |
| TRD005126 | 0.99 | 0.93 | 1.03 | 0.98 | 1.00 | 1.01 | 0.95 | 0.99 | 0.97 | 1.04 | 1.03 | >40nM |
| TRD005140 | 1.02 | 0.97 | 1.03 | 1.12 | 1.07 | 1.07 | 1.05 | 1.08 | 1.16 | 0.98 | 0.98 | >40nM |
| TRD005143 | 1.01 | 0.90 | 0.97 | 0.96 | 1.04 | 0.98 | 1.01 | 0.95 | 0.91 | 1.03 | 0.95 | >40nM |
| TRD005151 | 0.96 | 0.94 | 0.90 | 0.92 | 0.90 | 0.99 | 0.95 | 0.97 | 0.93 | 0.88 | 0.96 | >40nM |
| TRD005181 | 0.96 | 0.99 | 0.84 | 0.91 | 0.84 | 0.86 | 0.84 | 0.86 | 0.89 | 0.93 | 0.95 | >40nM |
| TRD005197 | 0.84 | 0.78 | 0.83 | 0.91 | 0.92 | 0.87 | 0.83 | 0.88 | 0.84 | 0.88 | 1.01 | >40nM |
| TRD005198 | 0.84 | 0.89 | 0.99 | 0.90 | 0.99 | 0.98 | 1.02 | 1.02 | 1.01 | 0.91 | 0.92 | >40nM |
| TRD005202 | 0.95 | 0.83 | 0.85 | 0.94 | 0.99 | 0.93 | 0.91 | 0.95 | 0.98 | 0.74 | 0.84 | >40nM |
| TRD005204 | 0.92 | 0.85 | 0.86 | 0.99 | 1.01 | 1.00 | 1.02 | 1.03 | 0.92 | 1.13 | 0.95 | >40nM |
| TRD005206 | 1.03 | 1.02 | 1.03 | 1.07 | 1.17 | 1.14 | 1.19 | 1.14 | 0.98 | 1.17 | 1.07 | >40nM |
| TRD005219 | 1.15 | 1.09 | 0.99 | 0.97 | 0.96 | 1.05 | 1.00 | 1.06 | 1.02 | 0.89 | 1.12 | >40nM |
| TRD005220 | 0.90 | 1.04 | 0.94 | 0.93 | 1.09 | 0.94 | 0.90 | 0.95 | 1.04 | 0.86 | 1.02 | >40nM |

**Example 24. Inhibition of Human ApoC3 in Huh7 Cells by siRNAs - 11 Concentration Point Inhibitory Activity**

siRNAs that showed 80% or higher *in vitro* inhibition (20% or lower mRNA remaining expression level) in Table 17 were subjected to off-target modification (modification in position 7 of the AS strand) in Huh7 cells using 11 concentration gradients and then Huh7 cell viability screening was carried out. Each siRNA sample for transfection was serially diluted 3-fold from the starting final concentration 40 nM to 11 concentration points.

Huh7 cells were cultured at 37 °C with 5% $CO_2$ in a DMEM high glucose medium containing 10% fetal bovine serum. 24 h prior to transfection, the Huh7 cells were inoculated into a 96-well plate at a density of 10 thousand cells per well. Each well contained 100 μL of medium.

The cells were transfected with siRNAs at final concentrations of 40 nM, 13.3 nM, 4.44 nM, 1.48 nM, 0.494 nM, 0.165 nM, 0.0549 nM, 0.0183 nM, 0.00609 nM, 0.00203 nM and 0.000677 nM using Lipofectamine RNAiMAX (ThermoFisher, 13778150) according to the instructions of the product. 24 h after treatment, the cells were lysed using TaqMan™ Fast Advanced Cells-to-CT™ Kit (ThermoFisher, A35378), and one-step reverse transcription and quantitative real-time PCR detection were carried out. The human ApoC3 mRNA level was measured and corrected based on the ACTIN internal reference gene level.

The results are expressed relative to the remaining percentage of human ApoC3 mRNA expression in cells treated with the control siRNA. The IC50 results of inhibition are shown in Table 41.

The experiment was carried out with reference to the cell viability screening (Cells-to-CT) in a 96-well plate in Example 21.

Table 41. Multi-dose inhibitory activity of siRNAs against human ApoC3 in Huh7 cells

| Double strand No. | 40nM | 13.3nM | 4.44nM | 1.48nM | 0.494nM | 0.165nM |
|---|---|---|---|---|---|---|
| TRD005874 | 3.5% | 3.8% | 6.2% | 9.1% | 12.8% | 22.8% |
| TRD005875 | 4.7% | 6.2% | 11.4% | 13.6% | 26.0% | 47.2% |
| TRD005878 | 5.8% | 12.3% | 10.1% | 11.8% | 23.4% | 38.1% |
| TRD005879 | 4.2% | 7.8% | 12.5% | 17.0% | 35.0% | 39.7% |
| TRD005882 | 7.7% | 8.6% | 12.8% | 14.6% | 22.2% | 37.8% |
| TRD005885 | 12.9% | 22.1% | 35.5% | 28.6% | 30.0% | 63.6% |
| TRD005891 | 6.7% | 10.5% | 15.3% | 21.6% | 28.9% | 49.3% |
| Double strand No. | 0.0549nM | 0.0183nM | 0.00609nM | 0.00203nM | 0.000677nM | Huh7 cell IC50 value (nM) |
| TRD005874 | 45.4% | 76.1% | 116.3% | 122.8% | 118.9% | 0.0457 |
| TRD005875 | 86.1% | 122.0% | 135.6% | 106.8% | 106.2% | 0.1585 |
| TRD005878 | 106.6% | 131.2% | 148.7% | 149.6% | 119.6% | 0.1349 |
| TRD005879 | 78.5% | 132.2% | 140.2% | 142.9% | 140.5% | 0.1318 |
| TRD005882 | 78.5% | 114.3% | 165.6% | 145.6% | 109.9% | 0.1072 |
| TRD005885 | 100.7% | 130.5% | 155.8% | 117.4% | 105.0% | 0.2239 |
| TRD005891 | 95.8% | 109.4% | 122.0% | 128.9% | 105.3% | 0.1862 |

**Example 25. siRNAs' On-Target Activity and Off-Target Level Validation by psiCHECK**

After siRNAs were modified (in position 7 of the AS strand) in HEK293A cells using 11 concentration gradients, *in vitro* molecular level simulation on-target and off-target activity screening was performed. The results show that the siRNAs of the present disclosure have low off-target activity while having high activity. See Example 16 for the experimental procedure. To improve detection sensitivity, a GSSM-5hits off-target plasmid, i.e. 5 identical GSSM sequences linked by TTCC, was constructed for the antisense strands of siRNAs.

The results are shown in Table 43 to Table 46. The results show that all the siRNAs had high-level *in vitro* on-target inhibitory activity (GSCM IC50 value less than 0.3 nM) and no significant off-target effect. Procedure of psiCHECK activity screening

In the HEK293A cell line, the activity of siRNAs was determined by performing psiCHECK activity assays. The experimental materials and instruments are detailed in Table 1 and Table 2 in Example 3.1, and the experimental results

are detailed in Table 43 to Table 46.

See Example 3.2 for the experimental procedure of psiCHECK activity screening, wherein the multi-concentration dilution protocol for siRNA samples is shown in Table 42.

Table 42. Multi-concentration dilution protocol for siRNAs

| siRNA concentration (μM) | Final concentration (nM) | Added water and siRNA |
|---|---|---|
| 20 | / | / |
| 4 | 40 | 4 μL siRNA + 16 μL H2O |
| 1.333333 | 13.33333 | 20 μL siRNA + 40 μL H2O |
| 0.444444 | 4.444444 | 20 μL siRNA + 40 μL H2O |
| 0.148148 | 1.481481 | 20 μL siRNA + 40 μL $H_2O$ |
| 0.049383 | 0.493827 | 20 μL siRNA + 40 μL $H_2O$ |
| 0.016461 | 0.164609 | 20 μL siRNA + 40 μL $H_2O$ |
| 0.005487 | 0.05487 | 20 μL siRNA + 40 μL $H_2O$ |
| 0.001829 | 0.01829 | 20 μL siRNA + 40 μL $H_2O$ |
| 0.00061 | 0.006097 | 20 μL siRNA + 40 μL $H_2O$ |
| 0.000203 | 0.002032 | 20 μL siRNA + 40 μL $H_2O$ |
| 6.77E-05 | 0.000677 | 20 μL siRNA + 40 μL $H_2O$ |

Table 43. Results of psiCHECK on-target activity screening of siRNAs (GSCM)

| Double strand No. | 40nM | 13.3nM | 4.44nM | 1.48nM | 0.494nM | 0.165nM |
|---|---|---|---|---|---|---|
| TRD005874 | 7.0% | 4.9% | 4.7% | 4.6% | 5.3% | 8.5% |
| TRD005875 | 27.1% | 19.8% | 14.0% | 12.4% | 12.6% | 18.7% |
| TRD005876 | 49.9% | 24.8% | 16.9% | 14.5% | 15.2% | 18.6% |
| TRD005877 | 16.6% | 8.2% | 6.0% | 6.5% | 9.5% | 19.8% |
| TRD005878 | 21.9% | 12.9% | 9.2% | 7.1% | 7.8% | 10.9% |
| TRD005879 | 15.1% | 8.0% | 4.6% | 4.8% | 4.7% | 6.5% |
| TRD005882 | 18.4% | 9.7% | 9.3% | 9.0% | 8.8% | 10.4% |
| TRD005883 | 38.3% | 22.4% | 13.0% | 13.4% | 19.3% | 38.6% |
| TRD005884 | 27.1% | 18.0% | 13.6% | 14.2% | 18.7% | 33.9% |
| TRD005886 | 49.5% | 30.0% | 19.5% | 17.0% | 18.0% | 30.6% |
| TRD005887 | 21.0% | 12.0% | 10.3% | 9.6% | 11.3% | 19.4% |
| TRD005889 | 50.7% | 32.9% | 25.8% | 28.5% | 39.2% | 58.7% |
| TRD005890 | 77.9% | 48.0% | 33.0% | 27.7% | 27.6% | 37.9% |
| TRD005891 | 27.0% | 16.6% | 12.2% | 10.1% | 8.9% | 12.0% |
| TRD005893 | 84.6% | 58.2% | 40.8% | 30.6% | 29.5% | 42.6% |
| Double strand No. | 0.0549nM | 0.0183nM | 0.00609nM | 0.00203nM | 0.000677nM | GSCM IC50 value (nM) |
| TRD005874 | 16.6% | 35.4% | 70.5% | 85.8% | 97.8% | 0.0115 |
| TRD005875 | 34.0% | 55.5% | 80.1% | 95.7% | 99.9% | 0.0229 |
| TRD005876 | 31.2% | 53.9% | 82.8% | 91.6% | 99.5% | 0.0214 |
| TRD005877 | 43.7% | 72.5% | 90.6% | 97.6% | 98.1% | 0.041 |

(continued)

| Double strand No. | 0.0549nM | 0.0183nM | 0.00609nM | 0.00203nM | 0.000677nM | GSCM IC50 value (nM) |
|---|---|---|---|---|---|---|
| TRD005878 | 21.5% | 51.9% | 74.9% | 86.5% | 98.0% | 0.017 |
| TRD005879 | 12.6% | 30.3% | 55.7% | 79.3% | 91.8% | 0.0076 |
| TRD005882 | 20.3% | 45.7% | 75.5% | 92.6% | 99.6% | 0.0148 |
| TRD005883 | 69.4% | 85.9% | 95.3% | 96.6% | 101.4% | 0.1023 |
| TRD005884 | 59.3% | 81.4% | 93.2% | 97.4% | 101.3% | 0.0759 |
| TRD005886 | 57.8% | 81.1% | 89.5% | 101.8% | 99.0% | 0.0646 |
| TRD005887 | 45.5% | 71.4% | 85.4% | 95.2% | 94.3% | 0.0417 |
| TRD005889 | 75.6% | 85.2% | 91.0% | 96.2% | 95.6% | 0.2399 |
| TRD005890 | 60.9% | 86.1% | 94.5% | 102.2% | 104.4% | 0.0813 |
| TRD005891 | 25.9% | 52.6% | 75.5% | 90.3% | 98.8% | 0.0186 |
| TRD005893 | 61.0% | 75.5% | 89.2% | 92.1% | 96.8% | 0.0851 |
| Note: since the transfection efficiency was low at the highest concentration (40 nM) due to the internal synthesis process, the experimental data corresponding to the highest concentration (40 nM) were discarded at the time of data processing. | | | | | | |

Table 44. Results of psiCHECK off-target activity screening of the seed regions of the AS strands of siRNAs (GSSM-5hits)

| Double strand No. | 40 nM | 13.3 nM | 4.44 nM | 1.48 nM | 0.494 nM | 0.165 nM | 0.0549 nM | 0.0183 nM | 0.00609 nM | 0.00203 nM | 0.000677 nM | IC50 value (nM) |
|---|---|---|---|---|---|---|---|---|---|---|---|---|
| TRD005875 | 1.00 | 0.97 | 0.98 | 1.00 | 1.04 | 1.06 | 1.00 | 1.07 | 1.01 | 1.16 | 1.03 | ND |
| TRD005882 | 0.72 | 0.75 | 0.89 | 0.92 | 1.01 | 0.96 | 0.92 | 1.01 | 0.90 | 1.00 | 0.91 | 80.4 |
| TRD005891 | 0.78 | 0.76 | 0.84 | 0.90 | 0.98 | 0.98 | 1.00 | 0.98 | 1.01 | 0.97 | 0.95 | 84.4 |
| TRD005874 | 0.72 | 0.69 | 0.74 | 0.93 | 0.98 | 1.00 | 0.99 | 0.98 | 0.95 | 1.12 | 0.93 | 59.0 |
| TRD005887 | 0.75 | 0.74 | 0.82 | 0.85 | 0.91 | 0.92 | 0.94 | 1.02 | 1.03 | 0.98 | 1.08 | 101.0 |
| Note: ND = undetectable. | | | | | | | | | | | | |

Table 45. Results of psiCHECK off-target activity screening of the SS strands of siRNAs (PSCM)

| Double strand No. | 40 nM | 13.3 nM | 4.44 nM | 1.48 nM | 0.494 nM | 0.165 nM | 0.0549 nM | 0.0183 nM | 0.00609 nM | 0.00203 nM | 0.000677 nM | IC50 value (nM) |
|---|---|---|---|---|---|---|---|---|---|---|---|---|
| TRD005874 | 0.57 | 0.60 | 0.69 | 0.79 | 0.87 | 0.99 | 0.97 | 1.02 | 1.01 | 1.00 | 0.97 | 26.2 |
| TRD005875 | 0.82 | 0.78 | 0.81 | 0.87 | 0.94 | 0.98 | 1.02 | 0.95 | 0.98 | 0.99 | 0.82 | 123.9 |
| TRD005877 | 0.95 | 0.78 | 0.76 | 0.73 | 0.80 | 0.85 | 0.99 | 0.95 | 1.03 | 1.08 | 0.93 | 291.2 |
| TRD005878 | 0.57 | 0.60 | 0.69 | 0.78 | 0.89 | 0.92 | 0.98 | 1.04 | 1.03 | 0.96 | 0.90 | 29.5 |
| TRD005879 | 0.65 | 0.64 | 0.72 | 0.80 | 0.89 | 0.95 | 1.02 | 1.06 | 1.03 | 1.06 | 0.98 | 48.4 |
| TRD005882 | 1.36 | 1.04 | 1.07 | 1.04 | 1.04 | 1.06 | 1.08 | 1.03 | 1.07 | 1.03 | 0.96 | ND |
| TRD005883 | 0.88 | 0.90 | 0.90 | 0.91 | 0.86 | 0.94 | 0.91 | 0.97 | 1.01 | 0.98 | 0.97 | 353.6 |
| TRD005884 | 0.92 | 0.86 | 0.94 | 0.97 | 1.01 | 1.00 | 0.98 | 1.00 | 0.98 | 0.95 | 0.97 | 244.5 |
| TRD005885 | 0.42 | 0.47 | 0.68 | 0.81 | 0.92 | 0.97 | 0.92 | 1.02 | 1.00 | 0.99 | 0.98 | 16.6 |
| TRD005887 | 0.72 | 0.70 | 0.78 | 0.85 | 0.95 | 0.98 | 1.04 | 1.02 | 1.06 | 0.93 | 0.89 | 61.8 |
| TRD005891 | 0.70 | 0.87 | 0.93 | 0.95 | 0.98 | 0.95 | 0.97 | 0.96 | 0.96 | 1.03 | 0.95 | 95.6 |
| TRD005892 | 0.73 | 0.61 | 0.78 | 0.91 | 0.98 | 0.99 | 0.97 | 0.97 | 1.00 | 1.03 | 0.97 | 19.8 |
| Note: ND = undetectable. | | | | | | | | | | | | |

Table 46. Results of psiCHECK off-target activity screening of the seed regions of the SS strands of siRNAs (PSSM)

| Double strand No. | 40 nM | 13.3 nM | 4.44 nM | 1.48 nM | 0.494 nM | 0.165 nM | 0.0549 nM | 0.0183 nM | 0.00609 nM | 0.00203 nM | 0.000677 nM | IC50 value (nM) |
|---|---|---|---|---|---|---|---|---|---|---|---|---|
| TRD005874 | 0.78 | 0.85 | 0.96 | 1.07 | 0.83 | 1.01 | 0.89 | 0.87 | 0.89 | 0.95 | 0.88 | 140.1 |
| TRD005875 | 1.19 | 1.00 | 0.87 | 0.89 | 0.91 | 0.94 | 0.99 | 0.99 | 0.96 | 0.98 | 0.90 | ND |
| TRD005877 | 1.05 | 0.85 | 0.72 | 0.82 | 0.86 | 0.87 | 0.92 | 0.96 | 1.07 | 0.99 | 0.88 | ND |
| TRD005878 | 0.70 | 0.64 | 0.69 | 0.81 | 0.81 | 0.81 | 0.97 | 1.01 | 0.96 | 0.90 | 0.91 | 46.5 |
| TRD005879 | 0.72 | 0.64 | 0.71 | 0.86 | 0.91 | 1.01 | 1.01 | 1.05 | 1.07 | 1.00 | 0.92 | 18.3 |
| TRD005882 | 1.37 | 1.10 | 1.06 | 1.06 | 1.06 | 1.05 | 1.04 | 1.02 | 1.02 | 0.98 | 0.94 | ND |
| TRD005883 | 1.21 | 1.09 | 1.00 | 0.91 | 0.91 | 0.95 | 0.97 | 1.04 | 1.05 | 1.04 | 1.02 | ND |
| TRD005884 | 0.98 | 0.95 | 0.95 | 1.06 | 1.07 | 1.04 | 1.09 | 1.08 | 1.03 | 1.05 | 0.99 | 699.4 |
| TRD005885 | 0.81 | 0.78 | 0.97 | 0.90 | 0.99 | 0.94 | 1.01 | 1.05 | 1.01 | 0.94 | 0.94 | 116.6 |
| TRD005887 | 0.95 | 0.88 | 0.88 | 0.99 | 1.03 | 1.03 | 1.04 | 1.08 | 1.03 | 1.00 | 1.01 | 334.6 |
| TRD005891 | 0.74 | 0.81 | 0.92 | 0.99 | 0.97 | 1.07 | 1.05 | 1.01 | 1.03 | 1.05 | 1.07 | 93.6 |
| TRD005892 | 0.77 | 0.70 | 0.83 | 0.95 | 0.96 | 1.00 | 1.06 | 1.05 | 1.08 | 1.05 | 1.09 | 26.4 |
| Note: ND = undetectable. | | | | | | | | | | | | |

**Example 26. Inhibition of Human ApoC3 in Huh7 Cells by siRNAs** - 11 **Concentration Point Inhibitory Activity**

After siRNAs were modified (in position 7 of the AS strand) in Huh7 cells using 11 concentration gradients, Huh7 cell viability screening was performed. Each siRNA sample for transfection was serially diluted 3-fold from the starting final concentration 20 nM to 11 concentration points.

Huh7 cells were cultured at 37 °C with 5% $CO_2$ in a DMEM high glucose medium containing 10% fetal bovine serum. 24 h prior to transfection, the Huh7 cells were inoculated into a 96-well plate at a density of 10 thousand cells per well. Each well contained 100 μL of medium.

The cells were transfected with siRNAs at final concentrations of 20 nM, 6.67 nM, 2.22 nM, 0.741 nM, 0.247 nM, 0.0823 nM, 0.0274 nM, 0.00914 nM, 0.00305 nM, 0.00102 nM and 0.000339 nM using Lipofectamine RNAi MAX (ThermoFisher, 13778150) according to the instructions of the product. 24 h after treatment, the total cellular RNA was extracted from the cells using a high-throughput cellular RNA extraction kit, and RNA reverse transcription and quantitative real-time PCR detection were carried out. The human ApoC3 mRNA level was measured and corrected based on the ACTIN internal reference gene level.

The results are expressed relative to the remaining percentage of human ApoC3 mRNA expression in cells treated with the control siRNA. The IC50 results of inhibition are shown in Table 53.

Experimental materials for cell viability screening (nucleic acid extractor) in a 96-well plate are shown in Table 1 and Table 2.

Experimental procedure of cell viability screening (nucleic acid extractor) in a 96-well plate:

I. Cell transfection

Reference was made to the procedure of cell transfection in Example 21.

The amounts of the components of the transfection complex are shown in Table 47:

Table 47. Amounts required for transfection complex in each well of a 96-well plate

| | Amount | Opti-MEM |
|---|---|---|
| siRNA | According to actual needs | 15 μL |
| RNAiMAX | 0.9 μL | 15 μL |

II. Extraction of cellular RNA using nucleic acid extractor (magnetic bead method)

1. Preparation: high-throughput cellular RNA extraction kit (FG0417-L/ FG0418-XL, magnetic bead method).

Table 48. Cellular RNA extraction kit components and storage conditions

| Cellular RNA extraction kit (FG0410-L, magnetic bead method) | | |
|---|---|---|
| Kit component | Volume (mL) | Storage conditions |
| Suspension of magnetic beads | 2.2 | 4 °C |
| Lysis solution LB | 22 | Room temperature |
| Buffer WB1 | 22 | Room temperature |
| Buffer WB2 | 5.5 | Room temperature |
| Eluent RFW | 5.5 | Room temperature |
| DNase I | 0.4 | -20 °C |
| Dnase dilution solution | 0.6 | -20 °C |
| 1 M DTT solution | 1 | -20 °C |

2. The following reagents were added to 6 deep-well plates.

Table 49. Addition of different reagent components and volumes to 6 deep-well plates

| | Reagent component | Volume (μL/well) |
|---|---|---|
| 96-deep-well plate 1 | Buffer WB1 | 150 |
| 96-deep-well plate 2 | DNase I Mix solution | 50 |
| 96-deep-well plate 3 | Isopropanol | 100 |
| | Magnetic bead | 20 |
| | Cell lysate supernatant | 200 |
| 96-deep-well plate 4 | Buffer WB2 | 200 |
| 96-deep-well plate 5 | Absolute ethanol | 200 |
| 96-deep-well plate 6 | Eluent RFW | 50 |
| Note: Absolute ethanol was added to each of the buffers WB1 and WB2 in the recommended amount on the label. | | |

Preparation of a cell lysate: 200 μL of lysis solution LB + 3.5 μL of 1 M DTT solution; the culture supernatant in the 96-well plate was completely aspirated, the mixture of solutions was added at 200 μL/well, and lysis was performed for 5 min.

DNase I Mix solution: 3.4 μL of DNase I + 5 μL of DNase dilution solution + 41.6 μL of 0.1% DEPC water (50 μL per well, mixed well). The prepared DNase I Mix was placed on ice.

Instrument program selection: cell RNA 96.

3. The 6 deep-well plates were placed into 6 corresponding cartridges of a nucleic acid extractor and marked, and tip combs were placed into 96-deep-well plate 3. The instrument was started, and a cellular RNA extraction program was run. After 35 min, the program was paused. 96-deep-well plate 2 was taken out and 220 μL of buffer WB1 was added to it. Then the cellular RNA extraction program was resumed.

4. After completion of the nucleic acid extraction and concentration measurement, the 96-deep-well plates were sealed with aluminum foil sealing film and fully marked. The plates could be stored in a refrigerator at 4 °C before use in reverse transcription or stored in a freezer at -40 °C.

III. Reverse transcription of cellular RNA

1. Preparation: (1) reverse transcription kit (Takara PrimeScript™ II 1st Strand cDNA Synthesis Kit (6210A); the shelf life was checked and the kit components were all stored in a freezer at -40 °C).

Table 50. Reverse transcription kit components

| Takara PrimeScript™ II 1st Strand cDNA Synthesis Kit (6210A) | |
|---|---|
| Kit component and concentration | Volume |
| PrimeScript II RTase (200 U/μL) | 50 μL |
| 5×PrimeScript II Buffer | 200 μL |
| RNase Inhibitor (40 U/μL) | 25 μL |
| dNTP Mixture (10 mM each) | 50 μL |
| Oligo dT Primer (50 μM) | 50 μL |
| Random 6 mers (50 μM) | 100 μL |
| RNase Free dH2O | 1 ml |

2. The following reaction mixture (Mix1) was prepared in a Microtube.

Table 51. Reaction mixture Mix1

| Reagent | Amount |
|---|---|
| Oligo dT Primer (50 μM) | 1 μL |
| dNTP Mixture (10 mM each) | 1 μL |
| Template RNA | Total RNA: 1 μg |
| RNase Free dH2O | Up to 10 μL |
| After 5 min of incubation at 65 °C, the mixture was quickly cooled on ice for 2 min. (Note: the above treatment can denature the template RNA, improving the reverse transcription efficiency.) | |

3. The following reverse transcription reaction mixture (Mix2) was prepared in a Microtube.

Table 52. Reaction mixture Mix2

| Reagent | Amount |
|---|---|
| 5×PrimeScript II Buffer | 4 μL |
| RNase Inhibitor (40 U/μL) | 0.5 μL (20 U) |
| PrimeScript II RTase (200 U/μL) | 1 μL (200 U) |
| RNase Free dH2O | 4.5 μL |
| Total | 10 μL |

10 μL of Mix2 was added to Mix1, making a total volume of 20 μL. Inversion was performed as follows: 42 °C 45 min, 95 °C 5 min, 4 °C Forever.

4. After the inversion was complete, 80 μL of DEPC water (final concentration: 10 ng/μL) was added to each tube, and the samples could be stored in a refrigerator at 4 °C before use in Taqman Q-PCR or stored in a freezer at -40 °C.

IV. Taqman probe Q-PCR assay. See Example 16 for the experimental procedure and Table 53 for the results.

Table 53. Multi-dose inhibitory activity of siRNAs against human ApoC3 in Huh7 cells

| Double strand No. | 20nM | 6.67nM | 2.22nM | 0.741nM | 0.247nM | 0.0823nM |
|---|---|---|---|---|---|---|
| TRD006884 | 1.7% | 2.6% | 4.1% | 7.4% | 25.1% | 55.2% |
| TRD006885 | 4.1% | 2.4% | 3.2% | 5.7% | 11.9% | 30.0% |
| TRD006886 | 2.8% | 3.0% | 4.5% | 7.6% | 17.4% | 45.8% |
| TRD006887 | 1.1% | 1.3% | 2.5% | 6.0% | 17.3% | 51.6% |
| TRD006888 | 1.1% | 2.4% | 1.8% | 2.7% | 4.9% | 12.4% |
| TRD006925 | 1.7% | 1.2% | 2.4% | 2.8% | 6.5% | 21.3% |
| TRD006928 | 0.9% | 0.9% | 1.2% | 1.8% | 2.6% | 5.2% |
| TRD006937 | 3.1% | 2.1% | 3.5% | 6.9% | 7.8% | 22.5% |
| TRD006964 | 3.2% | 3.9% | 2.4% | 6.2% | 8.8% | 23.0% |
| Double strand No. | 0.0274nM | 0.00914nM | 0.00305nM | 0.00102nM | 0.000339nM | Huh7 cell IC50 value (nM) |
| TRD006884 | 104.3% | 168.0% | 162.9% | 129.1% | 111.7% | 0.0933 |
| TRD006885 | 65.1% | 107.4% | 102.2% | 112.7% | 92.3% | 0.0912 |
| TRD006886 | 94.5% | 110.2% | 101.7% | 115.6% | 99.9% | 0.138 |
| TRD006887 | 65.9% | 171.1% | 135.3% | 135.9% | 105.2% | 0.1096 |
| TRD006888 | 32.3% | 66.7% | 116.0% | 109.9% | 101.0% | 0.0288 |

(continued)

| Double strand No. | 0.0274nM | 0.00914nM | 0.00305nM | 0.00102nM | 0.000339nM | Huh7 cell IC50 value (nM) |
|---|---|---|---|---|---|---|
| TRD006925 | 54.8% | 103.0% | 116.4% | 118.5% | 104.3% | 0.0871 |
| TRD006928 | 11.1% | 26.2% | 47.0% | 72.1% | 64.9% | 0.0031 |
| TRD006937 | 38.9% | 58.4% | 102.3% | 82.3% | 78.7% | 0.0195 |
| TRD006964 | 53.0% | 65.8% | 102.7% | 73.7% | 106.9% | 0.024 |

**Example 27. siRNAs' On-Target Activity and Off-Target Level Validation by psiCHECK**

*In vitro* molecular level simulation on-target and off-target level screening was performed on test compounds in HEK293A cells using 11 concentration gradients. The results show that the siRNAs of the present disclosure have low off-target activity while having high activity. The Psi-CHECK plasmids were purchased from Synbio Technologies (Suzhou) Co., Ltd. and Sangon Biotech (Shanghai) Co., Ltd. See Example 16 for the experimental procedure. To improve detection sensitivity, a GSSM-5hits off-target plasmid, i.e. 5 identical GSSM sequences linked by TTCC, was constructed for the antisense strands of siRNAs.

The results show that all 6 siRNAs had high-level *in vitro* on-target inhibitory activity (GSCM IC50 value less than 0.3 nM). The off-target evaluation (GSSM-5hits, PSCM, PSSM) results of the siRNAs show that 5 siRNAs showed no significant off-target effect.

In the HEK293A cell line, the activity of the 6 siRNAs was determined by performing psi-CHECK activity assays. The experimental results are detailed in Table 54 to Table 57.

Table 54. Results of psiCHECK on-target activity screening of siRNAs (GSCM)

| Double strand No. | 20nM | 6.67nM | 2.22nM | 0.741nM | 0.247nM | 0.0823nM |
|---|---|---|---|---|---|---|
| TRD006884 | 15.9% | 12.6% | 12.7% | 14.6% | 21.4% | 42.7% |
| TRD006885 | 11.5% | 8.4% | 7.2% | 6.7% | 9.3% | 23.7% |
| TRD006886 | 10.3% | 9.7% | 9.5% | 9.1% | 10.5% | 20.4% |
| TRD006887 | 15.8% | 13.2% | 13.2% | 18.3% | 33.5% | 60.9% |
| TRD006888 | 5.8% | 5.1% | 4.9% | 5.5% | 8.1% | 18.1% |
| TRD005205 | 25.1% | 18.8% | 19.6% | 24.8% | 52.0% | 75.8% |
| TRD006925 | 6.8% | 5.6% | 5.5% | 6.3% | 10.9% | 34.6% |
| TRD006971 | 4.8% | 4.5% | 4.5% | 4.3% | 5.6% | 11.7% |
| TRD006973 | 14.2% | 13.3% | 12.4% | 14.5% | 24.6% | 51.4% |
| TRD006975 | 12.5% | 11.5% | 11.6% | 11.2% | 17.5% | 35.9% |
| TRD006926 | 6.1% | 5.5% | 5.3% | 5.9% | 8.2% | 14.7% |
| TRD006927 | 5.7% | 5.5% | 5.4% | 5.7% | 7.2% | 11.8% |
| TRD006928 | 5.1% | 5.3% | 5.2% | 5.2% | 7.0% | 11.6% |
| TRD006929 | 14.9% | 13.2% | 12.7% | 14.7% | 28.3% | 53.0% |
| TRD006930 | 14.5% | 12.6% | 13.1% | 15.8% | 28.4% | 53.6% |
| TRD006931 | 16.8% | 14.1% | 13.5% | 15.2% | 24.2% | 44.3% |
| TRD006932 | 10.7% | 11.2% | 11.4% | 11.2% | 13.7% | 25.9% |
| TRD006933 | 11.1% | 10.5% | 11.1% | 10.6% | 13.6% | 21.2% |
| TRD006934 | 11.3% | 11.5% | 12.0% | 11.6% | 12.7% | 22.0% |
| TRD006935 | 18.7% | 15.2% | 14.1% | 15.9% | 27.6% | 52.5% |
| TRD006936 | 18.0% | 14.6% | 13.5% | 14.4% | 22.1% | 45.9% |

(continued)

| Double strand No. | 20nM | 6.67nM | 2.22nM | 0.741nM | 0.247nM | 0.0823nM |
|---|---|---|---|---|---|---|
| TRD006937 | 17.5% | 13.9% | 13.0% | 13.4% | 21.4% | 41.5% |
| TRD006938 | 12.3% | 9.3% | 8.3% | 7.7% | 10.7% | 21.8% |
| TRD006939 | 17.8% | 11.2% | 8.8% | 8.0% | 10.6% | 23.9% |
| TRD006940 | 9.9% | 8.8% | 8.4% | 7.8% | 11.0% | 22.3% |
| TRD006963 | 12.6% | 10.1% | 9.8% | 11.4% | 20.9% | 46.7% |
| TRD006964 | 11.8% | 10.1% | 10.5% | 11.5% | 20.1% | 39.9% |
| TRD006965 | 11.6% | 10.3% | 10.2% | 11.7% | 20.4% | 40.9% |
| TRD006966 | 13.5% | 10.6% | 11.8% | 17.5% | 39.2% | 74.5% |
| TRD005883 | 13.4% | 10.8% | 11.3% | 18.9% | 43.9% | 77.6% |
| Double strand No. | 0.0274nM | 0.00914nM | 0.00305nM | 0.00102nM | 0.000339nM | GSCM IC50 value (nM) |
| TRD006884 | 71.7% | 97.5% | 99.7% | 100.3% | 101.6% | 0.0753 |
| TRD006885 | 58.5% | 83.0% | 94.0% | 96.9% | 97.4% | 0.0349 |
| TRD006886 | 46.2% | 79.6% | 91.0% | 102.6% | 101.0% | 0.0271 |
| TRD006887 | 86.9% | 93.0% | 96.8% | 99.6% | 103.5% | 0.1433 |
| TRD006888 | 49.6% | 81.3% | 95.1% | 100.4% | 108.9% | 0.0282 |
| TRD005205 | 87.4% | 97.2% | 99.2% | 103.3% | 106.3% | 0.2847 |
| TRD006925 | 69.7% | 90.8% | 100.5% | 103.7% | 102.1% | 0.0531 |
| TRD006971 | 26.1% | 60.0% | 92.8% | 99.6% | 104.7% | 0.014 |
| TRD006973 | 75.9% | 95.3% | 100.6% | 98.4% | 97.4% | 0.094 |
| TRD006975 | 67.5% | 88.2% | 97.8% | 98.9% | 99.0% | 0.0555 |
| TRD006926 | 37.3% | 78.0% | 91.4% | 100.4% | 97.5% | 0.0198 |
| TRD006927 | 25.5% | 63.2% | 86.5% | 95.5% | 99.0% | 0.0129 |
| TRD006928 | 27.6% | 72.2% | 86.0% | 101.4% | 99.8% | 0.0151 |
| TRD006929 | 81.6% | 113.2% | 111.4% | 108.1% | 102.2% | 0.0905 |
| TRD006930 | 80.4% | 95.9% | 102.3% | 97.9% | 101.0% | 0.0912 |
| TRD006931 | 75.7% | 95.7% | 102.2% | 105.3% | 106.1% | 0.0656 |
| TRD006932 | 55.0% | 92.9% | 104.8% | 109.0% | 101.1% | 0.0326 |
| TRD006933 | 54.6% | 83.1% | 98.1% | 101.1% | 92.9% | 0.03 |
| TRD006934 | 48.2% | 88.6% | 97.3% | 119.5% | 105.7% | 0.0261 |
| TRD006935 | 81.8% | 96.9% | 97.9% | 104.7% | 102.6% | 0.0891 |
| TRD006936 | 76.5% | 96.4% | 100.2% | 99.5% | 90.3% | 0.0687 |
| TRD006937 | 70.0% | 91.3% | 93.3% | 95.1% | 92.3% | 0.0589 |
| TRD006938 | 58.1% | 93.1% | 96.3% | 100.4% | 94.1% | 0.0336 |
| TRD006939 | 61.5% | 92.2% | 97.3% | 100.7% | 98.4% | 0.036 |
| TRD006940 | 62.9% | 88.7% | 99.2% | 87.3% | 92.1% | 0.0366 |
| TRD006963 | 74.5% | 83.7% | 97.3% | 98.6% | 92.7% | 0.0676 |
| TRD006964 | 73.7% | 94.1% | 97.4% | 105.6% | 97.8% | 0.0593 |
| TRD006965 | 69.8% | 90.4% | 94.4% | 98.5% | 92.8% | 0.0584 |

(continued)

| Double strand No. | 0.0274nM | 0.00914nM | 0.00305nM | 0.00102nM | 0.000339nM | GSCM IC50 value (nM) |
|---|---|---|---|---|---|---|
| TRD006966 | 88.8% | 101.9% | 100.4% | 102.5% | 98.9% | 0.1711 |
| TRD005883 | 88.9% | 101.2% | 99.2% | 98.6% | 92.7% | 0.1995 |

Table 55. Results of psiCHECK off-target activity screening of the seed regions of the AS strands of siRNAs (GSSM-5hits)

| Double strand No. | 20nM | 6.67nM | 2.22nM | 0.741nM | 0.247nM | 0.0823nM |
|---|---|---|---|---|---|---|
| TRD006884 | 92.8% | 95.2% | 108.1% | 118.2% | 112.6% | 114.2% |
| TRD006885 | 85.1% | 93.6% | 106.3% | 115.6% | 117.8% | 107.1% |
| TRD006886 | 89.9% | 93.4% | 102.7% | 109.5% | 112.2% | 113.3% |
| TRD006887 | 63.2% | 63.6% | 89.5% | 106.7% | 113.7% | 106.5% |
| TRD006888 | 71.9% | 80.9% | 95.5% | 107.9% | 110.9% | 108.0% |
| TRD005205 | 48.2% | 42.9% | 55.7% | 78.9% | 96.3% | 104.2% |
| TRD006971 | 72.8% | 78.0% | 89.4% | 94.4% | 97.3% | 108.2% |
| TRD006973 | 41.5% | 44.2% | 73.6% | 93.8% | 104.1% | 122.2% |
| TRD006975 | 37.2% | 47.7% | 75.0% | 102.0% | 104.1% | 108.7% |
| Double strand No. | 0.0274nM | 0.00914nM | 0.00305nM | 0.00102nM | 0.000339nM | Fit IC50 value for GSSM-5hits (nM) |
| TRD006884 | 115.8% | 111.6% | 107.9% | 115.5% | 105.0% | 309 |
| TRD006885 | 113.1% | 106.2% | 110.9% | 111.9% | 108.6% | 131 |
| TRD006886 | 114.1% | 113.4% | 106.7% | 107.1% | 108.4% | 180 |
| TRD006887 | 107.0% | 105.0% | 105.0% | 114.5% | 113.3% | 24 |
| TRD006888 | 107.5% | 101.4% | 100.1% | 104.4% | 106.1% | 46 |
| TRD005205 | 103.8% | 105.0% | 112.0% | 102.5% | 106.4% | 6 |
| TRD006971 | 106.9% | 102.9% | 102.0% | 96.2% | 93.1% | 41 |
| TRD006973 | 111.2% | 108.6% | 110.8% | 104.0% | 97.8% | 8 |
| TRD006975 | 101.8% | 103.5% | 104.0% | 103.9% | 93.3% | 8 |

Table 56. Results of psiCHECK off-target activity screening of the SS strands of siRNAs (PSCM)

| Double strand No. | 20nM | 6.67nM | 2.22nM | 0.741nM | 0.247nM | 0.0823nM |
|---|---|---|---|---|---|---|
| TRD006884 | 81.7% | 87.8% | 102.8% | 118.3% | 116.1% | 113.8% |
| TRD006885 | 87.0% | 106.7% | 121.9% | 127.4% | 126.7% | 111.9% |
| TRD006886 | 104.0% | 103.4% | 99.3% | 95.1% | 105.1% | 109.6% |
| TRD006887 | 116.9% | 117.1% | 112.6% | 111.7% | 112.9% | 104.0% |
| TRD006888 | 79.0% | 89.0% | 95.1% | 105.6% | 110.0% | 111.9% |
| TRD005205 | 83.2% | 98.0% | 101.9% | 110.4% | 108.9% | 107.9% |
| TRD006925 | 76.7% | 89.6% | 103.4% | 104.6% | 110.0% | 120.0% |

(continued)

| Double strand No. | 20nM | 6.67nM | 2.22nM | 0.741nM | 0.247nM | 0.0823nM |
|---|---|---|---|---|---|---|
| TRD006971 | 76.5% | 86.2% | 94.5% | 99.8% | 103.8% | 119.7% |
| TRD006973 | 111.6% | 115.5% | 109.3% | 109.1% | 102.4% | 125.1% |
| TRD006975 | 119.4% | 118.6% | 130.2% | 107.8% | 98.3% | 104.0% |
| Double strand No. | 0.0274nM | 0.00914nM | 0.00305nM | 0.00102nM | 0.000339nM | Fit IC50 value for PSCM (nM) |
| TRD006884 | 121.3% | 105.8% | 108.8% | 110.9% | 104.9% | 90 |
| TRD006885 | 114.1% | 111.2% | 112.8% | 112.2% | 108.5% | 342 |
| TRD006886 | 116.2% | 107.2% | 100.8% | 107.9% | 105.6% | ND |
| TRD006887 | 104.1% | 102.0% | 104.6% | 108.1% | 107.2% | ND |
| TRD006888 | 116.9% | 104.8% | 99.0% | 105.5% | 105.4% | 72 |
| TRD005205 | 113.2% | 110.7% | 120.9% | 101.6% | 118.9% | 121 |
| TRD006925 | 117.9% | 107.5% | 106.3% | 97.9% | 94.3% | 70 |
| TRD006971 | 113.0% | 110.6% | 112.3% | 99.8% | 102.4% | 59 |
| TRD006973 | 111.6% | 104.2% | 101.6% | 98.4% | 92.0% | ND |
| TRD006975 | 97.1% | 98.8% | 104.0% | 97.8% | 95.6% | ND |
| **Note: ND = undetectable.** | | | | | | |

Table 57. Results of psiCHECK off-target activity screening of the seed regions of the SS strands of siRNAs (PSSM)

| Double strand No. | 20nM | 6.67nM | 2.22nM | 0.741nM | 0.247nM | 0.0823nM |
|---|---|---|---|---|---|---|
| TRD006884 | 108.3% | 103.8% | 77.5% | 111.3% | 105.7% | 109.0% |
| TRD006885 | 87.1% | 102.6% | 131.1% | 133.4% | 126.8% | 113.6% |
| TRD006886 | 105.4% | 109.7% | 110.3% | 110.1% | 107.9% | 108.1% |
| TRD006887 | 120.1% | 123.7% | 130.2% | 118.6% | 120.5% | 109.6% |
| TRD006888 | 69.5% | 87.3% | 94.1% | 103.7% | 108.1% | 107.4% |
| TRD005205 | 89.9% | 97.9% | 112.5% | 113.1% | 108.0% | 115.1% |
| TRD006925 | 81.3% | 92.4% | 102.2% | 107.8% | 109.2% | 126.1% |
| TRD006971 | 80.0% | 83.9% | 96.6% | 94.8% | 97.3% | 125.7% |
| TRD006973 | 109.0% | 110.4% | 109.1% | 103.1% | 102.5% | 116.0% |
| TRD006975 | 127.0% | 128.9% | 133.1% | 117.0% | 107.7% | 121.5% |
| Double strand No. | 0.0274nM | 0.00914nM | 0.00305nM | 0.00102nM | 0.000339nM | Fit IC50 value for PSSM (nM) |
| TRD006884 | 108.9% | 98.8% | 102.1% | 104.9% | 104.8% | ND |
| TRD006885 | 116.0% | 114.0% | 116.4% | 112.9% | 113.6% | 340 |
| TRD006886 | 110.5% | 110.5% | 105.6% | 103.5% | 98.7% | ND |
| TRD006887 | 107.5% | 105.5% | 108.9% | 109.0% | 109.8% | ND |
| TRD006888 | 110.4% | 103.1% | 99.8% | 103.3% | 105.2% | 46 |
| TRD005205 | 114.1% | 112.3% | 126.3% | 114.3% | 124.3% | 251 |

(continued)

| Double strand No. | 0.0274nM | 0.00914nM | 0.00305nM | 0.00102nM | 0.000339nM | Fit IC50 value for PSSM (nM) |
|---|---|---|---|---|---|---|
| TRD006925 | 111.4% | 103.3% | 104.9% | 105.2% | 91.6% | 94 |
| TRD006971 | 102.1% | 101.0% | 99.5% | 89.8% | 92.6% | 66 |
| TRD006973 | 108.6% | 104.1% | 101.3% | 98.3% | 95.7% | ND |
| TRD006975 | 106.9% | 108.2% | 116.3% | 110.3% | 97.0% | ND |
| **Note: ND = undetectable.** | | | | | | |

**Example 28. Inhibition of Human ApoC3 in Hep3B Cells by siRNAs - 11 Concentration Point Inhibitory Activity**

Hep3B cell viability screening was performed on test compounds in Hep3B cells using 11 concentration gradients. Each siRNA sample for transfection was serially diluted 3-fold from the starting final concentration 20 nM to 11 concentration points.

Hep3B cells were cultured at 37 °C with 5% COz in a MEM medium containing 10% fetal bovine serum. 24 h prior to transfection, the Hep3B cells were inoculated into a 96-well plate at a density of 10 thousand cells per well. Each well contained 100 μL of medium.

The cells were transfected with siRNAs at final concentrations of 20 nM, 6.67 nM, 2.22 nM, 0.741 nM, 0.247 nM, 0.0823 nM, 0.0274 nM, 0.00914 nM, 0.00305 nM, 0.00102 nM and 0.000339 nM using Lipofectamine RNAi MAX (ThermoFisher, 13778150) according to the instructions of the product. 24 h after treatment, the total cellular RNA was extracted from the cells using a high-throughput cellular RNA extraction kit, and RNA reverse transcription and quantitative real-time PCR detection were carried out. The human ApoC3 mRNA level was measured and corrected based on the ACTIN internal reference gene level.

The results are expressed relative to the remaining percentage of human ApoC3 mRNA expression in cells treated with the control siRNA. The IC50 results of inhibition are shown in Table 58.

Table 58. Multi-dose inhibitory activity of siRNAs against human ApoC3 in Hep3B cells

| No. | 20nM | 6.67nM | 2.22nM | 0.741nM | 0.247nM | 0.0823nM |
|---|---|---|---|---|---|---|
| TRD006884 | 2% | 2.0% | 3.5% | 4.3% | 12.6% | 20.5% |
| TRD006885 | 3% | 5.1% | 9.7% | 13.5% | 26.9% | 41.5% |
| TRD006886 | 5% | 8.6% | 14.0% | 20.6% | 53.2% | 88.1% |
| TRD006887 | 4% | 10.9% | 9.6% | 25.5% | 45.5% | 82.2% |
| TRD006888 | 3% | 6.1% | 10.6% | 19.8% | 12.4% | 30.1% |
| TRD006925 | 7% | 2.1% | 7.2% | 12.0% | 12.9% | 33.1% |
| TRD006966 | 4% | 3.6% | 12.1% | 28.1% | 40.0% | 115.0% |
| TRD006927 | 3% | 5.5% | 5.1% | 7.0% | 13.9% | 33.9% |
| TRD006928 | 3% | 4.9% | 3.6% | 5.2% | 11.0% | 19.8% |
| TRD006936 | 4% | 5.8% | 6.8% | 24.7% | 22.8% | 58.2% |
| TRD006937 | 10% | 12.8% | 17.6% | 24.4% | 19.1% | 53.9% |
| TRD006964 | 15% | 21.2% | 28.6% | 20.2% | 16.4% | 54.4% |
| TRD006965 | 36% | 23.7% | 30.3% | 36.2% | 37.7% | 61.7% |
| TRD006971 | 6% | 3.5% | 2.9% | 2.5% | 5.1% | 16.1% |
| TRD006973 | 5% | 3.0% | 5.0% | 9.6% | 29.8% | 56.1% |
| TRD006975 | 5% | 2.1% | 3.9% | 7.4% | 16.7% | 53.5% |

(continued)

| No. | 0.0274nM | 0.00914nM | 0.00305nM | 0.00102nM | 0.000339nM | Hep3B cell IC50 value (nM) |
|---|---|---|---|---|---|---|
| TRD006884 | 39.8% | 92.8% | 97.5% | 102.3% | 106.3% | 0.024 |
| TRD006885 | 83.2% | 115.9% | 141.0% | 118.3% | 124.5% | 0.0692 |
| TRD006886 | 110.0% | 190.3% | 187.7% | 162.3% | 145.3% | 0.2089 |
| TRD006887 | 123.6% | 160.3% | 148.0% | 179.4% | 134.5% | 0.195 |
| TRD006888 | 53.1% | 69.0% | 94.0% | 100.5% | 73.5% | 0.0288 |
| TRD006925 | 53.7% | 84.5% | 94.6% | 94.1% | 118.4% | 0.0363 |
| TRD006966 | 187.9% | 159.1% | 130.2% | 101.0% | 118.8% | 0.2089 |
| TRD006927 | 65.7% | 107.2% | 121.5% | 140.9% | 106.7% | 0.0457 |
| TRD006928 | 42.4% | 72.6% | 99.9% | 120.3% | 102.4% | 0.0214 |
| TRD006936 | 88.2% | 136.7% | 106.2% | 96.9% | 111.7% | 0.1 |
| TRD006937 | 111.7% | 116.2% | 114.8% | 109.3% | 107.3% | 0.0871 |
| TRD006964 | 97.0% | 101.5% | 111.3% | 108.0% | 89.6% | 0.0933 |
| TRD006965 | 117.9% | 156.2% | 133.6% | 147.2% | 121.7% | 0.1096 |
| TRD006971 | 35.0% | 57.8% | 102.0% | 104.3% | 95.9% | 0.0148 |
| TRD006973 | 78.7% | 114.1% | 118.0% | 124.7% | 114.6% | 0.0955 |
| TRD006975 | 101.3% | 108.0% | 110.5% | 131.6% | 111.8% | 0.0912 |

**Example 29. Inhibition of Human ApoC3 in Primary Human Hepatocytes (PHHs) by siRNAs - 11 Concentration Point Inhibitory Activity**

Primary human hepatocyte (PHH) viability screening was performed on test compounds in primary human hepatocytes (PHHs) using 11 concentration gradients. Each siRNA sample for transfection was serially diluted 3-fold from the starting final concentration 20 nM to 11 concentration points.

The primary human hepatocytes (PHHs) were cryopreserved in liquid nitrogen. 24 h prior to transfection, the primary human hepatocytes (PHHs) were thawed and then inoculated into a 96-well plate at a density of 40 thousand cells per well. Each well contained 100 μL of medium.

The cells were transfected with siRNAs at gradient final concentrations of 20 nM, 6.67 nM, 2.22 nM, 0.741 nM, 0.247 nM, 0.0823 nM, 0.0274 nM, 0.00914 nM, 0.00305 nM, 0.00102 nM and 0.000339 nM using Lipofectamine RNAi MAX (ThermoFisher, 13778150) according to the instructions of the product. 24 h after treatment, the total cellular RNA was extracted from the cells using a high-throughput cellular RNA extraction kit, and RNA reverse transcription and quantitative real-time PCR detection were carried out. The human ApoC3 mRNA level was measured and corrected based on the ACTIN internal reference gene level.

The results are expressed relative to the remaining percentage of human ApoC3 mRNA expression in cells treated with the control siRNA. The IC50 results of inhibition are shown in Table 59. All could effectively inhibit human ApoC3 mRNA expression.

Table 59. Multi-dose inhibitory activity of siRNAs against human ApoC3 in primary human hepatocytes (PHHs)

| Double strand No. | 20nM | 6.67nM | 2.22nM | 0.741nM | 0.247nM | 0.0823nM |
|---|---|---|---|---|---|---|
| TRD006884 | 7% | 5.5% | 9.2% | 28.2% | 22.4% | 46.8% |
| TRD006885 | 6% | 10.2% | 11.5% | 11.1% | 23.3% | 30.3% |
| TRD006888 | 7% | 5.8% | 10.1% | 13.8% | 20.2% | 40.8% |
| TRD006925 | 5% | 6.1% | 9.2% | 13.3% | 13.5% | 26.2% |
| TRD006928 | 10% | 7.1% | 8.0% | 7.5% | 12.5% | 25.5% |

(continued)

| Double strand No. | 20nM | 6.67nM | 2.22nM | 0.741nM | 0.247nM | 0.0823nM |
|---|---|---|---|---|---|---|
| TRD006937 | 5% | 7.9% | 9.5% | 17.3% | 26.2% | 54.2% |
| TRD006886 | 6.1% | 9.2% | 10.3% | 14.1% | 22.0% | 39.9% |
| TRD006971 | 3.9% | 5.6% | 6.2% | 6.9% | 9.4% | 14.2% |
| TRD006964 | 5% | 6.1% | 17.0% | 17.6% | 28.2% | 63.7% |
| Double strand No. | 0.0274nM | 0.00914nM | 0.00305nM | 0.00102nM | 0.000339nM | Primary human hepatocyte IC50 value (nM) |
| TRD006884 | 80.2% | 97.8% | 105.7% | 112.9% | 85.3% | 0.0756 |
| TRD006885 | 64.5% | 85.2% | 115.5% | 98.5% | 112.2% | 0.0427 |
| TRD006888 | 66.9% | 99.8% | 95.3% | 107.6% | 106.1% | 0.0574 |
| TRD006925 | 86.9% | 84.2% | 107.3% | 89.9% | 80.3% | 0.0336 |
| TRD006928 | 44.8% | 86.5% | 107.2% | 107.7% | 111.6% | 0.0267 |
| TRD006937 | 84.7% | 114.7% | 110.1% | 118.6% | 107.4% | 0.0953 |
| TRD006886 | 78.2% | 100.0% | 115.3% | 111.1% | 115.2% | 0.0631 |
| TRD006971 | 19.6% | 42.8% | 69.5% | 95.3% | 105.3% | 0.0071 |
| TRD006964 | 81.3% | 113.8% | 112.6% | 90.9% | 97.1% | 0.1202 |

**Example 30. Inhibition of Monkey ApoC3 in Primary Monkey Hepatocytes by siRNAs - 11 Concentration Point Inhibitory Activity**

Primary monkey hepatocyte viability screening was performed on test compounds in primary monkey hepatocytes using 11 concentration gradients. Each siRNA sample for transfection was serially diluted 3-fold from the starting final concentration 20 nM to 11 concentration points.

The cells were transfected with siRNAs at gradient final concentrations of 20 nM, 6.67 nM, 2.22 nM, 0.741 nM, 0.247 nM, 0.0823 nM, 0.0274 nM, 0.00914 nM, 0.00305 nM, 0.00102 nM and 0.000339 nM using Lipofectamine RNAi MAX (ThermoFisher, 13778150) according to the instructions of the product. Treatment solutions with the above concentrations were prepared in advance and added to a 96-well plate. The primary monkey hepatocytes were cryopreserved in liquid nitrogen. The primary monkey hepatocytes were thawed and then inoculated into the 96-well plate (with siRNA samples in it) at a density of 30 thousand cells per well. Each well contained 100 μL of medium.

24 h after reverse transfection treatment, the culture media were changed, and the culture was continued for 24 h. Then the total cellular RNA was extracted from the cells using a high-throughput cellular RNA extraction kit, and RNA reverse transcription and quantitative real-time PCR detection were carried out. The monkey ApoC3 mRNA level was measured and corrected based on the GAPDH internal reference gene level.

The results are expressed relative to the remaining percentage of monkey ApoC3 mRNA expression in cells treated with the control siRNA. The IC50 results of inhibition are shown in Table 61. All could effectively inhibit monkey ApoC3 mRNA expression in primary monkey hepatocytes.

Table 60. Taqman probe primers (10 μM working concentration)

| Primer name | SEQ ID NO | Primer sequence |
|---|---|---|
| mkApoc3-PF | SEQ ID NO:439 | GCCTGCCTGCTCTGTTCATC |
| mkApoc3-PR | SEQ ID NO:440 | AAGCCAAGAAGGGAGGTGTCC |
| mkApoc3-P | SEQ ID NO:441 | 5`6-FAM-TTGTTGCTGCCGTGCTGTCACTCCTGG-3`BHQ1 |
| mkGAPDH-PF | SEQ ID NO:442 | TCAAGATCGTCAGCAACGCC |

(continued)

| Primer name | SEQ ID NO | Primer sequence |
|---|---|---|
| mkGAPDH-PR | SEQ ID NO:443 | ACAGTCTTCTGGGTGGCAGT |
| mkGAPDH-P | SEQ ID NO:444 | 5`TET-ACCAACTGCTTAGCACCCCTGGCCA-3`BHQ2 |

Table 61. Multi-dose inhibitory activity of siRNAs against monkey ApoC3 in primary monkey hepatocytes

| Compound No. | 20nM | 6.67nM | 2.22nM | 0.741nM | 0.247nM | 0.0823nM |
|---|---|---|---|---|---|---|
| TRD006884 | 12.8% | 12.3% | 15.5% | 27.7% | 50.6% | 76.5% |
| TRD006888 | 1.6% | 3.0% | 4.7% | 5.1% | 7.3% | 9.5% |
| TRD006886 | 5.7% | 3.7% | 10.6% | 7.3% | 16.1% | 17.0% |
| TRD006964 | 2.4% | 2.9% | 4.7% | 5.4% | 10.5% | 15.4% |
| TRD006971 | 1.4% | 1.7% | 2.3% | 3.2% | 3.6% | 5.7% |
| TRD006925 | 1.4% | 1.6% | 2.3% | 2.6% | 3.6% | 6.1% |
| TRD006885 | 1.5% | 3.6% | 5.1% | 4.1% | 6.1% | 7.7% |
| Compound No. | 0.0274nM | 0.00914nM | 0.00305nM | 0.00102nM | 0.000339nM | IC50 value (nM) |
| TRD006884 | 112.6% | 114.3% | 131.3% | 108.6% | 112.1% | 0.2291 |
| TRD006888 | 12.8% | 27.9% | 67.4% | 110.7% | 100.9% | 0.0051 |
| TRD006886 | 42.1% | 74.9% | 87.6% | 110.4% | 100.9% | 0.0204 |
| TRD006964 | 36.5% | 82.6% | 82.1% | 108.0% | 92.6% | 0.0214 |
| TRD006971 | 8.1% | 21.7% | 52.4% | 90.1% | 100.2% | 0.0036 |
| TRD006925 | 11.3% | 27.0% | 58.6% | 100.8% | 97.7% | 0.0045 |
| TRD006885 | 19.1% | 29.3% | 65.4% | 94.2% | 93.3% | 0.0052 |

**Example 31. *In Vivo* Testing of siRNA Agents in Apoc3 Transgenic Mice**

To assess and evaluate the *in vivo* effect of certain ApoC3 siRNA agents, ApoC3 transgenic mice (The Jackson Laboratory, 006907-B6; CBA-Tg(APOC3)3707Bres/J) were purchased and used. Experiments were carried out with the ApoC3 transgenic mice and the human ApoC3 protein, triglyceride and total cholesterol levels in serum were measured as recommended by the manufacturers of the kits (Roche Cobas C311: CHOL2 & TRIGL; MSD Human ApoC3 antibody set (B21ZV-3)).

For normalization, the ApoC3 protein, triglyceride and total cholesterol levels for each animal at a time point were divided by the pre-treatment level of expression in that animal to determine the ratio of expression "normalized to pre-dose".

The ApoC3 protein, triglyceride and total cholesterol levels can be measured at various times before and after administration of ApoC3 siRNA agents. Unless otherwise noted herein, blood samples were collected from the submandibular area into centrifuge tubes with heparin sodium in them. After the blood samples were well mixed with heparin sodium, the tubes were centrifuged at 3,000×g for 5 min to separate the serum and stored at 4° C.

The ApoC3 transgenic mouse model described above was used. On day 0, each mouse was given a single subcutaneous administration of 200 μL of the respective siRNA agent dissolved in PBS (1×) or control (PBS (1×)) (i.e., the Vehicle group), which included the administration groups shown in Table 62 below.

Table 62. Administration groups of ApoC3 transgenic mice

| No. | Group | Dose (mg/kg) | Route of administration |
|---|---|---|---|
| 1 | Vehicle | NA | s.c. |

(continued)

| No. | Group | Dose (mg/kg) | Route of administration |
|---|---|---|---|
| 2 | TRD006884 | 3 | s.c. |
| 3 | TRD006888 | 3 | s.c. |
| 4 | TRD006886 | 3 | s.c. |
| 5 | TRD006925 | 3 | s.c. |
| 6 | TRD006971 | 3 | s.c. |

The injections of ApoC3 siRNA agents were performed between the skin and muscle (i.e. subcutaneous injections). Six mice in each group were tested (n = 6). Serum was collected from the mice on day -2 (pre-dose blood collection with an overnight fast), and day 7, day 14, day 21, day 28, day 35 and day 42. Mice were fasted overnight prior to each collection. The ApoC3 protein, triglyceride and total cholesterol levels in serum were determined on an instrument according to the recommendations of the agent manufacturers.

The ApoC3 protein, triglyceride and total cholesterol levels of each animal were normalized. For normalization, the ApoC3 protein, triglyceride and total cholesterol levels for each animal at a time point were each divided by the pre-treatment level of expression in that animal (in that case, on day -2) to determine the ratio of expression "normalized to pre-treatment".

Data from the experiments are shown below in Table 63 to Table 65 and in FIGs. 7 to FIG. 9. Each of the ApoC3 siRNA agents in each of the administration groups (i.e., groups 2 to 6) showed significant reductions in the ApoC3 protein, triglyceride and total cholesterol levels as compared to the control (group 1).

Table 63. Average total cholesterol (TC) normalized to pre-treatment

| Group ID | Compound No. | D7 | | D14 | | D21 | | D28 | |
|---|---|---|---|---|---|---|---|---|---|
| | | Average TC | Standard deviation (+/-) | Average TC | Standard deviation (+/-) | Average TC | Standard deviation (+/-) | Average TC | Standard deviation (+/-) |
| 1 | Vehicle | 1.141 | 0.275 | 1.112 | 0.154 | 0.962 | 0.270 | 1.054 | 0.297 |
| 2 | TRD006884 | 0.413 | 0.156 | 0.467 | 0.176 | 0.448 | 0.141 | 0.552 | 0.175 |
| 3 | TRD006888 | 0.357 | 0.211 | 0.391 | 0.190 | 0.321 | 0.148 | 0.355 | 0.191 |
| 4 | TRD006886 | 0.274 | 0.135 | 0.331 | 0.184 | 0.519 | 0.219 | 0.718 | 0.309 |
| 5 | TRD006925 | 0.355 | 0.132 | 0.488 | 0.204 | 0.522 | 0.214 | 0.746 | 0.269 |
| 6 | TRD006971 | 0.359 | 0.153 | 0.380 | 0.165 | 0.344 | 0.147 | 0.333 | 0.129 |

Table 64. Average triglyceride (TG) normalized to pre-treatment

| Group ID | Compound No. | D7 | | D14 | | D21 | | D28 | |
|---|---|---|---|---|---|---|---|---|---|
| | | Average TG | Standard deviation (+/-) | Average TG | Standard deviation (+/-) | Average TG | Standard deviation (+/-) | Average TG | Standard deviation (+/-) |
| 1 | Vehicle | 0.992 | 0.574 | 0.770 | 0.289 | 0.805 | 0.324 | 0.910 | 0.456 |
| 2 | TRD006884 | 0.143 | 0.102 | 0.165 | 0.178 | 0.264 | 0.200 | 0.428 | 0.332 |
| 3 | TRD006888 | 0.103 | 0.080 | 0.147 | 0.129 | 0.113 | 0.069 | 0.168 | 0.131 |
| 4 | TRD006886 | 0.105 | 0.075 | 0.189 | 0.152 | 0.391 | 0.311 | 0.573 | 0.380 |
| 5 | TRD006925 | 0.121 | 0.061 | 0.241 | 0.149 | 0.279 | 0.134 | 0.627 | 0.208 |
| 6 | TRD006971 | 0.102 | 0.080 | 0.106 | 0.086 | 0.129 | 0.117 | 0.087 | 0.076 |

Table 65. Average ApoC3 protein normalized to pre-treatment

| Group ID | Compound No. | D7 | | D14 | | D21 | |
|---|---|---|---|---|---|---|---|
| | | Average Apoc3 | Standard deviation (+/-) | Average Apoc3 | Standard deviation (+/-) | Average Apoc3 | Standard deviation (+/-) |
| 1 | Vehicle | 0.702 | 0.454 | 0.560 | 0.159 | 0.751 | 0.245 |
| 2 | TRD006884 | 0.065 | 0.041 | 0.056 | 0.039 | 0.151 | 0.167 |
| 3 | TRD006888 | 0.049 | 0.037 | 0.027 | 0.017 | 0.086 | 0.094 |
| 4 | TRD006886 | 0.085 | 0.062 | 0.115 | 0.058 | 0.259 | 0.098 |
| 5 | TRD006925 | 0.081 | 0.036 | 0.124 | 0.084 | 0.225 | 0.093 |
| 6 | TRD006971 | 0.043 | 0.028 | 0.030 | 0.017 | 0.052 | 0.041 |

**Example 32. Evaluation of Different Modifications in Positions 9 and Position 10 of AS strand**

In this experiment, the *in vivo* inhibition efficiency of the siRNA conjugates of the present disclosure with 2'-fluoro modifications at different sites against the target gene's mRNA expression level was investigated.

6- to 8-week-old male C57BL/6 mice were randomized into groups of 6, 3 mice per time point, and each group of mice was given test conjugates (TRD007047 and TRD006870), a control conjugate (TRD002218) and PBS.

All the animals were dosed once by subcutaneous injection based on their body weight. The siRNA conjugates were administered at a dose of 1 mg/kg (calculated based on siRNA) in a volume of 5 mL/kg. The mice were sacrificed 7 days after administration, and their livers were collected and stored with RNA later (Sigma Aldrich). Then, the liver tissue was homogenized using a tissue homogenizer, and the total RNA was extracted from the liver tissue using a tissue RNA extraction kit (FireGen Biomedicals, FG0412) by following the procedure described in the instructions. The total RNA was reverse-transcribed into cDNA, and the TTR mRNA expression level in liver tissue was measured by real-time fluorescence quantitative PCR. In the fluorescence quantitative PCR method, the glyceraldehyde 3-phosphate dehydrogenase (GAPDH) gene was used as an internal reference gene, and the TTR and GAPDH mRNA expression levels are measured using Taqman probe primers for TTR and GAPDH, respectively.

The TTR mRNA expression level was calculated according to the equation below: TTR mRNA expression = [(TTR mRNA expression in test group / GAPDH mRNA expression in test group) / (TTR mRNA expression in control group / GAPDH mRNA expression in control group)] × 100%

The compounds are shown in Table 66, the test compound grouping in mice is shown in Table 67, and the sequences of detection primers are shown in Table 68.

Table 66. Compounds

| Compound No. | SEQ ID NO | SS strand | SEQ ID NO | AS strand |
|---|---|---|---|---|
| TRD002218 | SEQ ID NO:445 | CmsAmsGmUmGfUmUfCf UfUmGmCmUmCmUm AmUmAm Am-L96 | SEQ ID NO:446 | UmsUfsAmUmAmGfAmGm CmAmAmGmAmAfCmAfC mUmGmsUmsUm |
| TRD007047 | SEQ ID NO:447 | CmsAmsGmUmGfUmUfCf UfUmGmCmUmCmUm AmUmAms Ams-NAG1 | SEQ ID NO:448 | UmsUfsAmUfAmGf**(-)hmpNA(A)**GmCfAmAmGf AmAfCmAfCmUfGmsUms Um |
| TRD006870 | SEQ ID NO:449 | CmsAmsGmUmGfUmUfCf UfUmGmCmUmCmUm AmUmAms Ams-NAG1 | SEQ ID NO:450 | UmsUfsAmUfAmGf**(-)hmpNA(A)**GmCmAfAmGf AmAfCmAfCmUfGmsUms Um |

Table 67. Test compound grouping in mice

| Compound No. | Dose | mRNA quantification | Number of animals | Note |
|---|---|---|---|---|
| PBS | - | D7, 28 | 6 | 3 mice per time point |
| TRD002218 | 1mpk s.c. | D7, 28 | 6 | 3 mice per time point |
| TRD007047 | 1mpk s.c. | D7, 28 | 6 | 3 mice per time point |
| TRD006870 | 1mpk s.c. | D7, 28 | 6 | 3 mice per time point |

Table 68. Sequences of detection primers

| Primer name | SEQ ID NO | Forward primer |
|---|---|---|
| mTTR-F | SEQ ID NO:451 | GGGAAGACCGCGGAGTCT |
| mTTR-R | SEQ ID NO:452 | CAGTTCTACTCTGTACACTCCTTCTACAAA |
| mTTR-P | SEQ ID NO:453 | 5'6-FAM-CTGCACGGGCTCACCACAGATGA-3'BHQ1 |
| mGAPDH-F | SEQ ID NO:454 | CGGCAAATTCAACGGCACAG |
| mGAPDH-R | SEQ ID NO:455 | CCACGACATACTCAGCACCG |
| mGAPDH-P | SEQ ID NO:456 | 5'TET-ACCATCTTCCAGGAGCGAGACCCCACT-3'BHQ2 |

28 days after administration, the *in vivo* inhibition efficiency of the siRNA conjugates of the present disclosure with F modifications at different sites against the target gene's mRNA expression level was shown in Table 69. siRNA compounds with F modifications at different sites inhibited more TTR mRNA expression than the positive control compound TRD002218 on day 28 after administration. The 9F and 10F modifications both showed high inhibition efficiency and the inhibitory effects were not significantly different, which indicates that the 9F and 10F modifications can mediate higher siRNA inhibition efficiency.

Table 69. Experimental results

| | | 7 days | | 28 days | |
|---|---|---|---|---|---|
| Platform 9/10F | Compound No. | Remaining mRNA | SD | Remaining mRNA | SD |
| | PBS | 100% | 11% | 100% | 9% |
| PC | TRD002218 | 31% | 7% | 49% | 5% |
| mTTR 9F | TRD007047 | 15% | 5% | 39% | 10% |
| mTTR 10F | TRD006870 | 13% | 4% | 36% | 3% |

## Claims

1. An siRNA, comprising a sense strand and an antisense strand, wherein each of the strands has 15 to 35 nucleotides; the antisense strand comprises a chemical modification of formula (I) or a tautomer modification thereof in at least one of nucleotide positions 2 to 8 of the 5' region thereof:

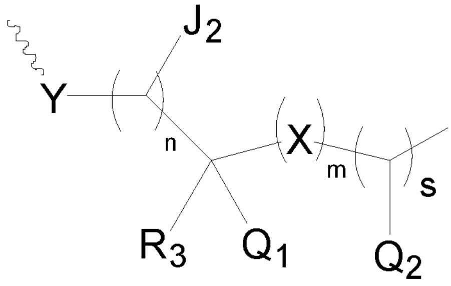

(I)

wherein Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
$J_2$ is H or $C_1$-$C_6$ alkyl;
n = 0, 1 or 2;
m = 0, 1 or 2;
s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$Q_1$ is

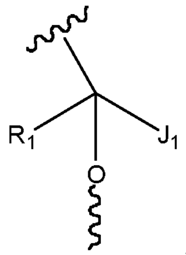

,

and $Q_2$ is $R_2$; or
$Q_1$ is $R_2$, and $Q_2$ is

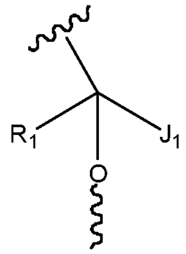

;

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
Ji is H or $C_1$-$C_6$ alkyl;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3; optionally, $R_1$ and $R_2$ are directly linked to form a ring;
B is a base;
wherein the chemical modification of formula (I) is not

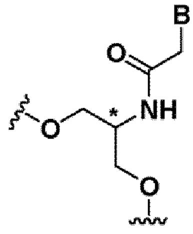
;

preferably, when X is NH-CO, $R_1$ is not H.

**2.** The siRNA according to claim 1, wherein the antisense strand comprises a chemical modification of formula (I-1) or a tautomer modification thereof in at least one of nucleotide positions 2 to 8 of the 5' region thereof:

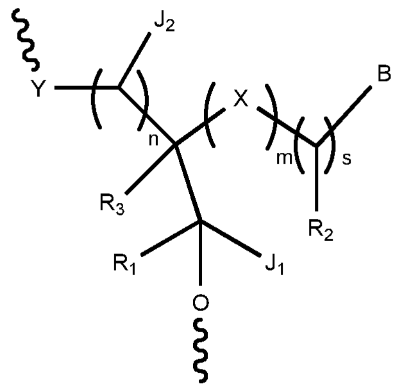
(formula I-1)

preferably,

Y is O or NH;
each X is independently selected from the group consisting of NH-CO, $CH_2$ and NH;
n = 0 or 1;
m = 0 or 1;
s = 0 or 1;
each Ji and each $J_2$ is independently H;
$R_1$ is selected from the group consisting of H, methyl and CHzOH;
$R_2$ is selected from the group consisting of H, OH, $NH_2$, methyl and CHzOH;
$R_3$ is selected from the group consisting of H, OH, $NH_2$, methyl and CHzOH;
optionally, $R_1$ and $R_2$ are directly linked to form a ring.

**3.** The siRNA according to claim 1, wherein the antisense strand comprises a chemical modification of formula (I-2) or a tautomer modification thereof in at least one of nucleotide positions 2 to 8 of the 5' region thereof:

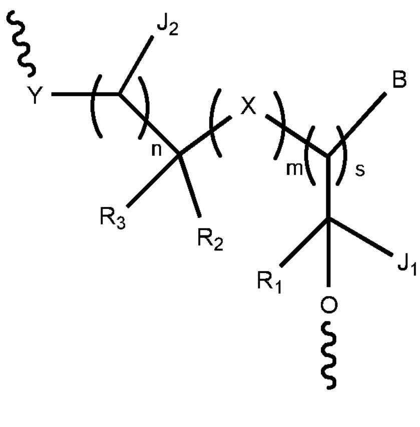
(I-2)

wherein

R$_1$ is selected from the group consisting of H, C$_1$-C$_6$ alkyl, C$_1$-C$_6$ alkoxy, C$_2$-C$_6$ alkenyl, C$_2$-C$_6$ alkynyl and (CH$_2$)$_q$R$_7$, wherein R$_7$ is selected from the group consisting of OH, halogen, methoxy, ethoxy, N$_3$, C$_2$-C$_6$ alkenyl and C$_2$-C$_6$ alkynyl, and q = 1, 2 or 3; R$_2$ is selected from the group consisting of H, C$_1$-C$_6$ alkyl, C$_1$-C$_6$ alkoxy, S-CH$_3$, NCH$_3$(CH$_3$), OCH$_2$CH$_2$OCH$_3$, -O-alkylamino and (CH$_2$)$_r$R$_8$, wherein R$_8$ is selected from the group consisting of OH, halogen, methoxy, ethoxy, N$_3$, C$_2$-C$_6$ alkenyl and C$_2$-C$_6$ alkynyl, and
r= 1,2 or 3;
optionally, R$_1$ and R$_2$ are directly linked to form a ring;
preferably,
Y is O or NH;
each X is independently selected from the group consisting of NH-CO, CH$_2$ and NH; n = 0 or 1;
m = 0 or 1;
s = 0 or 1;
each Ji and each J$_2$ is independently H;
R$_1$ is selected from the group consisting of H, methyl and CHzOH;
R$_2$ is selected from the group consisting of H, methyl and CHzOH;
R$_3$ is selected from the group consisting of H, OH, NH$_2$, methyl and CHzOH;
optionally, R$_1$ and R$_2$ are directly linked to form a ring.

**4.** The siRNA according to claim 1, wherein:

each X is independently selected from the group consisting of CR$_4$(R$_4$'), S, NR$_5$ and NH-CO, wherein R$_4$, R$_4$' and R$_5$ are each independently H, methyl, ethyl, n-propyl or isopropyl;
each Ji and each J$_2$ is independently H or methyl;
R$_3$ is selected from the group consisting of H, OH, F, Cl, NH$_2$, methyl, ethyl, n-propyl, isopropyl, methoxy, ethoxy, n-propoxy, isopropoxy, vinyl, allyl, ethynyl, propargyl, S-CH$_3$, NCH$_3$(CH$_3$), OCH$_2$CH$_2$OCH$_3$, -O-methylamino, -O-ethylamino and (CH$_2$)$_p$R$_6$, wherein R$_6$ is selected from the group consisting of OH, F, Cl, methoxy, ethoxy, N$_3$, vinyl, allyl, ethynyl and propargyl, and p = 1 or 2;
R$_1$ is selected from the group consisting of H, methyl, ethyl, n-propyl, isopropyl, methoxy, ethoxy, n-propoxy, isopropoxy, vinyl, allyl, ethynyl, propargyl and (CH$_2$)$_q$R$_7$, wherein R$_7$ is selected from the group consisting of OH, F, Cl, methoxy, ethoxy, N$_3$, vinyl, allyl, ethynyl and propargyl, and q = 1 or 2;
R$_2$ is selected from the group consisting of H, OH, F, Cl, NH$_2$, methyl, ethyl, n-propyl, isopropyl, methoxy, ethoxy, n-propoxy, isopropoxy, vinyl, allyl, ethynyl, propargyl, S-CH$_3$, NCH$_3$(CH$_3$), OCH$_2$CH$_2$OCH$_3$, -O-methylamino, -O-ethylamino and (CH$_2$)$_r$R$_8$, wherein R$_8$ is selected from the group consisting of OH, F, Cl, methoxy, ethoxy, N$_3$, vinyl, allyl, ethynyl, and propargyl, and r = 1 or 2;
optionally, R$_1$ and R$_2$ are directly linked to form a ring.

**5.** The siRNA according to any one of claims 1-4, wherein the chemical modification is selected from any one of the following structures:

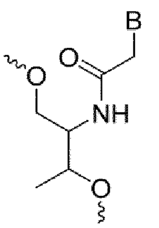 , 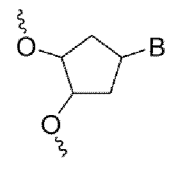 , 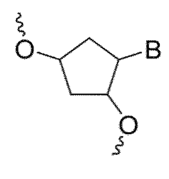 , 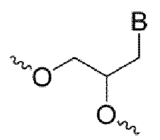 , 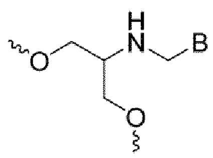 ,

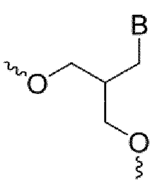 , 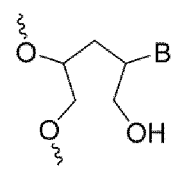 , 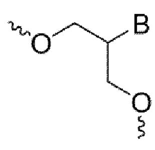 , 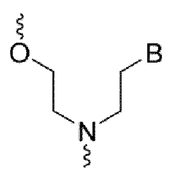 , 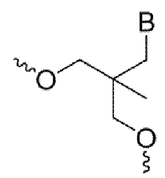 ,

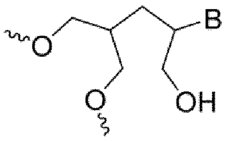 , 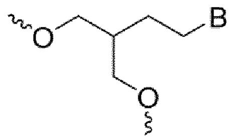 ,

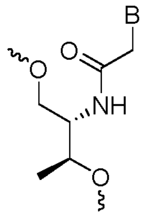 , 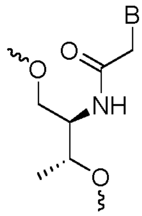 , 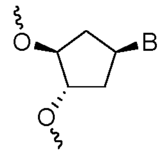 , 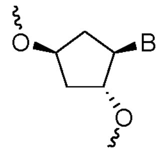 , 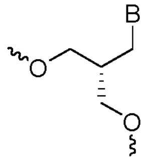 ,

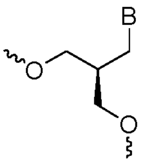 , 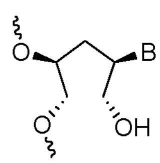 , 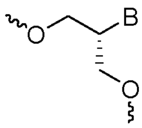 , 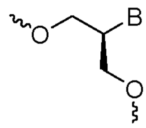 , 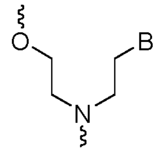 ,

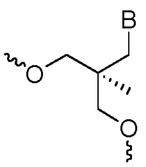 , 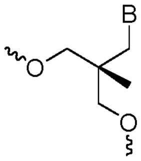 , 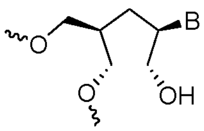 , 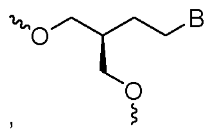 , 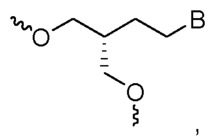 ,

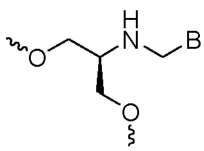 , 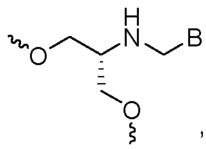 , 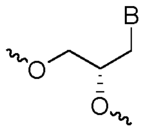 , 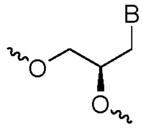 ,

preferably:

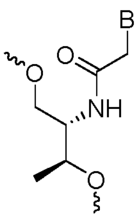 , 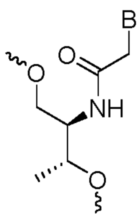 , 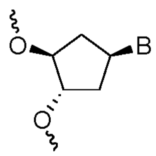 , 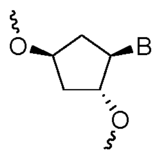 , 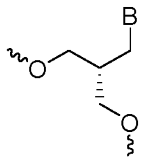 ,

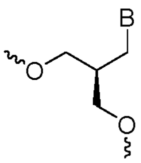 , 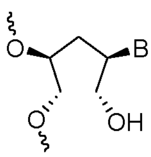 , 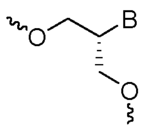 , 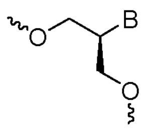 , 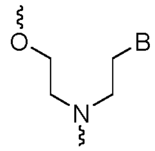 ,

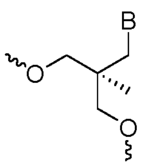 , 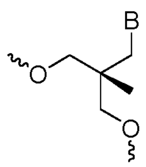

and more preferably:

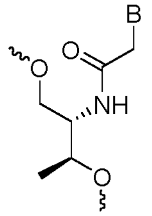 , 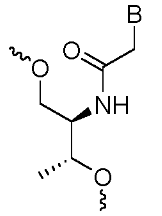 , 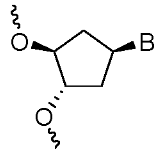 , 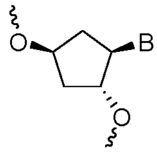 , 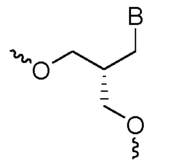 ,

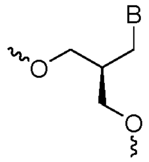

6. The siRNA according to any one of claims 1-5, wherein the antisense strand comprises the chemical modification of formula (I) or the tautomer modification thereof defined in any one of claims 1-5 at nucleotide position 5, 6 or 7, preferably 7, of the 5' region thereof.

7. The siRNA according to any one of claims 1-6, wherein in addition to the nucleotide modified by the chemical modification of formula (I) or the tautomer modification thereof defined in any one of claims 1-5, at least one otherwise modified nucleotide is also comprised in at least one of the sense strand and/or antisense strand;
preferably, in addition to the nucleotide modified by the chemical modification of formula (I) or the tautomer modification thereof defined in any one of claims 1-5, the other nucleotides in the sense strand and/or antisense strand are otherwise modified nucleotides.

8. The siRNA according to claim 7, wherein the otherwise modified nucleotides are each independently selected from the group consisting of a 2'-methoxy-modified nucleotide, a 2'-fluoro-modified nucleotide, and a 2'-deoxy-modified nucleotide.

9. The siRNA according to any one of claims 1-8, wherein:

in a 5'-end to 3'-end direction, nucleotides in positions 2, 6 and 14 of the antisense strand are each independently a 2'-fluoro-modified nucleotide;
preferably, in a 5'-end to 3'-end direction, nucleotides in positions 2, 6, 14 and 16 of the antisense strand are each independently a 2'-fluoro-modified nucleotide;
preferably, in a 5'-end to 3'-end direction, nucleotides in positions 2, 6, 12 and 14 of the antisense strand are each independently a 2'-fluoro-modified nucleotide;
more preferably, in a 5'-end to 3'-end direction, nucleotides in positions 2, 4, 6, 12, 14, 16 and 18 of the antisense strand are each independently a 2'-fluoro-modified nucleotide;
still more preferably, in a 5'-end to 3'-end direction, nucleotides in positions 2, 4, 6, 9, 12, 14, 16 and 18 of the antisense strand are each independently a 2'-fluoro-modified nucleotide;
still more preferably, in a 5'-end to 3'-end direction, nucleotides in positions 2, 4, 6, 10, 12, 14, 16 and 18 of the antisense strand are each independently a 2'-fluoro-modified nucleotide.

10. The siRNA according to any one of claims 1-9, wherein:

the sense strand has a nucleotide sequence of the formula shown below:
$5'\text{-}N_aN_aN_aN_aXN_aN_bN_bN_bN_aN_aN_aN_aN_aN_aN_aN_aN_aN_a\text{-}3'$;
wherein each X is independently $N_a$ or $N_b$; each $N_a$ and each $N_b$ independently represents a modified nucleotide or an unmodified nucleotide, and modifications on $N_a$ and $N_b$ are different; and/or,
the antisense strand has a nucleotide sequence of the formula shown below:
$5'\text{-}N_a'N_b'N_a'X'N_a'N_b'W'N_a'X'Y'N_a'X'N_a'N_b'N_a'X'N_a'X'N_a'N_a'N_a'\text{-}3'$;
wherein each X' is independently $N_a'$ or $N_b'$, and Y' is $N_a'$or $N_b'$; each $N_a'$ and each $N_b'$ independently represents a modified nucleotide or an unmodified nucleotide, wherein modifications on $N_a'$ and $N_b'$ are different; W' represents a nucleotide comprising the chemical modification of formula (I) or the tautomer modification thereof

defined in any one of claims 1-6;
preferably,
$N_a$ is a 2'-methoxy-modified nucleotide, and $N_b$ is a 2'-fluoro-modified nucleotide; and/or
$N_a'$ is a 2'-methoxy-modified nucleotide, and $N_b'$ is a 2'-fluoro-modified nucleotide.

**11.** The siRNA according to claim 10, wherein the antisense strand has a nucleotide sequence of the formula shown below:
5'-$N_a'N_b'N_a'N_b'N_a'N_b'W'N_a'X'Y'N_a'N_b'N_a'N_b'N_a'N_b'N_a'N_b'N_a'N_a'N_a'$-3';

wherein each X' is independently $N_a'$ or $N_b'$, and Y' is $N_a'$ or $N_b'$;
$N_a'$ is a 2'-methoxy-modified nucleotide;
$N_b'$ is a 2'-fluoro-modified nucleotide;
W' is as defined in claim 10.

**12.** The siRNA according to any one of claims 1-11, wherein the sense strand has a nucleotide sequence of the formula shown below:

5'-$N_aN_aN_aN_aN_aN_aN_bN_bN_bN_aN_aN_aN_aN_aN_aN_aN_aN_aN_a$-3'; or,
5'-$N_aN_aN_aN_aN_bN_aN_bN_bN_bN_aN_aN_aN_aN_aN_aN_aN_aN_aN_a$-3';
wherein $N_a$ is a 2'-methoxy-modified nucleotide, and $N_b$ is a 2'-fluoro-modified nucleotide.

**13.** The siRNA according to any one of claims 1-12, wherein the antisense strand has a nucleotide sequence of the formula shown below:

5'-$N_a'N_b'N_a'N_b'N_a'N_b'W'N_a'N_a'N_b'N_a'N_b'N_a'N_b'N_a'N_b'N_a'N_b'N_a'N_a'N_a'$-3'; or,
5'-$N_a'N_b'N_a'N_b'N_a'N_b'W'N_a'N_b'N_a'N_a'N_b'N_a'N_b'N_a'N_b'N_a'N_b'N_a'N_a'N_a'$-3';
wherein,
$N_a$ is a 2'-methoxy-modified nucleotide, and $N_b$ is a 2'-fluoro-modified nucleotide; and/or
$N_a'$ is a 2'-methoxy-modified nucleotide, and $N_b'$ is a 2'-fluoro-modified nucleotide;
W' is defined according to claim 10;
preferably, W' represents a nucleotide comprising a chemical modification selected from the group consisting of the following structures or a tautomer modification thereof;

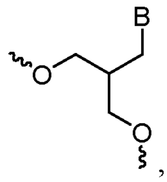 , 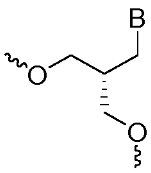 or 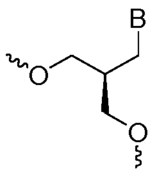 ;

wherein B is selected from the group consisting of guanine, adenine, cytosine and uracil;
preferably, B is a base corresponding to position 7 of the 5' region of the antisense strand.

**14.** The siRNA according to any one of claims 1-13, wherein at least one phosphoester group in the sense strand and/or the antisense strand is a phosphoester group with a modification group, preferably a phosphorothioate group.

**15.** The siRNA according to claim 14, wherein the phosphorothioate group is present in at least one of the positions selected from the group consisting of:

a position between the 1st and 2nd nucleotides of the 5' end of the sense strand;
a position between the 2nd and 3rd nucleotides of the 5' end of the sense strand;
an end of the 1st nucleotide of the 3' end of the sense strand;
a position between the 1st and 2nd nucleotides of the 3' end of the sense strand;
a position between the 2nd and 3rd nucleotides of the 3' end of the sense strand;
a position between the 1st and 2nd nucleotides of the 5' end of the antisense strand;
a position between the 2nd and 3rd nucleotides of the 5' end of the antisense strand;
an end of the 1st nucleotide of the 3' end of the antisense strand;
a position between the 1st and 2nd nucleotides of the 3' end of the antisense strand; and

a position between the 2nd and 3rd nucleotides of the 3' end of the antisense strand.

**16.** An siRNA conjugate, comprising:

the siRNA according to any one of claims 1-15; and
a conjugated group linked to the siRNA;
preferably, the conjugated group comprises a pharmaceutically acceptable targeting ligand and optionally a linker, and
the siRNA, the linker and the targeting ligand are sequentially linked covalently or non-covalently;
more preferably, the targeting ligand targets the liver;
still more preferably, the targeting ligand binds to an asialoglycoprotein receptor;
even more preferably, the targeting ligand includes a galactose cluster or a galactose derivative cluster, wherein the galactose derivative is selected from the group consisting of N-acetyl-galactosamine, N-trifluoroacetyl-galactosamine, N-propionyl-galactosamine, N-n-butyryl-galactosamine and N-isobutyrylgalactosamine.

**17.** A pharmaceutical composition, comprising:

the siRNA according to any one of claims 1-15; or
the siRNA conjugate according to claim 16.

**18.** A method for inhibiting expression of a target gene or mRNA thereof, comprising:
administering to a subject in need an effective amount or effective dose of the siRNA according to any one of claims 1-15, the siRNA conjugate according to claim 16 and/or the pharmaceutical composition according to claim 17.

**19.** A compound of formula (II) or a tautomer thereof,

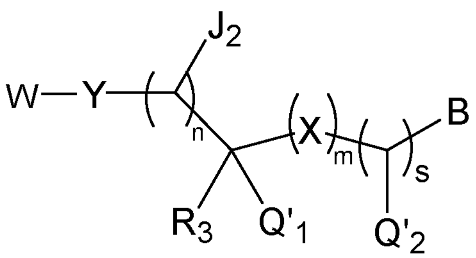

(II)

wherein:

Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
$J_2$ is H or $C_1$-$C_6$ alkyl;
n = 0, 1 or 2;
m = 0, 1 or 2;
s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$Q'_1$ is

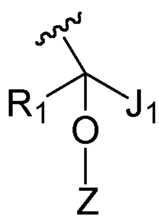

,

and $Q_2$ is $R_2$; or
$Q_1$ is $R_2$, and $Q'_2$ is

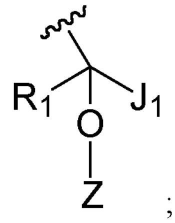

;

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
Ji is H or $C_1$-$C_6$ alkyl;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is a base; and
W is a leaving group; preferably, W is MMTr or DMTr;
Z is a phosphorus-containing active reaction group;
preferably, Z is

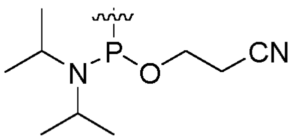

;

wherein the compound of formula (II) is not

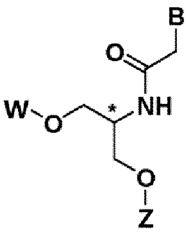

,

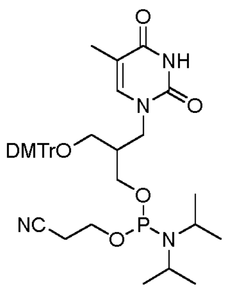

or

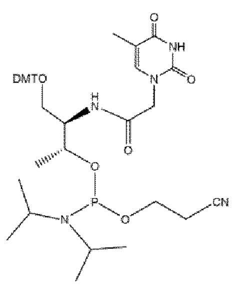

.

**20.** The compound of formula (II) or the tautomer thereof according to claim 19, when X is NH-CO, $R_1$ is not H; and the compound is not:

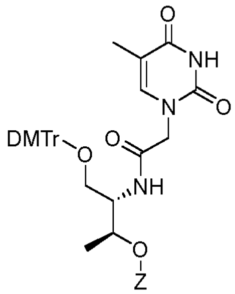
or
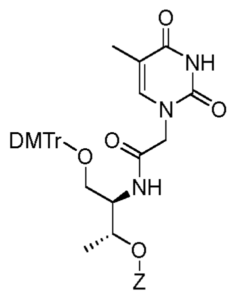
,

wherein Z is as defined in claim 19.

**21.** The compound of formula (II) or the tautomer thereof according to claim 19 or 20, being a compound of formula (II-1) or a tautomer thereof,

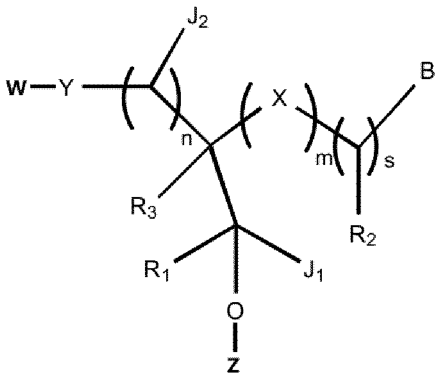

(II-1)

wherein:
W, Y, X, Z, B, Ji, $J_2$, n, m, s, $R_1$, $R_2$ and $R_3$ are as defined in claim 19.

**22.** The compound of formula (II) or the tautomer thereof according to claim 19 or 20, being a compound of formula (II-2) or a tautomer thereof,

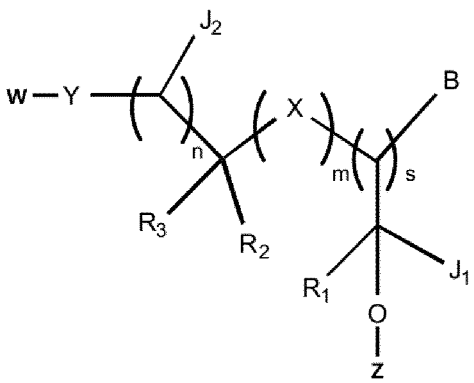

(II-2)

wherein $R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;

W, Y, X, Z, B, Ji, $J_2$, n, m, s and $R_3$ are as defined in claim 19.

**23.** The compound of formula (II) or the tautomer thereof according to claim 19 or 21, wherein:

each X is independently selected from the group consisting of $CR_4(R_4')$, S, $NR_5$ and NH-CO, wherein $R_4$, $R_4'$ and $R_5$ are each independently H or $C_1$-$C_3$ alkyl;
each Ji and each $J_2$ is independently H or $C_1$-$C_3$ alkyl;
$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_3$ alkyl, $C_1$-$C_3$ alkoxy, $C_2$-$C_4$ alkenyl, $C_2$-$C_4$ alkynyl and $(CH_2)_qR_7$, wherein $R_7$ is selected from the group consisting of OH, halogen, methoxy, ethoxy, $N_3$, $C_2$-$C_4$ alkenyl and $C_2$-$C_4$ alkynyl, and q = 1, 2 or 3;
$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_4$ alkenyl and $C_2$-$C_4$ alkynyl, and r = 1, 2 or 3;
optionally, $R_1$ and $R_2$ are directly linked to form a ring.

**24.** The compound of formula (II) or the tautomer thereof according to claim 19 or 21, wherein:

each X is independently selected from the group consisting of $CR_4(R_4')$, S, $NR_5$ and NH-CO, wherein $R_4$, $R_4'$ and $R_5$ are each independently H, methyl, ethyl, n-propyl or isopropyl;
each Ji and each $J_2$ is independently H or methyl;
$R_3$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino and $(CH_2)_pR_6$, wherein $R_6$ is selected from the group consisting of OH, F, Cl, methoxy, ethoxy, $N_3$, vinyl, allyl, ethynyl and propargyl, and p = 1 or 2;
$R_1$ is selected from the group consisting of H, methyl, ethyl, n-propyl, isopropyl, methoxy, ethoxy, n-propoxy, isopropoxy, vinyl, allyl, ethynyl, propargyl and $(CH_2)_qR_7$, wherein $R_7$ is selected from the group consisting of OH, F, Cl, methoxy, ethoxy, $N_3$, vinyl, allyl, ethynyl and propargyl, and q = 1 or 2;
$R_2$ is selected from the group consisting of H, OH, F, Cl, $NH_2$, methyl, ethyl, n-propyl, isopropyl, methoxy, ethoxy, n-propoxy, isopropoxy, vinyl, allyl, ethynyl, propargyl, S-$CH_3$, $NCH_3(CH_3)$, $OCH_2CH_2OCH_3$, -O-methylamino, -O-ethylamino and $(CH_2)_rR_8$, wherein $R_8$ is selected from the group consisting of OH, F, Cl, methoxy, ethoxy, $N_3$, vinyl, allyl, ethynyl, and propargyl, and r = 1 or 2;
optionally, $R_1$ and $R_2$ are directly linked to form a ring.

**25.** The compound of formula (II) or the tautomer thereof according to claim 21, wherein Y is O or NH; each X is independently selected from the group consisting of NH-CO, $CH_2$ and NH;

n = 0 or1; m = 0 or1; s = 0 or 1;
each Ji and each $J_2$ is independently H;
$R_1$ is selected from the group consisting of H, methyl and CHzOH;
$R_2$ is selected from the group consisting of H, OH, $NH_2$, methyl and CHzOH;
$R_3$ is selected from the group consisting of H, OH, $NH_2$, methyl and CHzOH;
optionally, $R_1$ and $R_2$ are directly linked to form a ring.

**26.** The compound of formula (II) or the tautomer thereof according to claim 22, wherein Y is O or NH; each X is independently selected from the group consisting of NH-CO, $CH_2$ and NH;

n = 0 or1; m = 0 or1; s = 0 or 1;
each Ji and each $J_2$ is independently H;
$R_1$ is selected from the group consisting of H, methyl and CHzOH;
$R_2$ is selected from the group consisting of H, methyl and CHzOH;
$R_3$ is selected from the group consisting of H, OH, $NH_2$, methyl and CHzOH;
optionally, $R_1$ and $R_2$ are directly linked to form a ring.

**27.** The compound of formula (II) or the tautomer thereof according to any one of claims 19-26, being selected from the group consisting of:

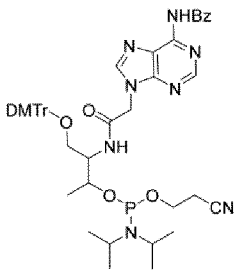 , 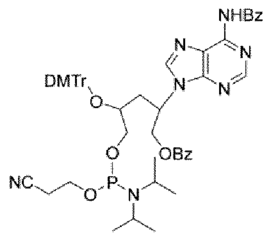 , 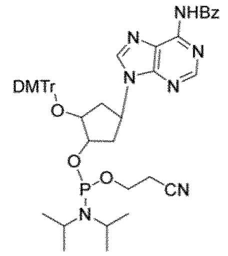 , 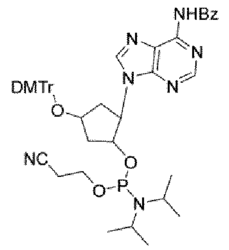 ,

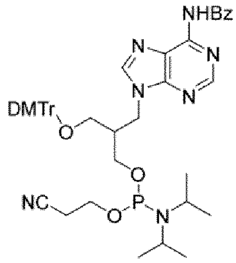

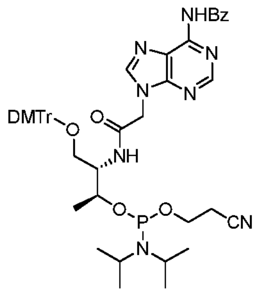 , 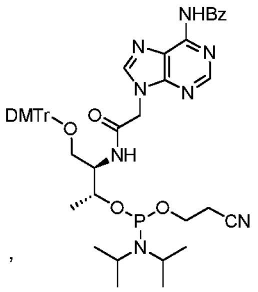 , 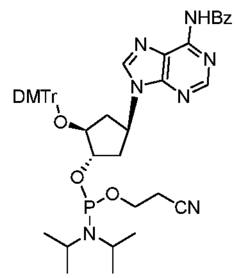 , 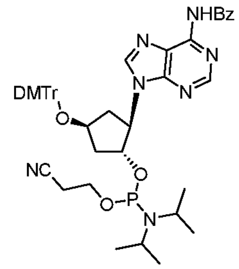 ,

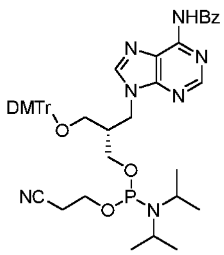 , 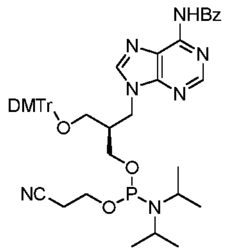 , 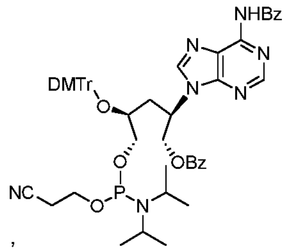 ;

and those where adenine is replaced with guanine, cytosine, uracil or thymine.

**28.** A compound of formula (III) or a tautomer thereof,

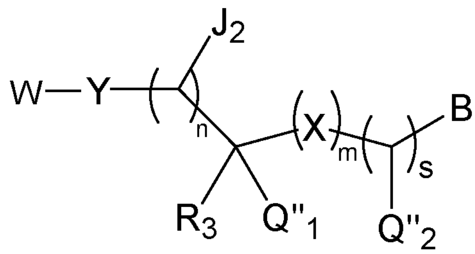 (III)

wherein:

Y is selected from the group consisting of O, NH and S;
each X is independently selected from the group consisting of $CR_4(R_4')$, S, $NR_5$ and NH-CO, wherein $R_4$, $R_4'$ and $R_5$ are each independently H or $C_1$-$C_6$ alkyl;
$J_2$ is H or $C_1$-$C_6$ alkyl;
n = 0, 1 or 2;
m = 0, 1 or 2;
s = 0 or 1;
$R_3$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$Q''_1$ is

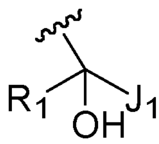,

and $Q_2$ is $R_2$; or
$Q_1$ is $R_2$, and $Q''_2$ is

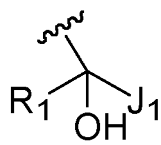;

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
Ji is H or $C_1$-$C_6$ alkyl;
$R_2$ is selected from the group consisting of H, OH, halogen, $NH_2$, $C_1$-$C_6$ alkyl, $C_1$-$C_6$ alkoxy, $C_2$-$C_6$ alkenyl, $C_2$-$C_6$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
B is a base; and
W is a leaving group; preferably, W is MMTr or DMTr;
wherein the compound is not:

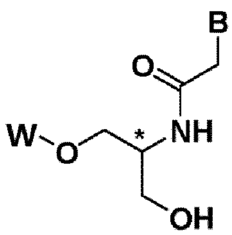,

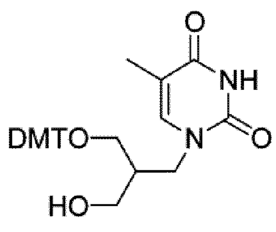,

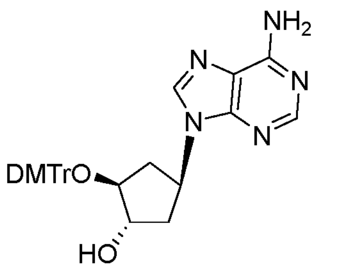,

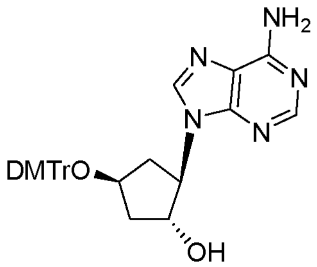

or

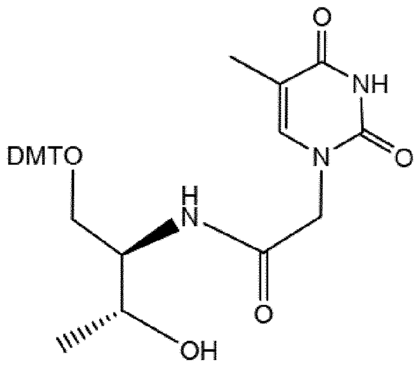

;

preferably, when X is NH-CO, $R_1$ is not H.

**29.** The compound of formula (III) or the tautomer thereof according to claim 28, being a compound of formula (III-1) or a tautomer thereof,

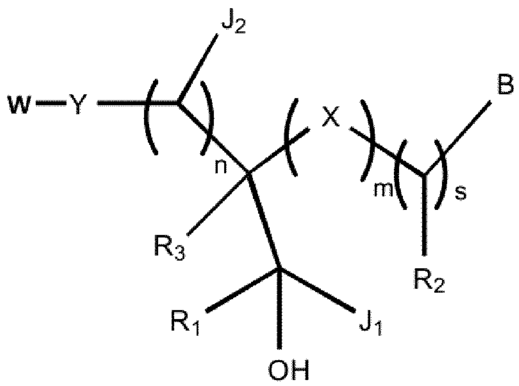

(III-1)

wherein preferably,

Y is O or NH; each X is independently selected from the group consisting of NH-CO, $CH_2$ and NH;
n = 0 or1; m = 0 or1; s = 0 or 1;
each Ji and each $J_2$ is independently H;
$R_1$ is selected from the group consisting of H, methyl and CHzOH;
$R_2$ is selected from the group consisting of H, OH, $NH_2$, methyl and CHzOH;
$R_3$ is selected from the group consisting of H, OH, $NH_2$, methyl and CHzOH;
optionally, $R_1$ and $R_2$ are directly linked to form a ring.

**30.** The compound of formula (III) or the tautomer thereof according to claim 28, being a compound of formula (111-2) or a tautomer thereof,

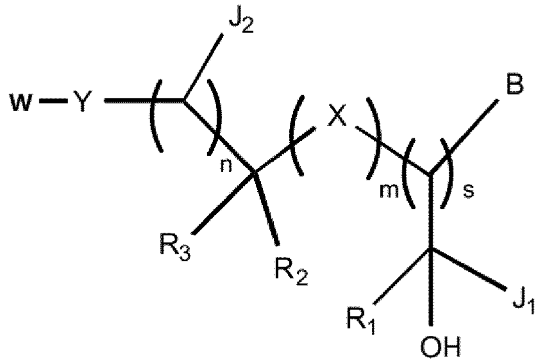

(III-2)

wherein:

$R_1$ is selected from the group consisting of H, $C_1$-$C_6$ alkyl, $C_1$-$C_6$ alkoxy, $C_2$-$C_6$ alkenyl, $C_2$-$C_6$ alkynyl and $(CH_2)_qR_7$, wherein $R_7$ is selected from the group consisting of OH, halogen, methoxy, ethoxy, $N_3$, $C_2$-$C_6$ alkenyl and $C_2$-$C_6$ alkynyl, and q = 1, 2 or 3;
$R_2$ is selected from the group consisting of H, $C_1$-$C_6$ alkyl, $C_1$-$C_6$ alkoxy, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_6$ alkenyl and $C_2$-$C_6$ alkynyl, and r = 1, 2 or 3;
optionally, $R_1$ and $R_2$ are directly linked to form a ring;
preferably,
Y is O or NH; each X is independently selected from the group consisting of NH-CO, $CH_2$ and NH;
n = 0 or1; m = 0 or1; s = 0 or 1;
each Ji and each $J_2$ is independently H;
$R_1$ is selected from the group consisting of H, methyl and CHzOH;
$R_2$ is selected from the group consisting of H, methyl and CHzOH;
$R_3$ is selected from the group consisting of H, OH, $NH_2$, methyl and CHzOH;
optionally, $R_1$ and $R_2$ are directly linked to form a ring.

**31.** The compound of formula (III) or the tautomer thereof according to claim 28, wherein:

each X is independently selected from the group consisting of $CR_4(R_4')$, S, $NR_5$ and NH-CO, wherein $R_4$, $R_4'$ and $R_5$ are each independently H or $C_1$-$C_3$ alkyl;
each Ji and each $J_2$ is independently H or $C_1$-$C_3$ alkyl;
$R_3$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_pR_6$, wherein $R_6$ is selected from the group consisting of OH, halogen, methoxy, ethoxy, $N_3$, $C_2$-$C_6$ alkenyl and $C_2$-$C_6$ alkynyl, and p = 1, 2 or 3;
$R_1$ is selected from the group consisting of H, $C_1$-$C_3$ alkyl, $C_1$-$C_3$ alkoxy, $C_2$-$C_4$ alkenyl, $C_2$-$C_4$ alkynyl and $(CH_2)_qR_7$, wherein $R_7$ is selected from the group consisting of OH, halogen, methoxy, ethoxy, $N_3$, $C_2$-$C_4$ alkenyl and $C_2$-$C_4$ alkynyl, and q = 1, 2 or 3;
$R_2$ is selected from the group consisting of H, OH, halogen, $NH_2$, $C_1$-$C_3$ alkyl, $C_1$-$C_3$ alkoxy, $C_2$-$C_4$ alkenyl, $C_2$-$C_4$ alkynyl, S-$CH_3$, $NCH_3(CH_3)$, $OCH_2CH_2OCH_3$, -O-alkylamino and $(CH_2)_rR_8$, wherein $R_8$ is selected from the group consisting of OH, halogen, methoxy, ethoxy, $N_3$, $C_2$-$C_4$ alkenyl and $C_2$-$C_4$ alkynyl, and r = 1, 2 or 3;
optionally, $R_1$ and $R_2$ are directly linked to form a ring.

**32.** The compound of formula (III) or the tautomer thereof according to any one of claims 29-31, being selected from any one of the following structures:

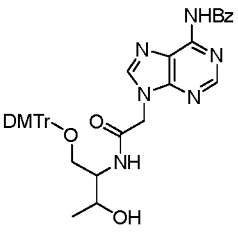,
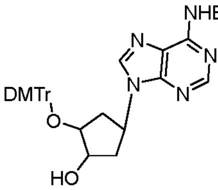,
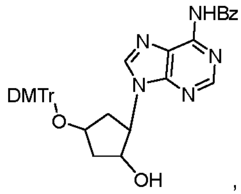,
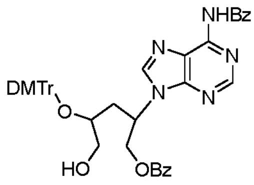

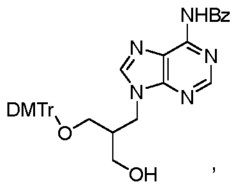,

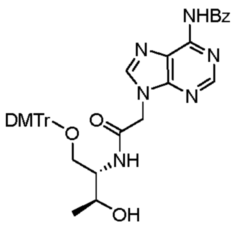,
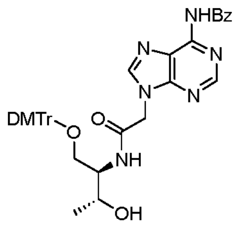,
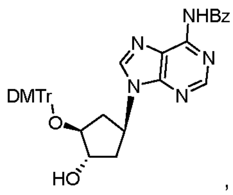,
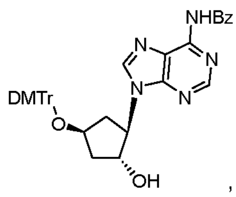,

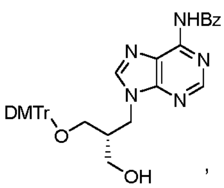,
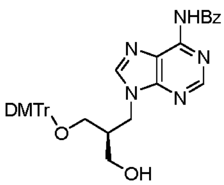,
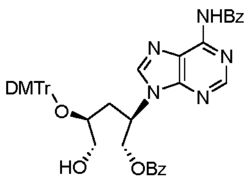

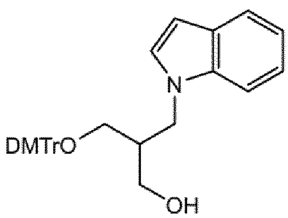,
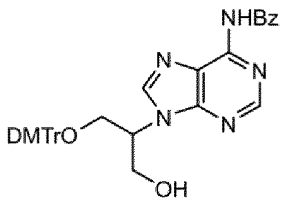,
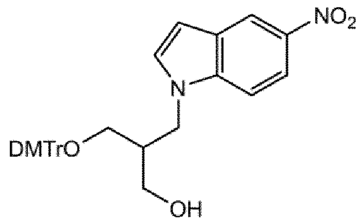,

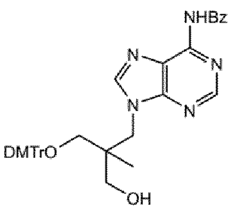,
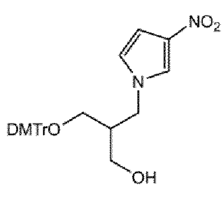,
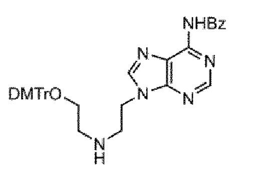,

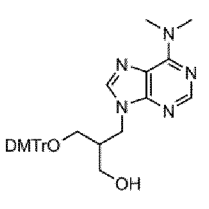,
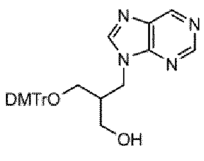,
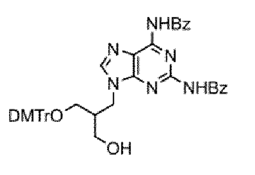,

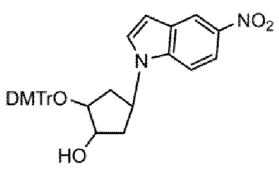 , 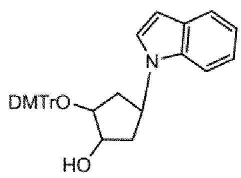 , 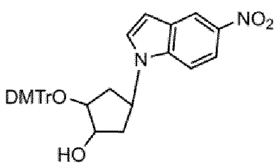 ,

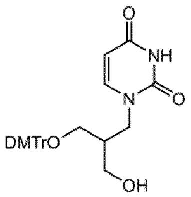 , 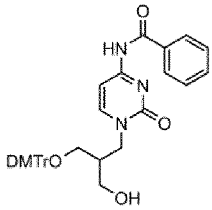 , 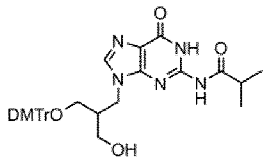 ,

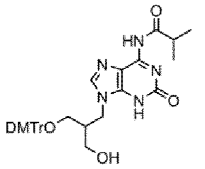 , 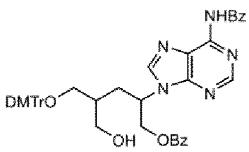 , 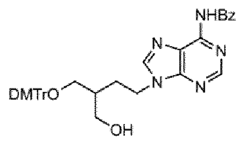 ,

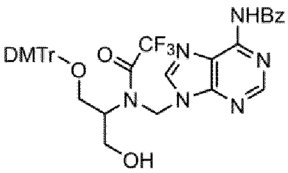 , 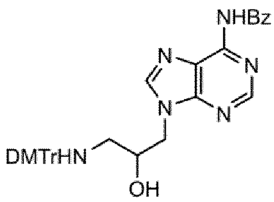 , 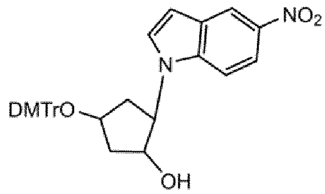

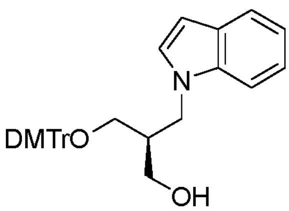 , 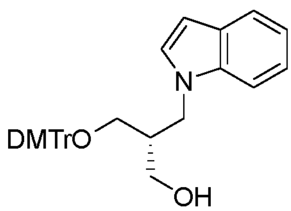 , 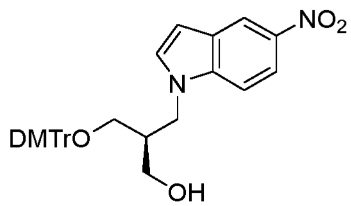 ,

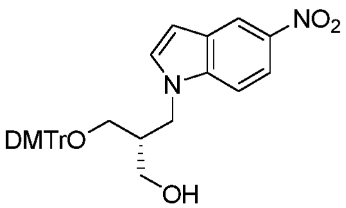 , 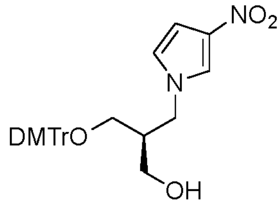 , 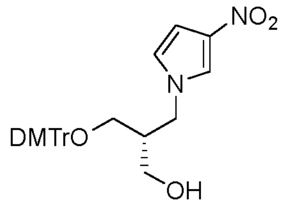 ,

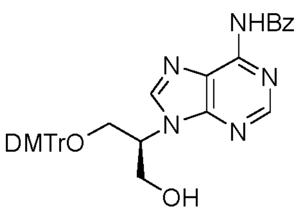 , 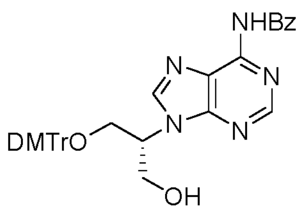 , 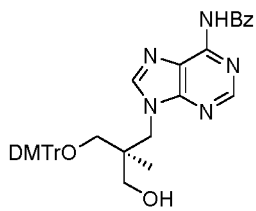 ,

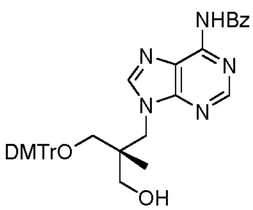,
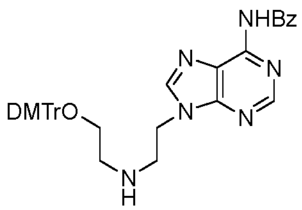,
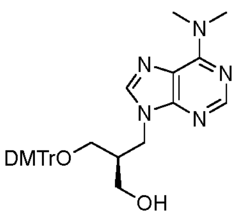,
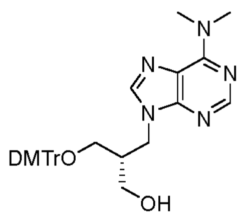,

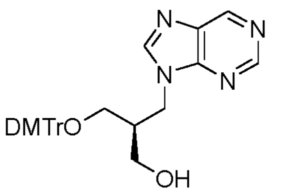,
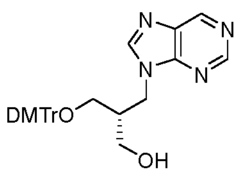,
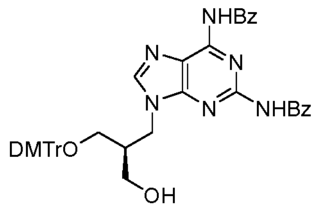,

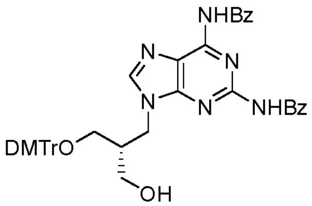,
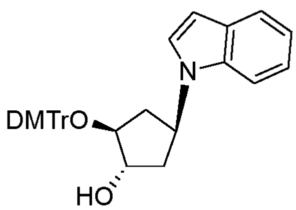,
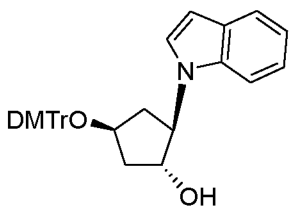,

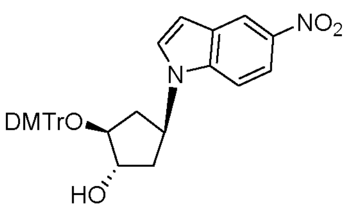,
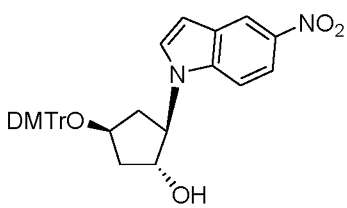,
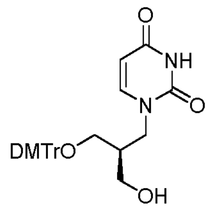,

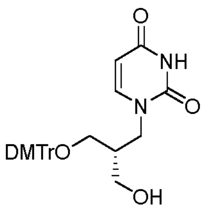,
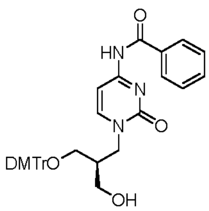,
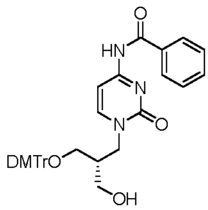,
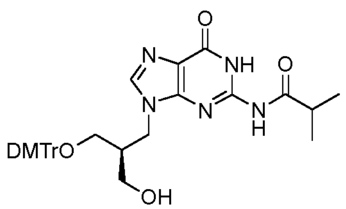,

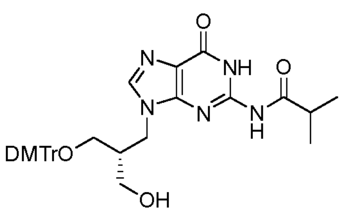,
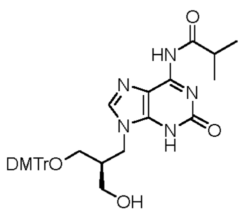,
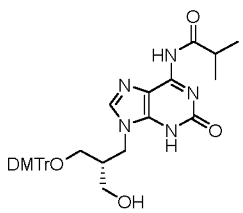,

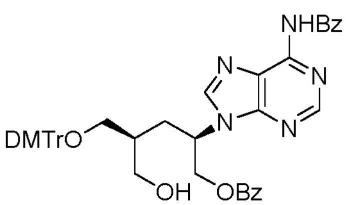,
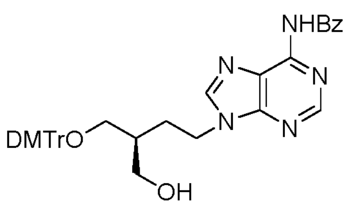,
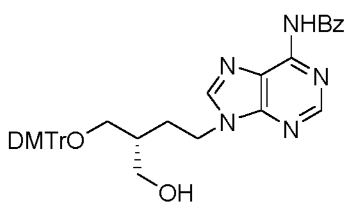,

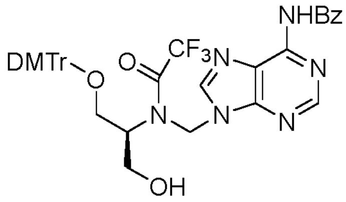,
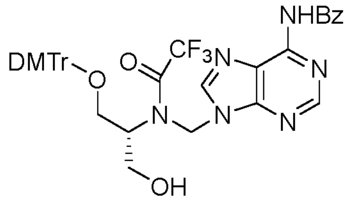,

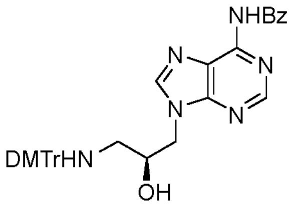
and
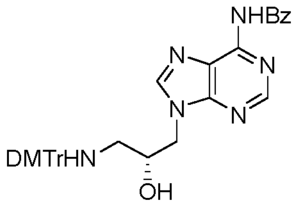,

and those where bases are replaced with adenine, guanine, 2,6-diaminopurine, 6-dimethylaminopurine, 2-aminopurine, cytosine, uracil, thymine, indole, 5-nitroindole or 3-nitropyrrole;

preferably being selected from the group consisting of:

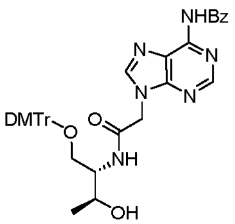,
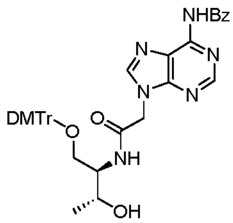,
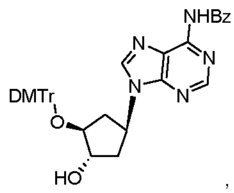,
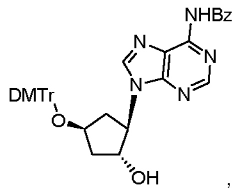,
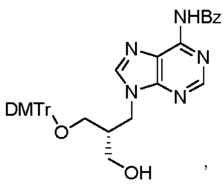,
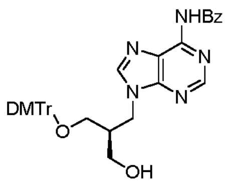,
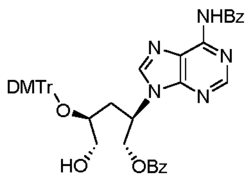,

and those compounds where adenine is replaced with guanine, cytosine, uracil or thymine.

**33.** A method for preparing the siRNA according to any one of claims 1-15 or the siRNA conjugate according to claim 16, comprising the following steps:

1) synthesizing the compound of formula (II) or the tautomer thereof according to any one of claims 19-27; and
2) synthesizing the siRNA or siRNA conjugate using the compound or the tautomer thereof of step 1);

wherein preferably, in step 1), the compound of formula (II) or the tautomer thereof is synthesized using the compound of formula (III) or the tautomer thereof according to any one of claims 28-32.

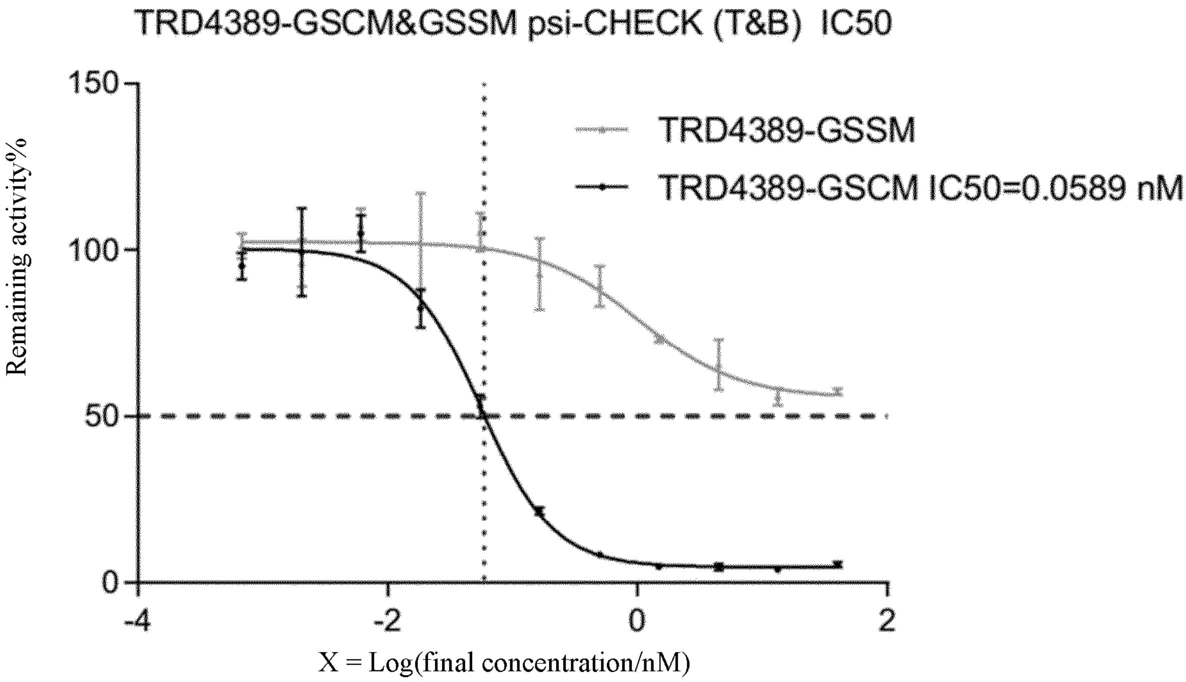
TRD4389-GSCM&GSSM psi-CHECK (T&B) IC50
150
100
50
0
-4
-2
0
2
Remaining activity%
X = Log(final concentration/nM)
TRD4389-GSSM
TRD4389-GSCM IC50=0.0589 nM

**FIG. 1A**

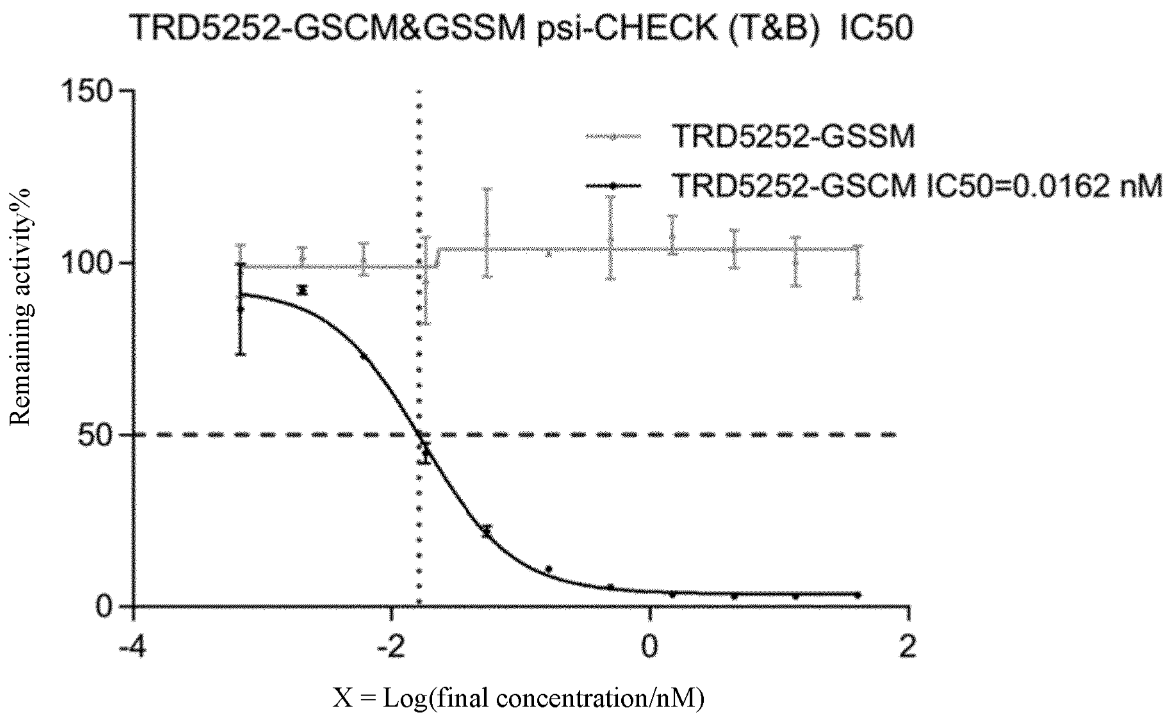
TRD5252-GSCM&GSSM psi-CHECK (T&B) IC50
150
100
50
0
-4
-2
0
2
Remaining activity%
X = Log(final concentration/nM)
TRD5252-GSSM
TRD5252-GSCM IC50=0.0162 nM

**FIG. 1B**

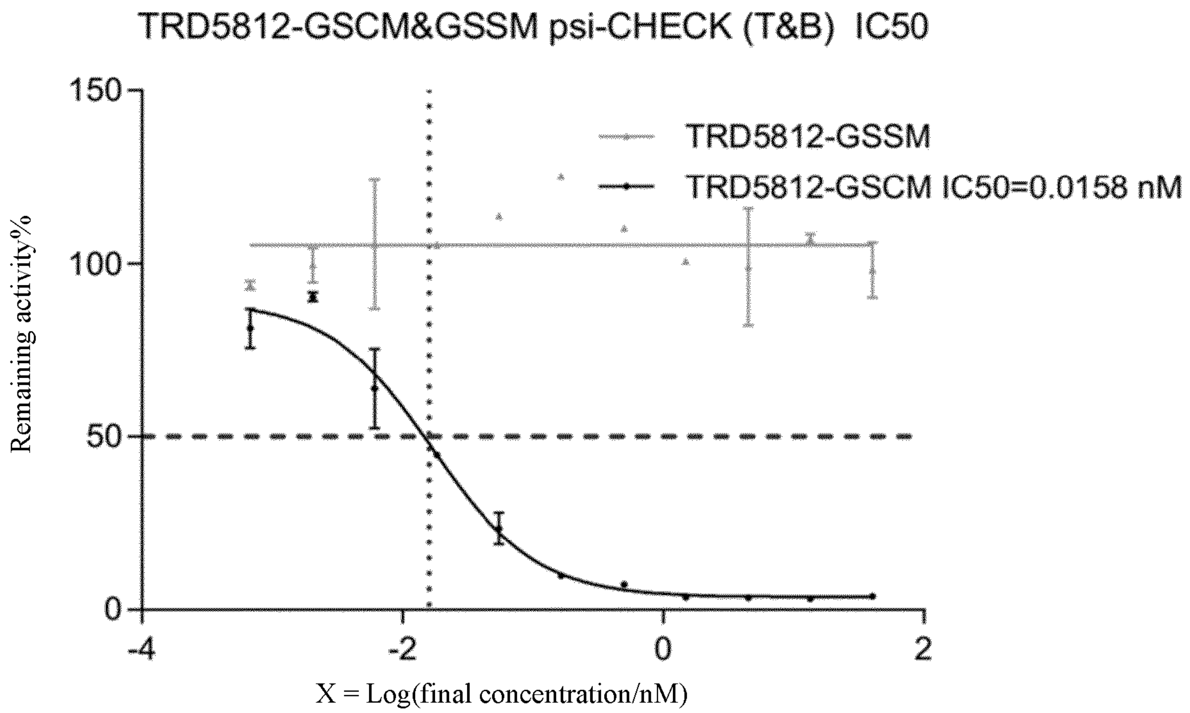
TRD5812-GSCM&GSSM psi-CHECK (T&B) IC50
TRD5812-GSSM
TRD5812-GSCM IC50=0.0158 nM
Remaining activity%
150
100
50
0
-4
-2
0
2
X = Log(final concentration/nM)

**FIG. 1C**

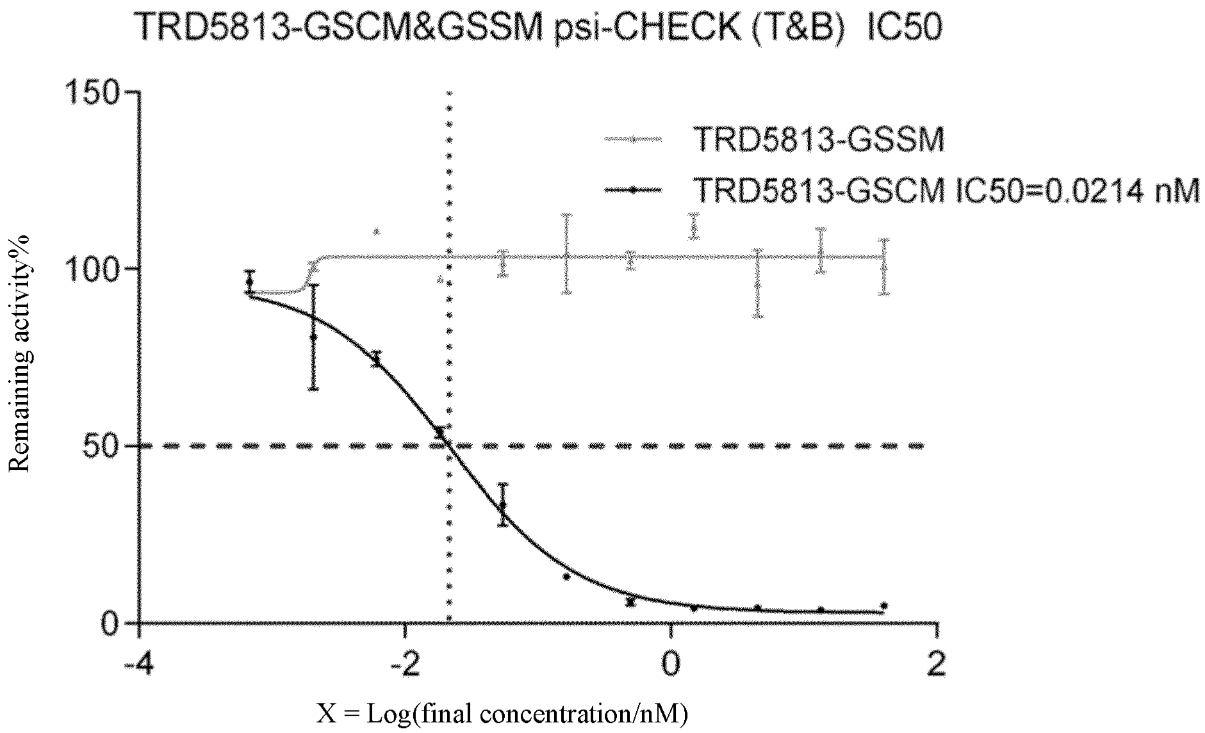
TRD5813-GSCM&GSSM psi-CHECK (T&B) IC50
TRD5813-GSSM
TRD5813-GSCM IC50=0.0214 nM
Remaining activity%
150
100
50
0
-4
-2
0
2
X = Log(final concentration/nM)

**FIG. 1D**

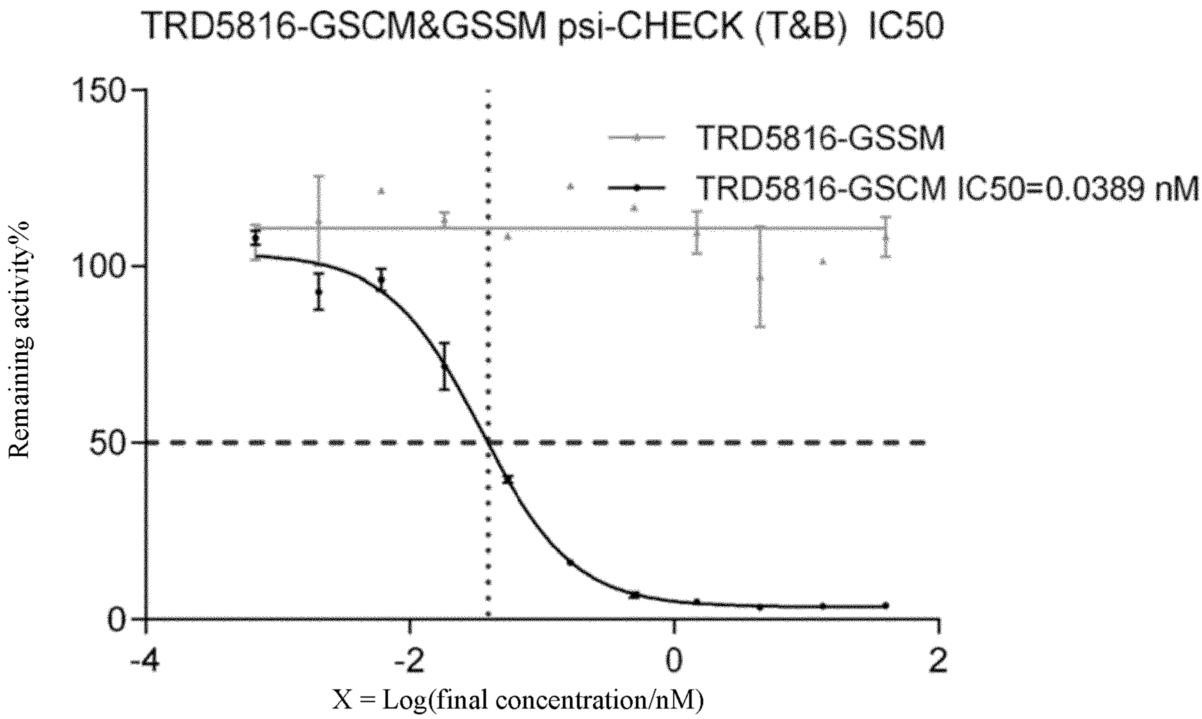
TRD5816-GSCM&GSSM psi-CHECK (T&B) IC50
150
100
50
0
-4
-2
0
2
Remaining activity%
X = Log(final concentration/nM)
TRD5816-GSSM
TRD5816-GSCM IC50=0.0389 nM

FIG. 1E

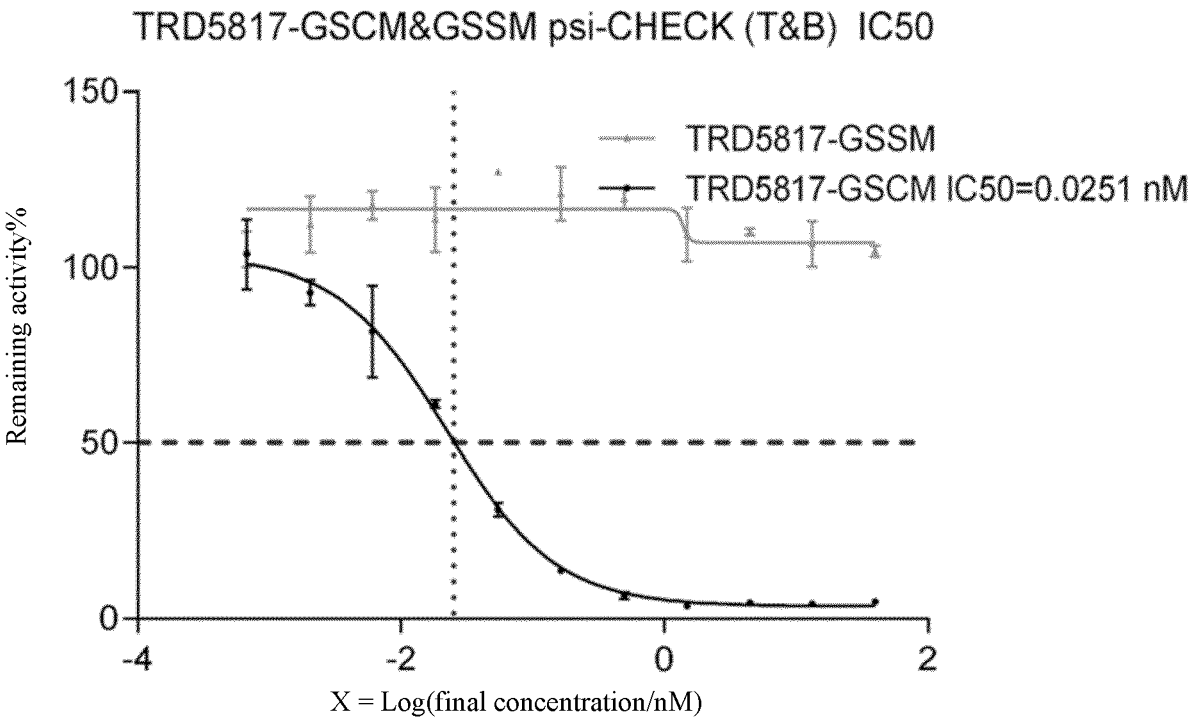
TRD5817-GSCM&GSSM psi-CHECK (T&B) IC50
150
100
50
0
-4
-2
0
2
Remaining activity%
X = Log(final concentration/nM)
TRD5817-GSSM
TRD5817-GSCM IC50=0.0251 nM

FIG. 1F

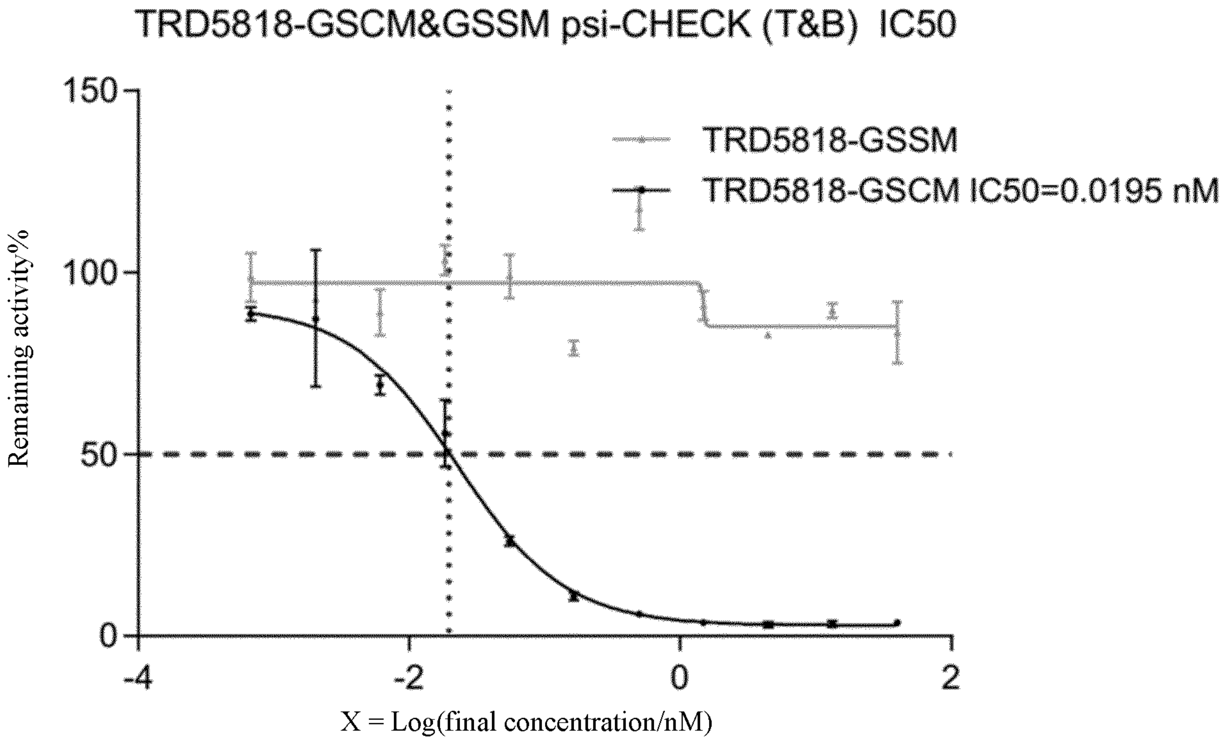
TRD5818-GSCM&GSSM psi-CHECK (T&B) IC50
TRD5818-GSSM
TRD5818-GSCM IC50=0.0195 nM
Remaining activity%
150
100
50
0
-4
-2
0
2
X = Log(final concentration/nM)

**FIG. 1G**

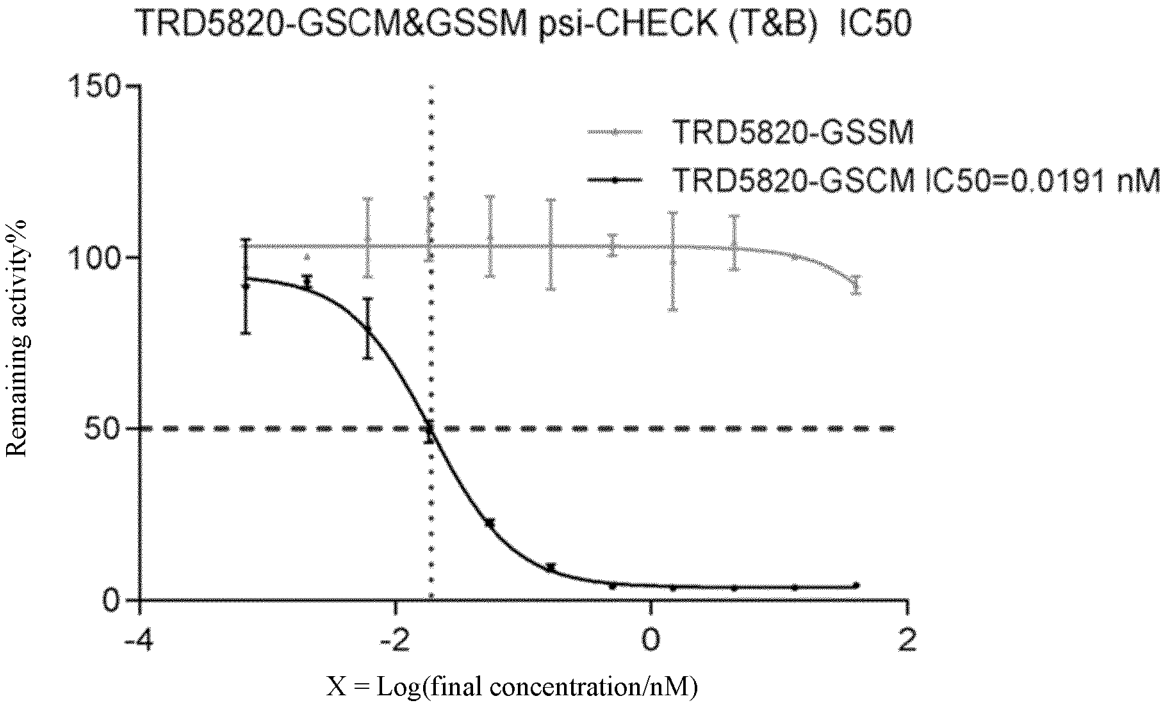
TRD5820-GSCM&GSSM psi-CHECK (T&B) IC50
TRD5820-GSSM
TRD5820-GSCM IC50=0.0191 nM
Remaining activity%
150
100
50
0
-4
-2
0
2
X = Log(final concentration/nM)

**FIG. 1H**

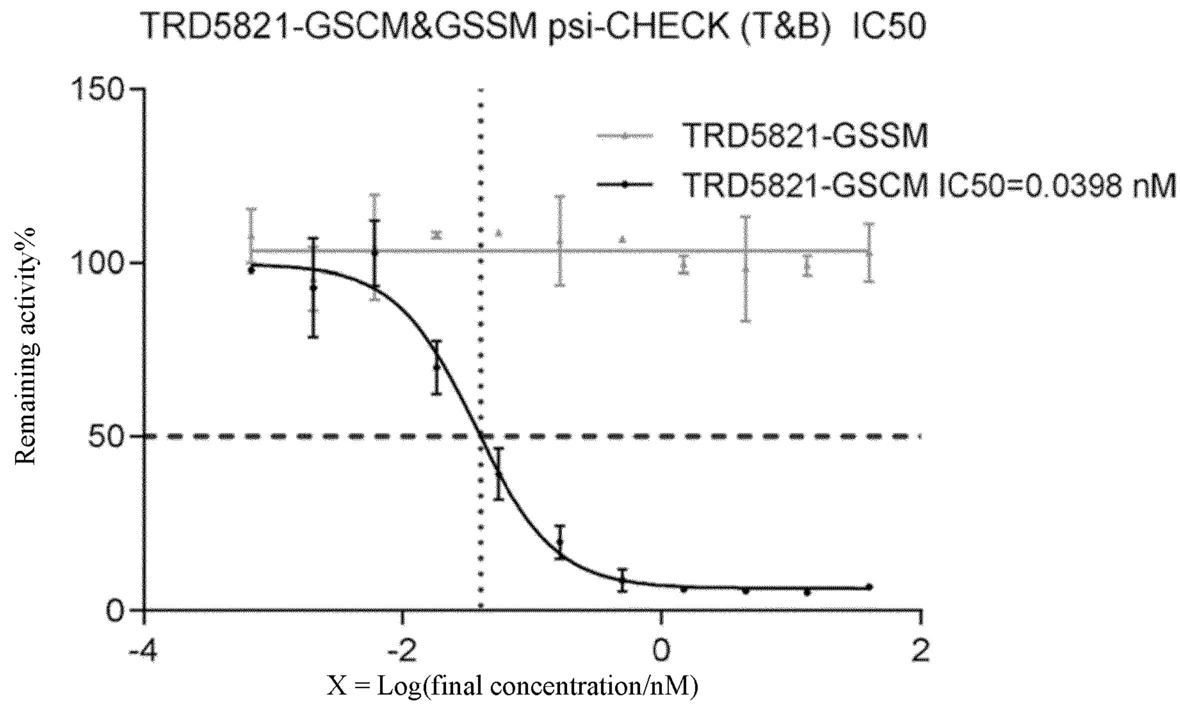
TRD5821-GSCM&GSSM psi-CHECK (T&B) IC50
TRD5821-GSSM
TRD5821-GSCM IC50=0.0398 nM
Remaining activity%
150
100
50
0
-4
-2
0
2
X = Log(final concentration/nM)

**FIG. 1I**

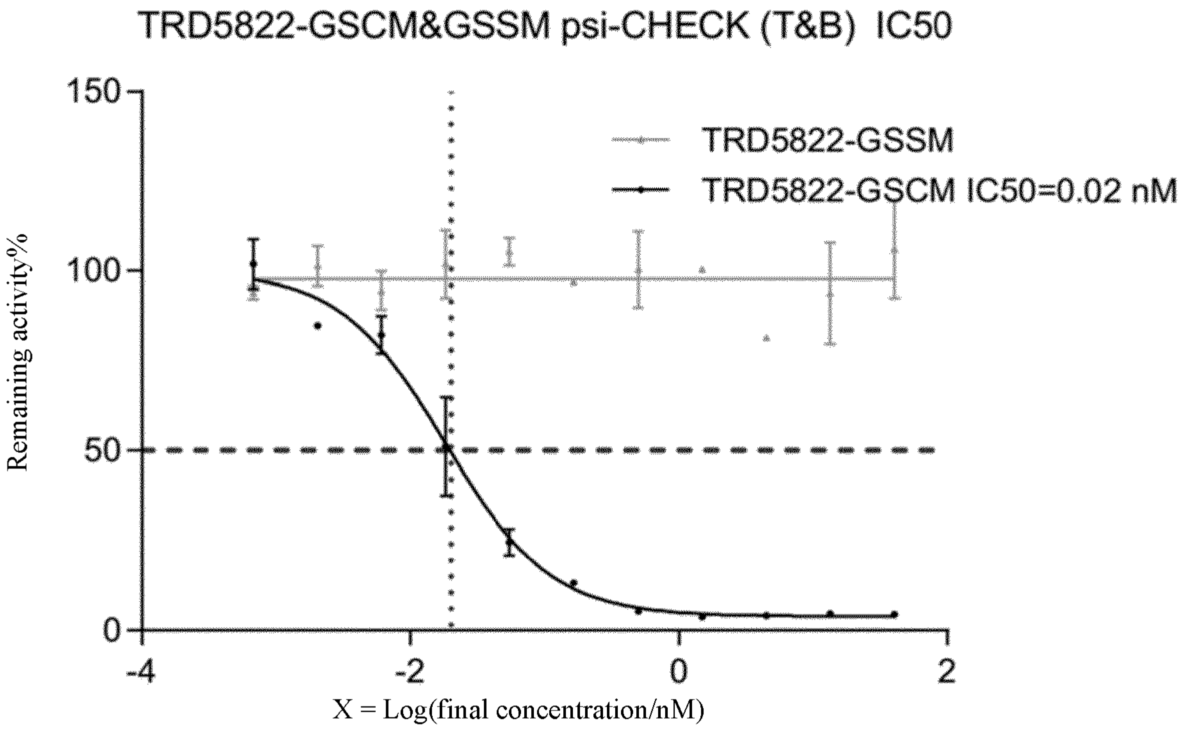
TRD5822-GSCM&GSSM psi-CHECK (T&B) IC50
TRD5822-GSSM
TRD5822-GSCM IC50=0.02 nM
Remaining activity%
150
100
50
0
-4
-2
0
2
X = Log(final concentration/nM)

**FIG. 1J**

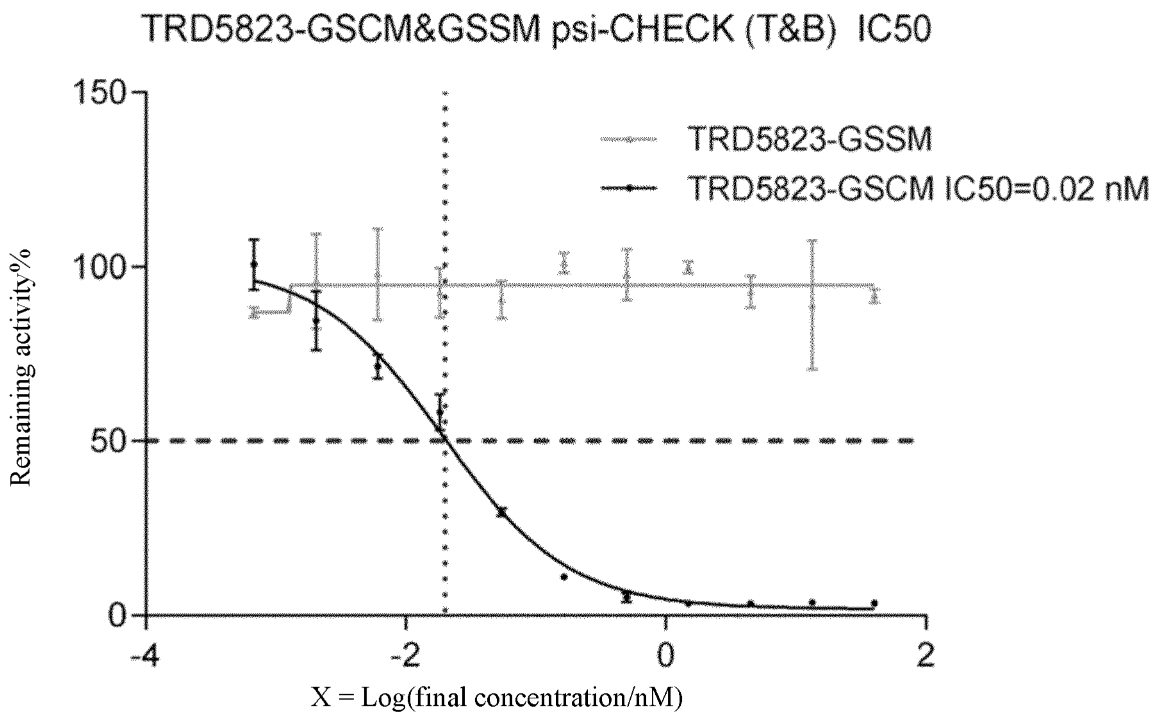
TRD5823-GSCM&GSSM psi-CHECK (T&B) IC50
TRD5823-GSSM
TRD5823-GSCM IC50=0.02 nM
Remaining activity%
150
100
50
0
-4
-2
0
2
X = Log(final concentration/nM)

**FIG. 1K**

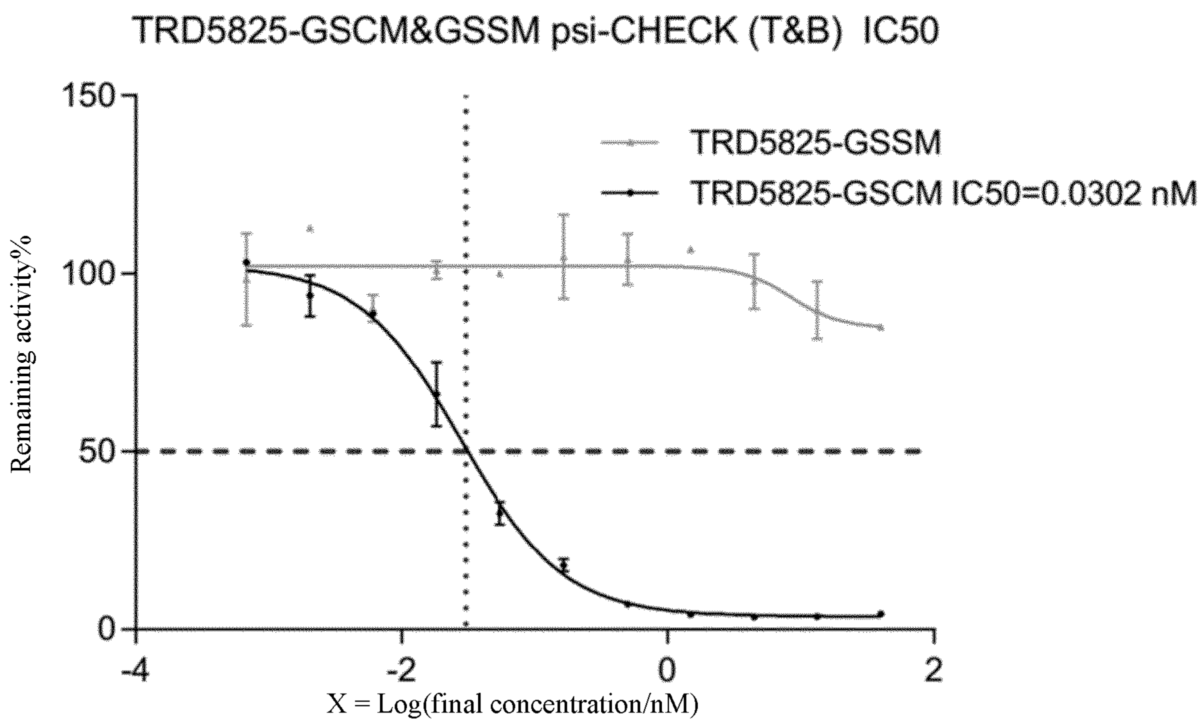
TRD5825-GSCM&GSSM psi-CHECK (T&B) IC50
TRD5825-GSSM
TRD5825-GSCM IC50=0.0302 nM
Remaining activity%
150
100
50
0
-4
-2
0
2
X = Log(final concentration/nM)

**FIG. 1L**

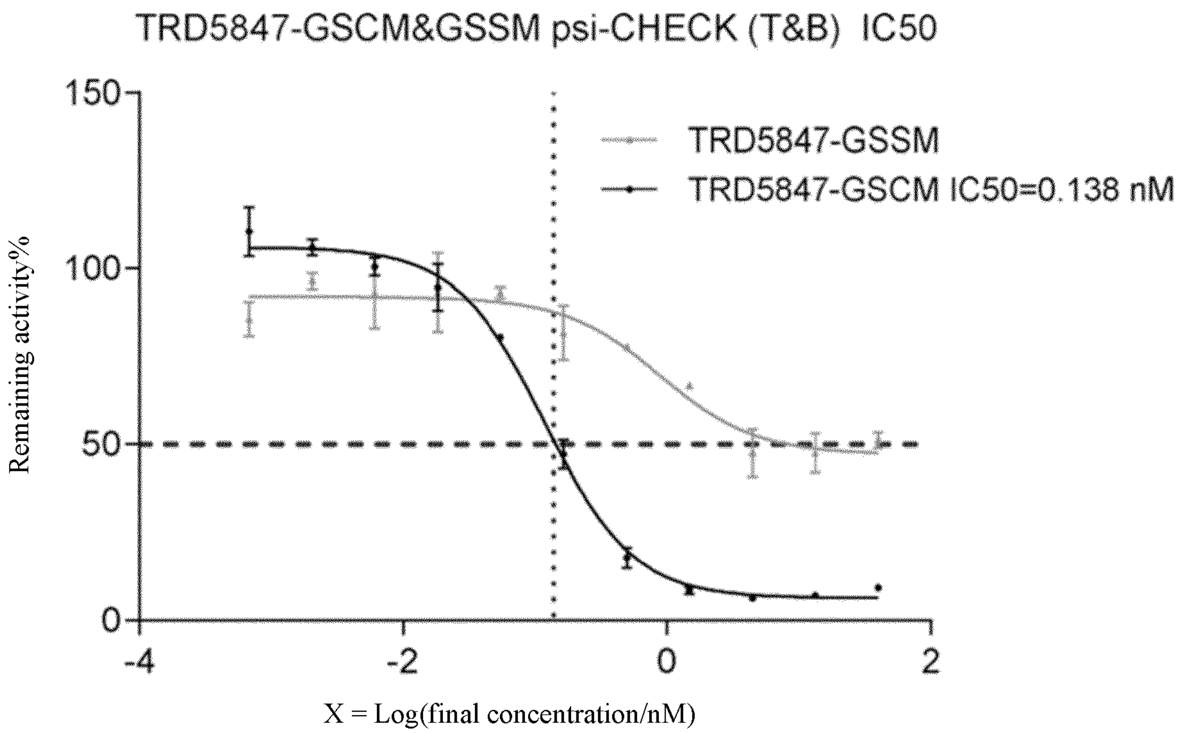
TRD5847-GSCM&GSSM psi-CHECK (T&B) IC50
150
100
50
0
-4
-2
0
2
Remaining activity%
X = Log(final concentration/nM)
TRD5847-GSSM
TRD5847-GSCM IC50=0.138 nM

**FIG. 2A**

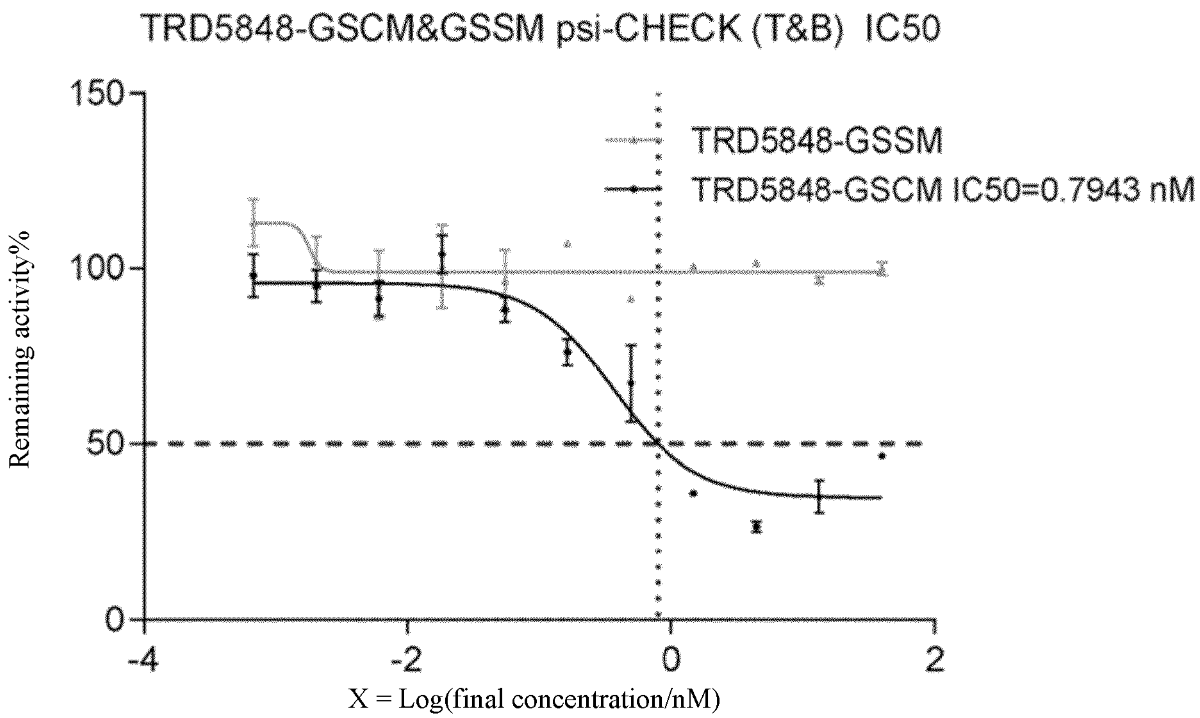
TRD5848-GSCM&GSSM psi-CHECK (T&B) IC50
150
100
50
0
-4
-2
0
2
Remaining activity%
X = Log(final concentration/nM)
TRD5848-GSSM
TRD5848-GSCM IC50=0.7943 nM

**FIG. 2B**

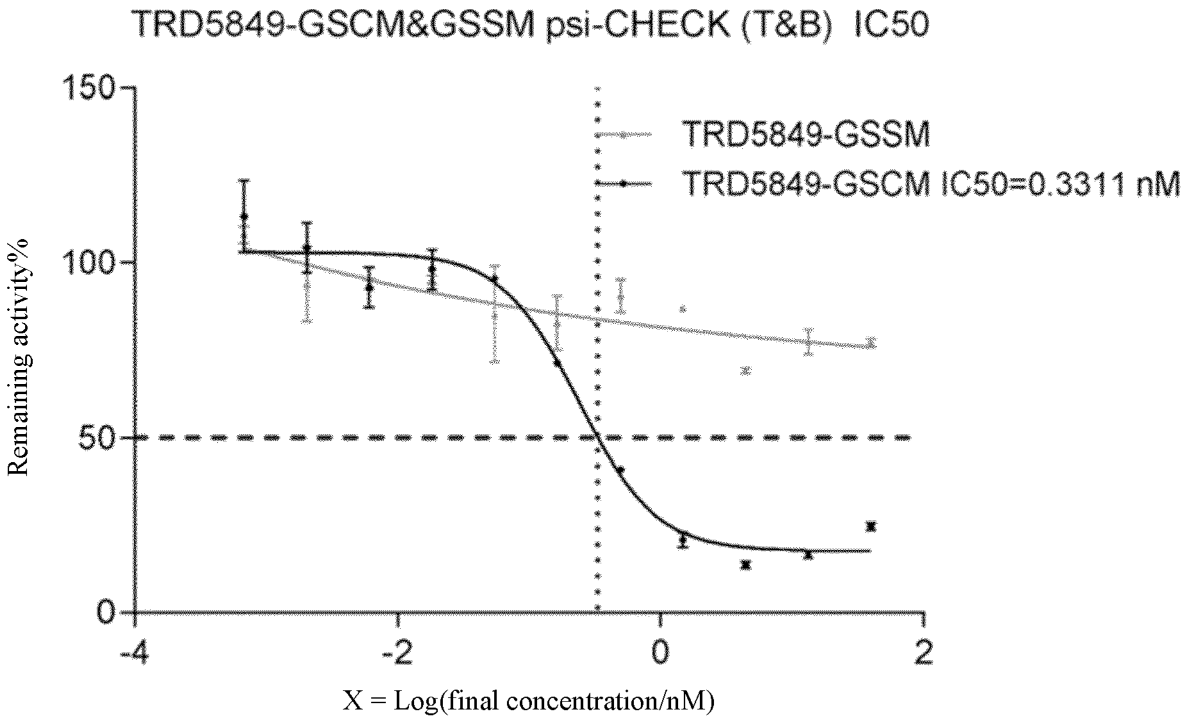
TRD5849-GSCM&GSSM psi-CHECK (T&B) IC50
TRD5849-GSSM
TRD5849-GSCM IC50=0.3311 nM
Remaining activity%
150
100
50
0
-4
-2
0
2
X = Log(final concentration/nM)

**FIG. 2C**

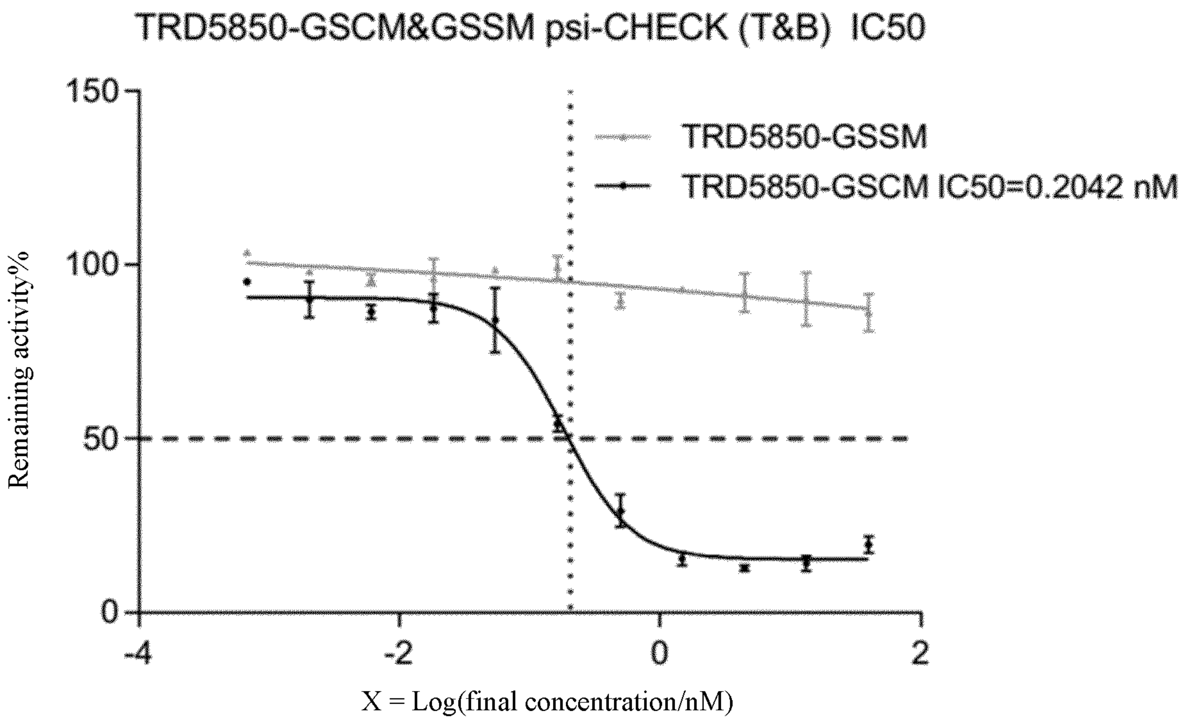
TRD5850-GSCM&GSSM psi-CHECK (T&B) IC50
TRD5850-GSSM
TRD5850-GSCM IC50=0.2042 nM
Remaining activity%
150
100
50
0
-4
-2
0
2
X = Log(final concentration/nM)

**FIG. 2D**

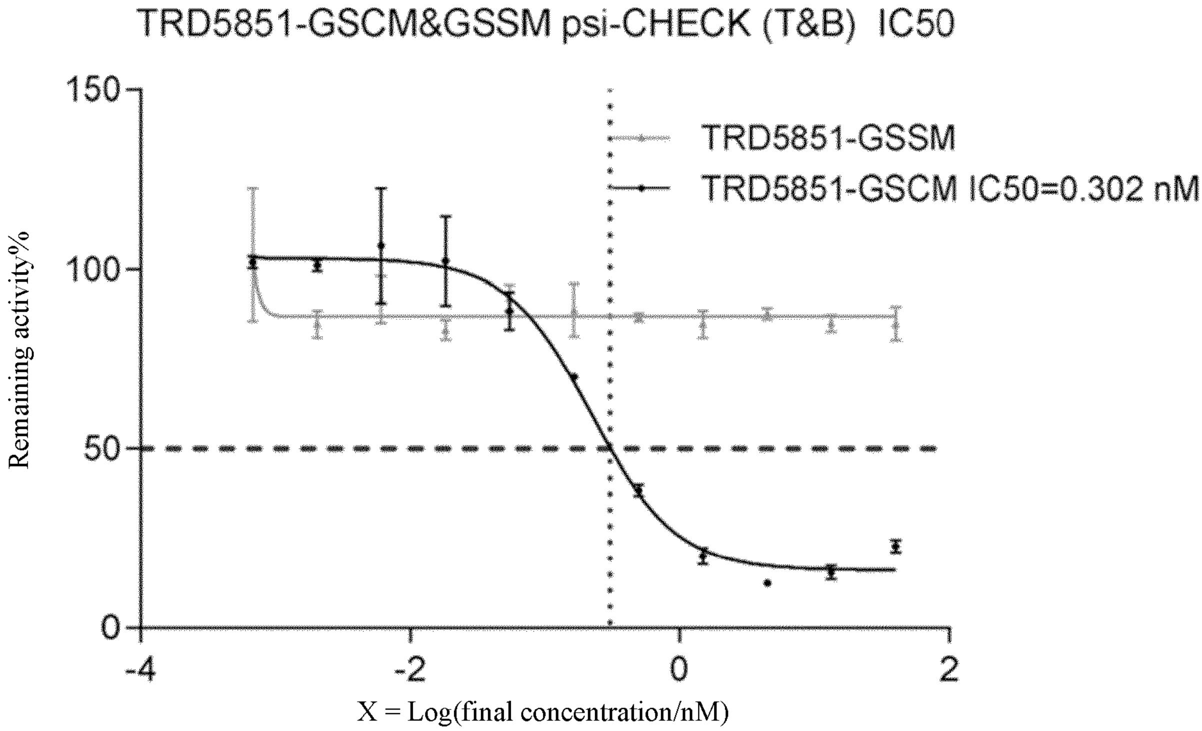
TRD5851-GSCM&GSSM psi-CHECK (T&B) IC50
TRD5851-GSSM
TRD5851-GSCM IC50=0.302 nM
Remaining activity%
150
100
50
0
-4
-2
0
2
X = Log(final concentration/nM)

FIG. 2E

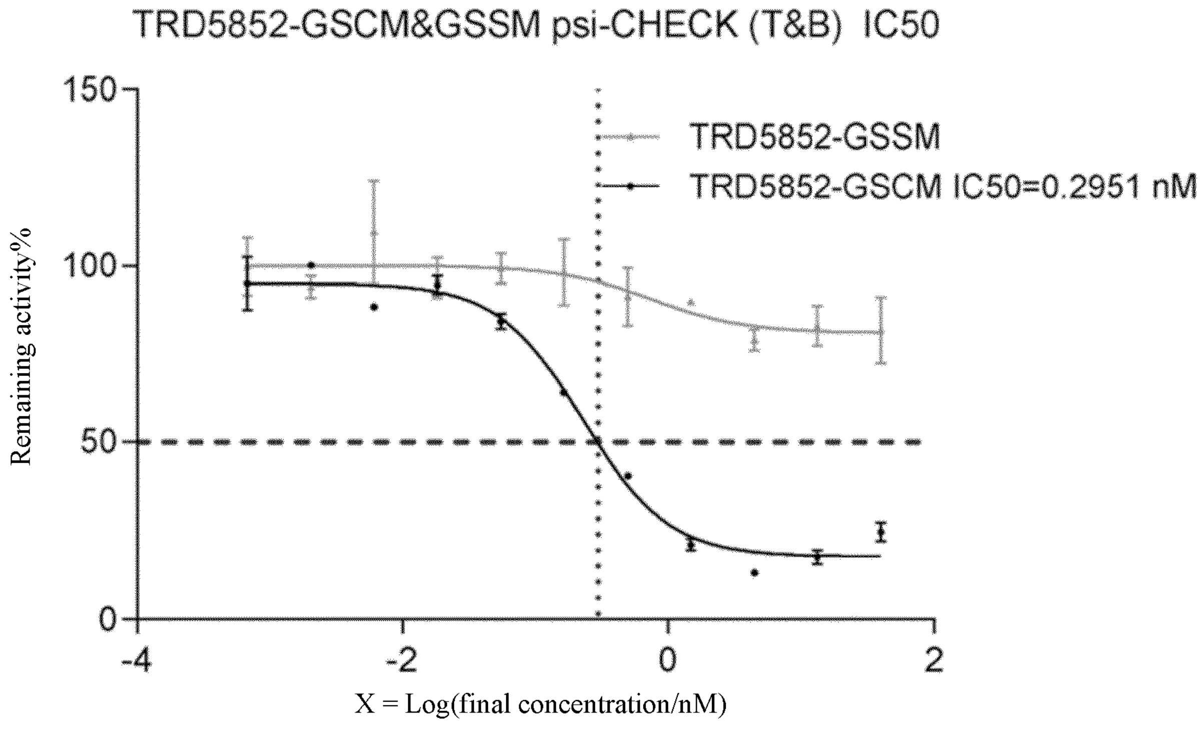
TRD5852-GSCM&GSSM psi-CHECK (T&B) IC50
TRD5852-GSSM
TRD5852-GSCM IC50=0.2951 nM
Remaining activity%
150
100
50
0
-4
-2
0
2
X = Log(final concentration/nM)

FIG. 2F

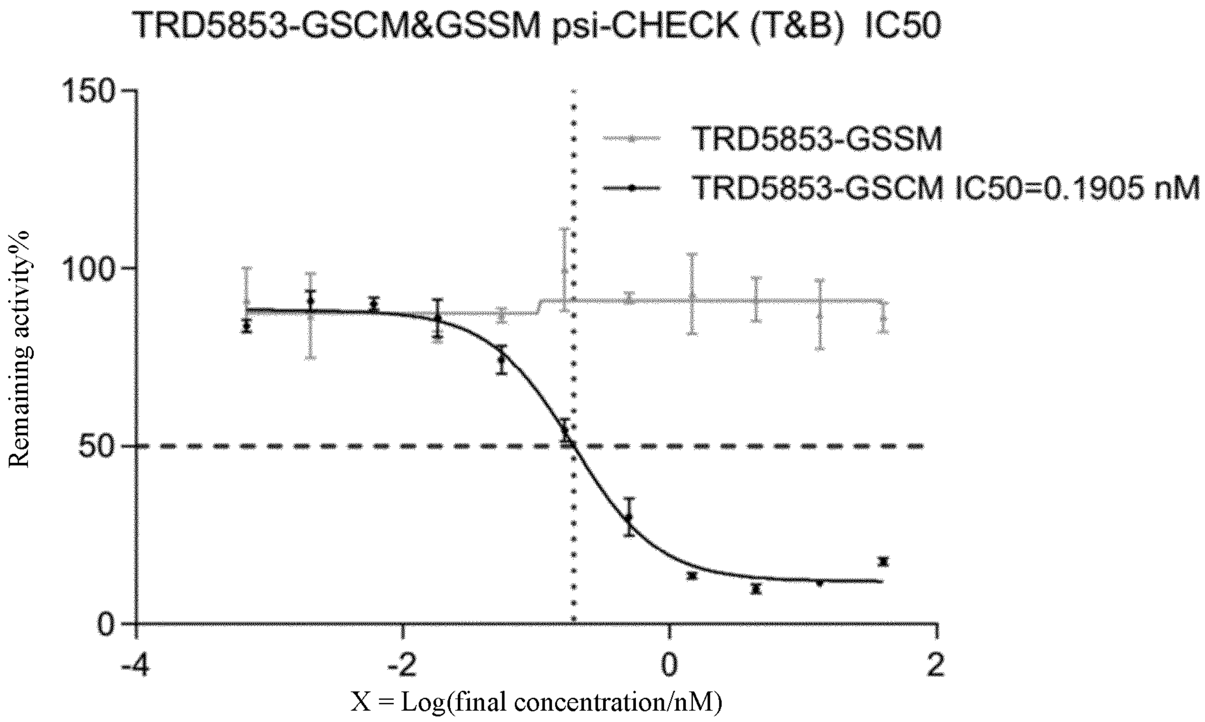
TRD5853-GSCM&GSSM psi-CHECK (T&B) IC50
TRD5853-GSSM
TRD5853-GSCM IC50=0.1905 nM
Remaining activity%
150
100
50
0
-4
-2
0
2
X = Log(final concentration/nM)

**FIG. 2G**

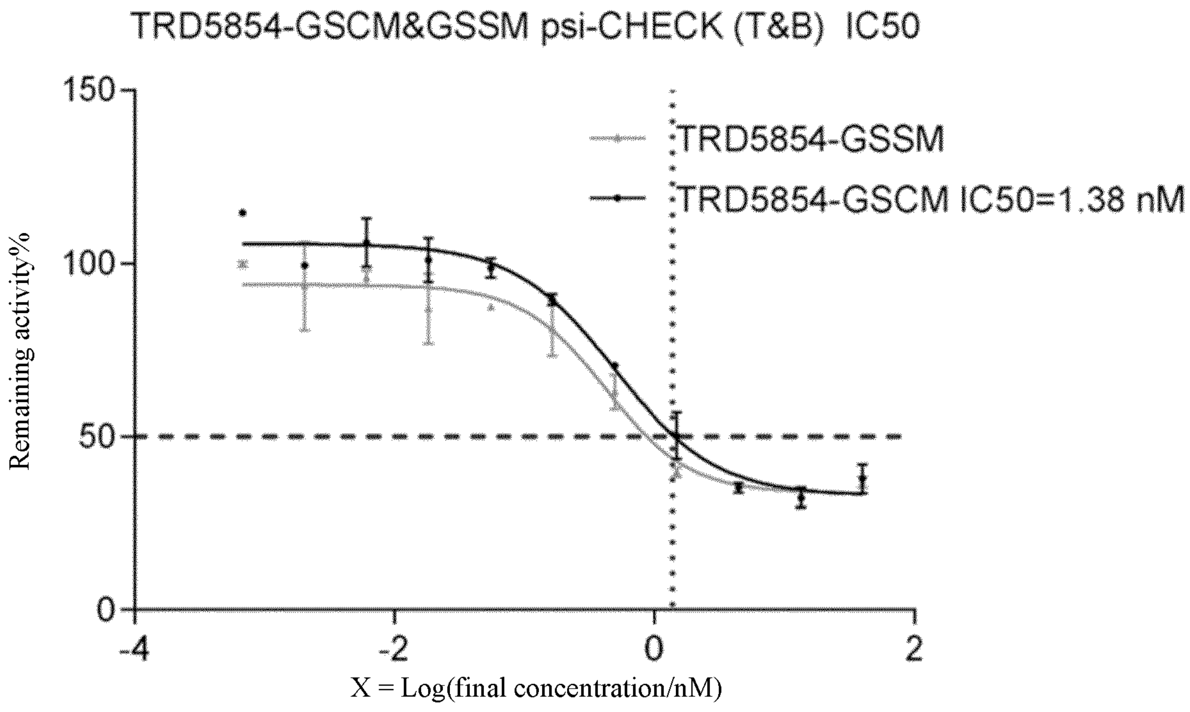
TRD5854-GSCM&GSSM psi-CHECK (T&B) IC50
TRD5854-GSSM
TRD5854-GSCM IC50=1.38 nM
Remaining activity%
150
100
50
0
-4
-2
0
2
X = Log(final concentration/nM)

**FIG. 3A**

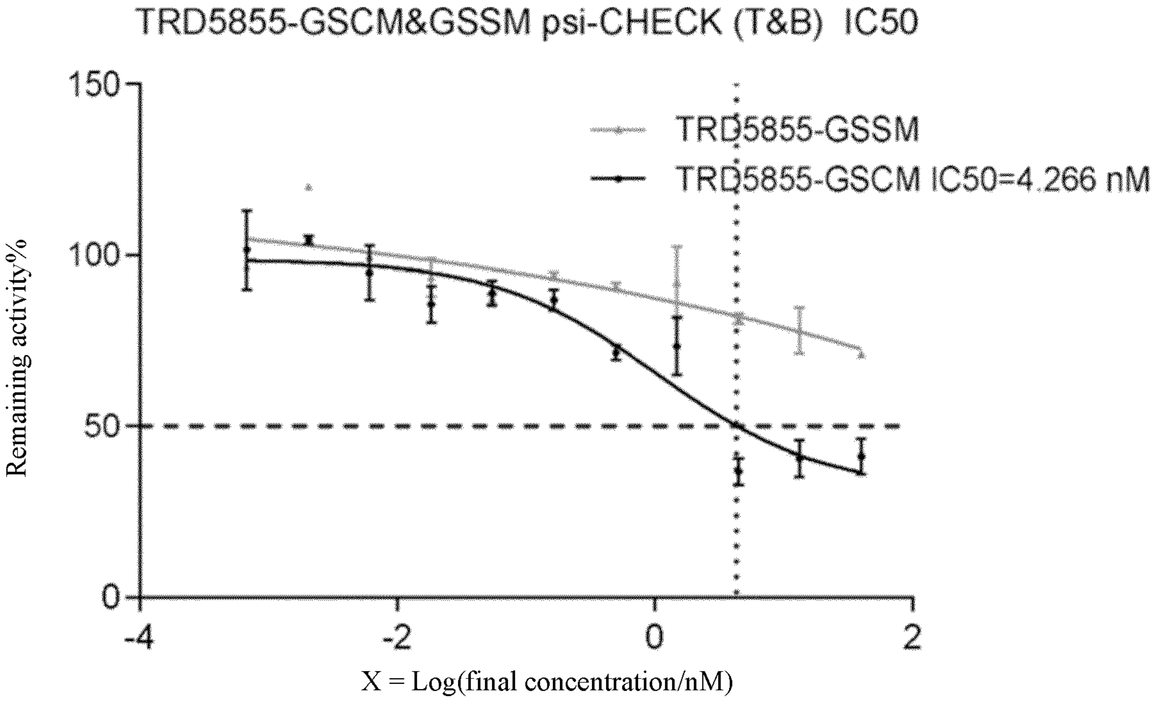
TRD5855-GSCM&GSSM psi-CHECK (T&B) IC50
TRD5855-GSSM
TRD5855-GSCM IC50=4.266 nM
Remaining activity%
150
100
50
0
-4
-2
0
2
X = Log(final concentration/nM)

**FIG. 3B**

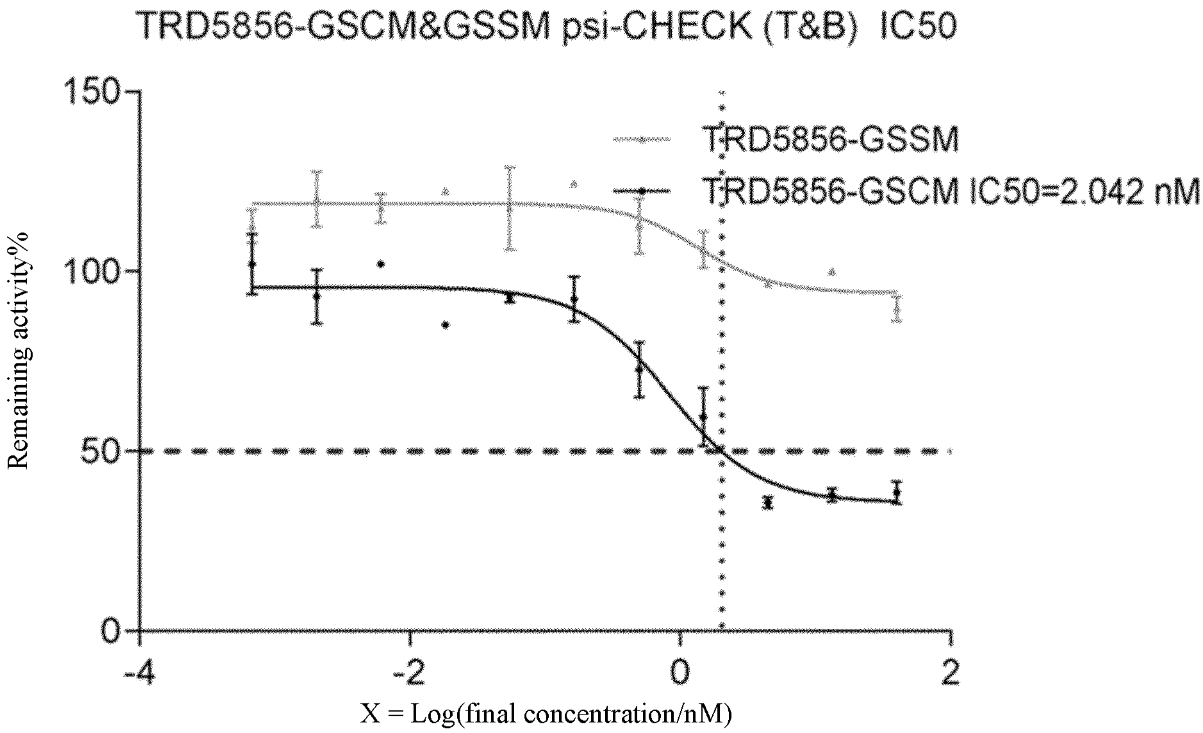
TRD5856-GSCM&GSSM psi-CHECK (T&B) IC50
TRD5856-GSSM
TRD5856-GSCM IC50=2.042 nM
Remaining activity%
150
100
50
0
-4
-2
0
2
X = Log(final concentration/nM)

**FIG. 3C**

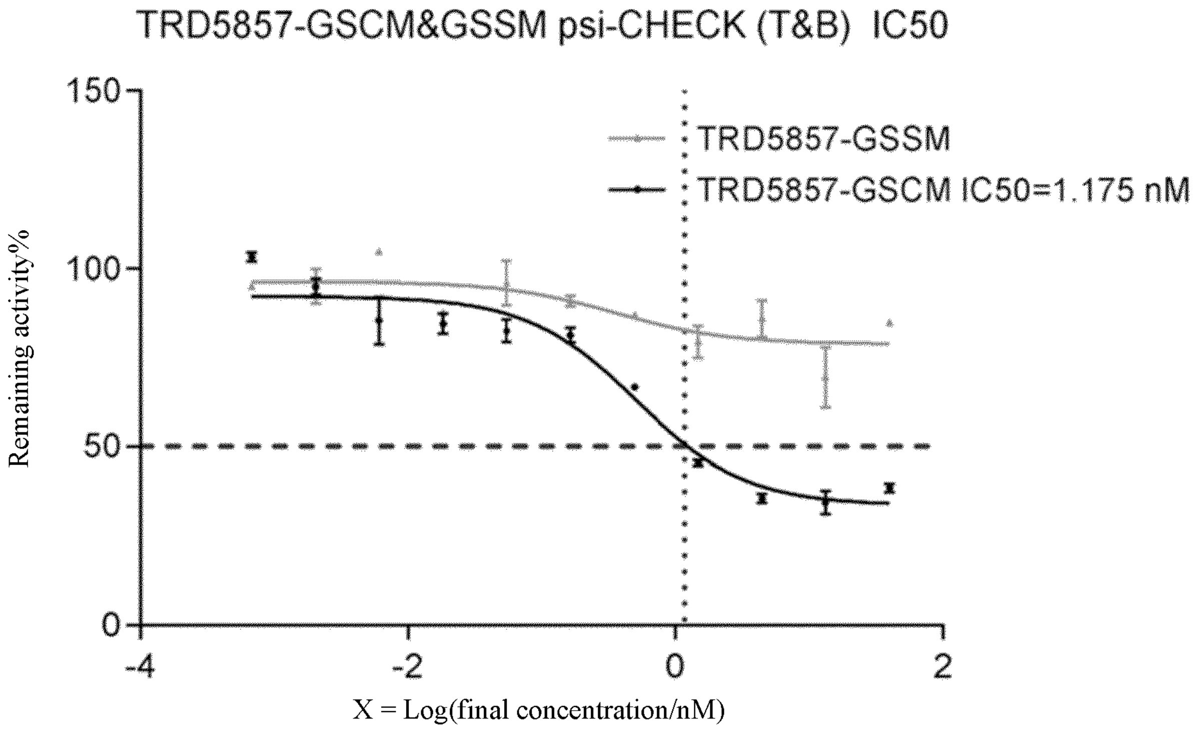
TRD5857-GSCM&GSSM psi-CHECK (T&B) IC50
TRD5857-GSSM
TRD5857-GSCM IC50=1.175 nM
Remaining activity%
150
100
50
0
-4
-2
0
2
X = Log(final concentration/nM)

FIG. 3D

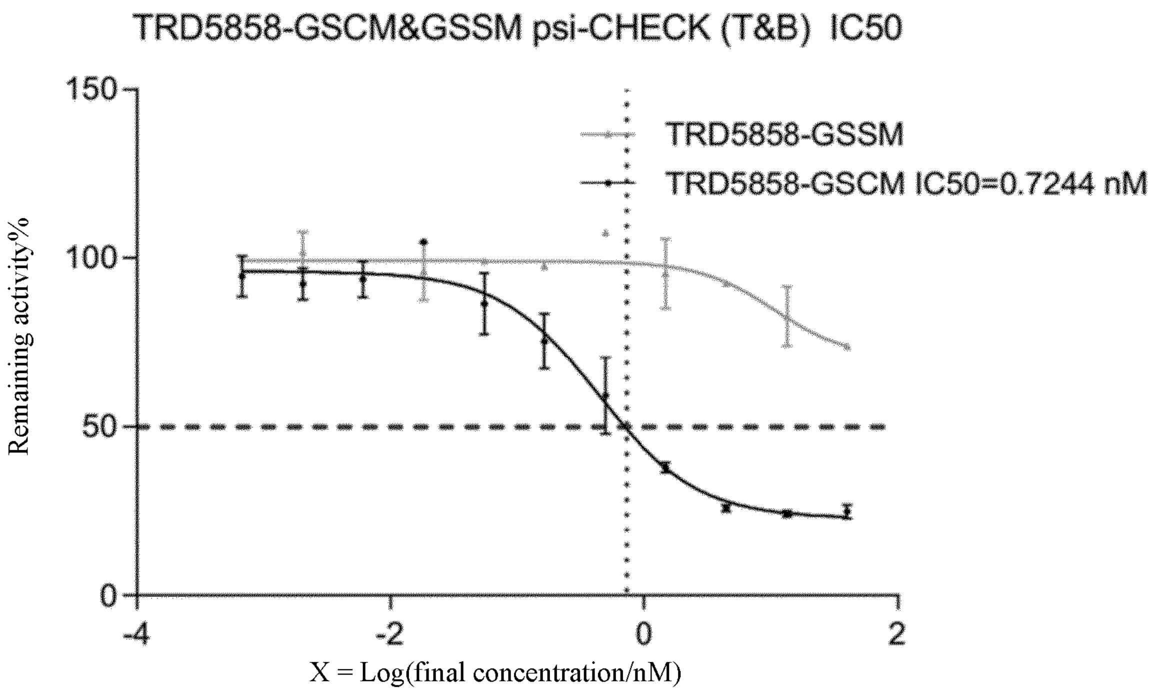
TRD5858-GSCM&GSSM psi-CHECK (T&B) IC50
TRD5858-GSSM
TRD5858-GSCM IC50=0.7244 nM
Remaining activity%
150
100
50
0
-4
-2
0
2
X = Log(final concentration/nM)

FIG. 3E

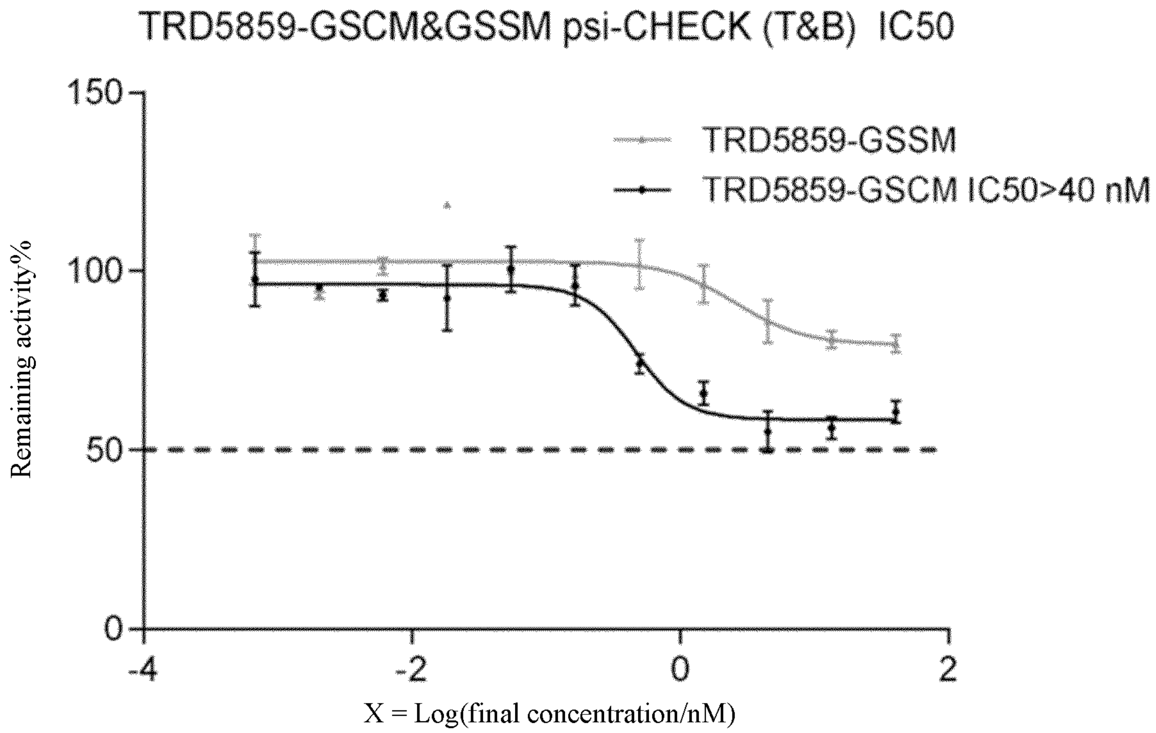
TRD5859-GSCM&GSSM psi-CHECK (T&B) IC50
150
100
50
0
-4
-2
0
2
TRD5859-GSSM
TRD5859-GSCM IC50>40 nM
Remaining activity%
X = Log(final concentration/nM)

**FIG. 3F**

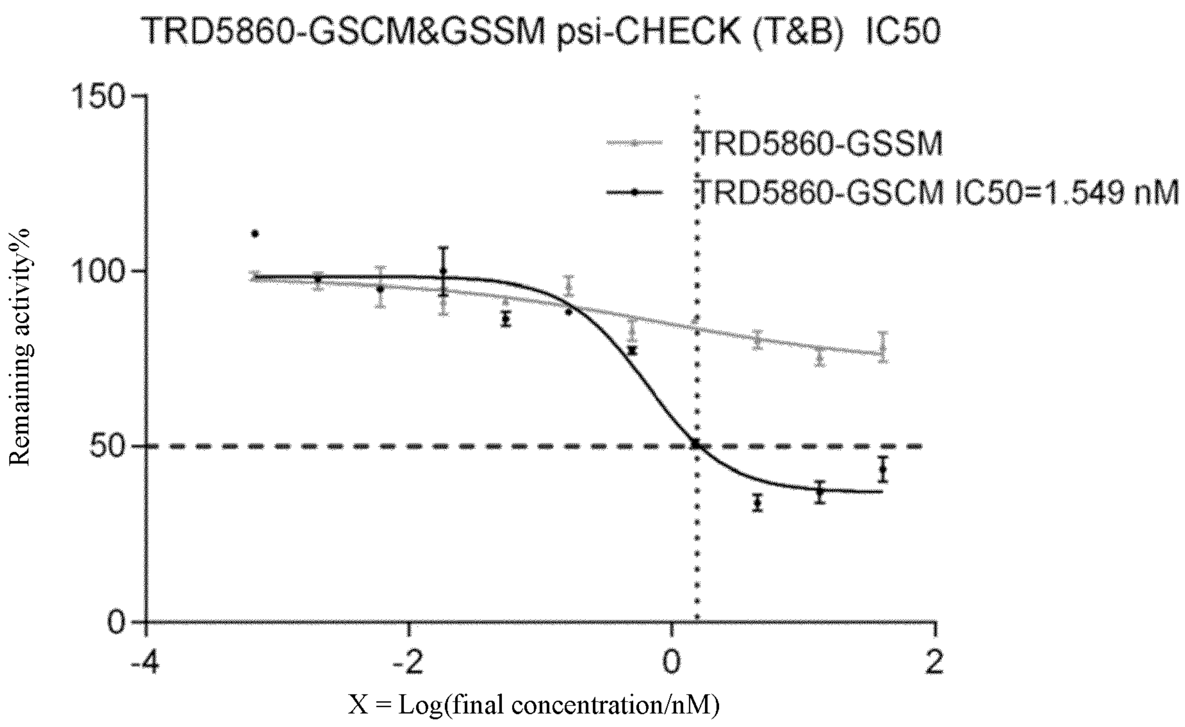
TRD5860-GSCM&GSSM psi-CHECK (T&B) IC50
150
100
50
0
-4
-2
0
2
TRD5860-GSSM
TRD5860-GSCM IC50=1.549 nM
Remaining activity%
X = Log(final concentration/nM)

**FIG. 3G**

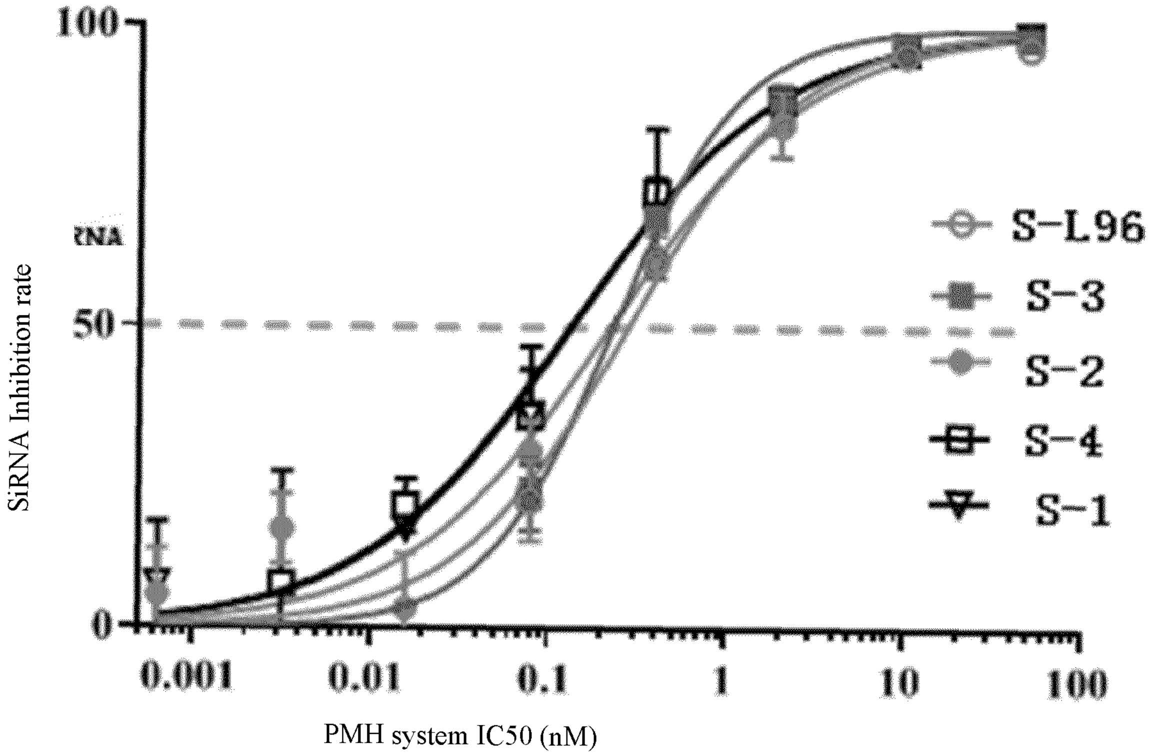
100
50
0
0.001
0.01
0.1
1
10
100
SiRNA Inhibition rate
PMH system IC50 (nM)
S-L96
S-3
S-2
S-4
S-1

**FIG. 4**

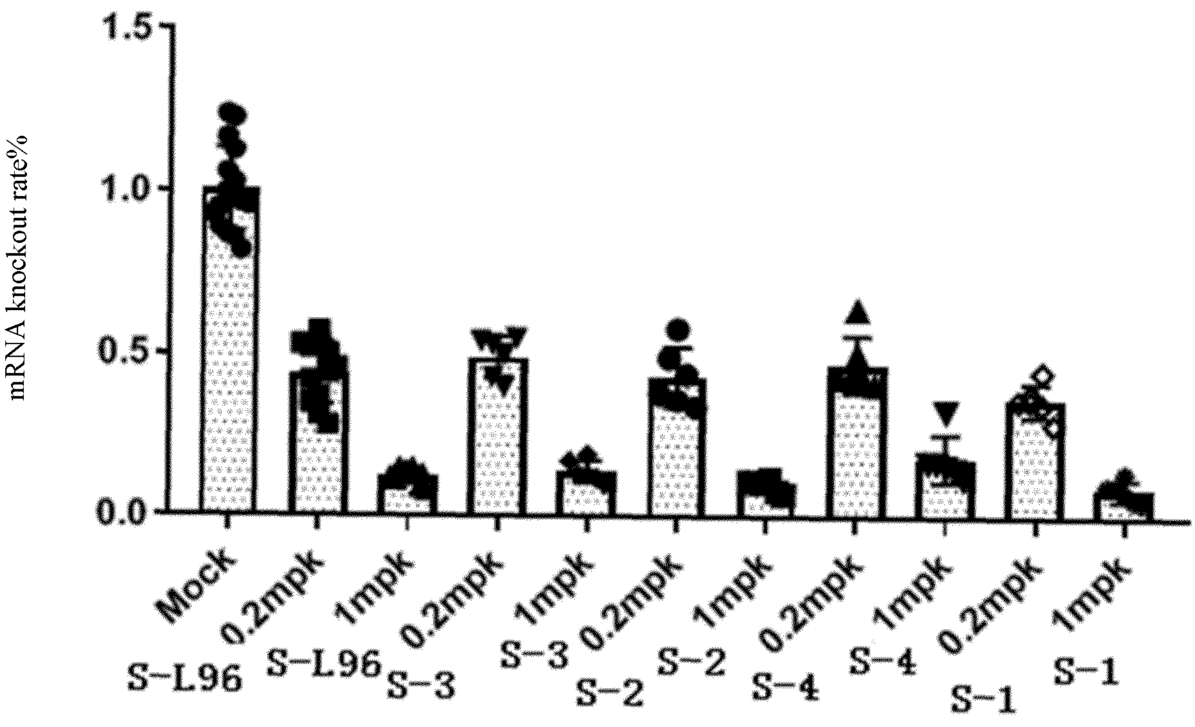
1.5
1.0
0.5
0.0
mRNA knockout rate%
Mock
S-L96
0.2mpk
S-L96
1mpk
S-3
0.2mpk
S-3
1mpk
S-2
0.2mpk
S-2
1mpk
S-4
0.2mpk
S-4
1mpk
S-1
0.2mpk
S-1
1mpk
S-1

**FIG. 5**

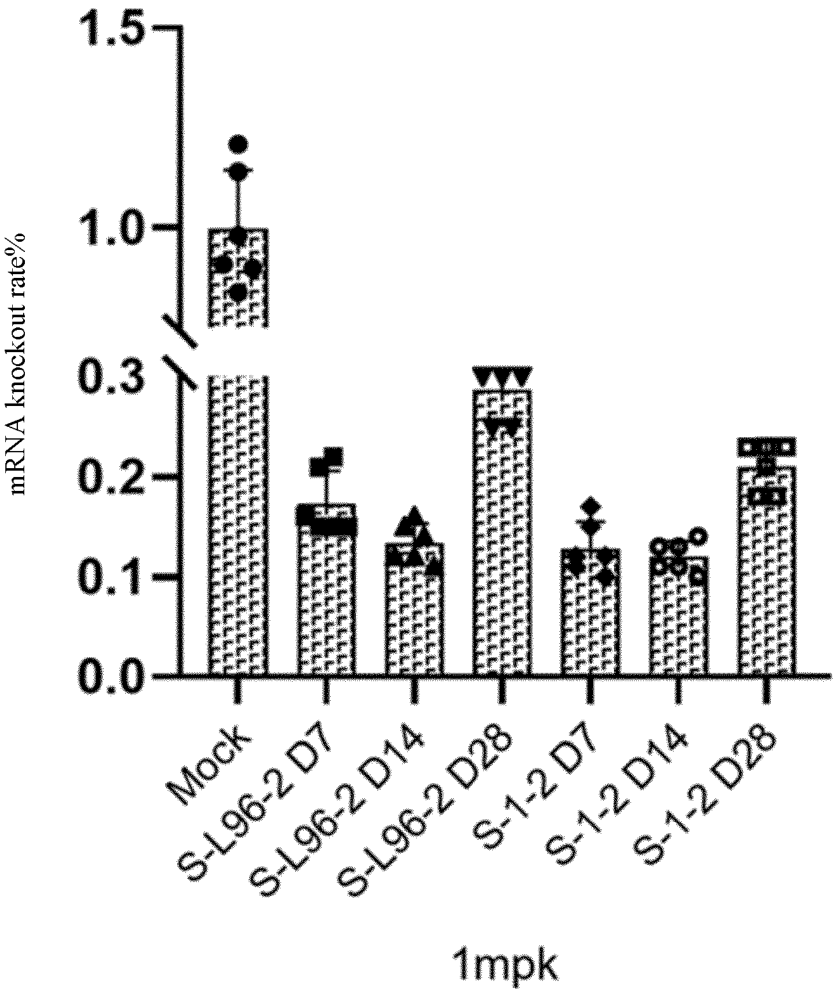
1.5
1.0
0.3
0.2
0.1
0.0
mRNA knockout rate%
Mock
S-L96-2 D7
S-L96-2 D14
S-L96-2 D28
S-1-2 D7
S-1-2 D14
S-1-2 D28
1mpk

FIG. 6

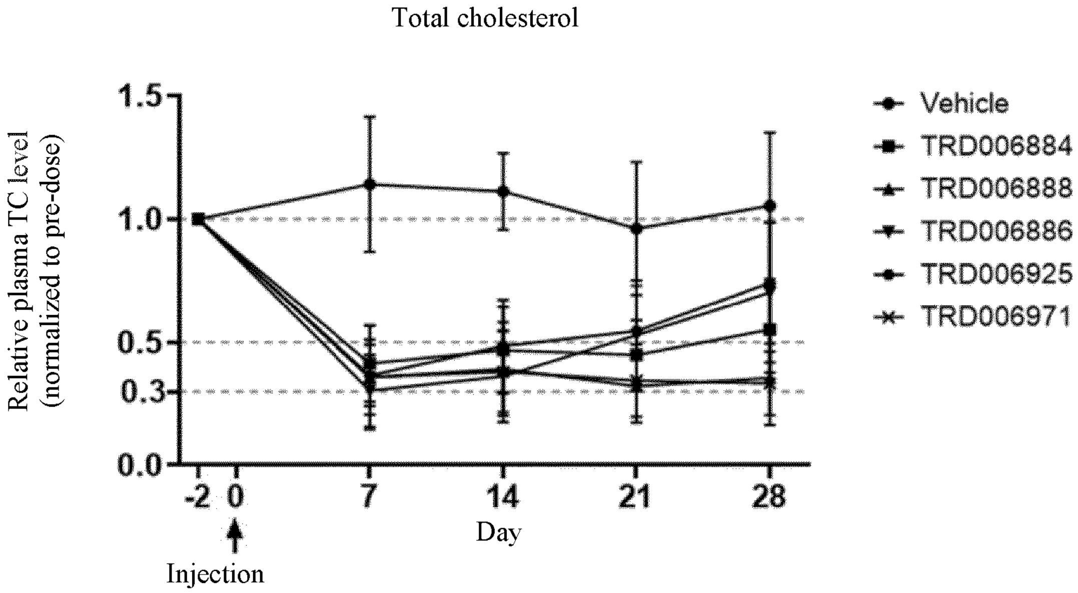
Total cholesterol
Relative plasma TC level (normalized to pre-dose)
1.5
1.0
0.5
0.3
0.0
-2
0
7
14
21
28
Day
Injection
Vehicle
TRD006884
TRD006888
TRD006886
TRD006925
TRD006971

FIG. 7

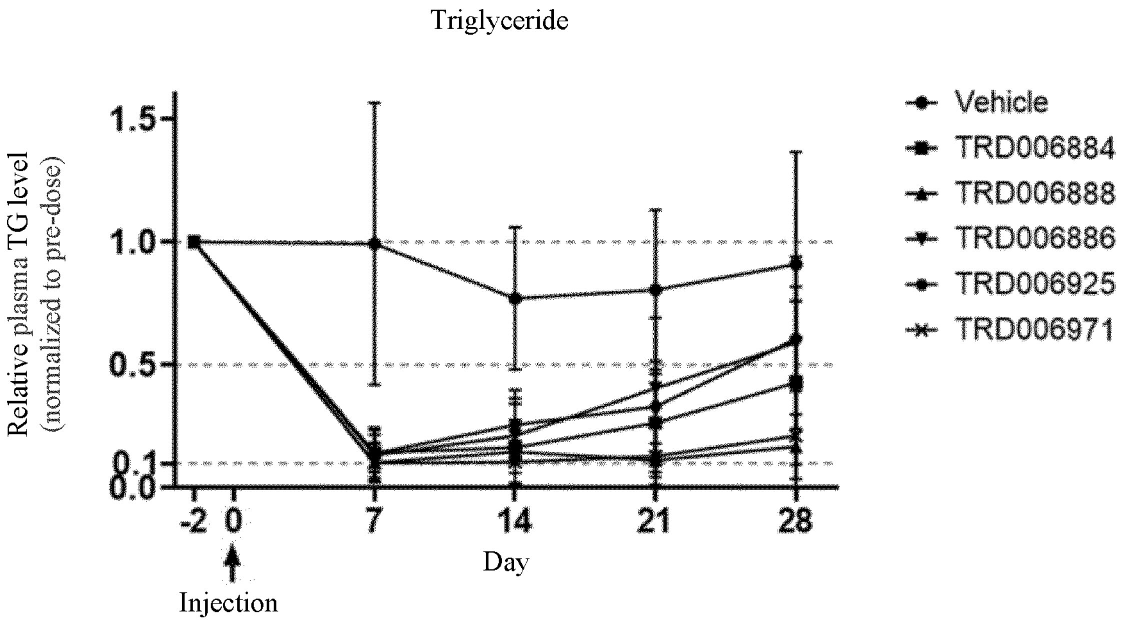
Triglyceride
Relative plasma TG level
(normalized to pre-dose)
1.5
1.0
0.5
0.1
0.0
-2
0
7
14
21
28
Day
Injection
Vehicle
TRD006884
TRD006888
TRD006886
TRD006925
TRD006971

**FIG. 8**

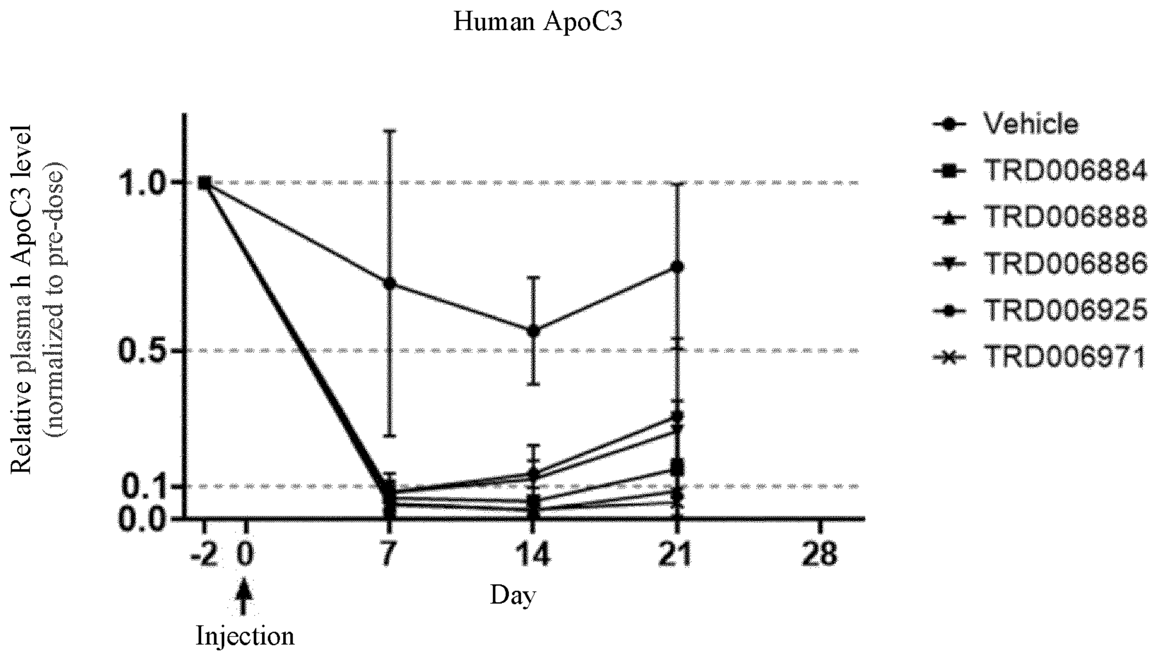
Human ApoC3
Relative plasma h ApoC3 level
(normalized to pre-dose)
1.0
0.5
0.1
0.0
-2
0
7
14
21
28
Day
Injection
Vehicle
TRD006884
TRD006888
TRD006886
TRD006925
TRD006971

**FIG. 9**

## INTERNATIONAL SEARCH REPORT

International application No.
**PCT/CN2021/110509**

**A. CLASSIFICATION OF SUBJECT MATTER**

C12N 15/113(2010.01)i; A61K 31/713(2006.01)i; A61K 48/00(2006.01)i

According to International Patent Classification (IPC) or to both national classification and IPC

**B. FIELDS SEARCHED**

Minimum documentation searched (classification system followed by classification symbols)

C12N; A61K

Documentation searched other than minimum documentation to the extent that such documents are included in the fields searched

Electronic data base consulted during the international search (name of data base and, where practicable, search terms used)

EPTXT; DWPI; USTXT; WOTXT; CNABS; CNTXT; CNKI; PUBMED; SPRINGERLINK; 百度学术; WEB OF SCIENCE; STN: 上海拓届, 黄金宇, 罗敏, 尹科, ANTISENSE, AS, SS, 有义链, 反义链, 正义链, 第2-8位, 第7位, modification, siRNA, 干扰RNA, 修饰, 甲氧基, off, target, 氟代, 脱靶, 氟代, 甲氧基, 甲基, 氨基, 亚磷酰胺

**C. DOCUMENTS CONSIDERED TO BE RELEVANT**

| Category* | Citation of document, with indication, where appropriate, of the relevant passages | Relevant to claim No. |
|---|---|---|
| X | CN 111378655 A (SUZHOU RIBO LIFE SCIENCE CO., LTD.) 07 July 2020 (2020-07-07) claims 1-19, description paragraphs 14, 126-139 | 1-33 |
| X | CN 111378656 A (SUZHOU RIBO LIFE SCIENCE CO., LTD.) 07 July 2020 (2020-07-07) claims 1-16, description paragraphs 22, 65-78 | 1-33 |
| X | CN 111378657 A (SUZHOU RIBO LIFE SCIENCE CO., LTD.) 07 July 2020 (2020-07-07) claims 1-19, description paragraphs 14, 126-139 | 1-33 |
| A | CN 108367022 A (NITTO DENKO CORPORATION) 03 August 2018 (2018-08-03) entire document | 1-33 |
| A | WO 2009102427 A2 (RXI PHARMACEUTICALS CORP.) 20 August 2009 (2009-08-20) entire document | 1-33 |
| A | CN 107201367 A (GUANGZHOU RIBOBIO CO., LTD.) 26 September 2017 (2017-09-26) entire document | 1-33 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 October 2021** | **03 November 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/110509**

**Box No. II Observations where certain claims were found unsearchable (Continuation of item 2 of first sheet)**

This international search report has not been established in respect of certain claims under Article 17(2)(a) for the following reasons:

1. [x] Claims Nos.: **18**
because they relate to subject matter not required to be searched by this Authority, namely:

[1] Claim 18 relates to a treatment method for diseases, and thus does not comply with PCT Rule 39.1 (iv). The present report is provided on the basis of claim 18 amended as pharmaceutical use claim.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/110509**

| Patent document cited in search report | Publication date (day/month/year) | Patent family member(s) | Publication date (day/month/year) |
|---|---|---|---|
| CN 111378655 A | 07 July 2020 | None | |
| CN 111378656 A | 07 July 2020 | None | |
| CN 111378657 A | 07 July 2020 | None | |
| CN 108367022 A | 03 August 2018 | US 10358647 B2 | 23 July 2019 |
| | | US 2017166898 A1 | 15 June 2017 |
| | | KR 20180086260 A | 30 July 2018 |
| | | EP 3386519 B1 | 24 March 2021 |
| | | AU 2016371624 A1 | 12 July 2018 |
| | | RU 2018125593 A3 | 06 April 2020 |
| | | EP 3816287 A1 | 05 May 2021 |
| | | EP 3386519 A4 | 07 November 2018 |
| | | BR 112018008344 A2 | 30 October 2018 |
| | | TW 201726920 A | 01 August 2017 |
| | | WO 2017106111 A1 | 22 June 2017 |
| | | PT 3386519 T | 27 April 2021 |
| | | US 2019323015 A1 | 24 October 2019 |
| | | EP 3386519 A1 | 17 October 2018 |
| | | CA 3005937 A1 | 22 June 2017 |
| | | JP 6457704 B2 | 23 January 2019 |
| | | AU 2016371624 B2 | 27 August 2020 |
| | | RU 2018125593 A | 13 January 2020 |
| | | JP 2018537104 A | 20 December 2018 |
| WO 2009102427 A2 | 20 August 2009 | CN 102016036 B | 08 April 2015 |
| | | WO 2009102427 A3 | 23 December 2009 |
| | | JP 2015133968 A | 27 July 2015 |
| | | EP 2247729 B1 | 01 May 2019 |
| | | JP 5697993 B2 | 08 April 2015 |
| | | US 2011039914 A1 | 17 February 2011 |
| | | JP 2011511636 A | 14 April 2011 |
| | | CA 2715289 C | 24 December 2019 |
| | | AU 2009213147 A1 | 20 August 2009 |
| | | JP 6170954 B2 | 26 July 2017 |
| | | EP 2247729 A2 | 10 November 2010 |
| | | IL 207548 D0 | 30 December 2010 |
| | | US 10131904 B2 | 20 November 2018 |
| | | US 10633654 B2 | 28 April 2020 |
| | | CA 2715289 A1 | 20 August 2009 |
| | | CN 104975020 A | 14 October 2015 |
| | | EP 3643782 A1 | 29 April 2020 |
| | | CN 104975020 B | 17 January 2020 |
| | | JP 2018007663 A | 18 January 2018 |
| | | US 2019169608 A1 | 06 June 2019 |
| | | CN 102016036 A | 13 April 2011 |
| CN 107201367 A | 26 September 2017 | CN 107164381 B | 14 April 2020 |
| | | CN 107164381 A | 15 September 2017 |
| | | CN 106047879 A | 26 October 2016 |
| | | CN 107460197 A | 12 December 2017 |
| | | CN 107201367 B | 27 December 2019 |
| | | CN 107164379 B | 02 June 2020 |

Form PCT/ISA/210 (patent family annex) (January 2015)

**INTERNATIONAL SEARCH REPORT**
**Information on patent family members**

International application No.
**PCT/CN2021/110509**

| Patent document cited in search report | Publication date (day/month/year) | Patent family member(s) | Publication date (day/month/year) |
|---|---|---|---|
| | | CN 107177597 B | 27 December 2019 |
| | | CN 107365771 A | 21 November 2017 |
| | | CN 107177597 A | 19 September 2017 |
| | | CN 107365771 B | 10 November 2020 |
| | | CN 107446925 A | 08 December 2017 |
| | | CN 107164379 A | 15 September 2017 |
| | | CN 107400669 A | 28 November 2017 |
| | | CN 107164380 B | 09 June 2020 |
| | | CN 107164380 A | 15 September 2017 |
| | | CN 107177598 B | 27 December 2019 |
| | | CN 107177598 A | 19 September 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010772542
- CN 202110244977
- CN 202110361502
- WO 2014025805 A1


### Non-patent literature cited in the description


- **JANAS, M.M. ; SCHLEGEL, M.K. ; HARBISON, C.E. et al.** Selection of GalNAc-conjugated siRNAs with limited off-target-driven rat hepatotoxicity. *Nat Commun,* 2018, vol. 9, 723
- *Molecular Therapy,* March 2018, vol. 26 (3
- **SEVERGINI et al.** *Cytotechnology,* 2012, vol. 64 (2), 187-195